(19) Europäisches Patentamt
European Patent Office
Office européen des brevets

(11) EP 1 883 700 B1

(12) **EUROPEAN PATENT SPECIFICATION**

(45) Date of publication and mention of the grant of the patent:
**15.12.2010 Bulletin 2010/50**

(21) Application number: **06723862.6**

(22) Date of filing: **24.03.2006**

(51) Int Cl.:
*C12N 15/82* (2006.01) *A01H 5/00* (2006.01)

(86) International application number:
**PCT/EP2006/002895**

(87) International publication number:
**WO 2006/100112 (28.09.2006 Gazette 2006/39)**

# (54) PLANTS HAVING INCREASED YIELD AND A METHOD FOR MAKING THE SAME

PFLANZEN MIT ERHÖHTEM ERTRAG UND VERFAHREN ZUR HERSTELLUNG DAVON

PLANTES A RENDEMENT AMELIORE ET LEUR PROCEDE DE PRODUCTION

(84) Designated Contracting States:
**AT BE BG CH CY CZ DE DK EE ES FI FR GB GR HU IE IS IT LI LT LU LV MC NL PL PT RO SE SI SK TR**

(30) Priority: **25.03.2005 EP 05102444**
**05.04.2005 US 668076 P**

(43) Date of publication of application:
**06.02.2008 Bulletin 2008/06**

(60) Divisional application:
**10157311.1**

(73) Proprietor: **CropDesign N.V.**
**9052 Zwijnaarde-Gent (BE)**

(72) Inventors:
- **FRANKARD, Valerie**
**B-1410 Waterloo (BE)**
- **MIRONOV, Vladimir**
**B-9000 Gent (BE)**

(74) Representative: **Mistry, Meeta**
**BASF SE**
**Global Intellectual Property**
**GVX - C6**
**67056 Ludwigshafen (DE)**

(56) References cited:
**WO-A-00/52171**
**WO-A-01/96579**
**WO-A-98/42851**
**US-A1- 2003 233 670**
**US-B1- 6 518 487**

- **DEWITTE WALTER ET AL: "Altered cell cycle distribution, hyperplasia, and inhibited differentiation in Arabidopsis caused by the D-type cyclin CYCD3" PLANT CELL, AMERICAN SOCIETY OF PLANT PHYSIOLOGISTS, ROCKVILLE, MD, US, vol. 15, no. 1, January 2003 (2003-01), pages 79-92, XP002292514 ISSN: 1040-4651**
- **RIOU-KHAMLICHI C ET AL: "Cytokinin activation of Arabidopsis cell division through a D-type cyclin" SCIENCE, AMERICAN ASSOCIATION FOR THE ADVANCEMENT OF SCIENCE,, US, vol. 283, no. 5407, 5 March 1999 (1999-03-05), pages 1541-1544, XP002134537 ISSN: 0036-8075**
- **RENAUDIN J-P ET AL: "PLANT CYCLINS: A UNIFIED NOMENCLATURE FOR PLANT A-, B- AND D-TYPE CYCLINS BASED ON SEQUENCE ORGANIZATION" PLANT MOLECULAR BIOLOGY, SPRINGER, DORDRECHT, NL, vol. 32, 1996, pages 1003-1018, XP002046019 ISSN: 0167-4412**
- **CHRIMES D ET AL: "Expression of fission yeast cdc25 driven by the wheat ADP-glucose pyrophosphorylase large subunit promoter reduces pollen viability and prevents transmission of the transgene in wheat" NEW PHYTOLOGIST, vol. 166, no. 1, April 2005 (2005-04), pages 185-192, ISSN: 0028-646X**
- **HEMERLY A ET AL: "DOMINANT NEGATIVE MUTANTS OF THE CDC2 KINASE UNCOUPLE CELL DIVISIONFROM ITERATIVE PLANT DEVELOPMENT" EMBO JOURNAL, OXFORD UNIVERSITY PRESS, SURREY, GB, vol. 14, no. 16, 1 January 1995 (1995-01-01), pages 3925-3936, XP002045514 ISSN: 0261-4189**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 883 700 B1

- LEIVA-NETO JOAO T ET AL: "A dominant negative mutant of cyclin-dependent kinase a reduces endoreduplication but not cell size or gene expression in maize endosperm" PLANT CELL, vol. 16, no. 7, July 2004 (2004-07), pages 1854-1869, ISSN: 1040-4651

## Description

The present invention relates generally to the field of molecular biology and concerns a method for increasing plant seed yield relative to corresponding wild type plants. More specifically, the present invention concerns a method for increasing plant seed yield comprising transforming a plant with a nucleic acid encoding a cyclin D3 (CYCD3) polypeptide under the control of an endosperm-specific promoter. The present invention also concerns plants comprising an isolated nucleic acid encoding a CYCD3 polypeptide under the control of an endosperm-specific promoter, which plants have increased seed yield relative to corresponding wild type plants. The invention also provides constructs useful in the methods of the invention.

The ever-increasing world population and the dwindling supply of arable land available for agriculture fuels research towards improving the efficiency of agriculture. Conventional means for crop and horticultural improvements utilise selective breeding techniques to identify plants having desirable characteristics. However, such selective breeding techniques have several drawbacks, namely that these techniques are typically labour intensive and result in plants that often contain heterogeneous genetic components that may not always result in the desirable trait being passed on from parent plants. Advances in molecular biology have allowed mankind to modify the germplasm of animals and plants. Genetic engineering of plants entails the isolation and manipulation of genetic material (typically in the form of DNA or RNA) and the subsequent introduction of that genetic material into a plant. Such technology has the capacity to deliver crops or plants having various improved economic, agronomic or horticultural traits.

A trait of particular economic interest is yield. Yield is normally defined as the measurable produce of economic value from a crop and may be defined in terms of quantity and/or quality. Yield is directly dependent on several factors, for example, the number and size of the organs, plant architecture (for example, the number of branches), seed production and more. Root development, nutrient uptake and stress tolerance may also be important factors in determining yield. Optimizing one of the abovementioned factors may therefore contribute to increasing crop yield.

A trait of particular economic interest is seed yield. Plant seeds are an important source of human and animal nutrition. Crops such as, corn, rice, wheat, canola and soybean account for over half of the total human caloric intake, whether through direct consumption of the seeds themselves or through consumption of meat products raised on processed seeds.

They are also a source of sugars, oils and many kinds of metabolites used in industrial processes. Seeds contain an embryo, the source of new shoots and roots after germination, and an endosperm, the source of nutrients for embryo growth, during germination and early growth of seedlings. The development of a seed involves many genes, and requires the transfer of metabolites from roots, leaves and stems into the growing seed. The endosperm, in particular, assimilates the metabolic precursors of carbohydrate polymers, oil and proteins and synthesizes them into storage macromolecules to fill out the grain.

The ability to increase plant yield, whether through altering seed-related traits, such as seed number, seed biomass, seed development, seed filling or any other seed-related trait, or whether by increasing the number and size of plant organs, or by influencing plant architecture (for example, the number of branches), root development, nutrient uptake or stress tolerance, would have many applications in agriculture, and even many non-agricultural uses, such as in the biotechnological production of substances such as pharmaceuticals, antibodies or vaccines.

One of the ways in which plant yield may be increased is by altering the inherent growth mechanisms of a plant. The inherent growth mechanisms of a plant reside in a highly ordered sequence of events collectively known as the 'cell cycle'. Progression through the cell cycle is fundamental to the growth and development of all multicellular organisms and is crucial to cell proliferation. The major components of the cell cycle are highly conserved in yeast, mammals, and plants. The cell cycle is typically divided into the following sequential phases: G0 - G1 - S - G2 - M. DNA replication or synthesis generally takes place during the S phase ("S" is for DNA synthesis) and mitotic segregation of the chromosomes occurs during the M phase (the "M" is for mitosis), with intervening gap phases, G1 (during which cells grow before DNA replication) and G2 (a period after DNA replication during which the cell prepares for division). Cell division is completed after cytokinesis, the last step of the M phase. Cells that have exited the cell cycle and that have become quiescent are said to be in the G0 phase. Cells in this phase can be stimulated to renter the cell cycle at the G1 phase. The "G" in G1, G2 and G0 stands for "gap". Completion of the cell cycle process allows each daughter cell during cell division to receive a full copy of the parental genome.

Cell division is controlled by two principal cell cycle events, namely initiation of DNA synthesis and initiation of mitosis. Each transition to each of these key events is controlled by a checkpoint represented by specific protein complexes (involved in DNA replication and division). The expression of genes necessary for DNA synthesis at the G1/S boundary is regulated by the E2F family of transcription factors in mammals and plant cells (La Thangue, 1994; Muller *et al.*, 2001; De Veylder *et al.*, 2002). Entry into the cell cycle is regulated/triggered by an E2F/Rb complex that integrates signals and allows activation of transcription of cell cycle genes. The transition between the different phases of the cell cycle, and therefore progression through the cell cycle, is driven by the formation and activation of different heterodimeric serine/threonine protein kinases, generally referred to as cyclin-dependent kinases (CDKs). A prerequisite for activity of these kinases is the physical association with a specific cyclin, the timing of activation being largely dependent upon cyclin expression. Cyclin binding induces conformational changes in the N-terminal lobe of the associating CDK and contributes to the localisation and substrate specificity of the complex. Monomeric CDKs are activated when they are associated with cyclins and thus have a kinase activity. Cyclin protein levels fluctuate in the cell cycle and therefore represent a major factor in determining timing of CDK activation. The periodic activation of these complexes containing cyclins and CDK during cell cycle mediates the temporal regulation of cell-cycle transitions (checkpoints).

Cyclins can be grouped into mitotic cyclins (designated A- and B-type cyclins in higher eukaryotes and CLBs in budding yeast) and G1-specific cyclins (designated D-type cyclins in mammals and CLNs in budding yeast). H-type cyclins regulate the activity of the CAKs (CDK-activating kinases). All four types of cyclins known in plants were identified mostly by analogy to their human counterparts. In *Arabidopsis,* ten A-type, nine B-type, ten D-type and one H-type cyclin have been described (Vandepoele *et al*., 2002).

The ten D-type cyclins in *Arabidopsis* are subdivided into seven subclasses, D1 to D7, which reflect their lack of high sequence similarity to each other, which is in contrast to the A-type and B-type cyclins. Only the D3 and D4 subclasses have more than one member, respectively three and two. Redundancy of the D3-type cyclins has been proposed previously as an explanation for the failure to observe mutant phenotypes upon knocking out of a single D3-type cyclin (Swaminathan *et al*., 2000). The two D3-type cyclins are linked via a recent segmental duplication, which suggests that these are functionally redundant. A similar hypothesis could hold for D4-type cyclins, because two out of three are located in a duplicated block.

The much larger divergence seen for D-type cyclins compared with A- and B-type cyclins might reflect the presumed role of D-type cyclins in integrating developmental signals and environmental cues into the cell cycle. For example, D3-type cyclins have been shown to respond to plant hormones, such as cytokinins and brassinosteroids, whereas CYCD2 and CYCD4 are activated earlier in G1 and react to sugar availability (for review, see Stals and Inzé, 2001).

Overexpression of the CYCD2;1 gene in tobacco was reported to increase cell division and increase overall plant growth rate with no morphological alterations (Cockcroft *et al*., 2000).

Overexpression in *Arabidopsis* of the CYCD3;1 gene under the control of a CaMV 35S promoter was reported to give plants with enlarged cotyledons, a dramatically reduced final plant size and distorted development. At a cellular level, cells are pushed from G1, causing ectopic cell divisions in both meristematic regions and in regions in which cell division is normally absent or limited. This increase in cell numbers is coupled to a decrease in cell size (Dewitte *et al*., 2003).

It is an object of the present invention to overcome some of the problems associated with the prior art expression of CYCD3 in plants.

It has now been found that transforming a plant with a nucleic acid encoding a CYCD3 polypeptide under the control of an endosperm-specific promoter gives plants having increased seed yield relative to corresponding wild type plants in particular relative to transgenic plants under the control of promoters which are not endosperm-specific. Therefore according to one embodiment of the present invention, there is provided a method for increasing plant seed yield, comprising transforming a plant with a nucleic acid encoding a CYCD3 polypeptide under the control of an endosperm-specific promoter.

Advantageously, performance of the methods according to the present invention results in plants having increased seed yield, relative to corresponding wild type plants.

The term "increased yield" as defined herein is taken to mean an increase in any one or more of the following, each relative to corresponding wild type plants: (i) increased biomass (weight) of one or more parts of a plant, which may include aboveground (harvestable) parts and/or (harvestable) parts below ground, especially increased root biomass; (ii) increased total seed yield, which includes an increase in seed biomass (seed weight) and which may be an increase in the seed weight per plant and/or on an individual seed basis and/or per hectare or acre; (iii) increased number of flowers ("florets") per panicle (iv) increased number of (filled) seeds; (v) increased seed size, which may also influence the composition of seeds; (vi) increased seed volume, which may also influence the composition of seeds (including oil, protein and carbohydrate total content and composition); (vii) increased individual seed area and/or seed perimeter; (viii) increased individual seed length and/or width; (ix) increased harvest index, which is expressed as a ratio of the yield of harvestable parts, such as seeds, over the total biomass; and (x) increased thousand kernel weight (TKW), which is extrapolated from the number of filled seeds counted and their total weight. An increased TKW may result from an increased seed size and/or seed weight. An increased TKW may result from an increase in embryo size (weight) and/or endosperm size (weight). An increase in seed size, seed volume, seed area and seed length may be due to an increase in specific parts of a seed, for example due to an increase in the size of the embryo and/or endosperm and/or aleurone and/or scutellum and/or cotyledons, or other parts of a seed.

Taking corn as an example, a yield increase may be manifested as one or more of the following: increase in the number of plants per hectare or acre, an increase in the number of ears per plant, an increase in the number of rows, number of kernels per row, kernel weight, thousand kernel weight, ear length/diameter, increase in the seed filling rate (which is the number of filled seeds divided by the total number of seeds and multiplied by 100), among others. Taking rice as an example, a yield increase may be manifested by an increase in one or more of the following: number of plants per hectare or acre, number of panicles per plant, number of spikelets per panicle, number of flowers per panicle, increase in the seed filling rate, increase in TKW, among others. An increase in yield may also result in modified architecture, or may occur as a result of modified architecture.

Performance of the methods of the invention result in plants having increased seed yield. In particular, such increased seed yield includes increased number of flowers per panicle, increased total seed yield, increased TKW and increased harvest index, each relative to corresponding wild type plants. Therefore, according to the present invention, there is provided a method for increasing seed yield in plants, which method comprises transforming a plant with a nucleic acid encoding a CYCD3 polypeptide under the control of an endosperm-specific promoter.

Since the transgenic plants according to the present invention have increased seed yield, it is likely that these plants exhibit an increased growth rate (during at least part of their life cycle), relative to the growth rate of corresponding wild type plants at a corresponding stage in their life cycle. The increased growth rate may be specific to one or more parts of a plant (including seeds), or may be throughout substantially the whole plant. A plant having an increased growth rate may even exhibit early flowering. The increase in growth rate may take place at one or more stages in the life cycle of a plant or during substantially the whole plant life cycle.

increased growth rate during the early stages in the life cycle of a plant may reflect enhanced vigour. The increase in growth rate may alter the harvest cycle of a plant allowing plants to be sown later and/or harvested sooner than would otherwise be possible. If the growth rate is sufficiently Increased, it may allow for the further sowing of seeds of the same plant species (for example sowing and harvesting of rice plants followed by sowing and harvesting of further rice plants all within one conventional growing period). Similarly, if the growth rate is sufficiently increased, it may allow for the further sowing of seeds of different plants species (for example the sowing and harvesting of rice plants followed by, for example, the sowing and optional harvesting of soybean, potato or any other suitable plant). Harvesting additional times from the same rootstock in the case of some crop plants may also be possible. Altering the harvest cycle of a plant may lead to an increase in annual biomass production per acre (due to an increase in the number of times (say in a year) that any particular plant may be grown and harvested). An increase in growth rate may also allow for the cultivation of transgenic plants in a wider geographical area than their wild-type counterparts, since the territorial limitations for growing a crop are often determined by adverse environmental conditions either at the time of planting (early season) or at the time of harvesting (late season). Such adverse conditions may be avoided if the harvest cycle is shortened. The growth rate may be determined by deriving various parameters from growth curves, such parameters may be: T-Mid (the time taken for plants to reach 50% of their maximal size) and T-90 (time taken for plants to reach 90% of their maximal size), amongst others.

Described herein are plants having an increased growth rate and a method for increasing plant growth rate relative to the growth rate of corresponding wild type plants, which method comprises transforming a plant with a nucleic acid encoding a CYCD3 polypeptide under the control of an endosperm-specific promoter. .

An increase in seed yield and/or growth rate occurs whether the plant is under non-stress conditions or whether the plant is exposed to various stresses compared to control plants. Plants typically respond to exposure to stress by growing more slowly. In conditions of severe stress, the plant may even stop growing altogether. Mild stress on the other hand is defined herein as being any stress to which a plant is exposed which does not result in the plant ceasing to grow altogether without the capacity to resume growth. Due to advances in agricultural practices (irrigation, fertilization, pesticide treatments) severe stresses are not often encountered in cultivated crop plants. As a consequence, the compromised growth induced by mild stress is often an undesirable feature for agriculture. Mild stresses are the typical stresses to which a plant may be exposed. These stresses may be the everyday biotic and/or abiotic (environmental) stresses to which a plant is exposed. Typical abiotic or environmental stresses include temperature stresses caused by atypical hot or cold/freezing temperatures; salt stress; water stress (drought or excess water). Chemicals may also cause abiotic stresses. Biotic stresses are typically those stresses caused by pathogens, such as bacteria, viruses, fungi and insects.

Advantageously, performance of the methods of the invention allows seed yield to be increased in any plant.

The term "plant" as used herein encompasses whole plants, ancestors and progeny of the plants and plant parts, including seeds, shoots, stems, leaves, roots (including tubers), flowers, and tissues and organs, wherein each of the aforementioned comprise the gene/nucleic acid of interest. The term "plant" also encompasses plant cells, suspension cultures, callus tissue, embryos, meristematic regions, gametophytes, sporophytes, pollen and microspores, again wherein each of the aforementioned comprise the gene/nucleic acid of interest.

Plants that are particularly useful in the methods of the invention include all plants which belong to the superfamily Viridiplantae, in particular monocotyledonous and dicotyledonous plants including fodder or forage legumes, ornamental plants, food crops, trees or shrubs selected from the list comprising *Acacia* spp., *Acer* spp., *Actinidia* spp., *Aesculus* spp., *Agathis australis, Albizia amara, Alsophila tricolor, Andropogon* spp., *Arachis* spp, *Areca catechu, Astelia fragrans, Astragalus cicer, Baikiaea plurijuga, Betula* spp., *Brassica* spp., *Bruguiera gymnorrhiza, Burkea africana, Butea fron-*

*dosa, Cadaba farinosa, Calliandra* spp, *Camellia sinensis, Canna indica, Capsicum* spp., *Cassia* spp., *Centroema pubescens, Chaenomeles* spp., *Cinnamomum cassia, Coffea arabica, Colophospermum mopane, Coronillia varia, Cotoneaster serotina, Crataegus* spp., *Cucumis* spp., *Cupressus* spp., *Cyathea dealbata, Cydonia oblonga, Cryptomeria japonica, Cymbopogon* spp., *Cynthea dealbata, Cydonia oblonga, Dalbergia monetaria, Davallia divaricata, Desmodium* spp., *Dicksonia squarosa, Diheteropogon amplectens, Dioclea* spp, *Dolichos* spp., *Doycnium rectum, Echinochloa pyramidalls, Ehrartia* spp., *Eleusine coracana, Eragrestis* spp., *Erythrina* spp., *Eucalyptus* spp., *Euclea schimperi, Eulalia villosa, Fagopyrum* spp., *Feijoa sellowiana, Fragaria* spp., *Flemingia* spp, *Freycinetia banksii, Geranium thunbergii, Ginkgo biloba, Glycine javanica, Gliricidia* spp, *Gossypium hirsutum, Grevillea* spp., *Guibourtia coleosperma, Hedysarum* spp., *Hemarthia altissima, Heteropogon contortus, Hordeum vulgare, Hyparrhenia rufa, Hypericum erectum, Hyperthelia dissoluta, Indigo incarnata, Iris* spp., *Leptarrhena pyrolifolia, Lespediza* spp., *Lettuca* spp., *Leucaena leucocephala, Loudetia simplex, Lotonus bainesii, Lotus* spp., *Macrotyloma axillare, Malus* spp., *Manihot esculenta, Medicago sativa, Metasequola glyptostroboldes, Musa sapientum, Nicotianum* spp., *Onobrychis* spp., *Omithopus* spp., *Oryza* spp., *Peltophorum africanum, Pennisetum* spp., *Persea gratissima, Petunia* spp., *Phaseolus* spp., *Phoenix canariensis, Phormium cookianum, Photinia* spp., *Picea glauca, Pinus* spp., *Pisum sativum, Podocarpus totara, Pogonarthria fleckii, Pogonarthria squarrosa, Populus* spp., *Prosopls cineraria, Pseudotsuga menziesii, Pterolobium stellatum, Pyrus communis, Quercus* spp., *Rhaphiolepsis umbellata, Rhopalostylis sapida, Rhus natalensis, Ribes grossularia, Ribes* spp., *Robinia pseudoacacia, Rosa* spp., *Rubus* spp., *Salix* spp., *Schyzachyrium sanguineum, Sciadopitys varticillata, Sequoia sempervirens, Sequoiadendron giganteum, Sorghum bicolor, Spinacia* spp., *Sporobolus fimbriatus, Stiburus alopecuroides, Stylosanthos humilis, Tadehagi* spp, *Taxodium distichum, Themeda triandra, Trifolium* spp., *Triticum* spp., *Tsuga heterophylla, Vaccinium* spp., *Vicia* spp., *Vitis vinifera, Watsonia pyramidata, Zantedeschla aethiopica, Zea mays,* amaranth, artichoke, asparagus, broccoli, Brussels sprouts, cabbage, canola, carrot, cauliflower, celery, collard greens, flax, kale, lentil, oilseed rape, okra, onion, potato, rice, soybean, strawberry, sugarbeet, sugarcane, sunflower, tomato, squash, tea and algae, amongst others.

According to a preferred embodiment of the present invention, the plant is a crop plant. Examples of such crop plants include soybean, sunflower, canola, alfalfa, rapeseed, cotton, tomato, potato and tobacco, amongst others. Further preferably, the plant is a monocotyledonous plant. One such example of a monocotyledonous plant is sugarcane. More preferably the plant is a cereal. Examples of such cereals include rice, maize, wheat, barley, millet, rye, sorghum and oats.

A CYCD3 polypeptide may be identified using different methods. For example, the query protein sequence may be BLASTed (for example, using BLAST default parameters for the gap opening penalty and the gap extension penalty) against a translated *Arabidopsis* nucleic acid sequence database. The first hit from the BLAST result will be an *Arabidopsis* CYCD3 polypeptide. Another method for identifying a CYCD3 polypeptide is by aligning the query sequence with known CYCD3 protein sequences, using for example the AlignX program from Vector NTI suite (InforMax, Bethesda, MD). Multiple alignments may then be carried out with a gap opening penalty of 10 and a gap extension of 0.01. Minor manual editing of the alignment may also be necessary in order to better position some conserved regions. If the query sequence is a CYCD3 polypeptide, it will align with the known CYCD3 polypeptide sequences.

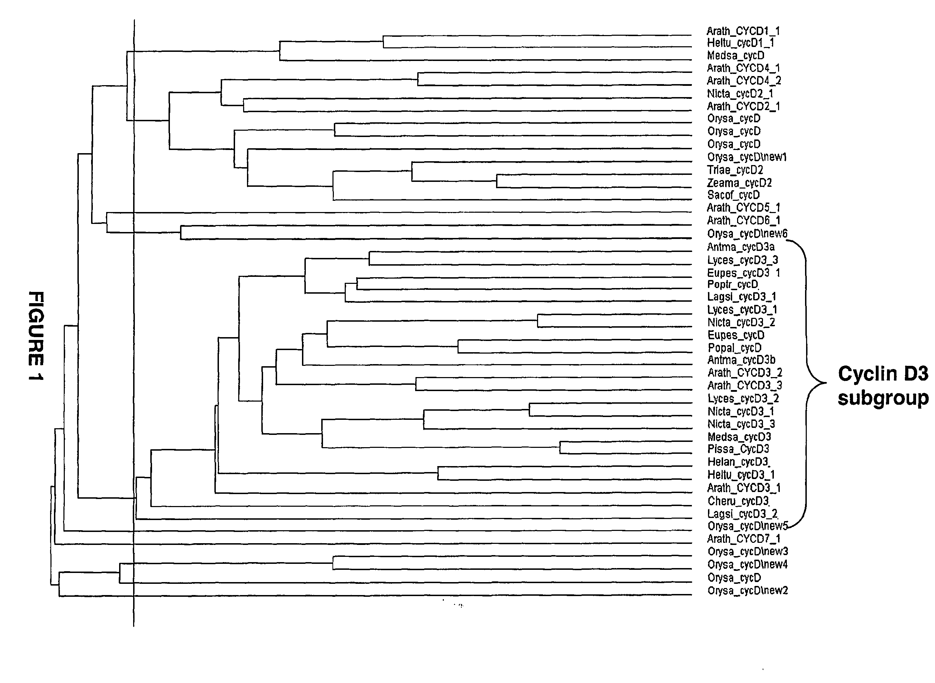

A "CYCD3 polypeptide" as defined herein refers to any polypeptide sequence which, when used in the construction of a cyclin or cyclin D phylogenetic tree, such as the one depicted in Fig. 1, falls into the cyclin D3-type group which includes CYCD3 polypeptides (and not other D-type cyclins, such as cyclin D1, D2, D4, D5, D6 and D7). Performance of the methods of the invention requires the use of nucleic acids encoding CYCD3 polypeptides. Reference herein to a nucleic acid encoding a CYCD3 polypeptide is to a nucleic acid encoding a CYCD3 polypeptide as defined above.

A person skilled in the art could readily determine whether any polypeptide sequence in question falls within the aforementioned definition using known techniques and software for the making of such a phylogenetic tree, such as a GCG, EBI or CLUSTAL package, using default parameters. Upon construction of such a phylogenetic tree, sequences clustering in the D3-type cyclin group will be considered to fall within the definition of a "CYCD3 polypeptide". Nucleic acids encoding such sequences will be useful in performing the methods of the invention.

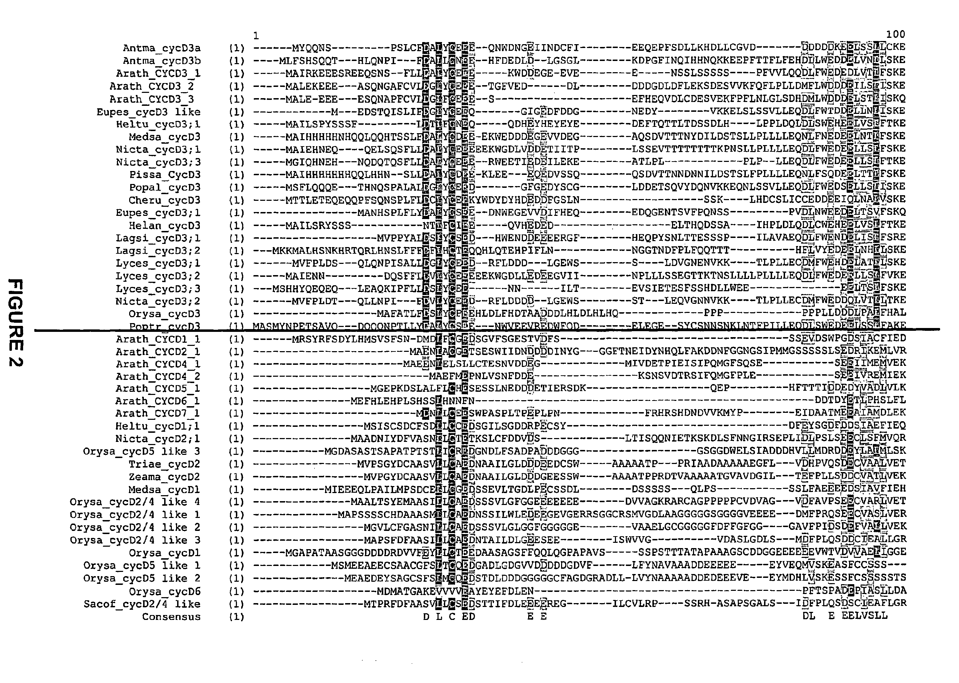

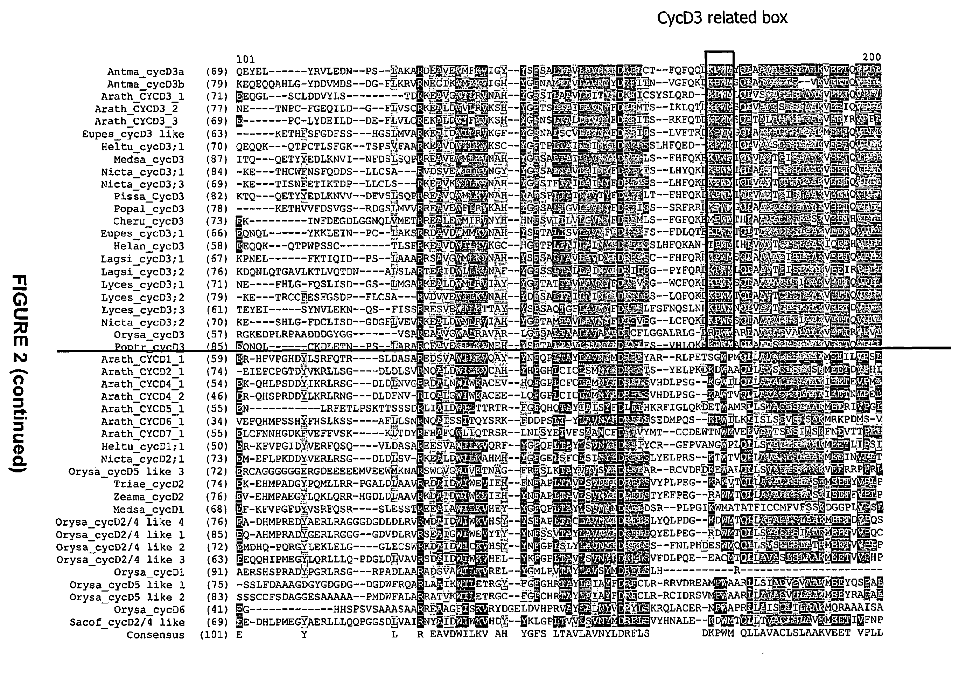

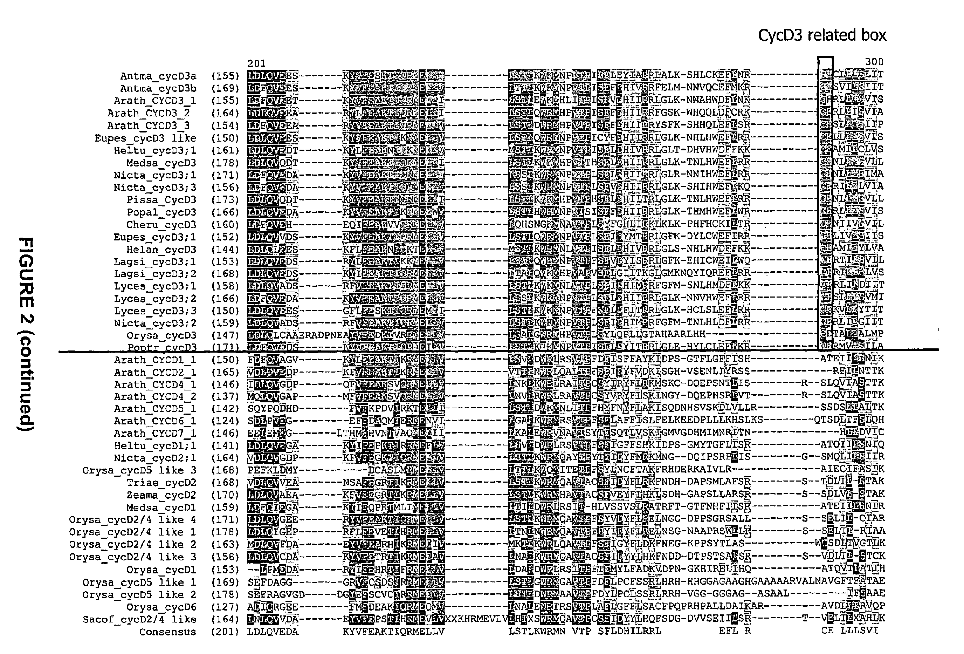

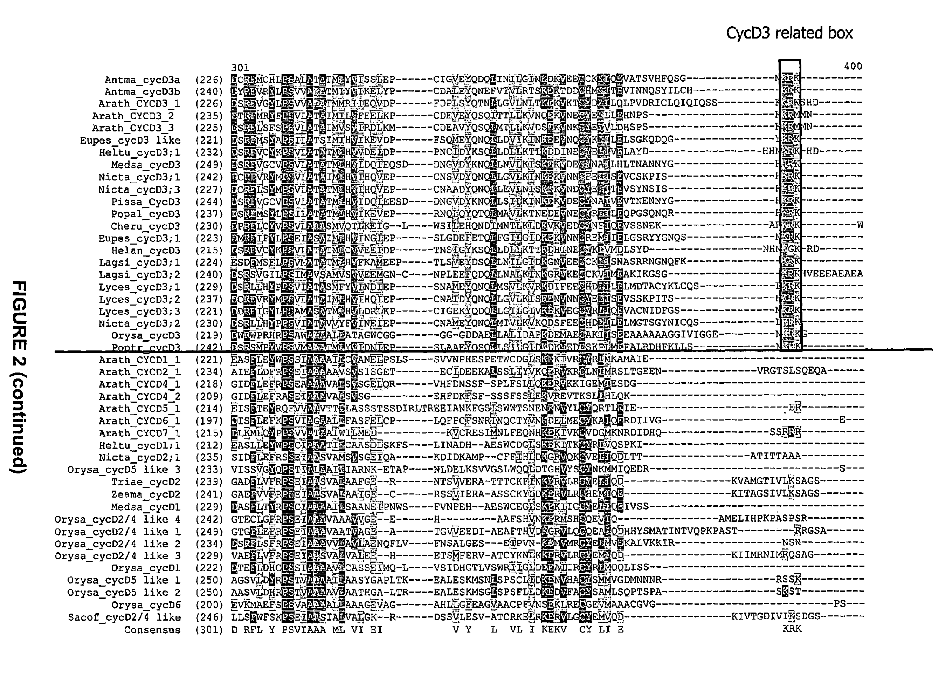

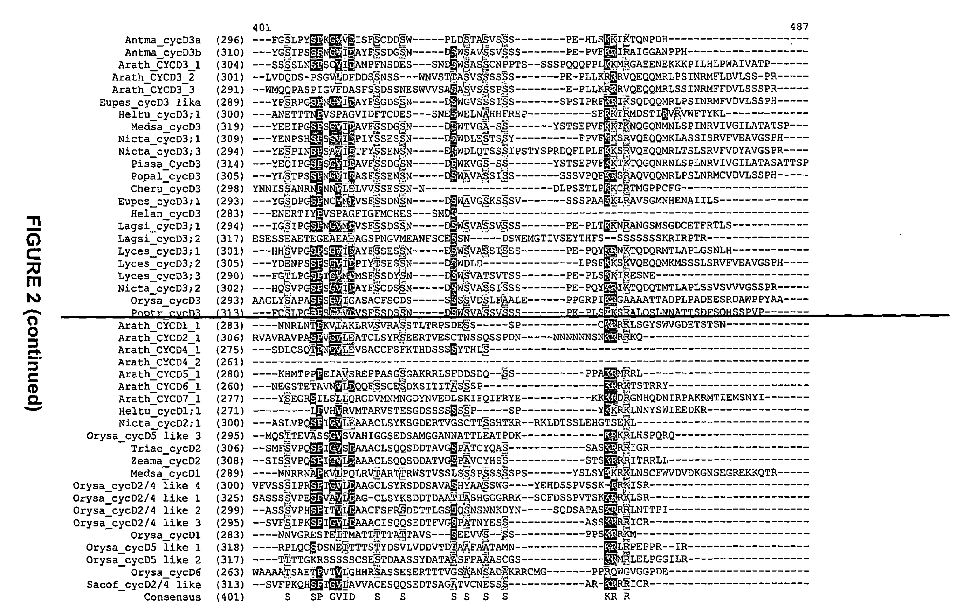

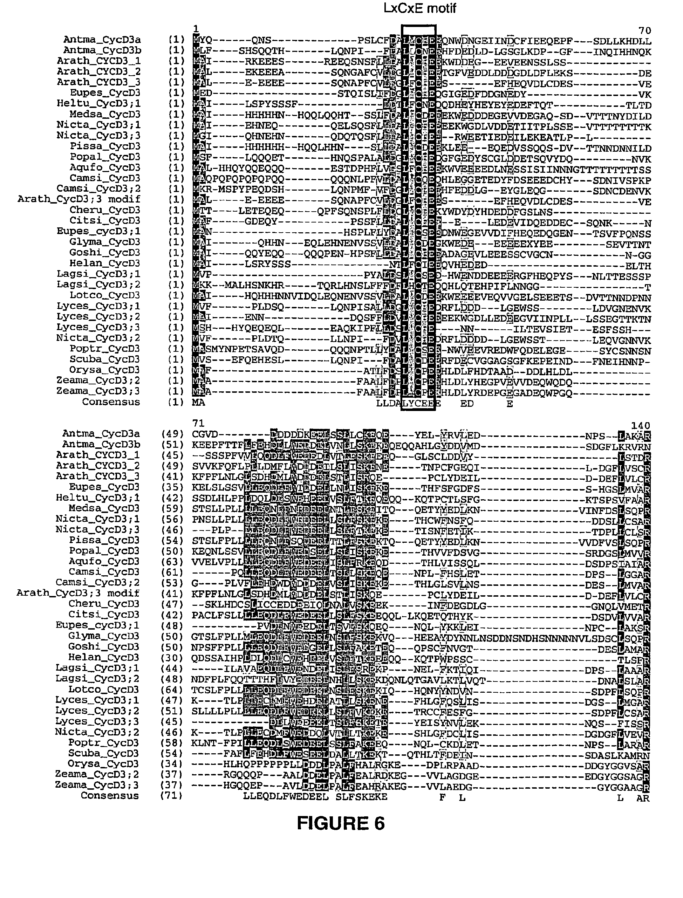

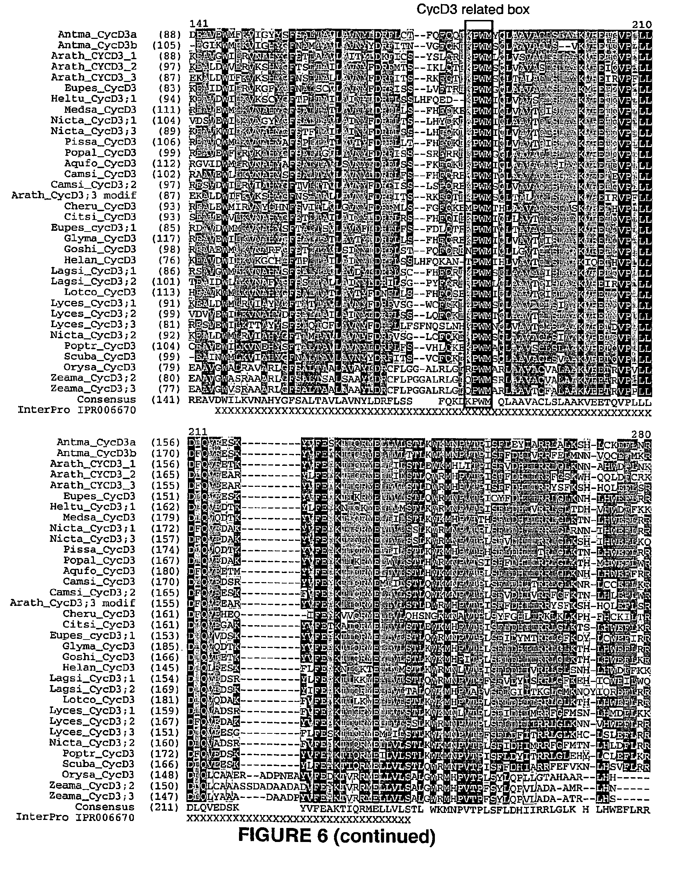

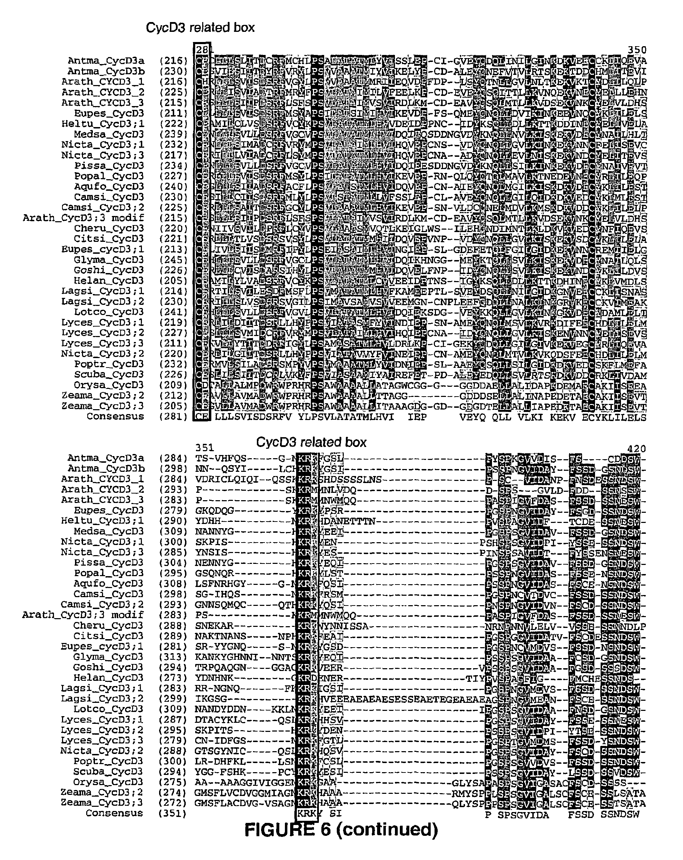

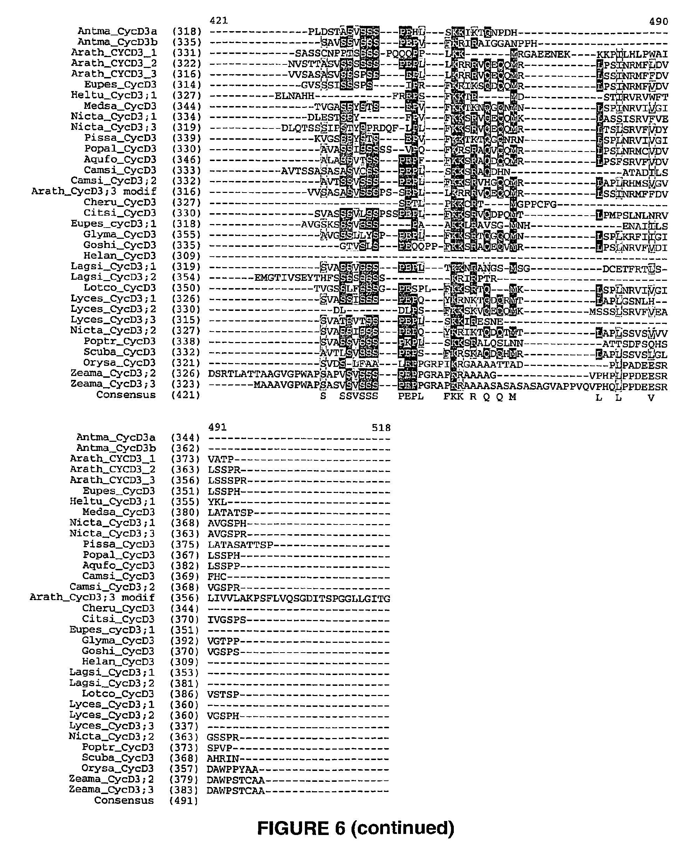

D3-type cyclins typically have the ability to bind and activate plant CDKs and Rb. In addition to a cyclin box and an LxCxE motif within the first 40 or so amino acids (which is characteristic of most D-type cyclins), D3-type cyclins may comprise one or more and preferably all of the conserved regions identified by the boxes shown in Figures 2 and 6. As shown in Figures 2 and 6, one mismatch within the boxes is allowed.

Examples of nucleic acids encoding CYCD3 polypeptides falling under the aforementioned definition of a CYCD3 polypeptide are given in Table 1 below. The CYCD3-encoding nucleic acids shown in Table 1 may be useful in performing the methods of the invention, i.e. to obtain plants having improved seed yield relative to corresponding wild type plants by transforming a plant with any one of these nucleic acids under the control of an endosperm-specific promoter. Variants of the CYCD3-encoding nucleic acids of Table 1 are also advantageously useful in the methods of the invention. SEQ ID NO: 1, SEQ ID NO: 48 or variants of either are preferred for use in the methods of the present invention.

Variants of a nucleic acid encoding a CYCD3 polypeptide as defined herein typically encode a substantial portion of the complete protein which may comprise in addition to a cyclin box and an LxCxE motif within the first 40 or so amino acids (which is characteristic of most D-type cyclins), one or more and preferably all of the conserved regions identified by the boxes shown In Figures 2 and 6 (as shown in Figures 2 and 6, one mismatch within the boxes is allowed).

Examples of CYCD3 polypeptides as defined hereinabove are shown in Table 1 (encoded by polynucleotide sequences with NCBI accession number). Preferred CYCD3 polypeptide sequence for the performance of the invention is represented by SEQ ID NO: 2, SEQ ID NO: 49 or a substantial portion of either.

The CYCD3 polypeptides may be the complete protein encoded by the nucleic acids, or may be portions of the encoded protein. Preferably, the nucleic acids provided herein encode CYCD3 polypeptides constituting a substantial portion of the complete protein which comprises, in addition to a cyclin box and an LxCxE motif within the first 40 or so amino acids (which is characteristic of most D-type cyclins), one or more and preferably all of the conserved regions identified by the boxes shown in Figures 2 and 6 (as shown in Figures 2 and 6, one mismatch within the boxes is allowed). The portion may be used in isolated form or it may be fused to other coding (or non coding) sequences in order to, for example, produce a protein that combines several activities. When fused to other coding sequences, the resulting polypeptide produced upon translation may be bigger than that predicted for the CYCD3 fragment.

**Table 1: Examples of nucleic acids encoding CYCD3 polypeptides**

| Name | NCBI nucleic acid accession number | Source | SEQ ID NO of nucleic acid | SEQ ID NO of polypeptide |
|---|---|---|---|---|
| Antma_cycD3a | AJ250397 | *Antirrhinum majus* | SEQ ID NO: 6 | SEQ ID NO: 7 |
| Antma_cycD3b | AJ250398 | *Antirrhinum majus* | SEQ ID NO: 8 | SEQ ID NO: 9 |
| Arath_CYCD3;1 | NM_119579.2 | *Arabidopsis thaliana* | SEQ ID NO:10 | SEQ ID NO: 11 |
| Arath_CYCD3;2 | NM_126126.2 | *Arabidopsis thaliana* | SEQ ID NO: 12 | SEQ ID NO: 13 |
| Arath_CYCD3;3 | NM_114867.2 | *Arabidopsis thaliana* | SEQ ID NO: 1 | SEQ ID NO: 2 |
| Eupes_cycD3;2 | AY340588 | *Euphorbia esula* | SEQ ID NO: 14 | SEQ ID NO: 15 |
| Eupes_cycD3;1 | AY340589 | *Euphorbia esula* | SEQ ID NO: 16 | SEQ ID NO: 17 |
| Helan_cycD3 | AY033440 | *Helianthus annuus* | SEQ ID NO: 18 | SEQ ID NO: 19 |
| Heltu_cycD3;1 | AY063461 | *Helianthus tuberosus* | SEQ ID NO: 20 | SEQ ID NO: 21 |
| Lagsi_cycD3;1 | AF519810 | *Lagenaria siceraria* | SEQ ID NO: 22 | SEQ ID NO: 23 |
| Lagsi_cycD3;2 | AF519811 | *Lagenaria siceraria* | SEQ ID NO: 24 | SEQ ID NO: 25 |
| Lyces_cycD3;1 | AJ002588 | *Lycopersicum esculentum* | SEQ ID NO: 26 | SEQ ID NO: 27 |
| Lyces_cycD3;2 | AJ002589 | *Lycoperslcum esculentum* | SEQ ID NO: 28 | SEQ ID NO: 29 |
| Lyces_cycD3;3 | AJ002590 | *Lycopersicum esculentum* | SEQ ID NO: 30 | SEQ ID NO: 31 |
| Medsa_cycD3 | X88864 | *Medicago sativa* | SEQ ID NO: 32 | SEQ ID NO: 33 |
| Nicta_cycD3;1 | AJ011893 | *Nicotlana tabacum* | SEQ ID NO: 34 | SEQ ID NO: 35 |
| Nicta_cycD3;2 | AJ011894 | *Nicotiana tabacum* | SEQ ID NO: 36 | SEQ ID NO: 37 |
| Nicta_cycD3;3 | AB015222 | *Nicotiana tabacum* | SEQ ID NO: 38 | SEQ ID NO: 39 |
| Orysa_cycD3-like | AK103499.1 | *Oryza sativa* | SEQ ID NO: 40 | SEQ ID NO: 41 |

(continued)

| Name | NCBI nucleic acid accession number | Source | SEQ ID NO of nucleic acid | SEQ ID NO of polypeptide |
|---|---|---|---|---|
| Plssa_cycD3 | AB008188 | *Pisum sativum* | SEQ ID NO: 42 | SEQ ID NO: 43 |
| Popal_cycD3 | AY230139 | *Populus alba* | SEQ ID NO: 44 | SEQ ID NO: 45 |
| Poptr_cycD3 | AF181993 | *Populus tremula x Populus tremuloides* | SEQ ID NO: 46 | SEQ ID NO: 47 |
| *Arath_ cycD3_modified | NA | *Arabidopsis thaliana* | SEQ ID NO: 48 | SEQ ID NO: 49 |
| Aqufo_CycD3 | DT755971.1 DT749271 | *Aquilegia formosa x Aquilegia pubescens* | SEQ ID NO: 50 | SEQ ID NO: 51 |
| Camsi_CycD3 | AB247282 | *Camellia sinensis* | SEQ ID NO: 52 | SEQ ID NO: 53 |
| Camsi_CycD3;2 | AB247283 | *Camellia sinensis* | SEQ ID NO: 54 | SEQ ID NO: 55 |
| Citsi_CycD3 | CX676162 CX676163 | *Citrus sinensis* | SEQ ID NO: 56 | SEQ ID NO: 57 |
| Glyma_CycD3 | AY439098 | *Glycine max* | SEQ ID NO: 58 | SEQ ID NO: 59 |
| Goshi_CycD3 | DT571998 DT543827.1 | *Gossypium hirsutum* | SEQ ID NO: 60 | SEQ ID NO: 61 |
| Lotco_CycD3 | AP008090 | *Lotus corniculatus* | SEQ ID NO: 62 | SEQ ID NO: 63 |
| Medtr_CycD3 | DY615448.1 | *Medicago trunculata* | SEQ ID NO: 64 | SEQ ID NO: 65 |
| Scuba_CycD3 | AB205135.1 | *Scutellarla baicalensis* | SEQ ID NO: 66 | SEQ ID NO: 67 |
| Zeama_CycD3 like 2 | DV509394.1 DV028752.1 | *Zea mays* | SEQ ID NO: 68 | SEQ ID NO: 69 |
| Zeama_CycD3 like 3 | DT948601.1 DT642394.1 | *Zea mays* | SEQ ID NO: 70 | SEQ ID NO: 71 |
| *Contains no stop codon, which generates a longer transcript; the resultant extra portion is not believed to affect overall function compared to a corresponding non-modified sequence (SEQ ID NO: 2). | | | | |

Also useful in the methods of in the present invention are variants of the CYCD3-encoding nucleic acids provided herein. Such variants may be derived from any natural or artificial source. The nucleic acid/gene or variant thereof may be isolated from a microbial source, such as yeast or fungi, or from a plant, algae or animal (including human) source. This nucleic acid may be modified from its native form in composition and/or genomic environment through deliberate human manipulation. The nucleic acid is preferably of plant origin, whether from the same plant species (for example to the one in which it is to be introduced) or whether from a different plant species. The nucleic acid may be isolated from a dicotyledonous species, preferably from the family Brassicaceae, further preferably from *Arabidopsis thaliana.* More preferably, the CYCD3-encoding nucleic acid isolated from *Arabldopsis thaliana* is represented by SEQ ID NO: 1 or SEQ ID NO: 48, and the CYCD3 polypeptide sequence is as represented by SEQ ID NO: 2 or SEQ ID NO: 49.

An example of a variant of a CYCD3-encoding nucleic acid/gene is a nucleic acid capable of hybridising under reduced stringency conditions, preferably under stringent conditions, with a CYCD3-encoding nucleic acid/gene encoding a polypeptide which, when used in the construction of a cyclin or cyclin D phylogenetic tree falls into a cyclin D3-type group which includes the CYCD3 as in SEQ ID NO: 2 or SEQ ID NO: 49. Preferably, a variant of a CYCD3-encoding nucleic acid/gene is a nucleic acid capable of hybridising to a nucleic acid encoding a CYCD3 polypeptide, which polypeptide comprises, in addition to a cyclin box and an LxCxE motif within the first 40 or so amino acids, one or more and preferably all of the conserved regions identified by the boxes shown in Figures 2 and 6 (as shown in Figures 2 and 6, one mismatch within the boxes is allowed). Preferred is a nucleic acid capable of hybridising to a nucleic acid represented by SEQ ID NO: 1 or SEQ ID NO: 48. Also useful in the methods of the invention is any nucleic acid capable of hybridising to any of the CYCD3-encoding nucleic acids shown in Table 1.

The term "hybridisation" as defined herein is a process wherein substantially homologous complementary nucleotide sequences anneal to each other. The hybridisation process can occur entirely in solution, i.e. both complementary nucleic acids are in solution. The hybridisation process can also occur with one of the complementary nucleic acids immobilised to a matrix such as magnetic beads, Sepharose beads or any other resin. The hybridisation process can furthermore occur with one of the complementary nucleic acids immobilised to a solid support such as a nitro-cellulose or nylon membrane or immobilised by e.g. photolithography to, for example, a siliceous glass support (the latter known as nucleic acid arrays or microarrays or as nucleic acid chips). In order to allow hybridisation to occur, the nucleic acid molecules are generally thermally or chemically denatured to melt a double strand into two single strands and/or to remove hairpins or other secondary structures from single stranded nucleic acids. The stringency of hybridisation is influenced by conditions such as temperature, salt concentration, ionic strength and hybridisation buffer composition.

"Stringent hybridisation conditions" and "stringent hybridisation wash conditions" in the context of nucleic acid hybridisation experiments such as Southern and Northern hybridisations are sequence dependent and are different under different environmental parameters. A person skilled in the art will be aware of various parameters which may be altered during hybridisation and washing and which will either maintain or change the stringency conditions.

The $T_m$ is the temperature under defined ionic strength and pH, at which 50% of the target sequence hybridises to a perfectly matched probe. The $T_m$ is dependent upon the solution conditions and the base composition and length of the probe. For example, longer sequences hybridise specifically at higher temperatures. The maximum rate of hybridisation is obtained from about 16°C up to 32°C below $T_m$. The presence of monovalent cations in the hybridisation solution reduce the electrostatic repulsion between the two nucleic acid strands thereby promoting hybrid formation; this effect is visible for sodium concentrations of up to 0.4M. Formamide reduces the melting temperature of DNA-DNA and DNA-RNA duplexes with 0.6 to 0.7°C for each percent formamide, and addition of 50% formamide allows hybridisaton to be performed at 30 to 45°C, though the rate of hybridisation will be lowered. Base pair mismatches reduce the hybridisation rate and the thermal stability of the duplexes. On average and for large probes, the $T_m$ decreases about 1°C per % base mismatch. The $T_m$ may be calculated using the following equations, depending on the types of hybrids:

1. DNA-DNA hybrids (Meinkoth and Wahl, Anal. Biochem., 138: 267-284, 1984):

$$T_m = 81.5°C + 16.6 x \log[Na^+]^a + 0.41 x \%[G/C^b] - 500 x [L^c]^{-1} - 0.61 x \% \text{ formamide}$$

2. DNA-DNA or RNA-RNA hybrids:

$$T_m = 79.8 + 18.5 (\log_{10}[Na^+]^a) + 0.58 (\%G/C^b) + 11.8 (\%G/C^b)^2 - 820/L^c$$

3. oligo-DNA or oligo-RNA[d] hybrids:

For <20 nucleotides: $T_m = 2 (l_n)$
For 20-35 nucleotides: $T_m = 22 + 1.46 (l_n)$

[a] or for other monovalent cation, but only accurate in the 0.01-0.4 M range.
[b] only accurate for %GC in the 30% to 75% range.
[c] L = length of duplex in base pairs.
[d] Oligo, oligonucleotide; $l_n$, effective length of primer = 2×(no. of G/C)+(no. of A/T).
*Note:* for each 1 % formamide, the $T_m$ is reduced by about 0.6 to 0.7°C, while the presence of 6 M urea reduces the $T_m$ by about 30°C

Specificity of hybridisation is typically the function of post-hybridisation washes. To remove background resulting from non-specific hybridisation, samples are washed with dilute salt solutions. Critical factors of such washes include the ionic strength and temperature of the final wash solution: the lower the salt concentration and the higher the wash temperature, the higher the stringency of the wash. Wash conditions are typically performed at or below hybridisation stringency. Generally, suitable stringent conditions for nucleic acid hybridisation assays or gene amplification detection procedures are as set forth above. Conditions of greater or less stringency may also be selected. Generally, low stringency conditions are selected to be about 50°C lower than the thermal melting point ($T_m$) for the specific sequence at a defined ionic strength and pH. Medium stringency conditions are when the temperature is 20°C below $T_m$, and high stringency

conditions are when the temperature is 10°C below $T_m$. For example, stringent conditions are those that are at least as stringent as, for example, conditions A-L; and reduced stringency conditions are at least as stringent as, for example, conditions M-R. Non-specific binding may be controlled using any one of a number of known techniques such as, for example, blocking the membrane with protein containing solutions, additions of heterologous RNA, DNA, and SDS to the hybridisation buffer, and treatment with RNase. Examples of hybridisation and wash conditions are listed in Table 2 below.

**Table 2: Examples of hybridisation and wash conditions**

| Stringency Condition | Polynucleotide Hybrid ± | Hybrid Length (bp) ‡ | Hybridization Temperature and Buffer † | Wash Temperature and Buffer† |
|---|---|---|---|---|
| A | DNA:DNA | > or equal to 50 | 65°C 1×SSC; or 42°C, 1×SSC and 50% formamide | 65°C; 0.3×SSC |
| B | DNA:DNA | <50 | Tb*; 1×SSC | Tb*; 1×SSC |
| C | DNA:RNA | > or equal to 50 | 67°C 1×SSC; or 45°C, 1×SSC and 50% formamide | 67°C; 0.3×SSC |
| D | DNA:RNA | <50 | Td*; 1×SSC | Td*; 1×SSC |
| E | RNA:RNA | > or equal to 50 | 70°C 1×SSC; or 50°C, 1×SSC and 50% formamide | 70°C; 0.3×SSC |
| F | RNA:RNA | <50 | Tf*; 1×SSC | Tf*; 1×SSC |
| G | DNA:DNA | > or equal to 50 | 65°C 4×SSC; or 45°C, 4×SSC and 50% formamide | 65°C; 1×SSC |
| H | DNA:DNA | <50 | Th*; 4 ×SSC | Th*; 4×SSC |
| I | DNA:RNA | > or equal to 50 | 67°C 4×SSC; or 45°C, 4×SSC and 50% formamide | 67°C; 1×SSC |
| J | DNA:RNA | <50 | Tj*: 4 ×SSC | Tj*; 4 ×SSC |
| K | RNA:RNA | > or equal to 50 | 70°C 4×SSC; or 40°C, 6×SSC and 50% formamide | 67°C; 1×SSC |
| L | RNA:RNA | <50 | T1*; 2 ×SSC | T1*; 2×SSC |
| M | DNA:DNA | > or equal to 50 | 50°C 4×SSC; or 40°C, 6×SSC and 50% formamide | 50°C; 2×SSC |
| N | DNA:DNA | <50 | Tn*; 6 ×SSC | Tn*; 6×SSC |
| O | DNA:RNA | > or equal to 50 | 55°C 4×SSC; or 42°C, 6×SSC and 50% formamide | 55°C; 2×SSC |
| P | DNA:RNA | <50 | Tp*; 6 ×SSC | Tp*; 6×SSC |
| Q | RNA:RNA | > or equal to 50 | 60°C 4×SSC; or 45°C, 6×SSC and 50% formamide | 60°C.; 2×SSC |

(continued)

| Stringency Condition | Polynucleotide Hybrid ± | Hybrid Length (bp) ‡ | Hybridization Temperature and Buffer † | Wash Temperature and Buffer† |
|---|---|---|---|---|
| R | RNA:RNA | <50 | Tr*; 4 ×SSC | Tr*; 4×SSC |

‡ The "hybrid length" is the anticipated length for the hybridising nucleic acid. When nucleic acids of known sequence are hybridised, the hybrid length may be determined by aligning the sequences and identifying the conserved regions described herein.
† SSPE (1×SSPE is 0.15M NaCl, 10mM $NaH_2PO_4$, and 1.25mM EDTA, pH7.4) may be substituted for SSC (1×SSC is 0.15M NaCl and 15mM sodium citrate) in the hybridisation and wash buffers; washes are performed for 15 minutes after hybridisation is complete. The hybridisations and washes may additionally include 5 × Denhardt's reagent, 0.5-1.0% SDS, 100 μg/ml denatured, fragmented salmon sperm DNA, 0.5% sodium pyrophosphate, and up to 50% formamide.
* Tb-Tr: The hybridisation temperature for hybrids anticipated to be less than 50 base pairs in length should be 5-10°C less than the melting temperature $T_m$ of the hybrids; the $T_m$ is determined according to the above-mentioned equations.
± The present invention also encompasses the substitution of any one, or more DNA or RNA hybrid partners with either a PNA, or a modified nucleic acid.

For the purposes of defining the level of stringency, reference can be made to Sambrook et al. (2001) Molecular Cloning: a laboratory manual, 3rd Edition Cold Spring Harbor Laboratory Press, CSH, New York or to Current Protocols in Molecular Biology, John Wiley & Sons, N.Y. (1989).

Nucleic acids encoding "homologues" of a CYCD3 polypeptide may also be useful in the present invention. Homologues encompass peptides, oligopeptides, polypeptides, proteins and enzymes having amino acid substitutions, deletions and/or insertions relative to the unmodified protein in question and having similar biological and functional activity as the unmodified protein from which they are derived. To produce such homologues, amino acids of the protein may be replaced by other amino acids having similar properties (such as similar hydrophobicity, hydrophilicity, antigenicity, propensity to form or break α-helical structures or β-sheet structures). Conservative substitution tables are well known in the art (see for example Creighton (1984) Proteins. W.H. Freeman and Company and Table 3 below).

Also encompassed by the term "homologues" are two special forms of homology, which include orthologous sequences and paralogous sequences, which encompass evolutionary concepts used to describe ancestral relationships of genes. The term "paralogous° relates to gene-duplications within the genome of a species leading to paralogous genes. The term "orthologous" relates to homologous genes in different organisms due to speciation. Examples of homologues of a CYCD3 polypeptide are given in Table 1 hereinabove.

Orthologues in, for example, monocot plant species may easily be found by performing a so-called reciprocal blast search. This may be done by a first blast involving blasting a query sequence (for example SEQ ID NO: 1, SEQ ID NO: 2, SEQ ID NO: 48 or SEQ ID NO: 49) against any sequence database, such as the publicly available NCBI database which may be found at: http://www.ncbi.nlm.nih.gov. BLASTn or TBLASTX may be used when starting from nucleotide sequence, or BLASTP or TBLASTN when starting from the protein, with standard default values. The BLAST results may optionally be filtered. The full-length sequences of either the filtered results or the non-filtered results are then BLASTed back (second BLAST) against the sequences of the organism from which the query sequence is derived. The results of the first and second BLASTs are then compared. A paralogue is identified if a high-ranking hit from the second blast is from the same species as from which the query sequence is derived; an orthologue is identified if a high-ranking hit is not from the same species as from which the query sequence Is derived. High-ranking hits are those having a low E-value. The lower the E-value, the more significant the score (or in other words the lower the chance that the hit was found by chance). Computation of the E-value is well known in the art. In the case of large families, ClustalW may be used, followed by a neighbour joining tree, to help visualize clustering of related genes and to identify orthologues and paralogues.

Orthologues and paralogues identified as described hereinabove are useful in performing the methods of the invention. According to the invention, there is provided a method for increasing plant seed yield, comprising transforming a plant with a nucleic acid encoding an orthologue or a paralogue of a CYCD3 polypeptide represented by SEQ ID NO: 2 or SEQ ID NO: 49, which nucleic acid is under the control of an endosperm-specific promoter.

A homologue may be in the form of a "substitutional variant" of a protein, i.e. where at least one residue in an amino acid sequence has been removed and a different residue inserted in its place. Amino acid substitutions are typically of single residues, but may be clustered depending upon functional constraints placed upon the polypeptide; insertions will usually be of the order of about 1 to 10 amino acid residues. Preferably, amino acid substitutions comprise conservative amino acid substitutions. Conservative substitution tables are readily available in the art. Table 3 below gives examples of conserved amino acid substitutions.

**Table 3: Examples of conserved amino acid substitutions**

| Residue | Conservative Substitutions | Residue | Conservative Substitutions |
|---|---|---|---|
| Ala | Ser | Leu | Ile; Val |
| Arg | Lys | Lys | Arg; Gln |
| Asn | Gln; His | Met | Leu; Ile |
| Asp | Glu | Phe | Met; Leu; Tyr |
| Gln | Asn | Ser | Thr; Gly |
| Cys | Ser | Thr | Ser; Val |
| Glu | Asp | Trp | Tyr |
| Gly | Pro | Tyr | Trp; Phe |
| His | Asn; Gln | Val | Ile; Leu |
| Ile | Leu, Val | | |

A homologue may also be in the form of an "insertional variant" of a protein, i.e. where one or more amino acid residues are introduced into a predetermined site in a protein. Insertions may comprise N-terminal and/or C-terminal fusions as well as intra-sequence insertions of single or multiple amino acids. Generally, insertions within the polypeptide sequence will be smaller than N- or C-terminal fusions, of the order of about 1 to 10 residues. Examples of N- or C-terminal fusion proteins or peptides include the binding domain or activation domain of a transcriptional activator as used in the yeast two-hybrid system, phage coat proteins, (histidine)-6-tag, glutathione S-transferase-tag, protein A, maltose-binding protein, dihydrofolate reductase, Tag-100 epitope, c-myc epitope, FLAG®-epitope, lacZ, CMP (calmodulin-binding peptide), HA epitope, protein C epitope and VSV epitope.

Homologues in the form of "deletion variants" of a protein are characterised by the removal of one or more amino acids from a protein.

Polypeptide variants of a protein may readily be made using peptide synthetic techniques well known in the art, such as solid phase peptide synthesis and the like, or by recombinant DNA manipulations. Methods for the manipulation of DNA sequences to produce substitution, insertion or deletion variants of a protein are well known in the art. For example, techniques for making substitution mutations at predetermined sites in DNA are well known to those skilled in the art and include M13 mutagenesis, T7-Gen *in vitro* mutagenesis (USB, Cleveland, OH), QuickChange Site Directed mutagenesis (Stratagene, San Diego, CA), PCR-mediated site-directed mutagenesis or other site-directed mutagenesis protocols.

The CYCD3 polypeptide may be a derivative. "Derivatives" of a protein encompass peptides, oligopeptides, polypeptides comprising naturally occurring altered (glycosylated, acylated, ubiquinated, prenylated, phosphorylated, myristoylated, sulphated etc) or non-naturally altered amino acid residues compared to the amino acid sequence of a naturally-occurring form of the polypeptide. A derivative may also comprise one or more non-amino acid substituents or additions compared to the amino acid sequence from which it is derived, for example a reporter molecule or other ligand, covalently or non-covalently bound to the amino acid sequence, such as a reporter molecule which is bound to facilitate its detection, and non-naturally occurring amino acid residues relative to the amino acid sequence of a naturally-occurring protein.

The CYCD3 polypeptide may be encoded by an alternative splice variant of a CYCD3-encoding nucleic acid/gene. The term "alternative splice variant" as used herein encompasses variants of a nucleic acid sequence in which selected introns and/or exons have been excised, maintained, replaced or added, or in which introns have been shortened or lengthened. Such variants will be ones in which the biological activity of the protein is retained, which may be achieved by selectively retaining functional segments of the protein. Such splice variants may be found in nature or may be manmade. Methods for making such splice variants are well known in the art.

According to the invention, there is provided a method for increasing plant seed yield, comprising transforming a plant with a splice variant of a nucleic acid encoding a CYCD3 polypeptide under the control of an endosperm-specific promoter.

Preferred splice variants are splice variants of a nucleic acid encoding a polypeptide which, when used in the construction of a cyclin or cyclin D phylogenetic tree, falls into the D3-type group which includes the CYCD3 represented by SEQ ID NO: 2 or SEQ ID NO: 49. Such splice variants may be splice variants of any of the nucleic acids mentioned in Table 1 above. Splice variants of SEQ ID NO: 1 or SEQ ID NO: 48 are particularly preferred for use in the methods of the invention.

The CYCD3 polypeptide may also be encoded by an allelic variant of a CYCD3-encoding nucleic acid/gene. Allelic variants exist in nature, and encompassed within the methods of the present invention is the use of these natural alleles. Allelic variants encompass Single Nucleotide Polymorphisms (SNPs), as well as Small Insertion/Deletion Polymorphisms (INDELs). The size of INDELs is usually less than 100 bp. SNPs and INDELs form the largest set of sequence variants in naturally occurring polymorphic strains of most organisms.

According to the invention, there is provided a method for increasing plant seed yield, comprising transforming a plant with an allelic variant of a nucleic acid encoding a CYCD3 polypeptide under the control of an endosperm-specific promoter.

Preferred allelic variants are allelic variants of a nucleic acid encoding a polypeptide which, when used in the construction of a cyclin or cyclin D phylogenetic tree falls into the D3-type group which includes the CYCD3 as in SEQ ID NO: 2 or SEQ ID NO: 49. Such allelic variants may be allelic variants of any of the nucleic acids mentioned in Table 1 above. Allelic variants of SEQ ID NO: 1 or SEQ ID NO: 48 are particularly preferred for use in the methods of the invention.

Site-directed mutagenesis and directed evolution are examples of technologies that enable the generation of novel CYCD3 variants.

Several methods are available to achieve site-directed mutagenesis, the most common being PCR based methods (current protocols in molecular biology. Wiley Eds. http://www.4ulr.com/products/currentprotocols/index.html).

Directed evolution, also known as gene shuffling, may also be used to generate variants of CYCD3-encoding nucleic acids. This consists of iterations of DNA shuffling followed by appropriate screening and/or selection to generate variants of CYCD3-encoding nucleic acids or portions thereof encoding CYCD3 polypeptides or portions thereof having a modified biological activity (Castle et al., (2004) Science 304(5674): 1151-4; US patents 5,811,238 and 6,395,547).

Therefore, the nucleic acid introduced into a plant may be one obtained through the techniques of site-directed mutagenesis or directed evolution or any other known method for the generation of such variant sequences.

The nucleic acid to be introduced into a plant may be a full-length nucleic acid or may be a variant sequence as hereinbefore defined. According to a preferred aspect of the present invention, increased expression of a CYCD3-encoding nucleic acid is envisaged. Methods for increasing expression of genes or gene products are well documented in the art and include, for example, overexpression driven by appropriate promoters, the use of transcription enhancers or translation enhancers. Isolated nucleic acids which serve as promoter or enhancer elements may be introduced in an appropriate position (typically upstream) of a non-heterologous form of a polynucleotide so as to upregulate expression of a CYCD3-encoding nucleic acid or variant thereof. For example, endogenous promoters may be altered *in vivo* by mutation, deletion, and/or substitution (see, Kmiec, U.S. Pat. No. 5,565,350; Zarling et al., WO 93/22443), or isolated promoters may be introduced into a plant cell in the proper orientation and distance from a gene of the present invention so as to control the expression of the gene. Methods for reducing the expression of genes or gene products are well documented in the art.

If polypeptide expression is desired, it is generally preferable to include a polyadenylation region at the 3'-end of a polynucleotide coding region. The polyadenylation region can be derived from the natural gene, from a variety of other plant genes, or from T-DNA. The 3' end sequence to be added may be derived from, for example, the nopaline synthase or octopine synthase genes, or alternatively from another plant gene, or less preferably from any other eukaryotic gene.

An intron sequence may also be added to the 5' untranslated region or the coding sequence of the partial coding sequence to increase the amount of the mature message that accumulates in the cytosol. Inclusion of a spliceable intron in the transcription unit in both plant and animal expression constructs has been shown to increase gene expression at both the mRNA and protein levels up to 1000-fold, Buchman and Berg, Mol. Cell biol. 8:4395-4405 (1988); Callis et al., Genes Dev. 1:1183-1200 (1987). Such intron enhancement of gene expression is typically greatest when placed near the 5' end of the transcription unit. Use of the maize introns Adh1-S Intron 1, 2, and 6, the Bronze-1 intron are known in the art. See generally, The Maize Handbook, Chapter 116, Freeling and Walbot, Eds., Springer, N.Y. (1994).

The invention also provides genetic constructs and vectors to facilitate introduction and/or expression of the nucleotide sequences useful in the methods according to the invention.

Therefore, there is provided a gene construct comprising:

(i) A nucleic acid encoding a CYCD3 polypeptide;
(ii) one or more endosperm-specific control sequences driving expression of the nucleic acid of (i) in the endosperm of seeds; and optionally
(iii) A transcription termination sequence.

The nucleic acid encoding a CYCD3 polypeptide may be any nucleic acid encoding a CYCD3 polypeptide as defined hereinabove. Particularly preferred are the nucleic acids described in Table 1, particularly the nucleic acid represented by SEQ ID NO: 1 or SEQ ID NO: 48. Also preferred are nucleic acid variants of the nucleic acids described in Table 1, such variants being as defined above.

Constructs useful in the methods according to the present invention may be constructed using recombinant DNA technology well known to persons skilled in the art. The gene constructs may be inserted into vectors, which may be commercially available, suitable for transforming into plants and suitable for expression of the gene of interest in the transformed cells. The invention therefore provides use of a gene construct as defined hereinabove in the methods of the invention.

Plants are transformed with a vector comprising the sequence of interest (i.e., a nucleic acid encoding a CYCD3 polypeptide). The sequence of interest is operably linked to one or more control sequences (at least to a promoter capable of preferentially driving expression of the nucleic acid in the endosperm of seeds). The terms "regulatory element", "control sequence" and "promoter" are all used interchangeably herein and are to be taken in a broad context to refer to regulatory nucleic acid sequences capable of effecting expression of the sequences to which they are ligated. Encompassed by the aforementioned terms are transcriptional regulatory sequences derived from a classical eukaryotic genomic gene (including the TATA box which is required for accurate transcription initiation, with or without a CCAAT box sequence) and additional regulatory elements (i.e. upstream activating sequences, enhancers and silencers) which alter gene expression in response to developmental and/or external stimuli, or in a tissue-specific manner. Also included within the term is a transcriptional regulatory sequence of a classical prokaryotic gene, in which case it may include a -35 box sequence and/or -10 box transcriptional regulatory sequences. The term "regulatory element also encompasses a synthetic fusion molecule or derivative that confers, activates or enhances expression of a nucleic acid molecule in a cell, tissue or organ. The term "operably linked" as used herein refers to a functional linkage between the promoter sequence and the gene of interest, such that the promoter sequence is able to initiate transcription of the gene of interest.

The promoter capable of preferentially expressing the nucleic acid in the endosperm of seeds is an endosperm-specific promoter. An endosperm-specific promoter refers to any promoter able to preferentially drive expression of the gene of interest in the endosperm. Reference herein to preferentially increasing expression in the endosperm of seeds is taken to mean increasing expression in the endosperm substantially to the exclusion of expression elsewhere in the plant, apart from any residual expression due to leaky promoters. For example, the prolamin promoter shows strong expression in the endosperm, with leakiness in meristem, more specifically the shoot meristem and/or discrimination centre in the meristem.

Preferably, the endosperm-specific promoter is a seed storage protein promoter, more preferably a promoter isolated from a prolamin gene, such as a rice prolamin RP6 (Wen et al., (1993) Plant Physiol 101(3):1115-6) promoter as represented by SEQ ID NO: 3 or a promoter of similar strength and/or a promoter with a similar expression pattern as the rice prolamin promoter. Similar strength and/or similar expression pattern may be analysed, for example, by coupling the promoters to a reporter gene and checking the function of the reporter gene in tissues of the plant. One well-known reporter gene is beta-glucuronidase and the colorimetric GUS stain used to visualize beta-glucuronidase activity in plant tissue. It should be clear that the applicability of the present invention is not restricted to the nucleic acid represented by SEQ ID NO: 1 or SEQ ID NO: 48, nor is the applicability of the invention restricted to expression of a nucleic acid encoding a CYCD3 polypeptide when driven by a prolamin promoter. Examples of other endosperm-specific promoters that may also be used In performing the methods of the invention are shown in Table 4 below.

**Table 4:** Examples of endosperm-specific promoters for use in the present invention

| GENE SOURCE | REFERENCE |
|---|---|
| glutelin (rice) | Takaiwa et al. (1986) Mol Gen Genet 208:15-22 Takaiwa et al. (1987) FEBS Letts. 221:43-47 |
| zein | Matzke et al., (1990) Plant Mol Biol 14(3): 323-32 |
| wheat LMW and HMW glutenin-1 | Colot et al. (1989) Mol Gen Genet 216:81-90 Anderson et al. (1989) NAR 17:461-2 |
| wheat SPA | Albani et a/. (1997) Plant Cell 9:171-184 |
| wheat gliadins | Rafalski et al. (1984) EMBO 3:1409-15 |
| barley *Itr1* promoter | Diaz et al. (1995) Mol Gen Genet 248(5):592-8 |
| barley B1, C, D, hordein | Cho et al. (1999) Theor Appl Genet 98:1253-62 Muller et al. (1993) Plant J 4:343-55 Sorenson et al. (1996) Mol Gen Genet 250:750-60 |
| barley DOF | Mena et al, (1998) Plant J 116(1): 53-62 |
| *blz2* | Onate et al. (1999) J Biol Chem 274(14):9175-82 |

(continued)

| GENE SOURCE | REFERENCE |
|---|---|
| synthetic promoter | Vicente-Carbajosa et al. (1998) Plant J 13:629-640 |
| rice prolamin NRP33 | Wu et al, (1998) Plant Cell Physiol 39(8) 885-889 |
| rice globulin Glb-1 | Wu et al. (1998) Plant Cell Physiol 39(8) 885-889 |
| rice globulin REB/OHP-1 | Nakase et al. (1997) Plant Molec Biol 33: 513-522 |
| rice ADP-glucose PP | Russell et al. (1997) Trans Res 6:157-68 |
| maize ESR gene family | Opsahl-Ferstad et al. (1997) Plant J 12:235-46 |
| sorgum kafirin | DeRose et al. (1996) Plant Molec Biol 32:1029-35 |

Optionally, one or more terminator sequences may also be used in the construct introduced into a plant. The term "terminator" encompasses a control sequence which is a DNA sequence at the end of a transcriptional unit which signals 3' processing and polyadenylation of a primary transcript and termination of transcription. Additional regulatory elements may include transcriptional as well as translational enhancers. Those skilled in the art will be aware of terminator and enhancer sequences that may be suitable for use in performing the invention. Such sequences would be known or may readily be obtained by a person skilled in the art.

The genetic constructs of the invention may further include an origin of replication sequence that is required for maintenance and/or replication in a specific cell type. One example is when a genetic construct is required to be maintained in a bacterial cell as an episomal genetic element (e.g. plasmid or cosmid molecule). Preferred origins of replication include, but are not limited to, the f1-ori and colE1.

The genetic construct may optionally comprise a selectable marker gene. As used herein, the term "selectable marker gene" includes any gene that confers a phenotype on a cell in which it is expressed to facilitate the identification and/or selection of cells that are transfected or transformed with a nucleic acid construct of the invention. Suitable markers may be selected from markers that confer antibiotic or herbicide resistance, that introduce a new metabolic trait or that allow visual selection. Examples of selectable marker genes include genes conferring resistance to antibiotics (such as nptII that phosphorylates neomycin and kanamycin, or hpt, phosphorylating hygromycin), to herbicides (for example bar which provides resistance to Basta; aroA or gox providing resistance against glyphosate), or genes that provide a metabolic trait (such as manA that allows plants to use mannose as sole carbon source). Visual marker genes result in the formation of colour (for example β-glucuronidase, GUS), luminescence (such as luciferase) or fluorescence (Green Fluorescent Protein, GFP, and derivatives thereof).

The present invention also encompasses plants (and parts thereof) obtainable by the methods according to the present invention, wherein said plants comprise a construct comprising a nucleic acid encoding a CYCD3 polypeptide under the control of an endosperm-specific promoter. The present invention therefore provides plants obtainable by the method according to the present invention, which plants have been transformed with a CYCD3-encoding nucleic acid under the control of an endosperm-specific promoter.

The invention also provides a method for the production of transgenic plants having increased seed yield, comprising transforming a plant with a CYCD3-encoding nucleic acid under the control of an endosperm-specific promoter.

More specifically, the present invention provides a method for the production of transgenic plants having increased seed yield, which method comprises:

(i) transforming a plant or plant cell with a nucleic acid encoding a CYCD3 polypeptide under the control of an endosperm-specific promoter; and
(ii) cultivating the plant cell under conditions promoting plant growth and development.

The increases in seed yield are as defined above.

The nucleic acid may be introduced directly into a plant cell or into the plant itself (including introduction into a tissue, organ or any other part of a plant). According to the present invention, the nucleic acid is introduced into a plant by transformation.

The term "transformation" as referred to herein encompasses the transfer of an exogenous polynucleotide into a host cell, irrespective of the method used for transfer. Plant tissue capable of subsequent clonal propagation, whether by organogenesis or embryogenesis, may be transformed with a genetic construct of the present invention and a whole plant regenerated from there. The particular tissue chosen will vary depending on the clonal propagation systems available for, and best suited to, the particular species being transformed. Exemplary tissue targets include leaf disks, pollen, embryos, cotyledons, hypocotyls, megagametophytes, callus tissue, existing meristematic tissue (e.g., apical meristem, axillary buds, and root meristems), and induced meristem tissue (e.g., cotyledon meristem and hypocotyl meristem). The polynucleotide may be transiently or stably introduced into a host cell and may be maintained non-integrated, for example, as a plasmid. Alternatively, it may be integrated into the host genome. The resulting transformed plant cell may then be used to regenerate a transformed plant in a manner known to persons skilled in the art.

Transformation of plant species is now a fairly routine technique. Advantageously, any of several transformation methods may be used to introduce the gene of interest into a suitable ancestor cell. Transformation methods include the use of liposomes, electroporation, chemicals that increase free DNA uptake, injection of the DNA directly into the plant, particle gun bombardment, transformation using viruses or pollen and microprojection. Methods may be selected from the calcium/polyethylene glycol method for protoplasts (Krens, F.A. et al., (1982) Nature 296, 72-74; Negrutiu I et al. (1987) Plant Mol Biol 8: 363-373); electroporation of protoplasts (Shillito R.D. et al. (1985) Bio/Technol 3, 1099-1102); microinjection into plant material (Crossway A et al., (1986) Mol. Gen Genet 202: 179-185); DNA or RNA-coated particle bombardment (Klein TM et al., (1987) Nature 327: 70) infection with (non-integrative) viruses and the like. Transgenic rice plants expressing a CYCD3-encoding nucleic acid/gene are preferably produced via *Agrobacterium*-mediated transformation using any of the well known methods for rice transformation, such as described in any of the following: published European patent application EP 1198985 A1, Aldemita and Hodges (Planta 199: 612-617, 1996); Chan et al. (Plant Mol Biol 22 (3): 491-506, 1993), Hiei et al. (Plant J 6 (2): 271-282, 1994). In the case of corn transformation, the preferred method is as described in either Ishida et a/. (Nat. Biotechnol 14(6): 745-50, 1996) or Frame et al. (Plant Physiol 129 (1): 13-22, 2002).

Generally after transformation, plant cells or cell groupings are selected for the presence of one or more markers which are encoded by plant-expressible genes co-transferred with the gene of interest, following which the transformed material is regenerated into a whole plant.

Following DNA transfer and regeneration, putatively transformed plants may be evaluated, for instance using Southern analysis, for the presence of the gene of interest, copy number and/or genomic organisation. Alternatively or additionally, expression levels of the newly introduced DNA may be monitored using Northern and/or Western analysis, both techniques being well known to persons having ordinary skill in the art.

The generated transformed plants may be propagated by a variety of means, such as by clonal propagation or classical breeding techniques. For example, a first generation (or T1) transformed plant may be selfed to give homozygous second-generation (or T2) transformants, and the T2 plants further propagated through classical breeding techniques.

The generated transformed organisms may take a variety of forms. For example, they may be chimeras of transformed cells and non-transformed cells; clonal transformants (e.g., all cells transformed to contain the expression cassette); grafts of transformed and untransformed tissues (e.g., in plants, a transformed rootstock grafted to an untransformed scion).

The present invention clearly extends to any plant cell or plant produced by any of the methods described herein, and to all plant parts and propagules thereof. The present invention extends further to encompass the progeny of a primary transformed or transfected cell, tissue, organ or whole plant that has been produced by any of the aforementioned methods, the only requirement being that progeny exhibit the same genotypic and/or phenotypic characteristic (s) as those produced by the parent in the methods according to the invention. The invention also includes host cells containing an isolated CYCD3-encoding nucleic acid. Preferred host cells according to the invention are plant cells. The invention also extends to harvestable parts of a plant such as, but not limited to seeds, leaves, fruits, flowers, stem cultures, rhizomes, tubers and bulbs. The invention furthermore relates to products directly derived from a harvestable part of such a plant, such as dry pellets or powders, oil, fat and fatty acids, starch or proteins.

The present invention also encompasses use of CYCD3-encoding nucleic acids and use of CYCD3 polypeptides.

One such use relates to increasing seed yield. The seed yield is as defined hereinabove and preferably includes one or more of the following: increased number of flowers per panicle, increased total seed yield, increased TKW and increased harvest index, each relative to corresponding wild type plants.

CYCD3-encoding nucleic acids or variants thereof, or CYCD3 polypeptides may find use in breeding programmes in which a DNA marker is identified which may be genetically linked to a CYCD3-encoding gene or variant thereof. The CYCD3-encoding nucleic acids/ genes or variants thereof, or CYCD3 polypeptides may be used to define a molecular marker. This DNA or protein marker may then be used in breeding programmes to select plants having increased seed yield. The CYCD3-encoding gene or variant thereof may, for example, be a nucleic acid as represented by SEQ ID NO: 1 or SEQ ID NO: 48.

Allelic variants of a CYCD3-encoding nucleic acid/gene may also find use in marker-assisted breeding programmes. Such breeding programmes sometimes require introduction of allelic variation by mutagenic treatment of the plants, using for example EMS mutagenesis; alternatively, the programme may start with a collection of allelic variants of so called "natural" origin caused unintentionally. Identification of allelic variants then takes place, for example, by PCR. This is followed by a step for selection of superior allelic variants of the sequence In question and which give increased seed yield. Selection is typically carried out by monitoring growth performance of plants containing different allelic variants of the sequence in question, for example, different allelic variants of SEQ ID NO: 1 or SEQ ID NO: 48. Growth performance may be monitored in a greenhouse or in the field. Further optional steps include crossing plants, in which the superior allelic variant was identified, with another plant. This could be used, for example, to make a combination of interesting phenotypic features.

A CYCD3-encoding nucleic acid or variant thereof may also be used as probes for genetically and physically mapping the genes that they are a part of, and as markers for traits linked to those genes. Such information may be useful in plant breeding in order to develop lines with desired phenotypes. Such use of CYCD3-encoding nucleic acids or variants thereof requires only a nucleic acid sequence of at least 15 nucleotides in length. The CYCD3-encoding nucleic acids or variants thereof may be used as restriction fragment length polymorphism (RFLP) markers. Southern blots (Sambrook J, Fritsch EF and Maniatis T (1989) Molecular Cloning, A Laboratory Manual) of restriction-digested plant genomic DNA may be probed with the CYCD3-encoding nucleic acids or variants thereof. The resulting banding patterns may then be subjected to genetic analyses using computer programs such as MapMaker (Lander et al. (1987) Genomics 1: 174-181) in order to construct a genetic map. In addition, the nucleic acids may be used to probe Southern blots containing restriction andonuclease-treated genomic DNAs of a set of individuals representing parent and progeny of a defined genetic cross. Segregation of the DNA polymorphisms is noted and used to calculate the position of the CYCD3-encoding nucleic acid or variant thereof in the genetic map previously obtained using this population (Botstein et al. (1980) Am. J. Hum. Genet. 32:314-331).

The production and use of plant gene-derived probes for use in genetic mapping is described in Bematzky and Tanksley (1986) Plant Mol. Biol. Reporter 4: 37-41. Numerous publications describe genetic mapping of specific cDNA clones using the methodology outlined above or variations thereof. For example, F2 intercross populations, backcross populations, randomly mated populations, near isogenic lines (NIL), and other sets of individuals may be used for mapping. Such methodologies are well known to those skilled in the art.

The nucleic acid probes may also be used for physical mapping (i.e., placement of sequences on physical maps; see Hoheisel et al. In: Non-mammalian Genomic Analysis: A Practical Guide, Academic press 1996, pp. 319-346, and references cited therein).

In another embodiment, the nucleic acid probes may be used in direct fluorescence *in situ* hybridization (FISH) mapping (Trask (1991) Trends Genet. 7:149-154). Although current methods of FISH mapping favor use of large clones (several kb to several hundred kb; see Laan et al. (1995) Genome Res. 5:13-20), improvements in sensitivity may allow performance of FISH mapping using shorter probes.

A variety of nucleic acid amplification-based methods for genetic and physical mapping may be carried out using the nucleic acids. Examples include allele-specific amplification (Kazazian (1989) J. Lab. Clin. Med 11:95-96), polymorphism of PCR-amplified fragments (CAPS; Sheffield et al. (1993) Genomics 16:325-332), allele-specific ligation (Landegren et al. (1988) Science 241:1077-1080), nucleotide extension reactions (Sokolov (1990) Nucleic Acid Res. 18:3671), Radiation Hybrid Mapping (Walter et al. (1997) Nat. Genet. 7:22-28) and Happy Mapping (Dear and Cook (1989) Nucleic Acid Res. 17:6795-6807). For these methods, the sequence of a nucleic acid is used to design and produce primer pairs for use in the amplification reaction or in primer extension reactions. The design of such primers is well known to those skilled in the art. In methods employing PCR-based genetic mapping, it may be necessary to identify DNA sequence differences between the parents of the mapping cross in the region corresponding to the instant nucleic acid sequence. This, however, is generally not necessary for mapping methods.

Yield increases are obtained in the methods of the invention by transforming a plant with a nucleic acid encoding a CYCD3 polypeptide under the control of an endosperm-specific promoter. However, such seed yield increases may also be obtained by other well known techniques, such as T-DNA activation, TILLING and homologous recombination.

T-DNA activation tagging (Hayashi et al. Science (1992) 1350-1353) involves insertion of T-DNA, usually containing a promoter (may also be a translation enhancer or an intron), in the genomic region of the gene of Interest or 10 kb up- or downstream of the coding region of a gene in a configuration such that the promoter directs expression of the targeted gene. Typically, regulation of expression of the targeted gene by its natural promoter is disrupted and the gene falls under the control of the newly introduced promoter. The promoter is typically embedded in a T-DNA. This T-DNA is randomly inserted into the plant genome, for example, through *Agrobacterium* infection and leads to overexpression of genes near the inserted T-DNA. The resulting transgenic plants show dominant phenotypes due to overexpression of genes close to the introduced promoter. The promoter to be introduced may be any endosperm-specific promoter.

The technique of TILLING (Targeted Induced Local Lesions In Genomes) may also be used to reproduce the effects of performing the methods of the invention. TILLING is a mutagenesis technology useful to generate and/or identify, and to eventually isolate a CYCD3-encoding nucleic acid with modified expression and/or activity. TILLING also allows selection of plants carrying such mutant variants. These mutant variants may exhibit modified expression, either in strength or in location or in timing (if the mutations affect the promoter, for example). TILLNG combines high-density mutagenesis with high-throughput screening methods. The steps typically followed in TILLING are: (a) EMS mutagenesis (Redei GP and Koncz C (1992) In Methods in Arabidopsis Research, Koncz C, Chua NH, Schell J, eds. Singapore, World Scientific Publishing Co, pp. 16-82; Feldmann et al., (1994) In Meyerowitz EM, Somerville CR, eds, Arabidopsis. Cold Spring Harbor Laboratory Press, Cold Spring Harbor, NY, pp 137-172; Lightner J and Caspar T (1998) In J Martinez-Zapater, J Salinas, eds, Methods on Molecular Biology, Vol. 82. Humana Press, Totowa, NJ, pp 91-104); (b) DNA preparation and pooling of individuals; (c) PCR amplification of a region of interest; (d) denaturation and annealing to allow formation of heteroduplexes; (e) DHPLC, where the presence of a heteroduplex in a pool is detected as an extra peak in the chromatogram; (f) identification of the mutant individual; and (g) sequencing of the mutant PCR product. Methods for TILLING are well known in the art (McCallum et al., (2000) Nat Biotechnol 18: 455-457; reviewed by Stemple (2004) Nat Rev Genet 5(2): 145-50). Plants carrying such mutant variants have preferentially increased expression of a CYCD3-encoding gene in the endosperm.

T-DNA activation and TILUNG are examples of technologies that enable the generation of genetic modifications (preferably in the locus of a gene encoding a CYCD3 polypeptide) that give preferentially increased expression of a nucleic acid encoding a CYCD3 polypeptide in the endosperm of plants. The locus of a gene is defined herein as a genomic region, which includes the gene of interest and 10 kb up- or downstream of the coding region.

Homologous recombination allows introduction in a genome of a selected nucleic acid at a defined selected position. Homologous recombination is a standard technology used routinely in biological sciences for lower organisms such as yeast or the moss *Physcomitrella.* Methods for performing homologous recombination in plants have been described not only for model plants (Offringa et al. (1990) EMBO J 9(10): 3077-84) but also for crop plants, for example rice (Terada et al. (2002) Nat Biotech 20(10): 1030-4; Iida and Terada (2004) Curr Opin Biotech 15(2): 132-8). The nucleic acid (which may be a CYCD3-encoding nucleic acid or variant thereof as hereinbefore defined) is targeted to the locus of a CYCD3 gene. The nucleic acid to be targeted may be an improved allele used to replace the endogenous gene or may be introduced in addition to the endogenous gene. The nucleic acid to be targeted is preferably the region controlling the natural expression of a nucleic acid encoding a CYCD3 polypeptide in a plant. An endosperm-specific promoter is introduced into this region, in addition to it, or replacing it partly or substantially all of it.

All the methods according to the present invention result in plants having increased seed yield, as described hereinbefore. These useful traits may also be combined with other economically advantageous traits, such as further yield-enhancing traits, tolerance to various stresses, traits modifying various architectural features and/or biochemical and/or physiological features.

**Description of figures**

The present invention will now be described with reference to the following figures in which:

**Fig. 1** is a multiple polypeptide alignment prepared using ClustalW and default values, followed by average distance tree computation. The CYCD3 polypeptide cluster is shown.

**Fig. 2** is an alignment of known plant CYCD protein sequences. The sequences were aligned using AlignX program from Vector NTI suite (InforMax, Bethesda, MD). Multiple alignment was done with a gap opening penalty of 10 and a gap extension of 0.01. Minor manual editing was also carried out where necessary to better position some conserved regions. The line shown indicates the separation of CYCD3 polypeptides from other D-type cyclins. A number of motifs specific to CYCD3 polypeptides are boxed.

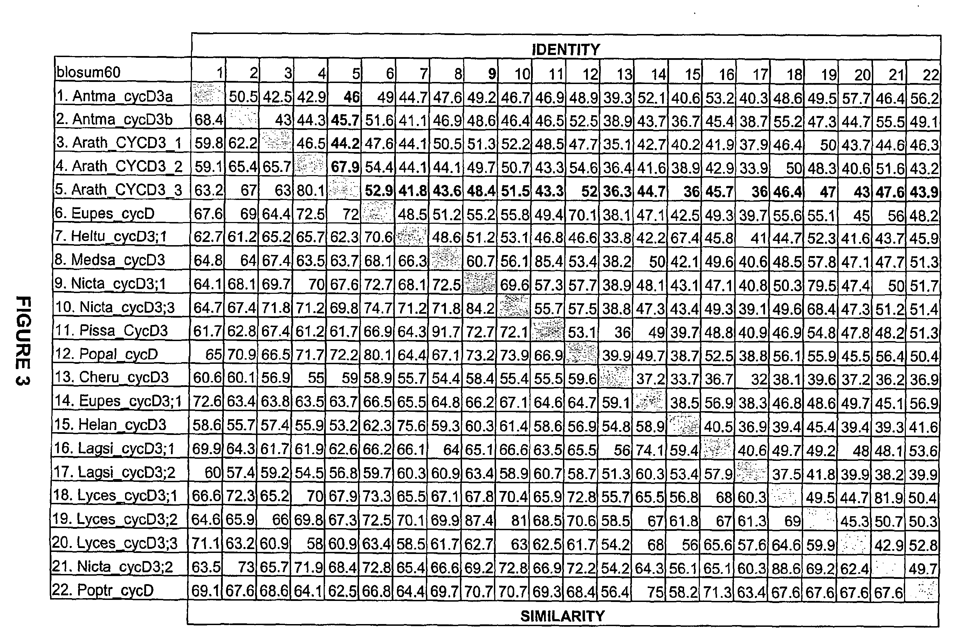

**Fig. 3** is a similarity/identity matrix prepared using MatGAT (Matrix Global Alignment Tool) which calculates the similarity and identity between every pair of polypeptide sequences in a given data set without requiring pre-alignment of the data. The program performs a series of pairwise alignments using the Myers and Miller global alignment algorithm (with a gap opening penalty of 12, and a gap extension penalty of 2). It then calculates similarity and identity using, for example, Blosum 60 as scoring matrix, and then places the results in a distance matrix. Sequence similarity is shown in the bottom half of the dividing line and sequence identity is shown in the top half of the dividing line. The sequence of SEQ ID NO: 2 is indicated as number 5 in the matrix. Polypeptide sequences having at least 30% sequence identity to the sequence of SEQ ID NO: 2 encompass CYCD3 polypeptides.

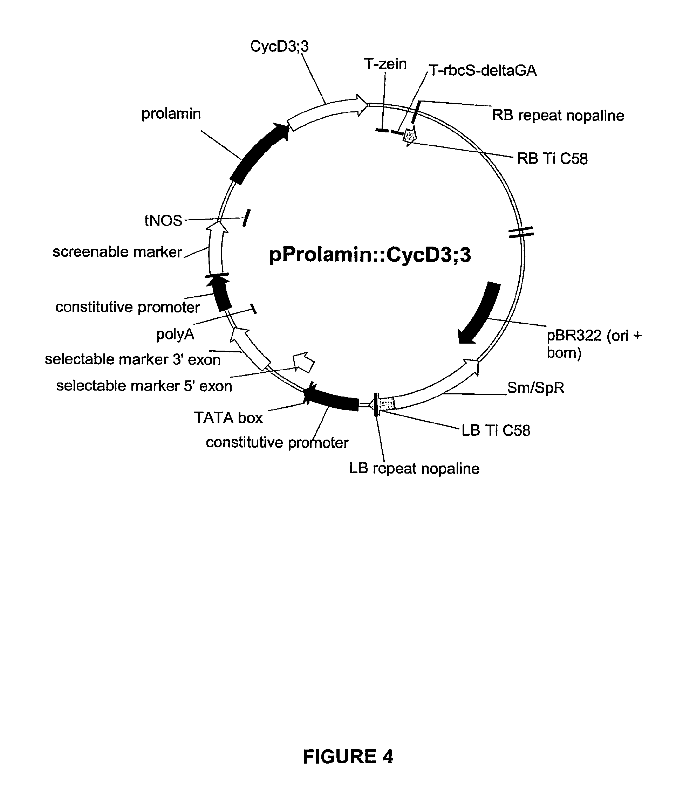

**Fig. 4** is a binary vector for expression in *Oryza sativa* of the *Arabidopsis thaliana* CycD3;3 gene under the control of the prolamin promoter.

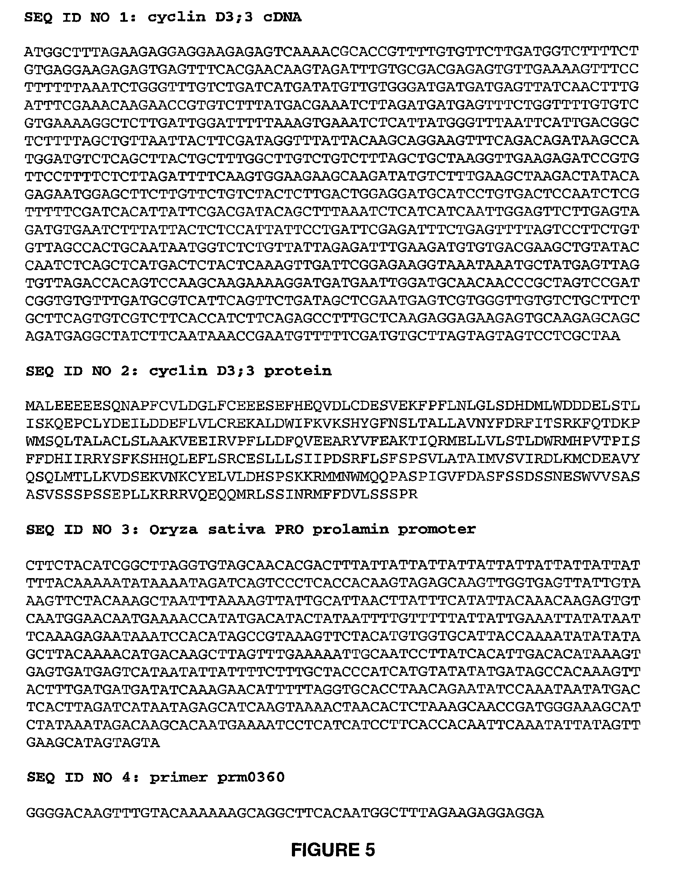

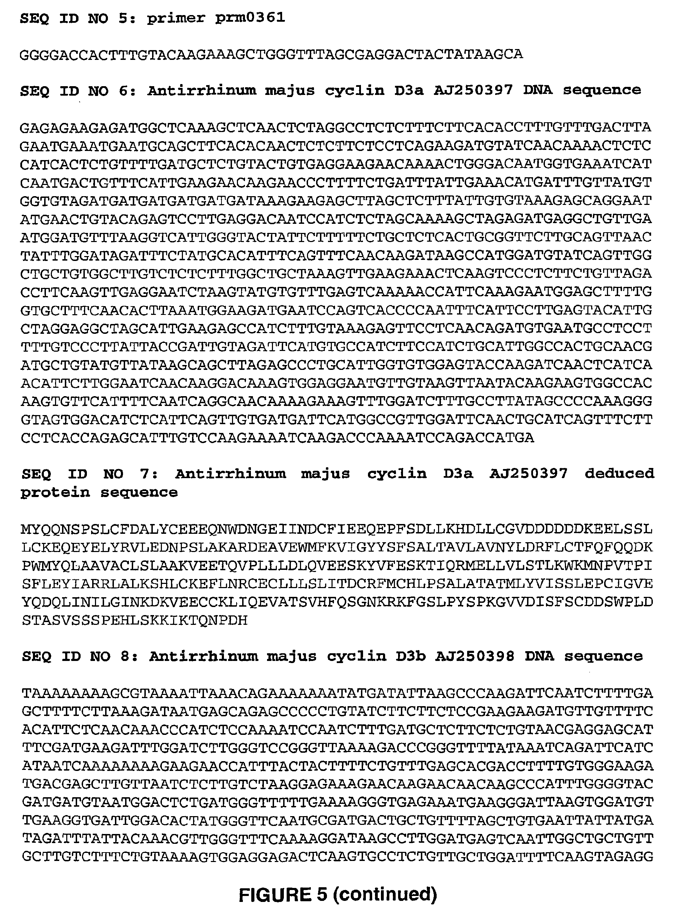

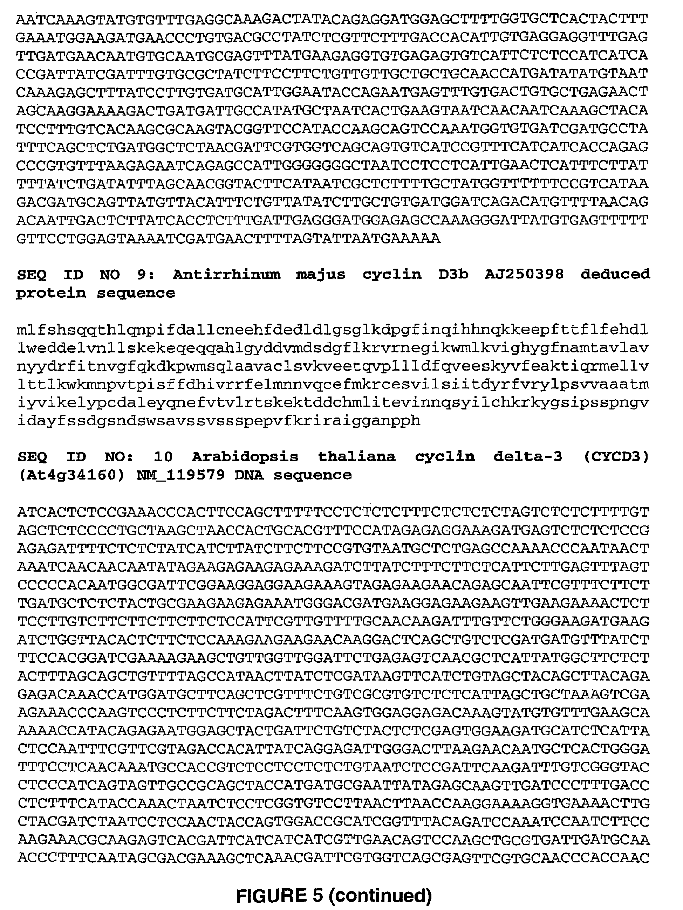

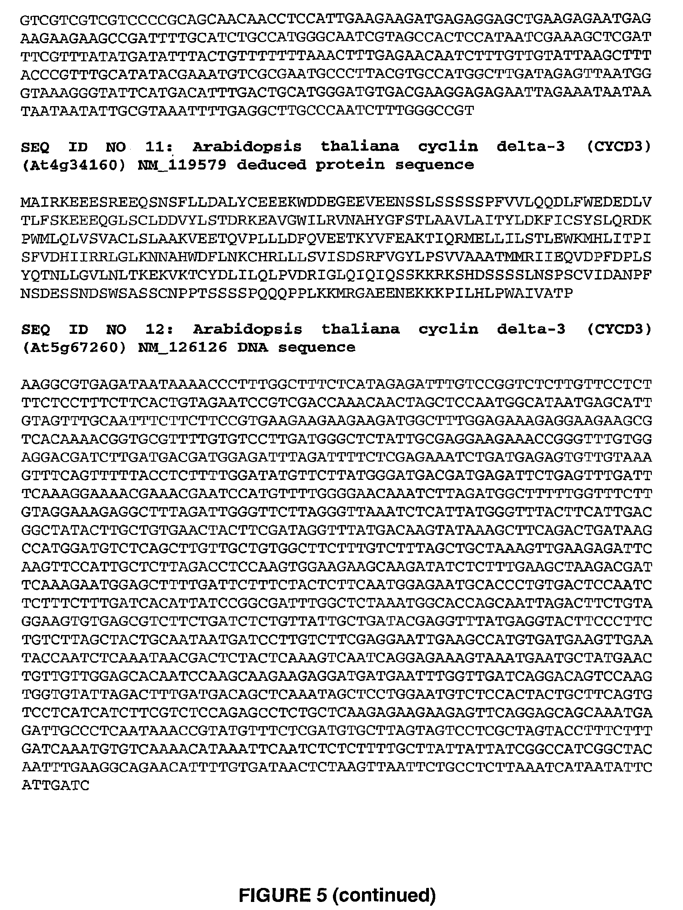

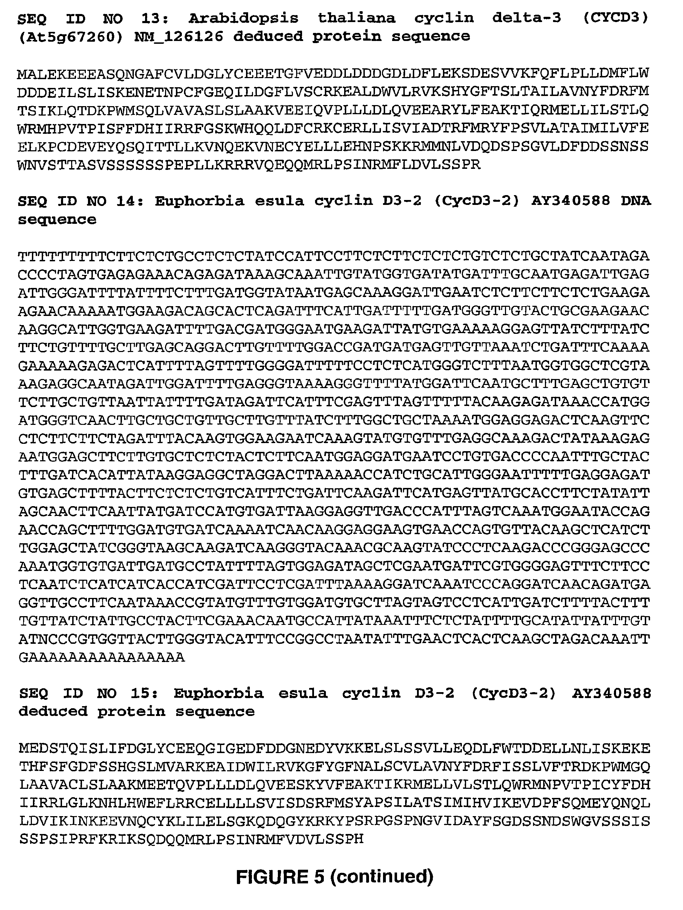

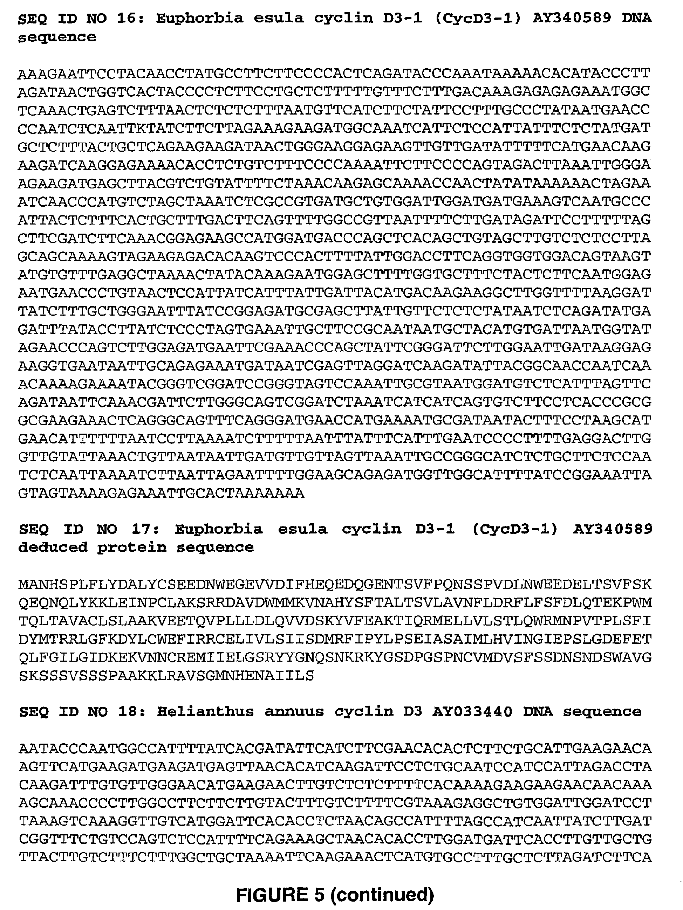

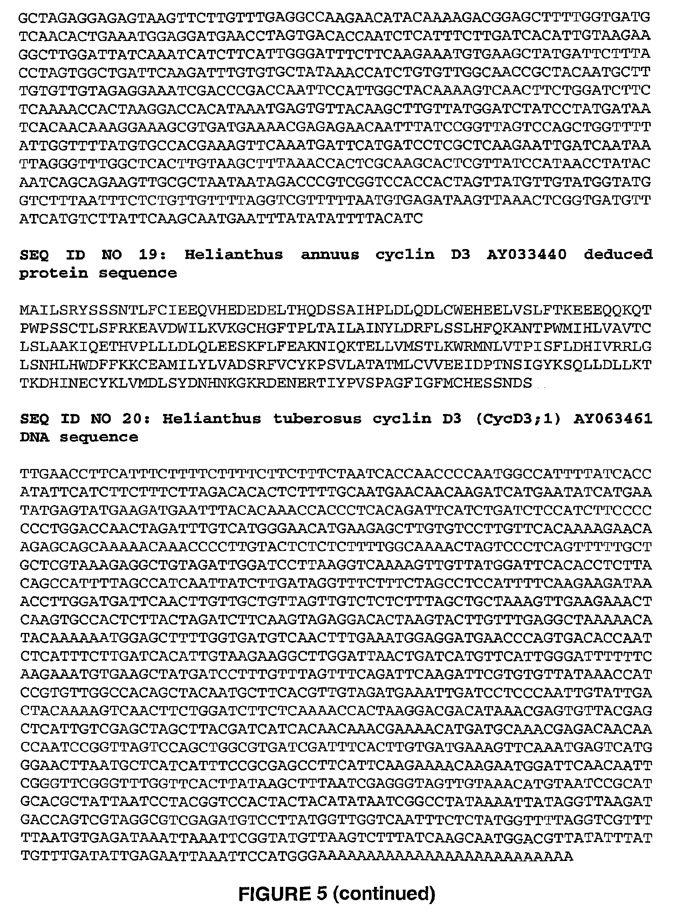

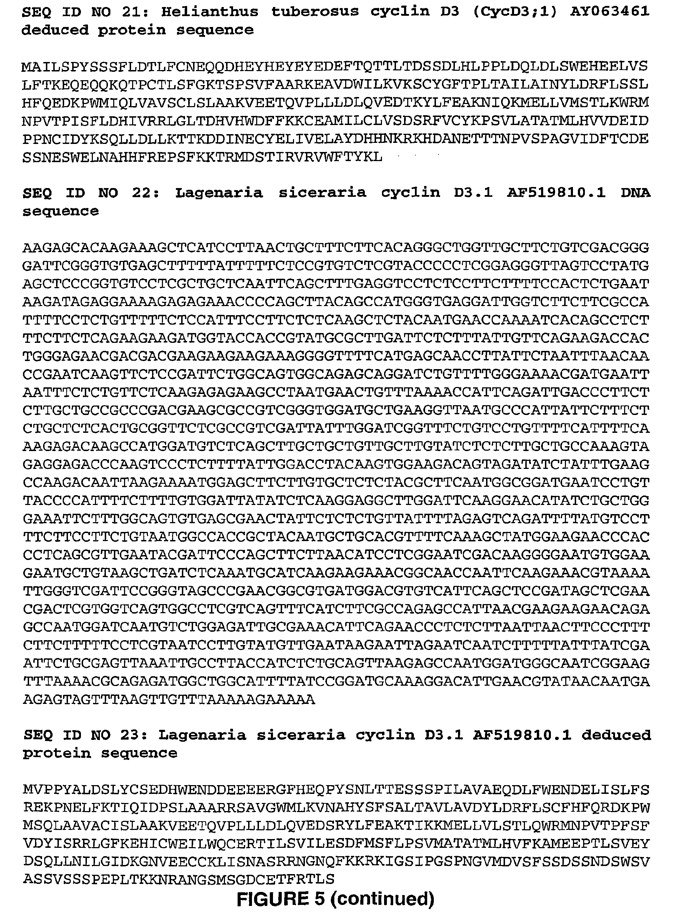

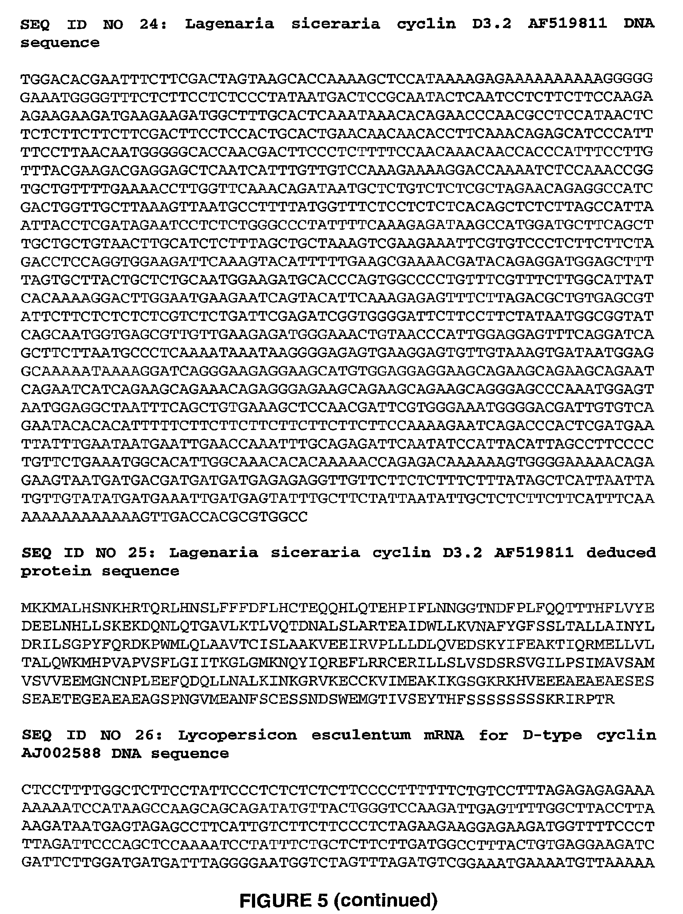

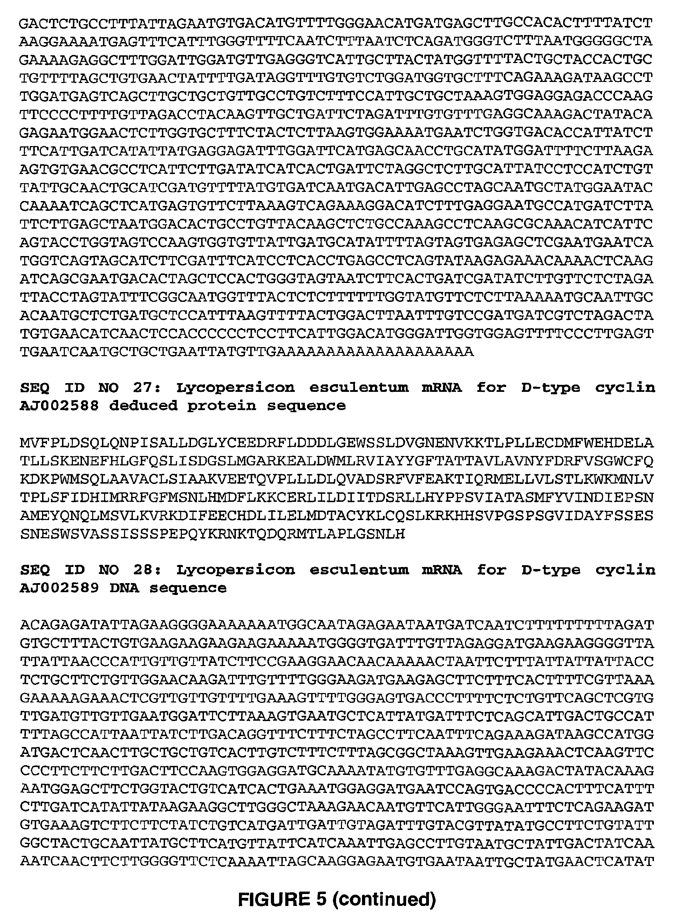

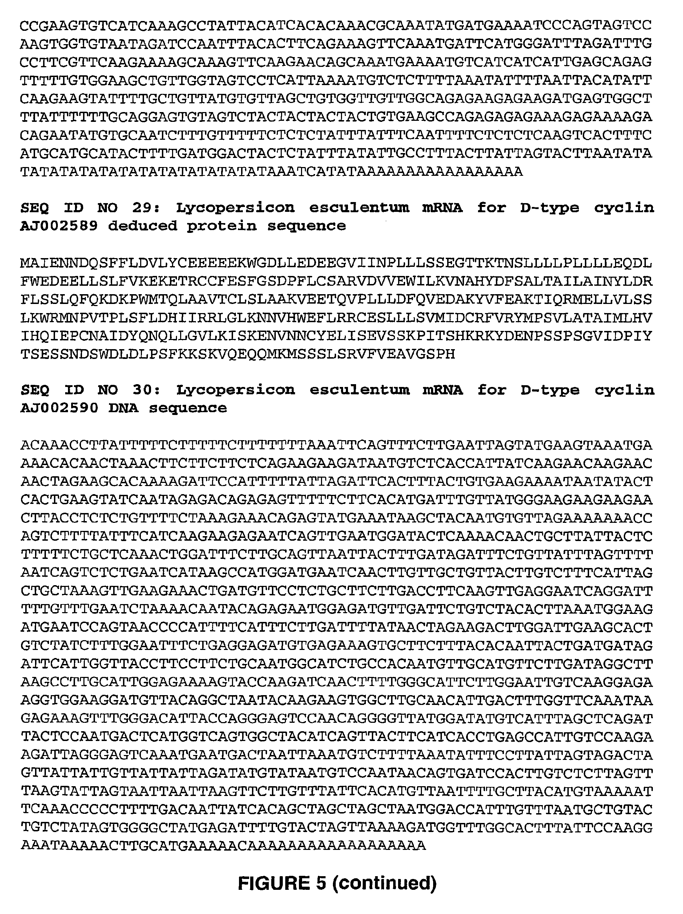

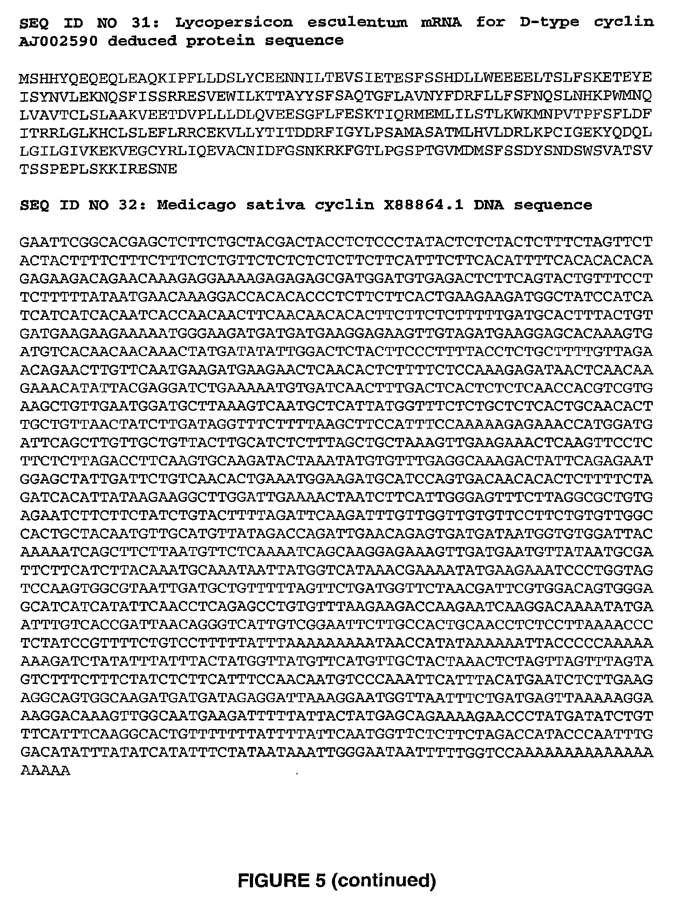

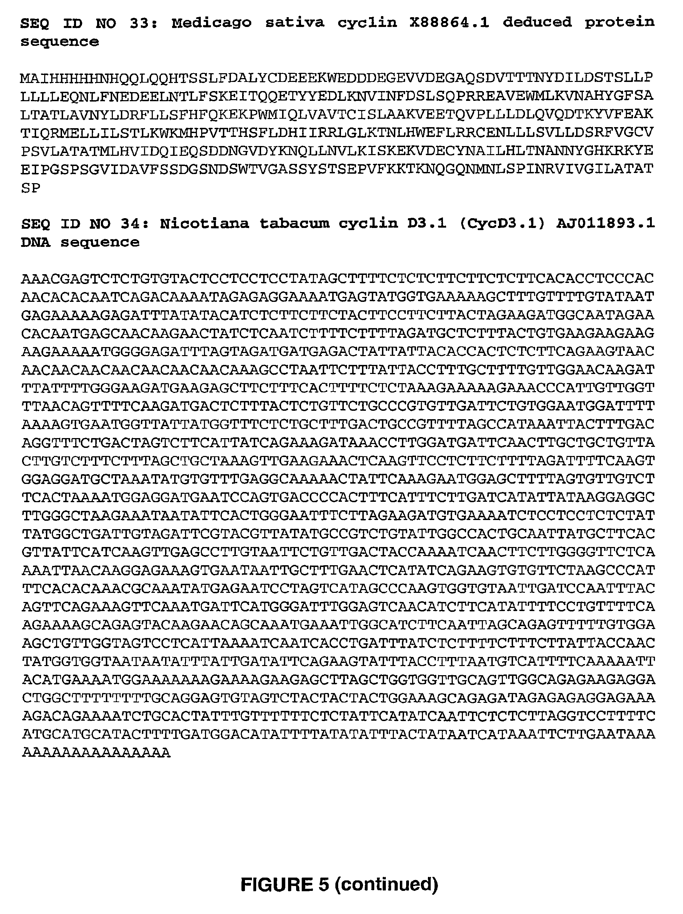

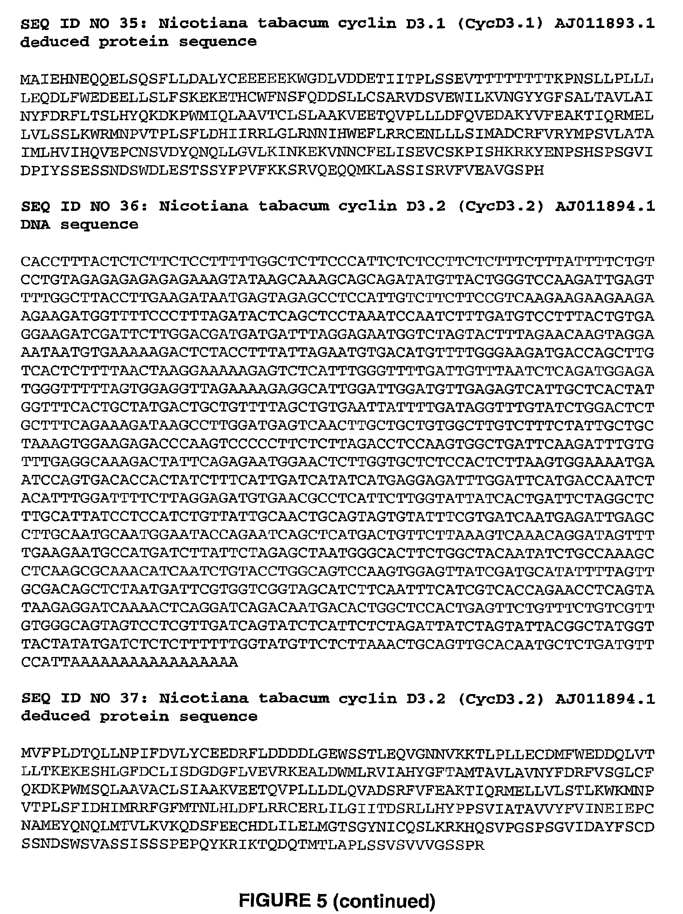

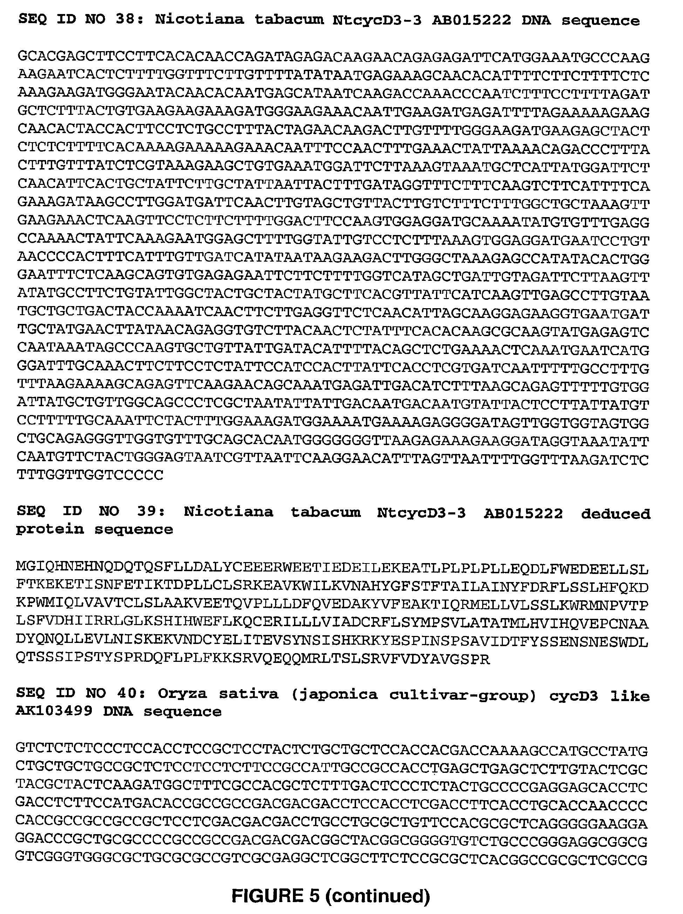

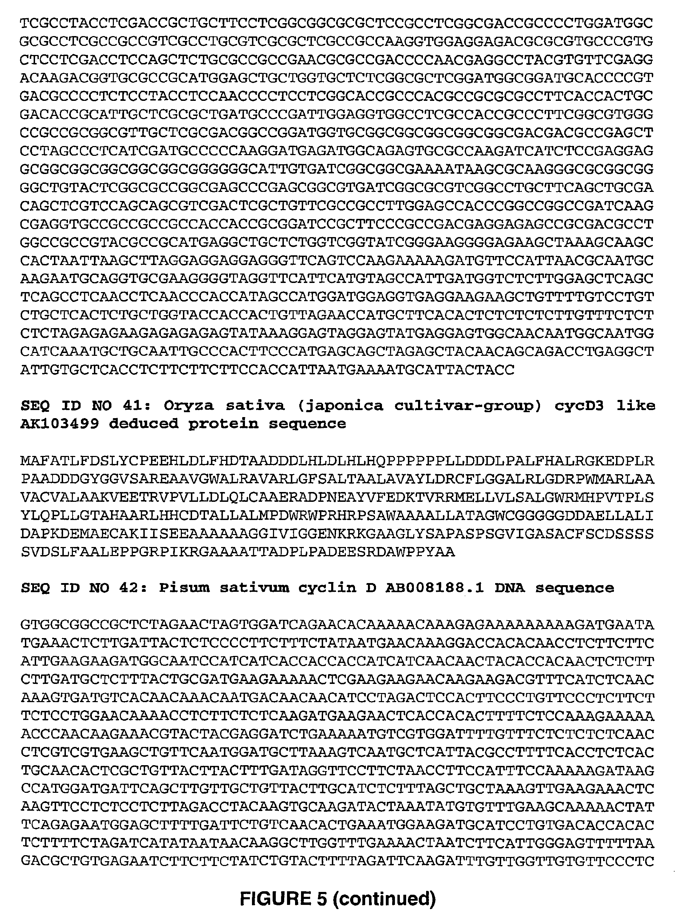

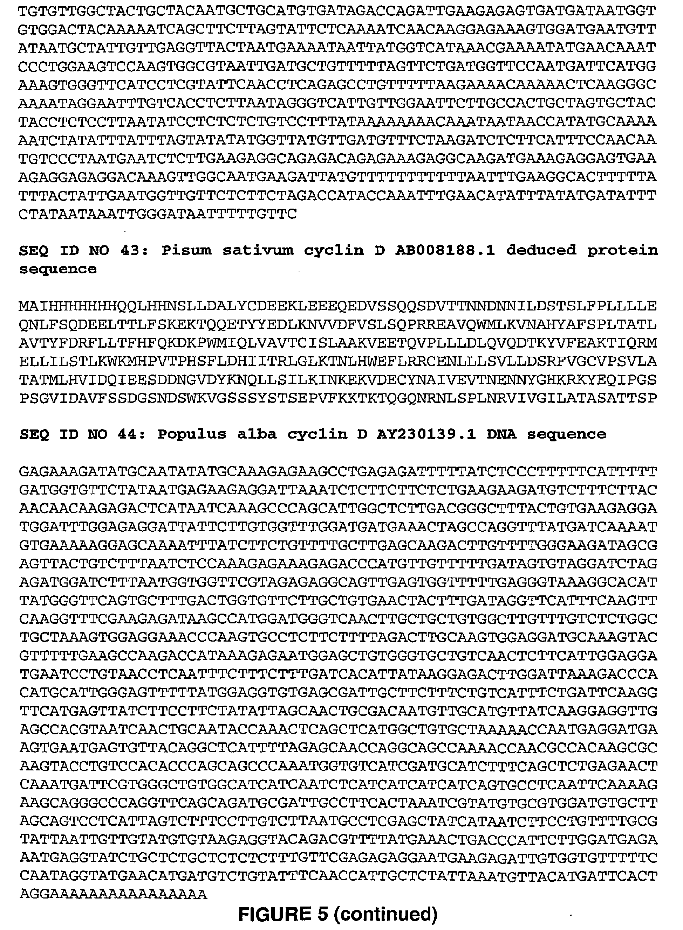

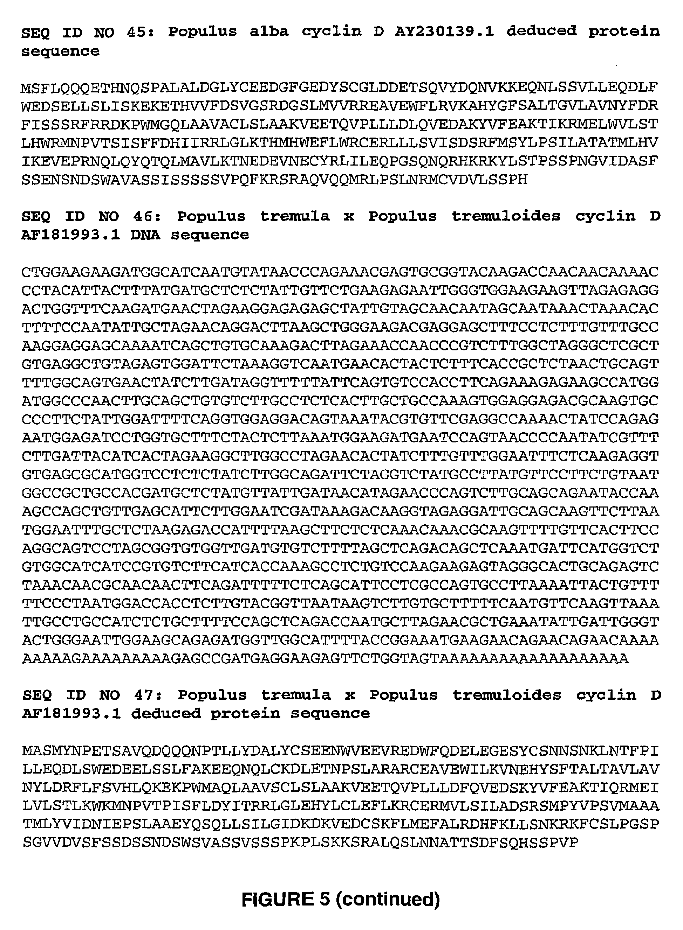

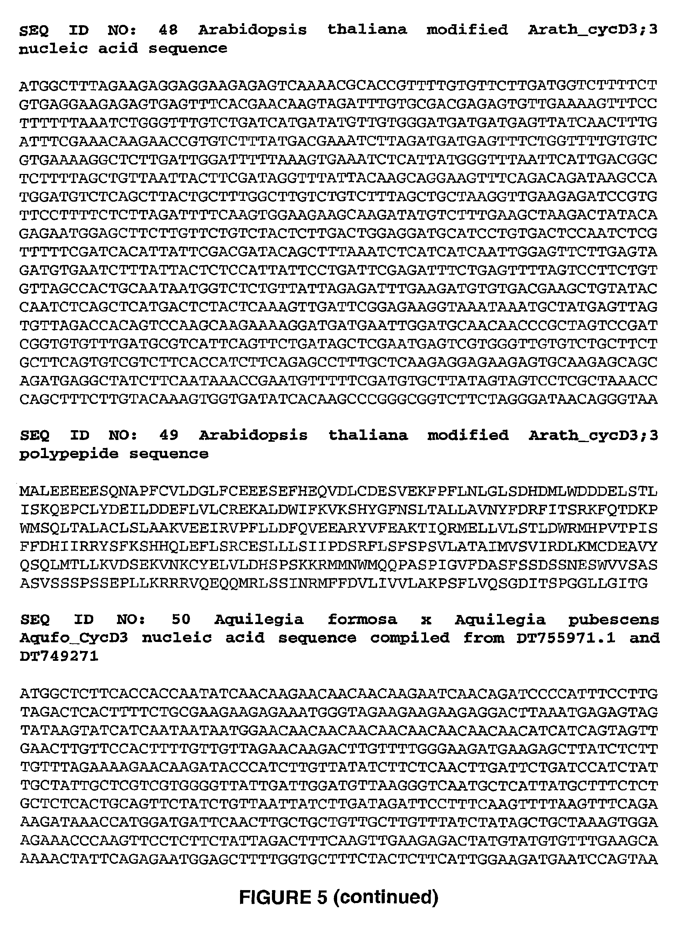

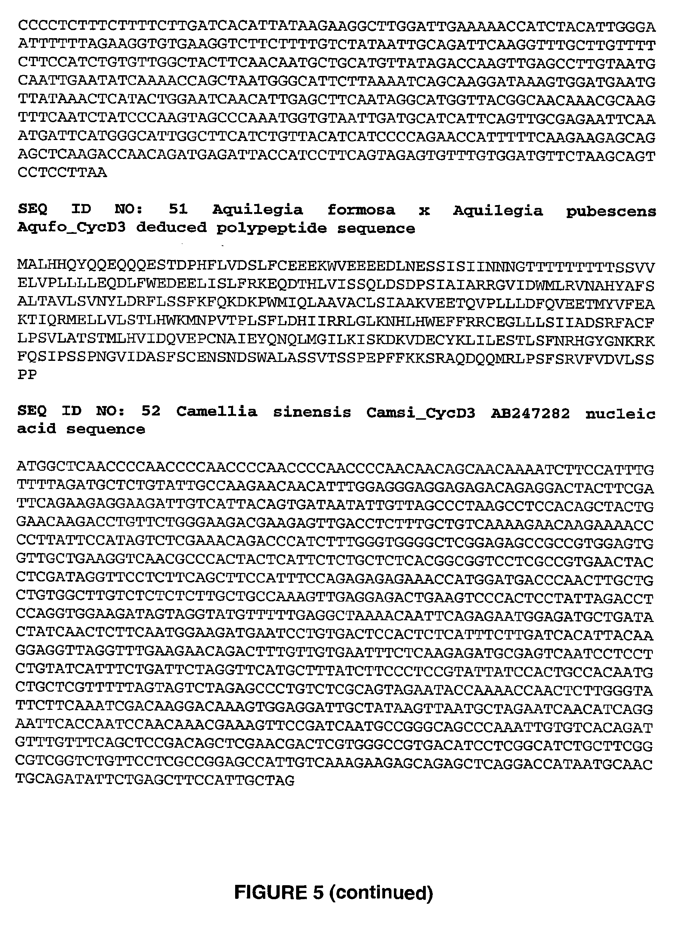

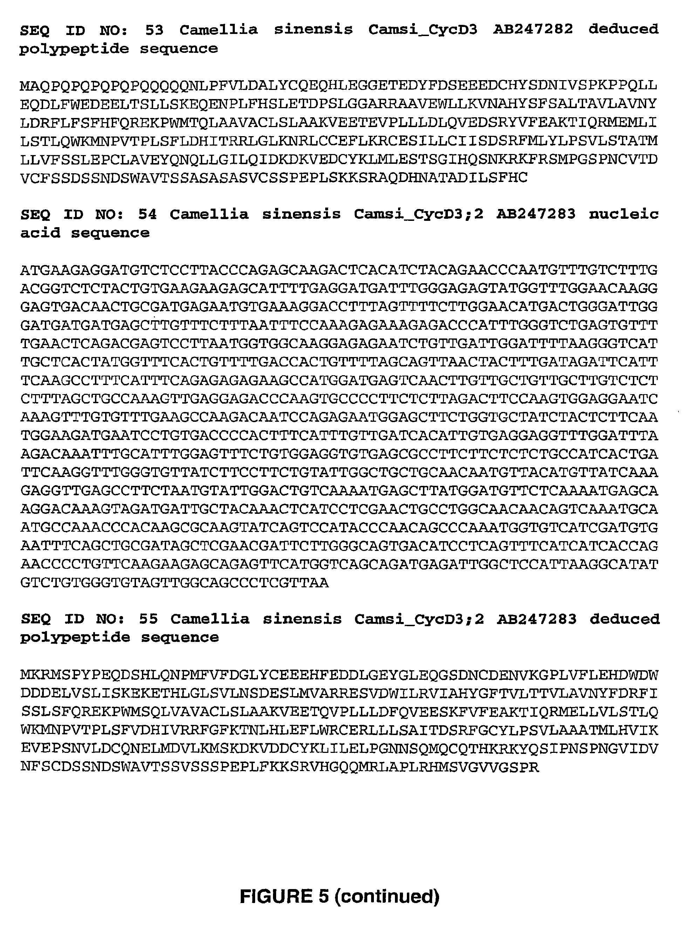

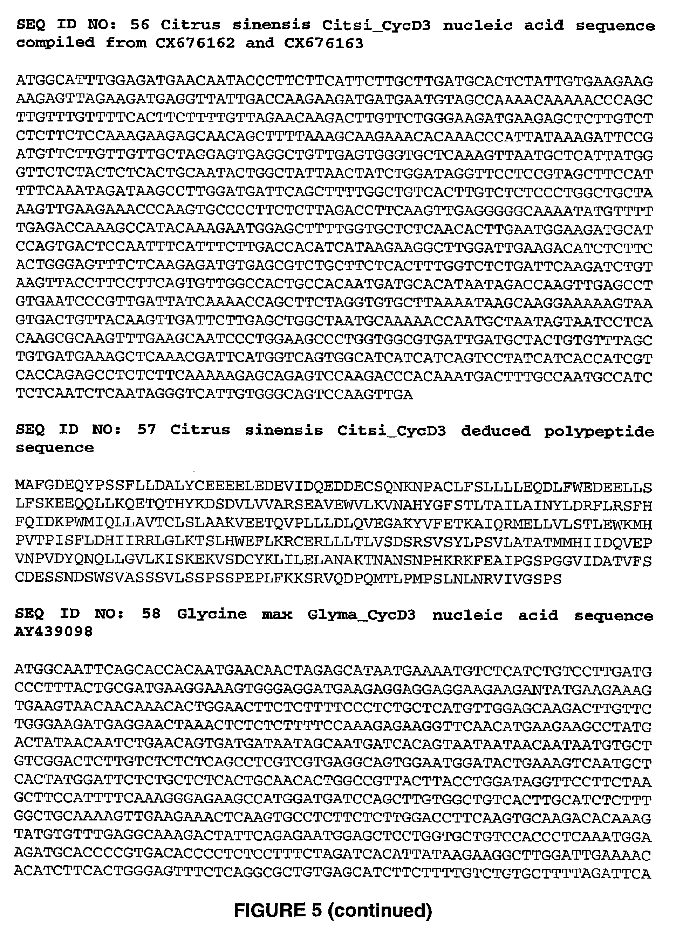

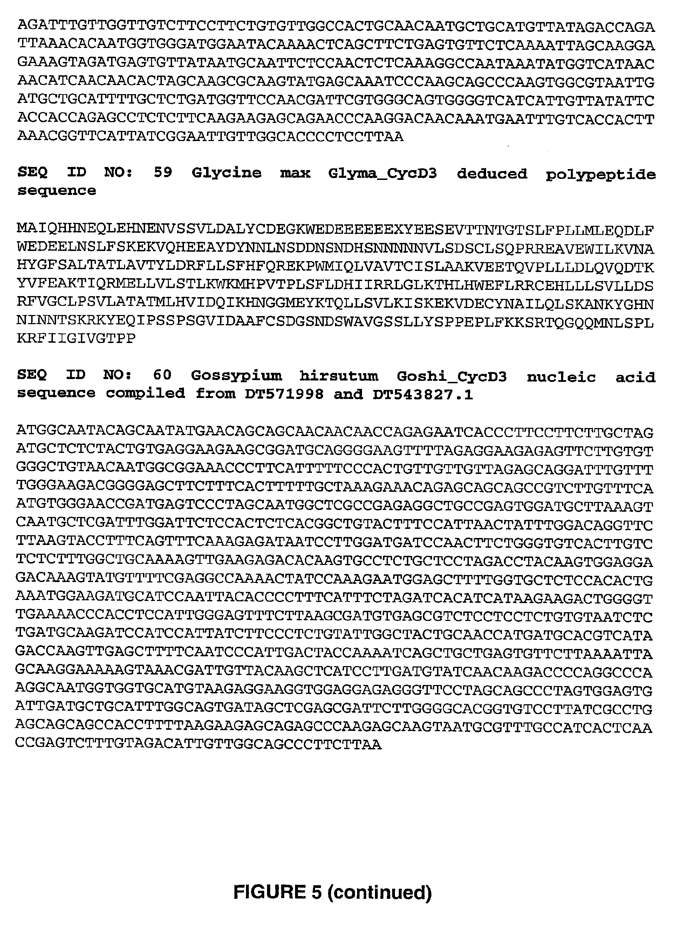

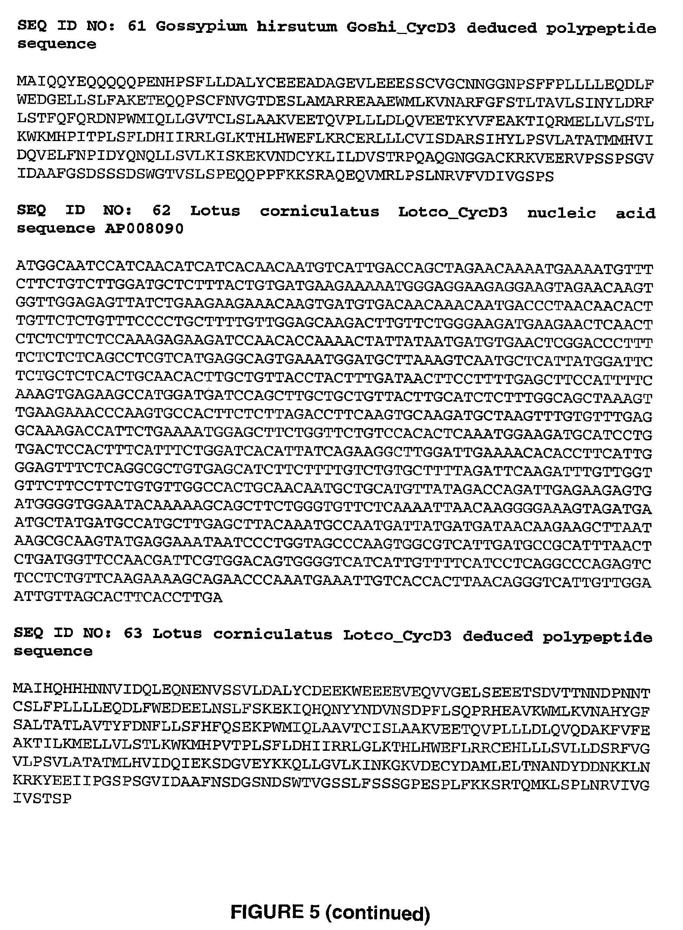

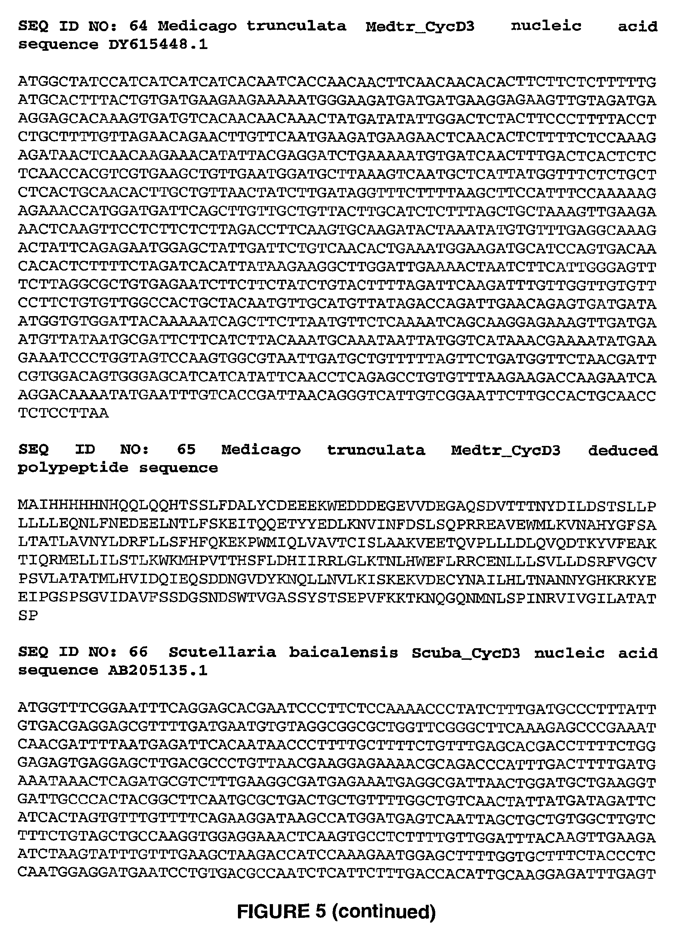

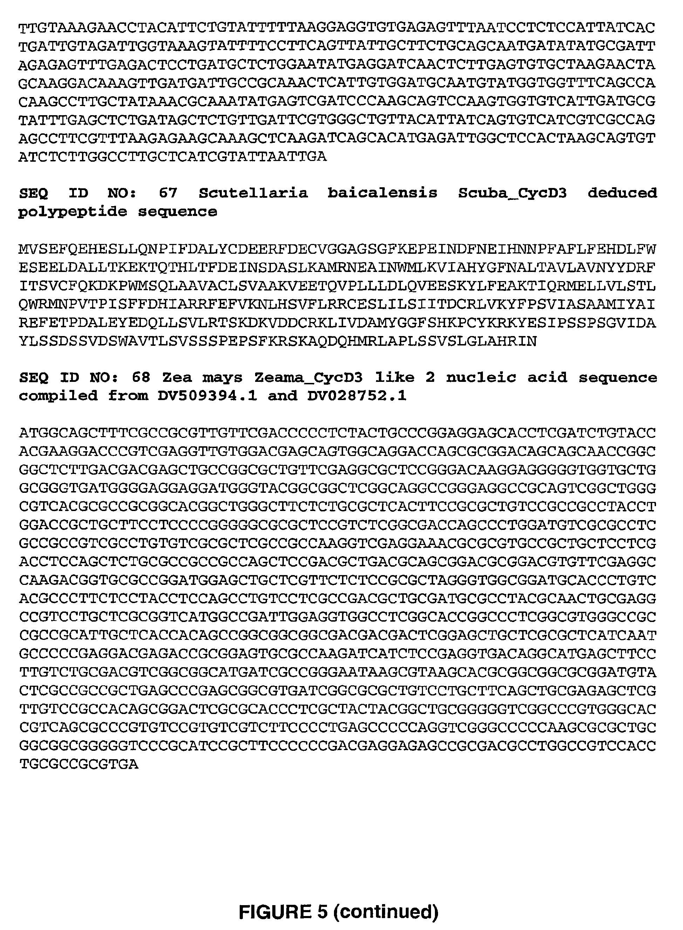

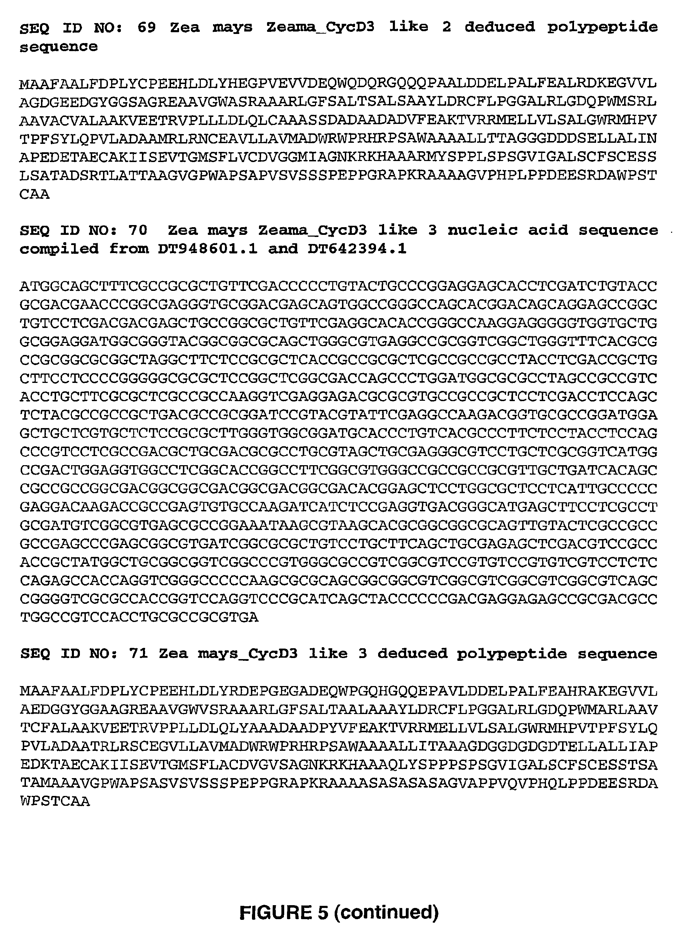

**Fig. 5** details examples of sequences useful in performing the methods according to the present invention.

**Fig. 6** is an alignment only of plant CYCD3 protein sequences. The sequences were aligned using AlignX program from Vector NTI suite (InforMax, Bethesda, MD). Multiple alignment was done with a gap opening penalty of 10 and a gap extension of 0.01. Minor manual editing was also carried out where necessary to better position some conserved regions. In addition to the cyclin box (marked as 'X' below the consensus sequence (Interpro ref: IPR006670)) and the LxCxE motif within the first 40 or so amino acids, a number of motifs specific to CYCD3 polypeptides are identified.

## Examples

The present invention will now be described with reference to the following examples, which are by way of illustration alone.

DNA manipulation: unless otherwise stated, recombinant DNA techniques are performed according to standard protocols described in (Sambrook (2001) Molecular Cloning: a laboratory manual, 3rd Edition Cold Spring Harbor Laboratory Press, CSH, New York) or in Volumes 1 and 2 of Ausubel *et al*. (1994), Current Protocols in Molecular Biology, Current Protocols. Standard materials and methods for plant molecular work are described in Plant Molecular Biology Labfase (1993) by R.D.D. Croy, published by BIOS Scientific Publications Ltd (UK) and Blackwell Scientific Publications (UK).

### *Example 1: Gene Cloning*

The *Arabldopsis* CycD3;3 was amplified by PCR using as template an *Arabldopsis thaliana* seedling cDNA library (Invitrogen, Paisley, UK). After reverse transcription of RNA extracted from seedlings, the cDNAs were cloned into pCMV Sport 6.0. Average insert size of the bank was 1.5 kb and original number of clones was of $1.59 \times 10^7$ cfu. Original titer was determined to be $9.6 \times 10^5$ cfu/ml after first amplification of $6 \times 10^{11}$ cfu/ml. After plasmid extraction, 200 ng of template was used in a 50 μl PCR mix. Primers prm0360 (sense, start codon in bold, AttB1 site in italic: 5' *GGGGACAAGTTTGTACAAAAAAGCAGGCTTCACAA*TGGCTTTAGAAGAGG AGGA 3') and prm0361 (reverse, complementary, stop codon in bold, AttB2 site in italic: 5' *GGGGACCACTTTGTACAAGAAAGCTGGGT*TTAGCGAGGACTACTACTAAGCA 3'), which include the AttB sites for Gateway recombination, were used for PCR amplification. PCR was performed using Hifi Taq DNA polymerase in standard conditions. A PCR fragment of 1086 bp was amplified and purified also using standard methods. The first step of the Gateway procedure, the BP reaction, was then performed, during which the PCR fragment recombines *in vivo* with the pDONR201 plasmid to produce, according to the Gateway terminology, an "entry clone", p0443. Plasmid pDONR201 was purchased from Invitrogen, as part of the Gateway® technology.

For the modified sequence of SEQ ID NO: 48/49, the reverse primer is: 5' *GGGGACCAC77TGTACAAGAAAGCTGGGT*TTAGCGAGGACTACTATAAGCA 3').

### *Example 2: Vector Construction*

The entry clone p0443 was subsequently used in an LR reaction with p0830, a destination vector used for *Oryza sativa* transformation. This vector contains as functional elements within the T-DNA borders: a plant selectable marker; a plant screenable marker, and a Gateway cassette intended for LR *in vivo* recombination with the sequence of interest already cloned in the entry clone. A prolamin promoter for endosperm-specific expression (PRO0090; SEQ ID NO: 3) is located upstream of this Gateway cassette.

After the LR recombination step, the resulting expression vector (see Figure 4) was transformed into *Agrobacterium* strain LBA4404 and subsequently to *Oryza sativa* plants. The resulting expression vector as shown in Figure 4 was transformed into *Agrobacterium* and subsequently into *Oryza sativa* plants. Transformed rice plants were allowed to grow and were then examined for the parameters described in Example 3.

### *Example* 3: *Evaluation and Results*

Approximately 15 to 20 independent T0 rice transformants were generated. The primary transformants were transferred from tissue culture chambers to a greenhouse for growing and harvest of T1 seed. Six events, of which the T1 progeny segregated 3:1 for presence/absence of the transgene, were retained. For each of these events, approximately 10 T1 seedlings containing the transgene (hetero- and homo-zygotes), and approximately 10 T1 seedlings lacking the transgene (nullizygotes), were selected by monitoring visual marker expression. The transgenic plants and the corresponding nullizygotes were grown side-by-side at random positions. From the stage of sowing until the stage of maturity the plants were passed several times through a digital imaging cabinet. At each time point digital images (2048x1536 pixels, 16 million colours) were taken of each plant from at least 6 different angles.

Five T1 events were further evaluated in the T2 generation following the same evaluation procedure as for the T1 generation but with more individuals per event.

**Statistical analysis: t-test and F-test**

A two factor ANOVA (analysis of variants) was used as a statistical model for the overall evaluation of plant phenotypic characteristics. An F-test was carried out on all the parameters measured of all the plants of all the events transformed with the gene of the present invention. The F-test was carried out to check for an effect of the gene over all the transformation events and to verify for an overall effect of the gene, also known as a global gene effect. The threshold for significance for a true global gene effect was set at a 5% probability level for the F-test. A significant F-test value points to a gene effect, meaning that it is not only the presence or position of the gene that is causing the differences in phenotype.

To check for an effect of the genes within an event, i.e., for a line-specific effect, a t-test was performed within each event using data sets from the transgenic plants and the corresponding null plants. "Null plants" or "null segregants" or "nullizygotes" are the plants treated in the same way as the transgenic plant, but from which the transgene has segregated. Null plants can also be described as the homozygous negative transformed plants. The threshold for significance for the t-test is set at 10% probability level. The results for some events can be above or below this threshold. This is based on the hypothesis that a gene might only have an effect in certain positions in the genome, and that the occurrence of this position-dependent effect is not uncommon. This kind of gene effect is also named herein a "line effect of the gene". The p-value is obtained by comparing the t-value to the t-distribution or alternatively, by comparing the F-value to the F-distribution. The p-value then gives the probability that the null hypothesis (i.e., that there is no effect of the transgene) is correct.

**3.1 Seed-related parameter measurements**

The mature primary panicles were harvested, bagged, barcode-labelled and then dried for three days in the oven at 37°C. The panicles were then threshed and all the seeds were collected and counted. The filled husks were separated from the empty ones using an airblowing device. The empty husks were discarded and the remaining fraction was counted again. The filled husks were weighed on an analytical balance. This procedure resulted in the set of seed-related parameters described below.

**3.1.1 Total number of flowers per panicle**

The total number of flowers per panicle as defined in the present invention is the ratio between the total number of seeds and the number of mature primary panicles. The percentage difference between two significant transgenic events and their corresponding nullizygotes in T2 is shown in Table 5. The P value of the significant events in the T2 evaluation is also shown. A significant P value indicates that the presence of the transgene relates to the increase in total number of flowers per panicle.

**Table 5:** Total number of flowers per panicle

| | % increase in T2 | P value per event |
|---|---|---|
| Significant event 1 | 13 | 0.0286 |
| Significant event 2 | 22 | 0.0007 |

**3.1.2 Total seed yield**

The total seed yield was measured by weighing all filled husks harvested from a plant. The percentage difference between three significant transgenic events and their corresponding nullizygotes in T2 is shown in Table 6. The P value of the significant events in the T2 evaluation is also shown. A significant P value indicates that the presence of the transgene relates to the increase in total seed yield.

**Table 6:** Total seed yield

| | % increase in T2 | P value per event |
|---|---|---|
| Significant event 1 | 31 | 0.1306 |
| Significant event 2 | 36 | 0.0826 |
| Significant event 3 | 37 | 0.0005 |

### 3.1.3 TKW

TKW in the present invention is extrapolated from the number of filled seeds counted and their total weight. The percentage difference between three significant transgenic events and their corresponding nullizygotes in T2 is shown in Table 7. The P value of the significant events in the T2 evaluation is also shown. A significant P value indicates that the presence of the transgene relates to the increase in TKW.

**Table 7:** TKW

| | % increase in T2 | P value per event |
|---|---|---|
| Significant event 1 | 6 | 0.0006 |
| Significant event 2 | 5 | 0.0009 |
| Significant event 3 | 4 | 0.0165 |

### 3.1.4 Harvest Index of plants

The harvest index in the present invention is defined as the ratio between the total seed yield and the above ground area ($mm^2$), multiplied by a factor $10^8$. The percentage difference between the three significant transgenic events and their corresponding nuilizygotes in T2 is shown in Table 8. The P value of the significant events in the T2 evaluation is also shown. A significant P value indicates that the presence of the transgene relates to the increase in harvest index.

**Table 8:** Harvest Index

| | % increase in T2 | P value per event |
|---|---|---|
| Significant event 1 | 30 | 0.0324 |
| Significant event 2 | 49 | 0.0014 |
| Significant event 3 | 15 | 0.0727 |

***Example 4: Comparative data pOleosin::cyclin D3;3***

Plants containing the above construct were produced and evaluated using the same procedures as described above for pProlamin::cyclinD3;3. The results of the T1 evaluation are shown in Tables 9 to 11 below. The percentage difference between transgenic plants and corresponding nullizygotes is shown in each of the tables. The p value of the F test is also shown.

**Table 9:** Aboveground Area

| Aboveground area | | |
|---|---|---|
| | % Difference | P value |
| T1 Overall | -12 | 0.0083 |

The p value of the F test was significant indicating that the expression of the transgene driven by this promoter significantly decreases aboveground area.

**Table 10:** Total Seed Weight

| Total Seed Weight | | |
|---|---|---|
| | % difference | P value |
| T1 Overall | -15 | 0.0858 |

The results show that the total weight of the seeds of transgenic plants was lower than the total seed weight of corresponding nulllizygotes.

**Table 11:** Number of Filled Seeds

| Number of Filled Seeds | | |
|---|---|---|
| | % difference | P value |
| T1 Overall | -17 | 0.0572 |

The results show that the number of filled seeds of transgenic plants was lower than the number of filled seeds of corresponding nulllizygotes.

## Claims

1. Method for increasing plant seed yield relative to corresponding wild type plants, comprising increasing expression in a plant of a nucleic acid encoding a cyclin D3 (CYCD3) polypeptide under the control of an endosperm-specific promoter,
wherein said increased expression is effected by

    (i) introducing by transformation a CYCD3-encoding nucleic acid under the control of an endosperm-specific promoter; and
    (ii) selecting for plants having increased seed yield.

2. Method according to claim 1, wherein said nucleic acid is capable of hybridising to a CYCD3-encoding nucleic acid of SEQ ID NO:1 or SEQ ID NO:48.

3. Method according to claim 1, wherein said nucleic acid encodes an orthologue or paralogue of the CYCD3 protein of SEQ ID NO: 2.

4. Method according to any one of claims 1 to 3, wherein said CYCD3-encoding nucleic acid is of plant origin, preferably from a dicotyledonous plant, further preferably from the family Brassicaceae, more preferably the nucleic acid is from *Arabidopsis thaliana.*

5. Method according to any one of 3 claims 1 to 4, wherein said endosperm-specific promoter is a prolamin promoter.

6. Method according to any one of claims 1 to 5, wherein said increased seed yield is selected from: increased number of flowers per panicle, increased total seed yield, increased TKW and increased harvest index.

7. Construct comprising:

    (i) a nucleic acid encoding a CYCD3 polypeptide;
    (ii) one or more endosperm-specific control sequences driving expression of the nucleic acid sequence of (i) in the endosperm of seeds; and optionally
    (iii) a transcription termination sequence.

8. Construct according to claim 7, wherein said control sequence is an endosperm-specific promoter.

9. Construct according to claim 8, wherein said endosperm-specific promoter is a prolamin promoter.

10. Construct according to claim 9, wherein said prolamin promoter is as represented by SEQ ID NO: 3.

11. Method for the production of a transgenic plant having increased seed yield, which method comprises:

(i) transforming a plant or plant cell with a construct according to any of claims 7 to 10; and
(ii) cultivating the plant cell under conditions promoting plant growth and development.

12. Transgenic plant comprising a construct according to any one of claims 7 to 10.

13. Transgenic plant according to claim 12, wherein said plant is a monocotyledonous plant, such as sugarcane or wherein the plant is a cereal, such as rice, maize, wheat, barley, millet, rye, oats or sorghum.

14. Harvestable parts of a plant according to any one of claims 12 or 13, wherein said harvestable parts comprise a construct according to any one of claims 7 to 10.

15. Harvestable parts of a plant according to claim 14, wherein said harvestable parts are seeds, and wherein harvestable parts comprise a construct according to any one of claims 7 to 10.

16. Use of a construct according to any of the claims 7 to 10 in improving seed yied, relative to corresponding wild type plants.

17. Use according to claim 16, wherein said seed yield is selected from: increased number of flowers per panicle, increased total seed yield, increased TKW and increased harvest index.

**Patentansprüche**

1. Verfahren zur Erhöhung des Samenertrags von Pflanzen relativ zu entsprechenden Wildtyp-Pflanzen, bei dem man die Expression einer für ein Polypeptid Cyclin D3 (CYCD3) codierenden Nukleinsäure unter der Kontrolle eines endospermspesifischen Promotors in einer Pflanze erhöht, wobei die erhöhte Expression durch

(i) Einführen einer CYCD3-codierenden Nukleinsäure unter der Kontrolle eines endosperm-spezifischen Promotors durch Transformation und
(ii) Selektionieren auf Pflanzen mit erhöhtem Samenertrag

bewirkt wird.

2. Verfahren nach Anspruch 1, wobei die Nukleinsäure zur Hybridisierung an eine CYCD3-codierende Nukleinsäure der SEQ ID NO: 1 oder SEQ ID NO: 48 fähig ist.

3. Verfahren nach Anspruch 1, wobei die Nukleinsäure für ein Ortholog oder Paralog des CYCD3-Proteins der SEQ ID NO: 2 codiert.

4. Verfahren nach einem der Ansprüche 1 bis 3, wobei die CYCD3-codierende Nukleinsäure pflanzlichen Ursprungs ist und vorzugsweise aus einer zweikeimblättrigen Pflanze, weiter bevorzugt aus der Familie Brassicaceae, stammt, wobei die Nukleinsäure stärker bevorzugt aus *Arabidopsis thaliana* stammt.

5. Verfahren nach einem der Ansprüche 1 bis 4, wobei es sich bei dem endospermspezifischen Promotor um einen Prolaminpromotor handelt.

6. Verfahren nach einem der Ansprüche 1 bis 5, wobei der erhöhte Samenertrag ausgewählt ist aus: erhöhte Anzahl von Blüten pro Rispe, erhöhter Samengesamtertrag, erhöhter TKW-Wert und erhöhter Ernteindex.

7. Konstrukt, umfassend:

(i) eine für ein CYCD3-Polypeptid codierende Nukleinsäure;
(ii) eine oder mehrere endospermspezifische Kontrollsequenzen, die die Expression der Nukleinsäuresequenz aus (i) im Endosperm von Samen treiben; und gegebenenfalls
(iii) eine Transkriptionsterminationssequenz.

**8.** Konstrukt nach Anspruch 7, wobei es sich bei der Kontrollsequenz um einen endospermspezifischen Promotor handelt.

**9.** Konstrukt nach Anspruch 8, wobei es sich bei dem endospermspezifischen Promotor um einen Prolaminpromotor handelt.

**10.** Konstrukt nach Anspruch 9, wobei der Prolaminpromotor wie durch SEQ ID NO: 3 dargestellt ist.

**11.** Verfahren zur Herstellung einer transgenen Pflanze mit erhöhtem Samenertrag, bei dem man

(i) eine Pflanze oder Pflanzenzelle mit einem Konstrukt nach einem der Ansprüche 7 bis 10 transformiert und
(ii) die Pflanzenzelle unter Pflansenwachstum und -entwicklung fördernden Bedingungen kultiviert.

**12.** Transgene Pflanze, umfassend ein Konstrukt nach einem der Ansprüche 7 bis 10.

**13.** Transgene Pflanze nach Anspruch 12, wobei es sich bei der Pflanze um eine einkeimblättrige Pflanze, wie etwa Zuckerrohr, handelt oder wobei es sich bei der Pflanze um ein Getreide, wie etwa Reis, Mais, Weisen, Gerste, Hirse, Roggen, Hafer oder Sorghum handelt.

**14.** Erntbare Teile einer Pflanze nach einem der Ansprüche 12 oder 13, wobei die erntbaren Teile ein Konstrukt nach einem der Ansprüche 7 bis 10 umfassen.

**15.** Erntbare Teile einer Pflanze nach Anspruch 14, wobei es sich bei den erntbaren Teilen um Samen handelt und wobei die erntbaren Teile ein Konstrukt nach einem der Ansprüche 7 bis 10 umfassen.

**16.** Verwendung eines Konstrukts nach einem der Ansprüche 7 bis 10 bei der Verbesserung des Samenertrags relativ zu entsprechenden Wildtyp-Pflanzen.

**17.** Verwendung nach Anspruch 16, wobei der Samenertrag ausgewählt ist aus: erhöhte Anzahl von Blühen pro Risse, erhöhter Samengesamtertrag, erhöhter TKW-Wert und erhöhter Ernteindex.

## Revendications

**1.** Méthode d'augmentation du rendement en graines d'une plante par rapport à des plantes correspondantes de type sauvage, comprenant l'augmentation de l'expression dans une plante d'un acide nucléique codant pour un polypeptide de cycline D3 (CYCD3) sous le contrôle d'un promoteur spécifique de l'endosperme, dans laquelle ladite expression accrue est affectée par

(i) l'introduction par transformation d'un acide nucléique codant pour CYCD3 sous le contrôle d'un promoteur spécifique de l'endosperme ; et
(ii) la sélection des plantes ayant un rendement accru en graines.

**2.** Méthode selon la revendication 1, dans laquelle ledit acide nucléique est capable de s'hybrider à un acide nucléique codant pour CYCD3 de SEQ ID n°1 ou de SEQ ID n°48.

**3.** Méthode selon la revendication 1, dans laquelle ledit acide nucléique code pour un orthologue ou paralogue de la protéine CYCD3 de SEQ ID n°2.

**4.** Méthode selon l'une quelconque des revendications 1 à 3, dans laquelle ledit acide nucléique codant pour CYCD3 est d'origine végétale, préférablement issu d'une dicotylédone, en outre préférablement issu de la famille des Brassicàcées, plus préférablement l'acide nucléique est issu d'*Arabidopsis thaliana.*

5. Méthode selon l'une quelconque des revendications 1 à 4, dans laquelle ledit promoteur spécifique de l'endosperme est un promoteur de prolamine.

6. Méthode selon l'une quelconque des revendications 1 à 5, dans laquelle ledit rendement accru en graines est choisi parmi : un nombre accru de fleurs par panicule, un rendement en graines total accru, un poids de mille grains accru et un indice de moisson accru.

7. Produit de recombinaison, comprenant :

   (i) un acide nucléique codant pour un polypeptide CYCD3 ;
   (ii) une ou plusieurs séquences de contrôle spécifiques de l'endosperme, conduisant l'expression de la séquence d'acide nucléique de (i) dans l'endosperme de graines ; et éventuellement
   (iii) une séquence de terminaison de la transcription.

8. Produit de recombinaison selon la revendication 7, dans lequel ladite séquence de contrôle est un promoteur spécifique de l'endosperme.

9. Produit de recombinaison selon la revendication 8, dans lequel ledit promoteur spécifique de l'endosperme est un promoteur de prolamine.

10. Produit de recombinaison selon la revendication 9, dans lequel ledit promoteur de prolamine est tel que représenté par SEQ ID n°3.

11. Méthode de production d'une plante transgénique ayant un rendement accru en graines, laquelle méthode comprend :

   (i) la transformation d'une plante ou d'une cellule végétale par un produit de recombinaison selon l'une quelconque des revendications 7 à 10 ; et
   (ii) la culture de la cellule végétale dans des conditions favorisant la croissante et le développement de la plante.

12. Plante transgénique comprenant un produit de recombinaison selon l'une quelconque des revendications 7 à 10.

13. Plante transgénique selon la revendication 12, dans laquelle ladite plante est une monocotylédone, telle que la canne à sucre ou dans laquelle la plante est une céréale, telle que le riz, le maïs, le blé, l'orge, le millet, le seigle, l'avoine ou le sorgho.

14. Parties récoltables d'une plante selon l'une quelconque des revendications 12 à 13, dans lesquelles lesdites parties récoltables comprennent un produit de recombinaison selon l'une quelconque des revendications 7 à 10.

15. Parties récoltables d'une plante selon la revendication 14, dans lesquelles lesdites partiels récoltables sont des graines, et dans lesquelles lesdites parties récoltables comprennent un produit de recombinaison selon l'une quelconque des revendications 7 à 10.

16. Utilisation d'un produit de recombinaison selon l'une quelconque des revendications 7 à 10, dans l'amélioration du rendement en graines par rapport à des plantes correspondantes de type saurage.

17. Utilisation selon la revendication 16, dans laquelle ledit rendement en graines est choisi parmi : un nombre accru de fleurs par panicule, un rendement en graines total accru, un poids de mille grains accru et un indice de moisson accru.

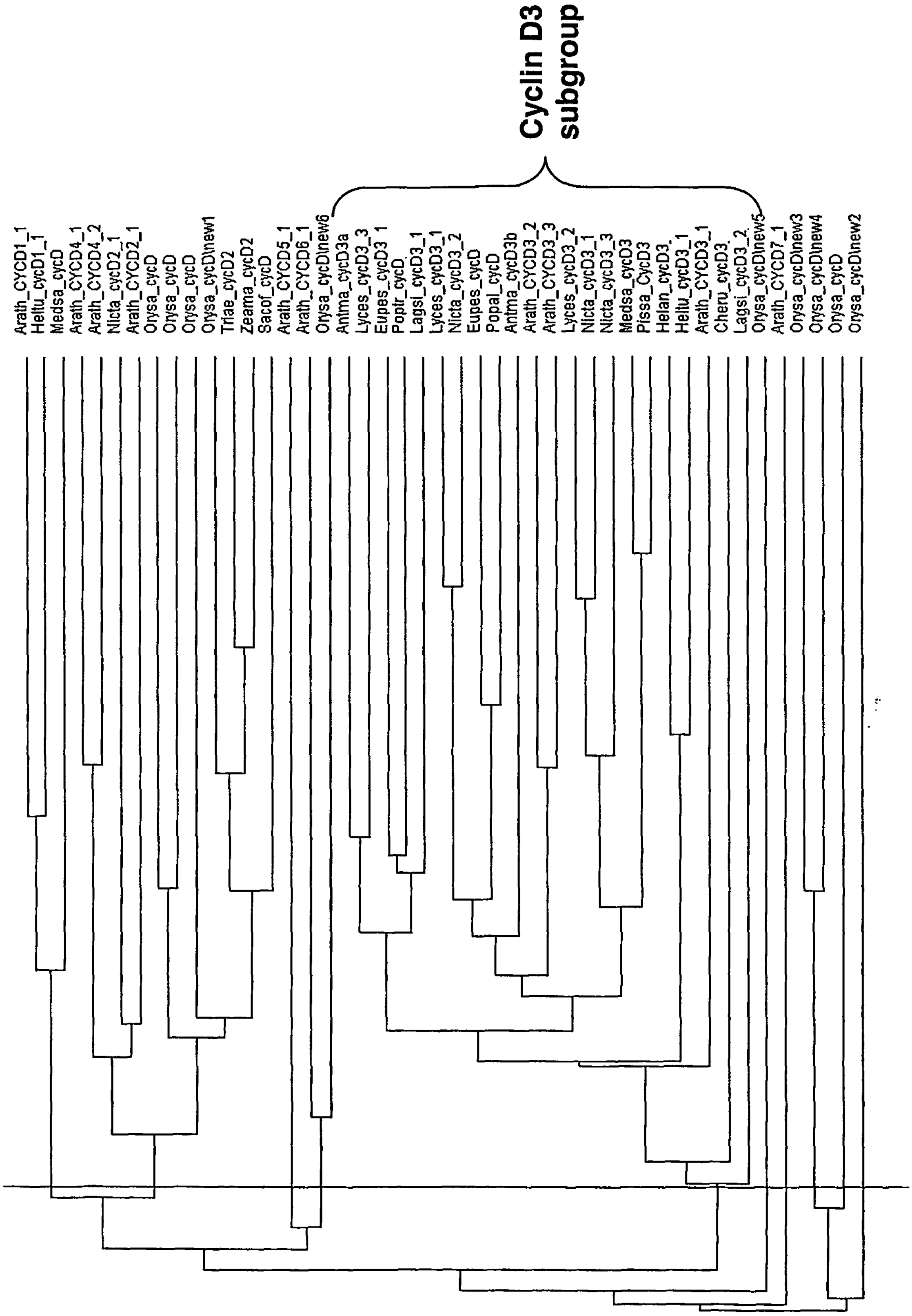
Cyclin D3
subgroup
Arath_CYCD1_1
Heltu_cycD1_1
Medsa_cycD
Arath_CYCD4_1
Arath_CYCD4_2
Nicta_cycD2_1
Arath_CYCD2_1
Orysa_cycD
Orysa_cycD
Orysa_cycD
Orysa_cycDlnew1
Triae_cycD2
Zeama_cycD2
Sacof_cycD
Arath_CYCD5_1
Arath_CYCD6_1
Orysa_cycDlnew6
Antma_cycD3a
Lyces_cycD3_3
Eupes_cycD3 1
Poptr_cycD
Lagsi_cycD3_1
Lyces_cycD3_1
Nicta_cycD3_2
Eupes_cycD
Popal_cycD
Antma_cycD3b
Arath_CYCD3_2
Arath_CYCD3_3
Lyces_cycD3_2
Nicta_cycD3_1
Nicta_cycD3_3
Medsa_CycD3
Pissa_CycD3
Helan_cycD3
Heltu_cycD3_1
Arath_CYCD3_1
Cheru_cycD3
Lagsi_cycD3_2
Orysa_cycDlnew5
Arath_CYCD7_1
Orysa_cycDlnew3
Orysa_cycDlnew4
Orysa_cycD
Orysa_cycDlnew2

**FIGURE 1**

```
                          1                                                                                                   100
Antma_cycD3a          (1) ------MYQQNS---------PSLCFDALYCEEE--QNWDNGEIINDCFI--------EEQEPFSDLLKHDLLCGVD-------DDDDKEELSSLLCKE
Antma_cycD3b          (1) ----MLFSHSQQT---HLQNPI---FDALLCNEE--HFDEDLD--LGSGL--------KDPGFINQIHHNQKKEEPFTTFLFEHDILWEDDELVNLLSKE
Arath_CYCD3_1         (1) -----MAIRKEEESREEQSNS--FLLDALYCEEE-----KWDDEGE-EVE--------E-----NSSLSSSSS----PFVVLQQDLFWEDEDLVTLFSKE
Arath_CYCD3_2         (1) -----MALEKEEE---ASQNGAFCVLDGLYCEEE--TGFVED------DL--------DDDGDLDFLEKSDESVVKFQFLPLLDMFLWDDDEILSLISKE
Arath_CYCD3_3         (1) -----MALE-EEE---ESQNAPFCVLDGLFCEEE--S--------------------EFHEQVDLCDESVEKFPFLNLGLSDHDMLWDDDELSTLISKQ
Eupes_cycD3 like      (1) ------------M---EDSTQISLIFDGLYCEEQ-------GIGEDFDDG--------NEDY--------VKKELSLSSVLLEQDLFWTDDELLNLISKE
Heltu_cycD3;1         (1) -----MAILSPYSSSF---------LDTLFCNEQ-----QDHEYHEYEYE--------DEFTQTTLTDSSDLH----LPPLDQLDLSWEHEELVSLFTKE
Medsa_cycD3           (1) -----MAIHHHHHNHQQLQQHTSSLFDALYCDEE-EKWEDDDEGEVVDEG--------AQSDVTTTNYDILDSTSLLPLLLLEQNLFNEDEELNTLFSKE
Nicta_cycD3;1         (1) -----MAIEHNEQ----QELSQSFLLDALYCEEEEEKWGDLVDDETIITP--------LSSEVTTTTTTTKPNSLLPLLLLEQDLFWEDEELLSLFSKE
Nicta_cycD3;3         (1) -----MGIQHNEH---NQDQTQSFLLDALYCEEE--RWEETIEDEILEKE--------ATLPL------------PLP--LLEQDLFWEDEELLSLFTKE
Pissa_CycD3           (1) -----MAIHHHHHHHQQLHHN--SLLDALYCDEE-KLEE---EQEDVSSQ--------QSDVTTNNDNNILDSTSLFPLLLLEQNLFSQDEELTTLFSKE
Popal_cycD3           (1) -----MSFLQQQE---THNQSPALALDGLYCEED-------GFGEDYSCG--------LDDETSQVYDQNVKKEQNLSSVLLEQDLFWEDSELLSLISKE
Cheru_cycD3           (1) -----MTTLETEQEQQPFSQNSPLFLDCLYCEEKYWDYDYHDEDDFGSLN------------------SSK----LHDCSLICCEDDEEIQLNALVSKE
Eupes_cycD3;1         (1) ----------------MANHSPLFLYDALYCSEE--DNWEGEVVDIFHEQ--------EDQGENTSVFPQNSS---------PVDLNWEEDELTSVFSKQ
Helan_cycD3           (1) -----MAILSRYSSS-----------NTLFCIEE-----QVHEDED-------------------ELTHQDSSA----IHPLDLQDLCWEHEELVSLFTKE
Lagsi_cycD3;1         (1) -----------------MVPPYALDSLYCSED---HWENDDEEEERGF--------HEQPYSNLTTESSSP----ILAVAEQDLFWENDELISLFSRE
Lagsi_cycD3;2         (1) ---MKKMALHSNKHRTQRLHNSLFFFDFLHCTEQQHLQTEHPIFLN------------NGGTNDFPLFQQTTT----------HFLVYEDEELNHLLSKE
Lyces_cycD3;1         (1) ------MVFPLDS---QLQNPISALLDGLYCEED--RFLDDD---LGEWS--------S-----LDVGNENVKK-----TLPLLECDMFWEHDELATLLSKE
Lyces_cycD3;2         (1) -----MAIENN---------DQSFFLEVLYCEEEEEKWGDLLEDEEGVII--------NPLLLSSEGTTKTNSLLLLPLLLLEQDLFWEDEELLSLFVKE
Lyces_cycD3;3         (1) ---MSHHYQEQEQ---LEAQKIPFLLDSLYCEEE------NN------ILT--------EVSIETESFSSHDLLWEE--------------EELTSLFSKE
Nicta_cycD3;2         (1) ------MVFPLDT---QLLNPI---FDVLYCEED--RFLDDDD--LGEWS--------ST---LEQVGNNVKK----TLPLLECDMFWEDDQLVTLLTKE
Orysa_cycD3           (1) -----------------MAFATLFDSLYCPEEHLDLFHDTAADDDLHLDLHLHQ-------------PPP-----------PPPLLDDDLPALFHAL
Poptr_cycD3           (1) MASMYNPETSAVQ---DQQQNPTLLYDALYCSEE---NWEEVREDWFQD--------ELEGE--SYCSNNSNKLNTEPLLLLEQDLSWEDEELLSLFAKE
Arath_CYCD1_1         (1) ------MRSYRFSDYLHMSVSFSN-DMDLFCGEDSGVFSGESTVDFS---------------------------------SSEVDSWPGDSIACFIED
Arath_CYCD2_1         (1) -----------------------MAENLACGETSESWIIDNDDDDINYG---GGFTNEIDYNHQLFAKDDNFGGNGSIPMMGSSSSSLSEDRIKEMLVR
Arath_CYCD4_1         (1) ----------------------MAEENLELSLLCTESNVDDEG-------------MIVDETPIEISIPQMGFSQSE-----------SEEIIMEMVEK
Arath_CYCD4_2         (1) --------------------------MAEFMEPNLVSNFDDE----------------KSNSVDTRSIFQMGFPLE------------SEEIVREMIEK
Arath_CYCD5_1         (1) ------------------MGEPKDSLALFLCHESESSLNEDDDETIERSDK-------------------QEP---------HFTTTIDDEDYVADLVLK
Arath_CYCD6_1         (1) ---------------MEFHLEHPLSHSSLHNNFN-----------------------------------------------DDTDYETLPHSLFL
Arath_CYCD7_1         (1) ------------------------MDNLLCEESWPASPLTPEPLPN-------------FRHRSHDNDVVKMYP--------EIDAATMEEAIAMDLEK
Heltu_cycD1;1         (1) -----------------MSISCSDCFSDLLCCDDSGILSGDDRPECSY---------------------------------DFEYSGDFDDSIAEFIEQ
Nicta_cycD2;1         (1) ---------------MAADNIYDFVASNLLCTETKSLCFDDVDS-------------LTISQQNIETKSKDLSFNNGIRSEPLIDLPSLSEECLSFMVQR
Orysa_cycD5 like 3    (1) -----------MGDASASTSAPATPTSTLICREDGNDLFSADPADDDGGG------------------GSGGDWELSIADDDHVLLMDRDDEYLAEMLSK
Triae_cycD2           (1) ----------------MVPSGYDCAASVLLCAEDNAAILGLDDDDEEDCSW-----AAAATP---PRIAADAAAAAEGFL---VDHPVQSDECVAALVET
Zeama_cycD2           (1) ----------------MVPGYDCAASVLLCAEDNAAILGLDDDGEESSW-----AAAATPPRDTVAAAAATGVAVDGIL---TEFPLLSDDCVATLVEK
Medsa_cycD1           (1) -----------MIEEEQLPAILMPSDCELLCGEDSSEVLTGDLPECSSDL--------DSSSSS---QLPS-----------SSLFAEEEEDSIAVFIEH
Orysa_cycD2/4 like 4  (1) ---------------MAALTSYEMAASIDLLCAEDSSSVLGFGGEEEEEE--------DVVAGKRARCAGPPPPCVDVAG---VDFAVPSEECVARLVET
Orysa_cycD2/4 like 1  (1) -------------MAPSSSSCHDAAASMLLCAEDNSSILWLEDEEGEVGERRSGGCRSMVGDLAAGGGGGSGGGGVEEEE---DMFPRQSEECVASLVER
Orysa_cycD2/4 like 2  (1) -----------------MGVLCFGASNILLCAEDSSSVLGLGGFGGGGGE--------VAAELGCGGGGGFDFFGFGG----GAVFPIDSDEFVAALVEK
Orysa_cycD2/4 like 3  (1) -----------------MAPSFDFAASIDLLCAEDNTAILDLGEESEE---------ISWVVG----------VDASLGDLS---MDFPLQSDDCIEALLGR
Orysa_cycD1           (1) -----MGAPATAASGGGDDDDRDVVFEYLLCTEDDAASAGSFFQQLQGPAPAVS-----SSPSTTTATAPAAAGSCDDGGEEEEEEVWTVDVVAELIGGE
Orysa_cycD5 like 1    (1) ------------MSMEEAEECSAACGFSLTCQEDGADLGDGVVDDDDDDGDVF------LFYNAVAAADDEEEEE-----EYVEQMVSKEASFCCSSS---
Orysa_cycD5 like 2    (1) -------------MEAEDEYSAGCSFSEMCQEDSTDLDDDGGGGGCFAGDGRADLL-LVYNAAAAADDEDEEEVE---EYMDHLVSKESSFCSSSSSTS
Orysa_cycD6           (1) -------------------MDMATGAKEVVVVEAYEYEFDLEN-----------------------------------------PFTSPADEPIASLLDA
Sacof_cycD2/4 like    (1) -----------------MTPRFDFAASVLLCSEDSTTIFDLEEEEREG-------ILCVLRP----SSRH-ASAPSGALS---IDFPLQSDSCIEAFLGR
Consensus             (1)                           D L C ED        E E                                  DL  E EELVSLL
```

FIGURE 2

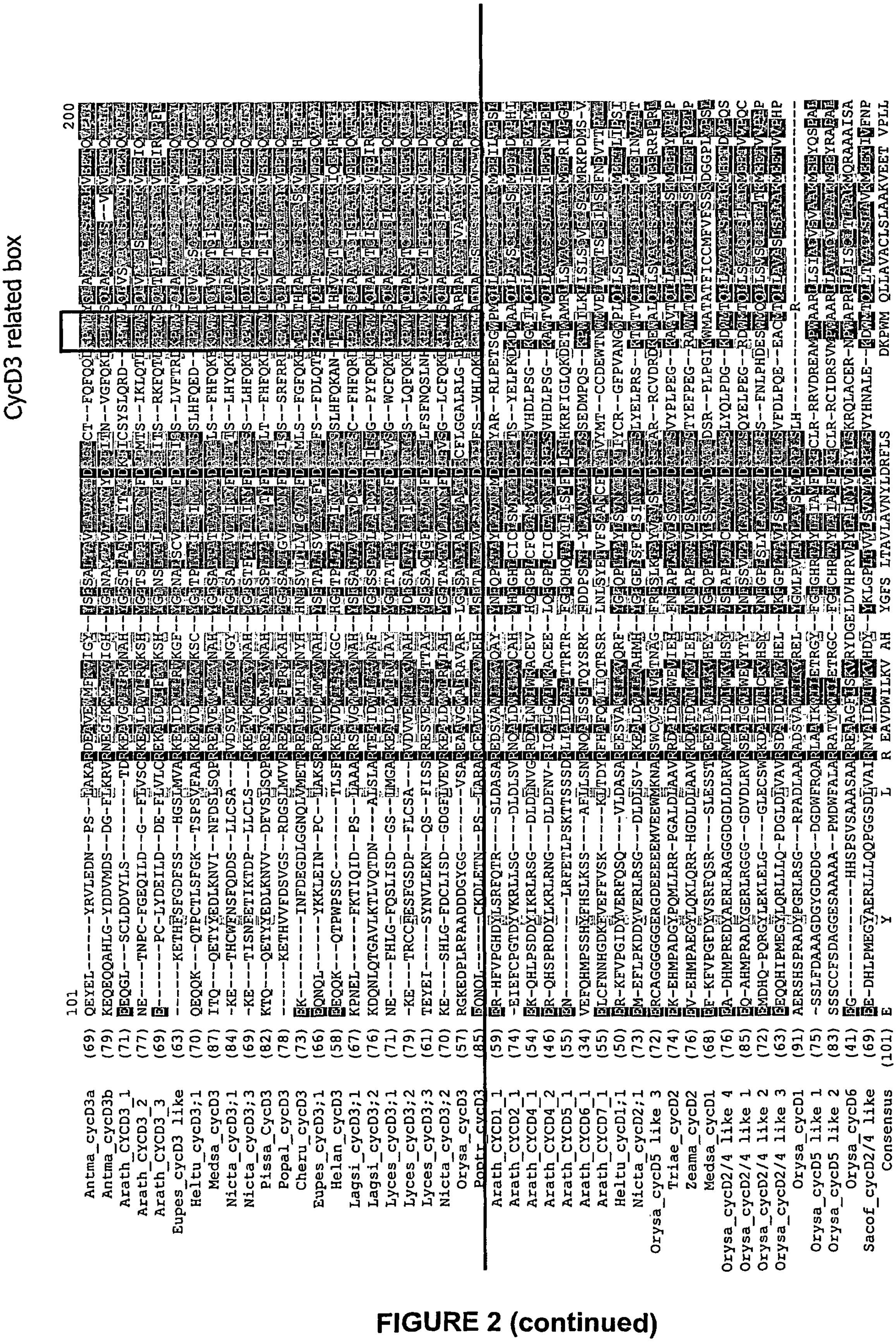


FIGURE 2 (continued)

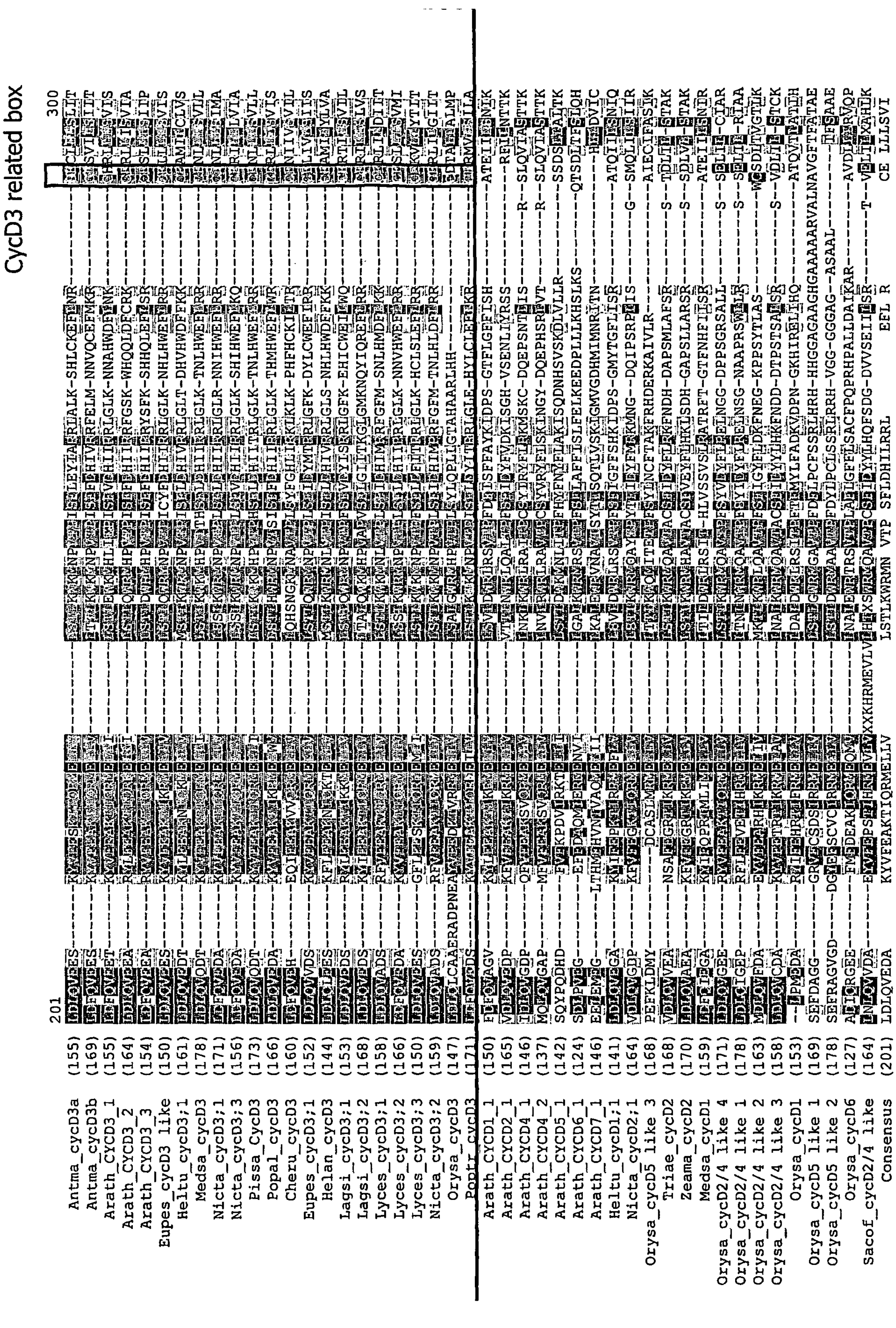


FIGURE 2 (continued)

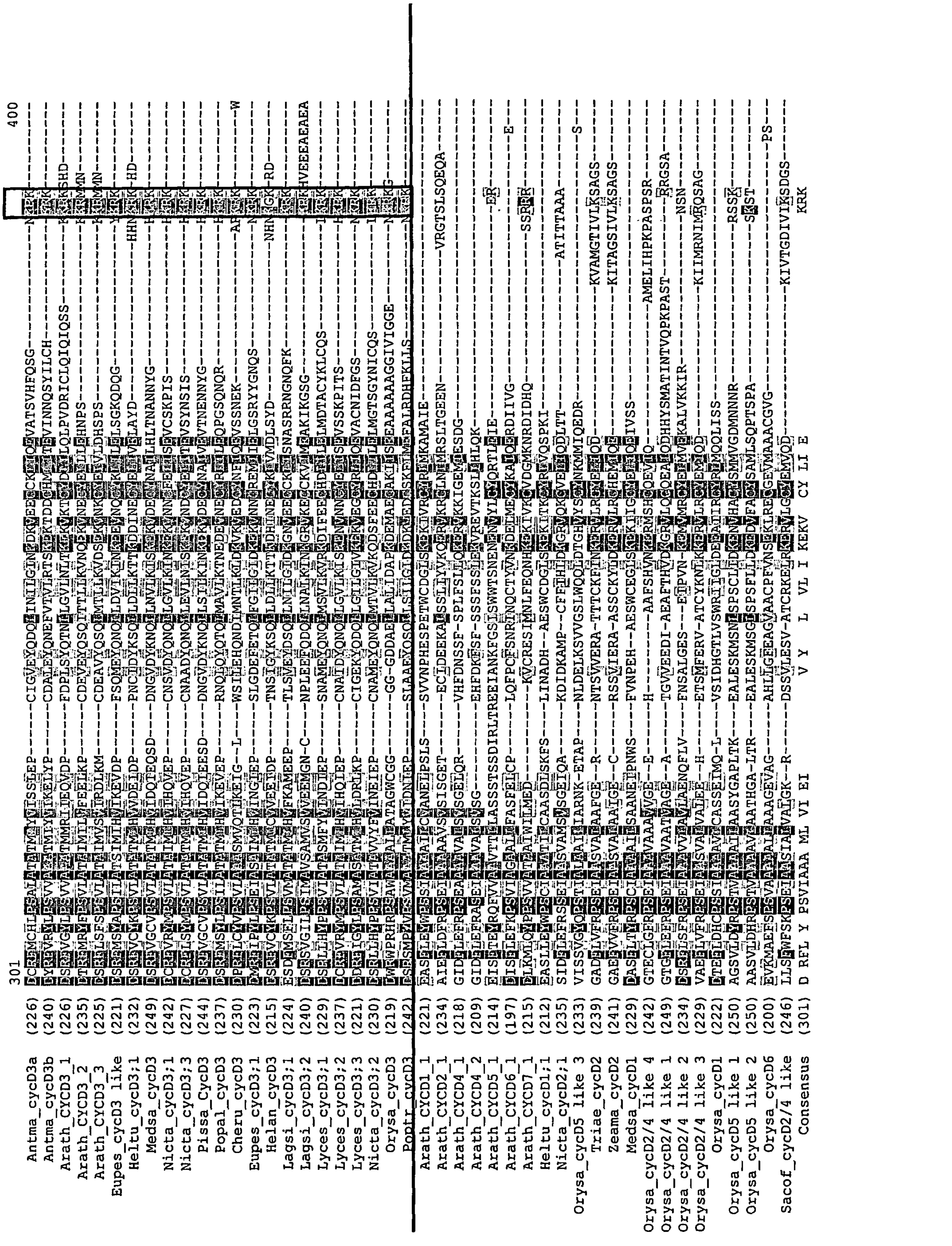


FIGURE 2 (continued)

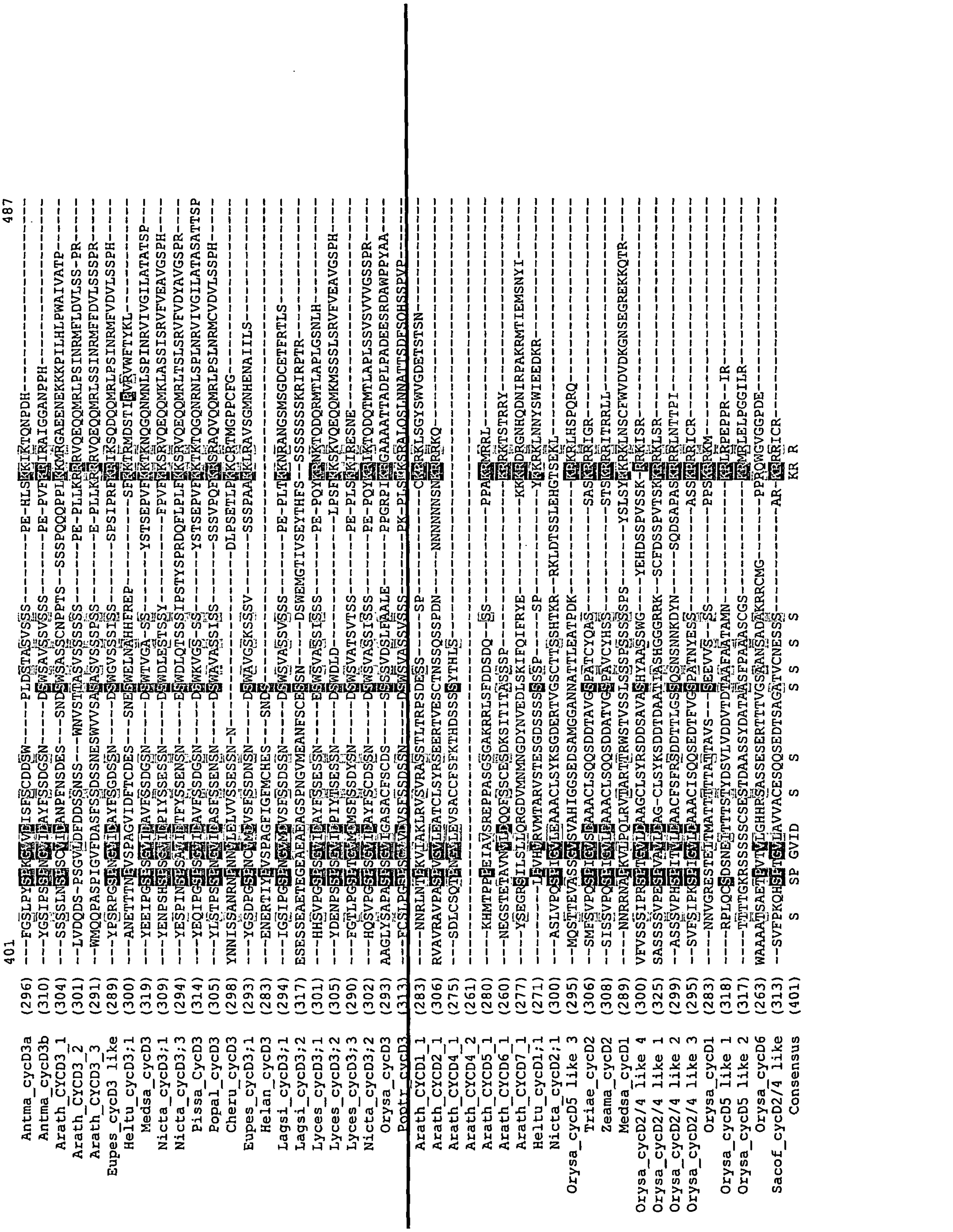

FIGURE 2 (continued)

| | IDENTITY | | | | | | | | | | | | | | | | | | | | | |
|---|---|---|---|---|---|---|---|---|---|---|---|---|---|---|---|---|---|---|---|---|---|---|
| blosum60 | 1 | 2 | 3 | 4 | 5 | 6 | 7 | 8 | **9** | 10 | 11 | 12 | 13 | 14 | 15 | 16 | 17 | 18 | 19 | 20 | 21 | 22 |
| 1. Antma_cycD3a | | 50.5 | 42.5 | 42.9 | **46** | 49 | 44.7 | 47.6 | 49.2 | 46.7 | 46.9 | 48.9 | 39.3 | 52.1 | 40.6 | 53.2 | 40.3 | 48.6 | 49.5 | 57.7 | 46.4 | 56.2 |
| 2. Antma_cycD3b | 68.4 | | 43 | 44.3 | **45.7** | 51.6 | 41.1 | 46.9 | 48.6 | 46.4 | 46.5 | 52.5 | 38.9 | 43.7 | 36.7 | 45.4 | 38.7 | 55.2 | 47.3 | 44.7 | 55.5 | 49.1 |
| 3. Arath_CYCD3_1 | 59.8 | 62.2 | | 46.5 | **44.2** | 47.6 | 44.1 | 50.5 | 51.3 | 52.2 | 48.5 | 47.7 | 35.1 | 42.7 | 40.2 | 41.9 | 37.9 | 46.4 | 50 | 43.7 | 44.6 | 46.3 |
| 4. Arath_CYCD3_2 | 59.1 | 65.4 | 65.7 | | **67.9** | 54.4 | 44.1 | 44.1 | 49.7 | 50.7 | 43.3 | 54.6 | 36.4 | 41.6 | 38.9 | 42.9 | 33.9 | 50 | 48.3 | 40.6 | 51.6 | 43.2 |
| 5. Arath_CYCD3_3 | 63.2 | 67 | 63 | 80.1 | | **52.9** | **41.8** | **43.6** | **48.4** | **51.5** | **43.3** | **52** | **36.3** | **44.7** | **36** | **45.7** | **36** | **46.4** | **47** | **43** | **47.6** | **43.9** |
| 6. Eupes_cycD | 67.6 | 69 | 64.4 | 72.5 | 72 | | 48.5 | 51.2 | 55.2 | 55.8 | 49.4 | 70.1 | 38.1 | 47.1 | 42.5 | 49.3 | 39.7 | 55.6 | 55.1 | 45 | 56 | 48.2 |
| 7. Heltu_cycD3;1 | 62.7 | 61.2 | 65.2 | 65.7 | 62.3 | 70.6 | | 48.6 | 51.2 | 53.1 | 46.8 | 46.6 | 33.8 | 42.2 | 67.4 | 45.8 | 41 | 44.7 | 52.3 | 41.6 | 43.7 | 45.9 |
| 8. Medsa_cycD3 | 64.8 | 64 | 67.4 | 63.5 | 63.7 | 68.1 | 66.3 | | 60.7 | 56.1 | 85.4 | 53.4 | 38.2 | 50 | 42.1 | 49.6 | 40.6 | 48.5 | 57.8 | 47.1 | 47.7 | 51.3 |
| 9. Nicta_cycD3;1 | 64.1 | 68.1 | 69.7 | 70 | 67.6 | 72.7 | 68.1 | 72.5 | | 69.6 | 57.3 | 57.7 | 38.9 | 48.1 | 43.1 | 47.1 | 40.8 | 50.3 | 79.5 | 47.4 | 50 | 51.7 |
| 10. Nicta_cycD3;3 | 64.7 | 67.4 | 71.8 | 71.2 | 69.8 | 74.7 | 71.2 | 71.8 | 84.2 | | 55.7 | 57.5 | 38.8 | 47.3 | 43.4 | 49.3 | 39.1 | 49.6 | 68.4 | 47.3 | 51.2 | 51.4 |
| 11. Pissa_CycD3 | 61.7 | 62.8 | 67.4 | 61.2 | 61.7 | 66.9 | 64.3 | 91.7 | 72.7 | 72.1 | | 53.1 | 36 | 49 | 39.7 | 48.8 | 40.9 | 46.9 | 54.8 | 47.8 | 48.2 | 51.3 |
| 12. Popal_cycD | 65 | 70.9 | 66.5 | 71.7 | 72.2 | 80.1 | 64.4 | 67.1 | 73.2 | 73.9 | 66.9 | | 39.9 | 49.7 | 38.7 | 52.5 | 38.8 | 56.1 | 55.9 | 45.5 | 56.4 | 50.4 |
| 13. Cheru_cycD3 | 60.6 | 60.1 | 56.9 | 55 | 59 | 58.9 | 55.7 | 54.4 | 58.4 | 55.4 | 55.5 | 59.6 | | 37.2 | 33.7 | 36.7 | 32 | 38.1 | 39.6 | 37.2 | 36.2 | 36.9 |
| 14. Eupes_cycD3;1 | 72.6 | 63.4 | 63.8 | 63.5 | 63.7 | 66.5 | 65.5 | 64.8 | 66.2 | 67.1 | 64.6 | 64.7 | 59.1 | | 38.5 | 56.9 | 38.3 | 46.8 | 48.6 | 49.7 | 45.1 | 56.9 |
| 15. Helan_cycD3 | 58.6 | 55.7 | 57.4 | 55.9 | 53.2 | 62.3 | 75.6 | 59.3 | 60.3 | 61.4 | 58.6 | 56.9 | 54.8 | 58.9 | | 40.5 | 36.9 | 39.4 | 45.4 | 39.4 | 39.3 | 41.6 |
| 16. Lagsi_cycD3;1 | 69.9 | 64.3 | 61.7 | 61.9 | 62.6 | 66.2 | 66.1 | 64 | 65.1 | 66.6 | 63.5 | 65.5 | 56 | 74.1 | 59.4 | | 40.6 | 49.7 | 49.2 | 48 | 48.1 | 53.6 |
| 17. Lagsi_cycD3;2 | 60 | 57.4 | 59.2 | 54.5 | 56.8 | 59.7 | 60.3 | 60.9 | 63.4 | 58.9 | 60.7 | 58.7 | 51.3 | 60.3 | 53.4 | 57.9 | | 37.5 | 41.8 | 39.9 | 38.2 | 39.9 |
| 18. Lyces_cycD3;1 | 66.6 | 72.3 | 65.2 | 70 | 67.9 | 73.3 | 65.5 | 67.1 | 67.8 | 70.4 | 65.9 | 72.8 | 55.7 | 65.5 | 56.8 | 68 | 60.3 | | 49.5 | 44.7 | 81.9 | 50.4 |
| 19. Lyces_cycD3;2 | 64.6 | 65.9 | 66 | 69.8 | 67.3 | 72.5 | 70.1 | 69.9 | 87.4 | 81 | 68.5 | 70.6 | 58.5 | 67 | 61.8 | 67 | 61.3 | 69 | | 45.3 | 50.7 | 50.3 |
| 20. Lyces_cycD3;3 | 71.1 | 63.2 | 60.9 | 58 | 60.9 | 63.4 | 58.5 | 61.7 | 62.7 | 63 | 62.5 | 61.7 | 54.2 | 68 | 56 | 65.6 | 57.6 | 64.6 | 59.9 | | 42.9 | 52.8 |
| 21. Nicta_cycD3;2 | 63.5 | 73 | 65.7 | 71.9 | 68.4 | 72.8 | 65.4 | 66.6 | 69.2 | 72.8 | 66.9 | 72.2 | 54.2 | 64.3 | 56.1 | 65.1 | 60.3 | 88.6 | 69.2 | 62.4 | | 49.7 |
| 22. Poptr_cycD | 69.1 | 67.6 | 68.6 | 64.1 | 62.5 | 66.8 | 64.4 | 69.7 | 70.7 | 70.7 | 69.3 | 68.4 | 56.4 | 75 | 58.2 | 71.3 | 63.4 | 67.6 | 67.6 | 67.6 | 67.6 | |
| | SIMILARITY | | | | | | | | | | | | | | | | | | | | | |

FIGURE 3

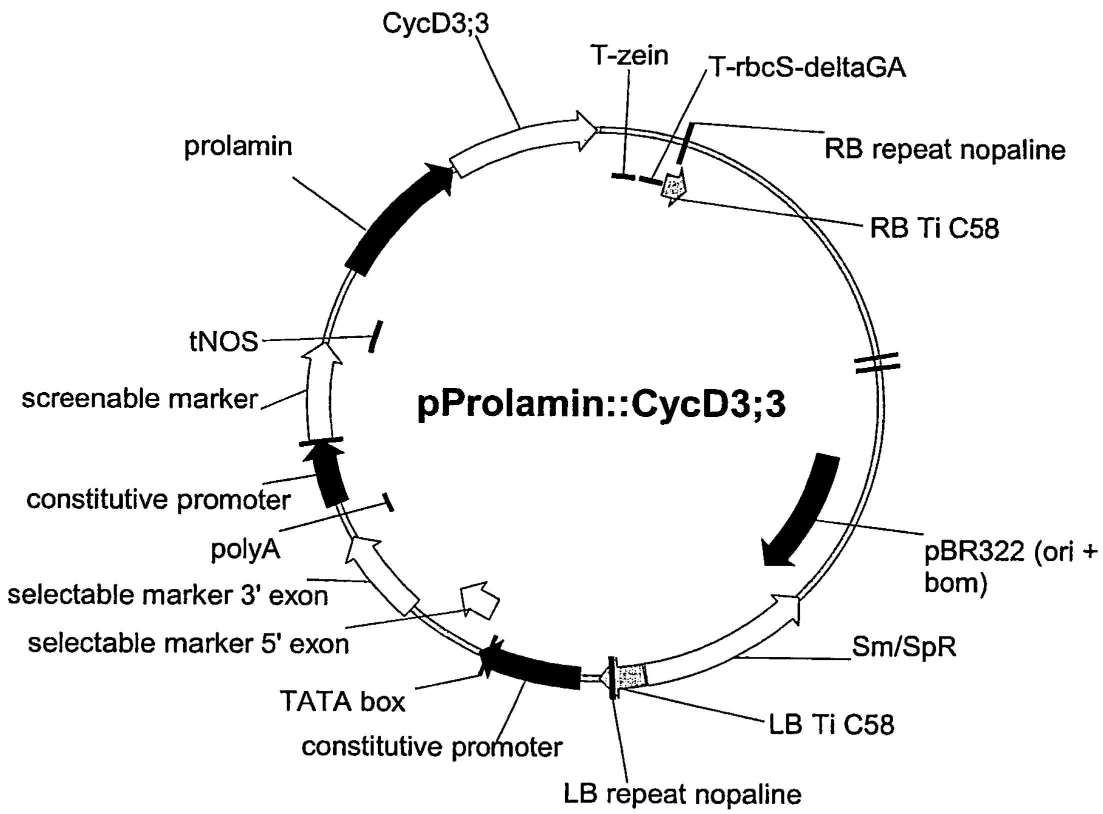
CycD3;3
T-zein
T-rbcS-deltaGA
RB repeat nopaline
prolamin
RB Ti C58
tNOS
screenable marker
pProlamin::CycD3;3
constitutive promoter
polyA
pBR322 (ori + bom)
selectable marker 3' exon
selectable marker 5' exon
Sm/SpR
TATA box
LB Ti C58
constitutive promoter
LB repeat nopaline

FIGURE 4

**SEQ ID NO 1: cyclin D3;3 cDNA**

ATGGCTTTAGAAGAGGAGGAAGAGAGTCAAAACGCACCGTTTTGTGTTCTTGATGGTCTTTTCT
GTGAGGAAGAGAGTGAGTTTCACGAACAAGTAGATTTGTGCGACGAGAGTGTTGAAAAGTTTCC
TTTTTTAAATCTGGGTTTGTCTGATCATGATATGTTGTGGGATGATGATGAGTTATCAACTTTG
ATTTCGAAACAAGAACCGTGTCTTTATGACGAAATCTTAGATGATGAGTTTCTGGTTTTGTGTC
GTGAAAAGGCTCTTGATTGGATTTTTAAAGTGAAATCTCATTATGGGTTTAATTCATTGACGGC
TCTTTTAGCTGTTAATTACTTCGATAGGTTTATTACAAGCAGGAAGTTTCAGACAGATAAGCCA
TGGATGTCTCAGCTTACTGCTTTGGCTTGTCTGTCTTTAGCTGCTAAGGTTGAAGAGATCCGTG
TTCCTTTTCTCTTAGATTTTCAAGTGGAAGAAGCAAGATATGTCTTTGAAGCTAAGACTATACA
GAGAATGGAGCTTCTTGTTCTGTCTACTCTTGACTGGAGGATGCATCCTGTGACTCCAATCTCG
TTTTTCGATCACATTATTCGACGATACAGCTTTAAATCTCATCATCAATTGGAGTTCTTGAGTA
GATGTGAATCTTTATTACTCTCCATTATTCCTGATTCGAGATTTCTGAGTTTTAGTCCTTCTGT
GTTAGCCACTGCAATAATGGTCTCTGTTATTAGAGATTTGAAGATGTGTGACGAAGCTGTATAC
CAATCTCAGCTCATGACTCTACTCAAAGTTGATTCGGAGAAGGTAAATAAATGCTATGAGTTAG
TGTTAGACCACAGTCCAAGCAAGAAAAGGATGATGAATTGGATGCAACAACCCGCTAGTCCGAT
CGGTGTGTTTGATGCGTCATTCAGTTCTGATAGCTCGAATGAGTCGTGGGTTGTGTCTGCTTCT
GCTTCAGTGTCGTCTTCACCATCTTCAGAGCCTTTGCTCAAGAGGAGAAGAGTGCAAGAGCAGC
AGATGAGGCTATCTTCAATAAACCGAATGTTTTTCGATGTGCTTAGTAGTAGTCCTCGCTAA

**SEQ ID NO 2: cyclin D3;3 protein**

MALEEEEESQNAPFCVLDGLFCEEESEFHEQVDLCDESVEKFPFLNLGLSDHDMLWDDDELSTL
ISKQEPCLYDEILDDEFLVLCREKALDWIFKVKSHYGFNSLTALLAVNYFDRFITSRKFQTDKP
WMSQLTALACLSLAAKVEEIRVPFLLDFQVEEARYVFEAKTIQRMELLVLSTLDWRMHPVTPIS
FFDHIIRRYSFKSHHQLEFLSRCESLLLSIIPDSRFLSFSPSVLATAIMVSVIRDLKMCDEAVY
QSQLMTLLKVDSEKVNKCYELVLDHSPSKKRMMNWMQQPASPIGVFDASFSSDSSNESWVVSAS
ASVSSSPSSEPLLKRRRVQEQQMRLSSINRMFFDVLSSSPR

**SEQ ID NO 3: Oryza sativa PRO prolamin promoter**

CTTCTACATCGGCTTAGGTGTAGCAACACGACTTTATTATTATTATTATTATTATTATTATTAT
TTTACAAAAATATAAAATAGATCAGTCCCTCACCACAAGTAGAGCAAGTTGGTGAGTTATTGTA
AAGTTCTACAAAGCTAATTTAAAAGTTATTGCATTAACTTATTTCATATTACAAACAAGAGTGT
CAATGGAACAATGAAAACCATATGACATACTATAATTTTGTTTTTATTATTGAAATTATATAAT
TCAAAGAGAATAAATCCACATAGCCGTAAAGTTCTACATGTGGTGCATTACCAAAATATATATA
GCTTACAAAACATGACAAGCTTAGTTTGAAAAATTGCAATCCTTATCACATTGACACATAAAGT
GAGTGATGAGTCATAATATTATTTTCTTTGCTACCCATCATGTATATATGATAGCCACAAAGTT
ACTTTGATGATGATATCAAAGAACATTTTTAGGTGCACCTAACAGAATATCCAAATAATATGAC
TCACTTAGATCATAATAGAGCATCAAGTAAAACTAACACTCTAAAGCAACCGATGGGAAAGCAT
CTATAAATAGACAAGCACAATGAAAATCCTCATCATCCTTCACCACAATTCAAATATTATAGTT
GAAGCATAGTAGTA

**SEQ ID NO 4: primer prm0360**

GGGGACAAGTTTGTACAAAAAAGCAGGCTTCACAATGGCTTTAGAAGAGGAGGA

## FIGURE 5

**SEQ ID NO 5: primer prm0361**

GGGGACCACTTTGTACAAGAAAGCTGGGTTTAGCGAGGACTACTATAAGCA

**SEQ ID NO 6: Antirrhinum majus cyclin D3a AJ250397 DNA sequence**

GAGAGAAGAGATGGCTCAAAGCTCAACTCTAGGCCTCTCTTTCTTCACACCTTTGTTTGACTTA
GAATGAAATGAATGCAGCTTCACACAACTCTCTTCTCCTCAGAAGATGTATCAACAAAACTCTC
CATCACTCTGTTTTGATGCTCTGTACTGTGAGGAAGAACAAAACTGGGACAATGGTGAAATCAT
CAATGACTGTTTCATTGAAGAACAAGAACCCTTTTCTGATTTATTGAAACATGATTTGTTATGT
GGTGTAGATGATGATGATGATGATAAAGAAGAGCTTAGCTCTTTATTGTGTAAAGAGCAGGAAT
ATGAACTGTACAGAGTCCTTGAGGACAATCCATCTCTAGCAAAAGCTAGAGATGAGGCTGTTGA
ATGGATGTTTAAGGTCATTGGGTACTATTCTTTTTCTGCTCTCACTGCGGTTCTTGCAGTTAAC
TATTTGGATAGATTTCTATGCACATTTCAGTTTCAACAAGATAAGCCATGGATGTATCAGTTGG
CTGCTGTGGCTTGTCTCTCTTTGGCTGCTAAAGTTGAAGAAACTCAAGTCCCTCTTCTGTTAGA
CCTTCAAGTTGAGGAATCTAAGTATGTGTTTGAGTCAAAAACCATTCAAAGAATGGAGCTTTTG
GTGCTTTCAACACTTAAATGGAAGATGAATCCAGTCACCCCAATTTCATTCCTTGAGTACATTG
CTAGGAGGCTAGCATTGAAGAGCCATCTTTGTAAAGAGTTCCTCAACAGATGTGAATGCCTCCT
TTTGTCCCTTATTACCGATTGTAGATTCATGTGCCATCTTCCATCTGCATTGGCCACTGCAACG
ATGCTGTATGTTATAAGCAGCTTAGAGCCCTGCATTGGTGTGGAGTACCAAGATCAACTCATCA
ACATTCTTGGAATCAACAAGGACAAAGTGGAGGAATGTTGTAAGTTAATACAAGAAGTGGCCAC
AAGTGTTCATTTTCAATCAGGCAACAAAAGAAAGTTTGGATCTTTGCCTTATAGCCCCAAAGGG
GTAGTGGACATCTCATTCAGTTGTGATGATTCATGGCCGTTGGATTCAACTGCATCAGTTTCTT
CCTCACCAGAGCATTTGTCCAAGAAAATCAAGACCCAAAATCCAGACCATGA

**SEQ ID NO 7: Antirrhinum majus cyclin D3a AJ250397 deduced protein sequence**

MYQQNSPSLCFDALYCEEEQNWDNGEIINDCFIEEQEPFSDLLKHDLLCGVDDDDDDKEELSSL
LCKEQEYELYRVLEDNPSLAKARDEAVEWMFKVIGYYSFSALTAVLAVNYLDRFLCTFQFQQDK
PWMYQLAAVACLSLAAKVEETQVPLLLDLQVEESKYVFESKTIQRMELLVLSTLKWKMNPVTPI
SFLEYIARRLALKSHLCKEFLNRCECLLLSLITDCRFMCHLPSALATATMLYVISSLEPCIGVE
YQDQLINILGINKDKVEECCKLIQEVATSVHFQSGNKRKFGSLPYSPKGVVDISFSCDDSWPLD
STASVSSSPEHLSKKIKTQNPDH

**SEQ ID NO 8: Antirrhinum majus cyclin D3b AJ250398 DNA sequence**

TAAAAAAAAGCGTAAAATTAAACAGAAAAAAATATGATATTAAGCCCAAGATTCAATCTTTTGA
GCTTTTCTTAAAGATAATGAGCAGAGCCCCCTGTATCTTCTTCTCCGAAGAAGATGTTGTTTTC
ACATTCTCAACAAACCCATCTCCAAAATCCAATCTTTGATGCTCTTCTCTGTAACGAGGAGCAT
TTCGATGAAGATTTGGATCTTGGGTCCGGGTTAAAAGACCCGGGTTTTATAAATCAGATTCATC
ATAATCAAAAAAAAGAAGAACCATTTACTACTTTTCTGTTTGAGCACGACCTTTTGTGGGAAGA
TGACGAGCTTGTTAATCTCTTGTCTAAGGAGAAAGAACAAGAACAACAAGCCCATTTGGGGTAC
GATGATGTAATGGACTCTGATGGGTTTTTGAAAAGGGTGAGAAATGAAGGGATTAAGTGGATGT
TGAAGGTGATTGGACACTATGGGTTCAATGCGATGACTGCTGTTTTAGCTGTGAATTATTATGA
TAGATTTATTACAAACGTTGGGTTTCAAAAGGATAAGCCTTGGATGAGTCAATTGGCTGCTGTT
GCTTGTCTTTCTGTAAAAGTGGAGGAGACTCAAGTGCCTCTGTTGCTGGATTTTCAAGTAGAGG

## FIGURE 5 (continued)

AATCAAAGTATGTGTTTGAGGCAAAGACTATACAGAGGATGGAGCTTTTGGTGCTCACTACTTT
GAAATGGAAGATGAACCCTGTGACGCCTATCTCGTTCTTTGACCACATTGTGAGGAGGTTTGAG
TTGATGAACAATGTGCAATGCGAGTTTATGAAGAGGTGTGAGAGTGTCATTCTCTCCATCATCA
CCGATTATCGATTTGTGCGCTATCTTCCTTCTGTTGTTGCTGCTGCAACCATGATATATGTAAT
CAAAGAGCTTTATCCTTGTGATGCATTGGAATACCAGAATGAGTTTGTGACTGTGCTGAGAACT
AGCAAGGAAAAGACTGATGATTGCCATATGCTAATCACTGAAGTAATCAACAATCAAAGCTACA
TCCTTTGTCACAAGCGCAAGTACGGTTCCATACCAAGCAGTCCAAATGGTGTGATCGATGCCTA
TTTCAGCTCTGATGGCTCTAACGATTCGTGGTCAGCAGTGTCATCCGTTTCATCATCACCAGAG
CCCGTGTTTAAGAGAATCAGAGCCATTGGGGGGGCTAATCCTCCTCATTGAACTCATTTCTTAT
TTTATCTGATATTTAGCAACGGTACTTCATAATCGCTCTTTTGCTATGGTTTTTTCCGTCATAA
GACGATGCAGTTATGTTACATTTCTGTTATATCTTGCTGTGATGGATCAGACATGTTTTAACAG
ACAATTGACTCTTATCACCTCTTTGATTGAGGGATGGAGAGCCAAAGGGATTATGTGAGTTTTT
GTTCCTGGAGTAAAATCGATGAACTTTTAGTATTAATGAAAAA

**SEQ ID NO 9: Antirrhinum majus cyclin D3b AJ250398 deduced protein sequence**

mlfshsqqthlqnpifdallcneehfdedldlgsglkdpgfinqihhnqkkeepfttflfehdl
lweddelvnllskekeqeqqahlgyddvmdsdgflkrvrnegikwmlkvighygfnamtavlav
nyydrfitnvgfqkdkpwmsqlaavaclsvkveetqvplllldfqveeskyvfeaktiqrmellv
lttlkwkmnpvtpisffdhivrrfelmnnvqcefmkrcesvilsiitdyrfvrylpsvvaaatm
iyvikelypcdaleyqnefvtvlrtskektddchmlitevinnqsyilchkrkygsipsspngv
idayfssdgsndswsavssvssspepvfkriraigganpph

**SEQ ID NO: 10 Arabidopsis thaliana cyclin delta-3 (CYCD3) (At4g34160) NM_119579 DNA sequence**

ATCACTCTCCGAAACCCACTTCCAGCTTTTTCCTCTCTCTTTCTCTCTCTAGTCTCTCTTTTGT
AGCTCTCCCCTGCTAAGCTAACCACTGCACGTTTCCATAGAGAGGAAAGATGAGTCTCTCTCCG
AGAGATTTTCTCTCTATCATCTTATCTTCTTCCGTGTAATGCTCTGAGCCAAAACCCAATAACT
AAATCAACAACAATATAGAAGAGAAGAGAAAGATCTTATCTTTCTTCTCATTCTTGAGTTTAGT
CCCCCACAATGGCGATTCGGAAGGAGGAAGAAAGTAGAGAAGAACAGAGCAATTCGTTTCTTCT
TGATGCTCTCTACTGCGAAGAAGAGAAATGGGACGATGAAGGAGAAGAAGTTGAAGAAAACTCT
TCCTTGTCTTCTTCTTCTTCTCCATTCGTTGTTTTGCAACAAGATTTGTTCTGGGAAGATGAAG
ATCTGGTTACACTCTTCTCCAAAGAAGAAGAACAAGGACTCAGCTGTCTCGATGATGTTTATCT
TTCCACGGATCGAAAAGAAGCTGTTGGTTGGATTCTGAGAGTCAACGCTCATTATGGCTTCTCT
ACTTTAGCAGCTGTTTTAGCCATAACTTATCTCGATAAGTTCATCTGTAGCTACAGCTTACAGA
GAGACAAACCATGGATGCTTCAGCTCGTTTCTGTCGCGTGTCTCTCATTAGCTGCTAAAGTCGA
AGAAACCCAAGTCCCTCTTCTTCTAGACTTTCAAGTGGAGGAGACAAAGTATGTGTTTGAAGCA
AAAACCATACAGAGAATGGAGCTACTGATTCTGTCTACTCTCGAGTGGAAGATGCATCTCATTA
CTCCAATTTCGTTCGTAGACCACATTATCAGGAGATTGGGACTTAAGAACAATGCTCACTGGGA
TTTCCTCAACAAATGCCACCGTCTCCTCCTCTCTGTAATCTCCGATTCAAGATTTGTCGGGTAC
CTCCCATCAGTAGTTGCCGCAGCTACCATGATGCGAATTATAGAGCAAGTTGATCCCTTTGACC
CTCTTTCATACCAAACTAATCTCCTCGGTGTCCTTAACTTAACCAAGGAAAAGGTGAAAACTTG
CTACGATCTAATCCTCCAACTACCAGTGGACCGCATCGGTTTACAGATCCAAATCCAATCTTCC
AAGAAACGCAAGAGTCACGATTCATCATCATCGTTGAACAGTCCAAGCTGCGTGATTGATGCAA
ACCCTTTCAATAGCGACGAAAGCTCAAACGATTCGTGGTCAGCGAGTTCGTGCAACCCACCAAC

**FIGURE 5 (continued)**

GTCGTCGTCGTCCCCGCAGCAACAACCTCCATTGAAGAAGATGAGAGGAGCTGAAGAGAATGAG
AAGAAGAAGCCGATTTTGCATCTGCCATGGGCAATCGTAGCCACTCCATAATCGAAAGCTCGAT
TTCGTTTATATGATATTTACTGTTTTTTAAACTTTGAGAACAATCTTTGTTGTATTAAGCTTT
ACCCGTTTGCATATACGAAATGTCGCGAATGCCCTTACGTGCCATGGCTTGATAGAGTTAATGG
GTAAAGGGTATTCATGACATTTGACTGCATGGGATGTGACGAAGGAGAGAATTAGAAATAATAA
TAATAATATTGCGTAAATTTTGAGGCTTGCCCAATCTTTGGGCCGT

**SEQ ID NO 11: Arabidopsis thaliana cyclin delta-3 (CYCD3) (At4g34160) NM_119579 deduced protein sequence**

MAIRKEEESREEQSNSFLLDALYCEEEKWDDEGEEVEENSSLSSSSSPFVVLQQDLFWEDEDLV
TLFSKEEEQGLSCLDDVYLSTDRKEAVGWILRVNAHYGFSTLAAVLAITYLDKFICSYSLQRDK
PWMLQLVSVACLSLAAKVEETQVPLLLDFQVEETKYVFEAKTIQRMELLILSTLEWKMHLITPI
SFVDHIIRRLGLKNNAHWDFLNKCHRLLLSVISDSRFVGYLPSVVAAATMMRIIEQVDPFDPLS
YQTNLLGVLNLTKEKVKTCYDLILQLPVDRIGLQIQIQSSKKRKSHDSSSSLNSPSCVIDANPF
NSDESSNDSWSASSCNPPTSSSSPQQQPPLKKMRGAEENEKKKPILHLPWAIVATP

**SEQ ID NO 12: Arabidopsis thaliana cyclin delta-3 (CYCD3) (At5g67260) NM_126126 DNA sequence**

AAGGCGTGAGATAATAAAACCCTTTGGCTTTCTCATAGAGATTTGTCCGGTCTCTTGTTCCTCT
TTCTCCTTTCTTCACTGTAGAATCCGTCGACCAAACAACTAGCTCCAATGGCATAATGAGCATT
GTAGTTTGCAATTTCTTCTTCCGTGAAGAAGAAGAAGATGGCTTTGGAGAAAGAGGAAGAAGCG
TCACAAAACGGTGCGTTTTGTGTCCTTGATGGGCTCTATTGCGAGGAAGAAACCGGGTTTGTGG
AGGACGATCTTGATGACGATGGAGATTTAGATTTTCTCGAGAAATCTGATGAGAGTGTTGTAAA
GTTTCAGTTTTTACCTCTTTTGGATATGTTCTTATGGGATGACGATGAGATTCTGAGTTTGATT
TCAAAGGAAAACGAAACGAATCCATGTTTTGGGGAACAAATCTTAGATGGCTTTTTGGTTTCTT
GTAGGAAAGAGGCTTTAGATTGGGTTCTTAGGGTTAAATCTCATTATGGGTTTACTTCATTGAC
GGCTATACTTGCTGTGAACTACTTCGATAGGTTTATGACAAGTATAAAGCTTCAGACTGATAAG
CCATGGATGTCTCAGCTTGTTGCTGTGGCTTCTTTGTCTTTAGCTGCTAAAGTTGAAGAGATTC
AAGTTCCATTGCTCTTAGACCTCCAAGTGGAAGAAGCAAGATATCTCTTTGAAGCTAAGACGAT
TCAAAGAATGGAGCTTTTGATTCTTTCTACTCTTCAATGGAGAATGCACCCTGTGACTCCAATC
TCTTTCTTTGATCACATTATCCGGCGATTTGGCTCTAAATGGCACCAGCAATTAGACTTCTGTA
GGAAGTGTGAGCGTCTTCTGATCTCTGTTATTGCTGATACGAGGTTTATGAGGTACTTCCCTTC
TGTCTTAGCTACTGCAATAATGATCCTTGTCTTCGAGGAATTGAAGCCATGTGATGAAGTTGAA
TACCAATCTCAAATAACGACTCTACTCAAAGTCAATCAGGAGAAAGTAAATGAATGCTATGAAC
TGTTGTTGGAGCACAATCCAAGCAAGAAGAGGATGATGAATTTGGTTGATCAGGACAGTCCAAG
TGGTGTATTAGACTTTGATGACAGCTCAAATAGCTCCTGGAATGTCTCCACTACTGCTTCAGTG
TCCTCATCATCTTCGTCTCCAGAGCCTCTGCTCAAGAGAAGAAGAGTTCAGGAGCAGCAAATGA
GATTGCCCTCAATAAACCGTATGTTTCTCGATGTGCTTAGTAGTCCTCGCTAGTACCTTTCTTT
GATCAAATGTGTCAAAACATAAATTCAATCTCTCTTTTGCTTATTATTATCGGCCATCGGCTAC
AATTTGAAGGCAGAACATTTTGTGATAACTCTAAGTTAATTCTGCCTCTTAAATCATAATATTC
ATTGATC

**FIGURE 5 (continued)**

**SEQ ID NO 13: Arabidopsis thaliana cyclin delta-3 (CYCD3) (At5g67260) NM_126126 deduced protein sequence**

MALEKEEEASQNGAFCVLDGLYCEEETGFVEDDLDDDGDLDFLEKSDESVVKFQFLPLLDMFLW
DDDEILSLISKENETNPCFGEQILDGFLVSCRKEALDWVLRVKSHYGFTSLTAILAVNYFDRFM
TSIKLQTDKPWMSQLVAVASLSLAAKVEEIQVPLLLDLQVEEARYLFEAKTIQRMELLILSTLQ
WRMHPVTPISFFDHIIRRFGSKWHQQLDFCRKCERLLISVIADTRFMRYFPSVLATAIMILVFE
ELKPCDEVEYQSQITTLLKVNQEKVNECYELLLEHNPSKKRMMNLVDQDSPSGVLDFDDSSNSS
WNVSTTASVSSSSSSPEPLLKRRRVQEQQMRLPSINRMFLDVLSSPR

**SEQ ID NO 14: Euphorbia esula cyclin D3-2 (CycD3-2) AY340588 DNA sequence**

TTTTTTTTCTTCTCTGCCTCTCTATCCATTCCTTCTCTTCTCTCTGTCTCTGCTATCAATAGA
CCCCTAGTGAGAGAAACAGAGATAAAGCAAATTGTATGGTGATATGATTTGCAATGAGATTGAG
ATTGGGATTTTATTTTCTTTGATGGTATAATGAGCAAAGGATTGAATCTCTTCTTCTCTGAAGA
AGAACAAAAATGGAAGACAGCACTCAGATTTCATTGATTTTGATGGGTTGTACTGCGAAGAAC
AAGGCATTGGTGAAGATTTTGACGATGGGAATGAAGATTATGTGAAAAAGGAGTTATCTTTATC
TTCTGTTTTGCTTGAGCAGGACTTGTTTTGGACCGATGATGAGTTGTTAAATCTGATTTCAAAA
GAAAAAGAGACTCATTTTAGTTTTGGGGATTTTTCCTCTCATGGGTCTTTAATGGTGGCTCGTA
AAGAGGCAATAGATTGGATTTTGAGGGTAAAAGGGTTTTATGGATTCAATGCTTTGAGCTGTGT
TCTTGCTGTTAATTATTTTGATAGATTCATTTCGAGTTTAGTTTTTACAAGAGATAAACCATGG
ATGGGTCAACTTGCTGCTGTTGCTTGTTTATCTTTGGCTGCTAAAATGGAGGAGACTCAAGTTC
CTCTTCTTCTAGATTTACAAGTGGAAGAATCAAAGTATGTGTTTGAGGCAAAGACTATAAAGAG
AATGGAGCTTCTTGTGCTCTCTACTCTTCAATGGAGGATGAATCCTGTGACCCCAATTTGCTAC
TTTGATCACATTATAAGGAGGCTAGGACTTAAAAACCATCTGCATTGGGAATTTTTGAGGAGAT
GTGAGCTTTTACTTCTCTCTGTCATTTCTGATTCAAGATTCATGAGTTATGCACCTTCTATATT
AGCAACTTCAATTATGATCCATGTGATTAAGGAGGTTGACCCATTTAGTCAAATGGAATACCAG
AACCAGCTTTTGGATGTGATCAAAATCAACAAGGAGGAAGTGAACCAGTGTTACAAGCTCATCT
TGGAGCTATCGGGTAAGCAAGATCAAGGGTACAAACGCAAGTATCCCTCAAGACCCGGGAGCCC
AAATGGTGTGATTGATGCCTATTTTAGTGGAGATAGCTCGAATGATTCGTGGGGAGTTTCTTCC
TCAATCTCATCATCACCATCGATTCCTCGATTTAAAAGGATCAAATCCCAGGATCAACAGATGA
GGTTGCCTTCAATAAACCGTATGTTTGTGGATGTGCTTAGTAGTCCTCATTGATCTTTTACTTT
TGTTATCTATTGCCTACTTCGAAACAATGCCATTATAAATTTCTCTATTTTGCATATTATTTGT
ATNCCCGTGGTTACTTGGGTACATTTCCGGCCTAATATTTGAACTCACTCAAGCTAGACAAATT
GAAAAAAAAAAAAAAA

**SEQ ID NO 15: Euphorbia esula cyclin D3-2 (CycD3-2) AY340588 deduced protein sequence**

MEDSTQISLIFDGLYCEEQGIGEDFDDGNEDYVKKELSLSSVLLEQDLFWTDDELLNLISKEKE
THFSFGDFSSHGSLMVARKEAIDWILRVKGFYGFNALSCVLAVNYFDRFISSLVFTRDKPWMGQ
LAAVACLSLAAKMEETQVPLLLDLQVEESKYVFEAKTIKRMELLVLSTLQWRMNPVTPICYFDH
IIRRLGLKNHLHWEFLRRCELLLLSVISDSRFMSYAPSILATSIMIHVIKEVDPFSQMEYQNQL
LDVIKINKEEVNQCYKLILELSGKQDQGYKRKYPSRPGSPNGVIDAYFSGDSSNDSWGVSSSIS
SSPSIPRFKRIKSQDQQMRLPSINRMFVDVLSSPH

## FIGURE 5 (continued)

**SEQ ID NO 16: Euphorbia esula cyclin D3-1 (CycD3-1) AY340589 DNA sequence**

AAAGAATTCCTACAACCTATGCCTTCTTCCCCACTCAGATACCCAAATAAAAACACATACCCTT
AGATAACTGGTCACTACCCCTCTTCCTGCTCTTTTTGTTTCTTTGACAAAGAGAGAGAAATGGC
TCAAACTGAGTCTTTAACTCTCTCTTTAATGTTCATCTTCTATTCCTTTGCCCTATAATGAACC
CCAATCTCAATTKTATCTTCTTAGAAAGAAGATGGCAAATCATTCTCCATTATTTCTCTATGAT
GCTCTTTACTGCTCAGAAGAAGATAACTGGGAAGGAGAAGTTGTTGATATTTTTCATGAACAAG
AAGATCAAGGAGAAAACACCTCTGTCTTTCCCCAAAATTCTTCCCCAGTAGACTTAAATTGGGA
AGAAGATGAGCTTACGTCTGTATTTTCTAAACAAGAGCAAAACCAACTATATAAAAAACTAGAA
ATCAACCCATGTCTAGCTAAATCTCGCCGTGATGCTGTGGATTGGATGATGAAAGTCAATGCCC
ATTACTCTTTCACTGCTTTGACTTCAGTTTTGGCCGTTAATTTTCTTGATAGATTCCTTTTTAG
CTTCGATCTTCAAACGGAGAAGCCATGGATGACCCAGCTCACAGCTGTAGCTTGTCTCTCCTTA
GCAGCAAAAGTAGAAGAGACACAAGTCCCACTTTTATTGGACCTTCAGGTGGTGGACAGTAAGT
ATGTGTTTGAGGCTAAAACTATACAAAGAATGGAGCTTTTGGTGCTTTCTACTCTTCAATGGAG
AATGAACCCTGTAACTCCATTATCATTTATTGATTACATGACAAGAAGGCTTGGTTTTAAGGAT
TATCTTTGCTGGGAATTTATCCGGAGATGCGAGCTTATTGTTCTCTCTATAATCTCAGATATGA
GATTTATACCTTATCTCCCTAGTGAAATTGCTTCCGCAATAATGCTACATGTGATTAATGGTAT
AGAACCCAGTCTTGGAGATGAATTCGAAACCCAGCTATTCGGGATTCTTGGAATTGATAAGGAG
AAGGTGAATAATTGCAGAGAAATGATAATCGAGTTAGGATCAAGATATTACGGCAACCAATCAA
ACAAAAGAAAATACGGGTCGGATCCGGGTAGTCCAAATTGCGTAATGGATGTCTCATTTAGTTC
AGATAATTCAAACGATTCTTGGGCAGTCGGATCTAAATCATCATCAGTGTCTTCCTCACCCGCG
GCGAAGAAACTCAGGGCAGTTTCAGGGATGAACCATGAAAATGCGATAATACTTTCCTAAGCAT
GAACATTTTTTAATCCTTAAAATCTTTTTAATTTATTTCATTTGAATCCCCTTTTGAGGACTTG
GTTGTATTAAACTGTTAATAATTGATGTTGTTAGTTAAATTGCCGGGCATCTCTGCTTCTCCAA
TCTCAATTAAAATCTTAATTAGAATTTTGGAAGCAGAGATGGTTGGCATTTTATCCGGAAATTA
GTAGTAAAAGAGAAATTGCACTAAAAAAA

**SEQ ID NO 17: Euphorbia esula cyclin D3-1 (CycD3-1) AY340589 deduced protein sequence**

MANHSPLFLYDALYCSEEDNWEGEVVDIFHEQEDQGENTSVFPQNSSPVDLNWEEDELTSVFSK
QEQNQLYKKLEINPCLAKSRRDAVDWMMKVNAHYSFTALTSVLAVNFLDRFLFSFDLQTEKPWM
TQLTAVACLSLAAKVEETQVPLLLDLQVVDSKYVFEAKTIQRMELLVLSTLQWRMNPVTPLSFI
DYMTRRLGFKDYLCWEFIRRCELIVLSIISDMRFIPYLPSEIASAIMLHVINGIEPSLGDEFET
QLFGILGIDKEKVNNCREMIIELGSRYYGNQSNKRKYGSDPGSPNCVMDVSFSSDNSNDSWAVG
SKSSSVSSSPAAKKLRAVSGMNHENAIILS

**SEQ ID NO 18: Helianthus annuus cyclin D3 AY033440 DNA sequence**

AATACCCAATGGCCATTTTATCACGATATTCATCTTCGAACACACTCTTCTGCATTGAAGAACA
AGTTCATGAAGATGAAGATGAGTTAACACATCAAGATTCCTCTGCAATCCATCCATTAGACCTA
CAAGATTTGTGTTGGGAACATGAAGAACTTGTCTCTCTTTTCACAAAAGAAGAAGAACAACAAA
AGCAAACCCCTTGGCCTTCTTCTTGTACTTTGTCTTTTCGTAAAGAGGCTGTGGATTGGATCCT
TAAAGTCAAAGGTTGTCATGGATTCACACCTCTAACAGCCATTTTAGCCATCAATTATCTTGAT
CGGTTTCTGTCCAGTCTCCATTTCAGAAAGCTAACACACCTTGGATGATTCACCTTGTTGCTG
TTACTTGTCTTTCTTTGGCTGCTAAAATTCAAGAAACTCATGTGCCTTTGCTCTTAGATCTTCA

**FIGURE 5 (continued)**

GCTAGAGGAGAGTAAGTTCTTGTTTGAGGCCAAGAACATACAAAAGACGGAGCTTTTGGTGATG
TCAACACTGAAATGGAGGATGAACCTAGTGACACCAATCTCATTTCTTGATCACATTGTAAGAA
GGCTTGGATTATCAAATCATCTTCATTGGGATTTCTTCAAGAAATGTGAAGCTATGATTCTTTA
CCTAGTGGCTGATTCAAGATTTGTGTGCTATAAACCATCTGTGTTGGCAACCGCTACAATGCTT
TGTGTTGTAGAGGAAATCGACCCGACCAATTCCATTGGCTACAAAAGTCAACTTCTGGATCTTC
TCAAAACCACTAAGGACCACATAAATGAGTGTTACAAGCTTGTTATGGATCTATCCTATGATAA
TCACAACAAAGGAAAGCGTGATGAAAACGAGAGAACAATTTATCCGGTTAGTCCAGCTGGTTTT
ATTGGTTTTATGTGCCACGAAAGTTCAAATGATTCATGATCCTCGCTCAAGAATTGATCAATAA
TTAGGGTTTGGCTCACTTGTAAGCTTTAAACCACTCGCAAGCACTCGTTATCCATAACCTATAC
AATCAGCAGAAGTTGCGCTAATAATAGACCCGTCGGTCCACCACTAGTTATGTTGTATGGTATG
GTCTTTAATTTCTCTGTTGTTTTAGGTCGTTTTTAATGTGAGATAAGTTAAACTCGGTGATGTT
ATCATGTCTTATTCAAGCAATGAATTTATATATTTTACATC

**SEQ ID NO 19: Helianthus annuus cyclin D3 AY033440 deduced protein sequence**

MAILSRYSSSNTLFCIEEQVHEDEDELTHQDSSAIHPLDLQDLCWEHEELVSLFTKEEEQQKQT
PWPSSCTLSFRKEAVDWILKVKGCHGFTPLTAILAINYLDRFLSSLHFQKANTPWMIHLVAVTC
LSLAAKIQETHVPLLLDLQLEESKFLFEAKNIQKTELLVMSTLKWRMNLVTPISFLDHIVRRLG
LSNHLHWDFFKKCEAMILYLVADSRFVCYKPSVLATATMLCVVEEIDPTNSIGYKSQLLDLLKT
TKDHINECYKLVMDLSYDNHNKGKRDENERTIYPVSPAGFIGFMCHESSNDS

**SEQ ID NO 20: Helianthus tuberosus cyclin D3 (CycD3;1) AY063461 DNA sequence**

TTGAACCTTCATTTCTTTTCTTTTCTTCTTTCTAATCACCAACCCCAATGGCCATTTTATCACC
ATATTCATCTTCTTTCTTAGACACACTCTTTTGCAATGAACAACAAGATCATGAATATCATGAA
TATGAGTATGAAGATGAATTTACACAAACCACCCTCACAGATTCATCTGATCTCCATCTTCCCC
CCCTGGACCAACTAGATTTGTCATGGGAACATGAAGAGCTTGTGTCCTTGTTCACAAAAGAACA
AGAGCAGCAAAAACAAACCCCCTTGTACTCTCTCTTTTGGCAAAACTAGTCCCTCAGTTTTTGCT
GCTCGTAAAGAGGCTGTAGATTGGATCCTTAAGGTCAAAAGTTGTTATGGATTCACACCTCTTA
CAGCCATTTTAGCCATCAATTATCTTGATAGGTTTCTTTCTAGCCTCCATTTTCAAGAAGATAA
ACCTTGGATGATTCAACTTGTTGCTGTTAGTTGTCTCTCTTTAGCTGCTAAAGTTGAAGAAACT
CAAGTGCCACTCTTACTAGATCTTCAAGTAGAGGACACTAAGTACTTGTTTGAGGCTAAAAACA
TACAAAAAATGGAGCTTTTGGTGATGTCAACTTTGAAATGGAGGATGAACCCAGTGACACCAAT
CTCATTTCTTGATCACATTGTAAGAAGGCTTGGATTAACTGATCATGTTCATTGGGATTTTTTC
AAGAAATGTGAAGCTATGATCCTTTGTTTAGTTTCAGATTCAAGATTCGTGTGTTATAAACCAT
CCGTGTTGGCCACAGCTACAATGCTTCACGTTGTAGATGAAATTGATCCTCCCAATTGTATTGA
CTACAAAAGTCAACTTCTGGATCTTCTCAAAACCACTAAGGACGACATAAACGAGTGTTACGAG
CTCATTGTCGAGCTAGCTTACGATCATCACAACAAACGAAAACATGATGCAAACGAGACAACAA
CCAATCCGGTTAGTCCAGCTGGCGTGATCGATTTCACTTGTGATGAAAGTTCAAATGAGTCATG
GGAACTTAATGCTCATCATTTCCGCGAGCCTTCATTCAAGAAAACAAGAATGGATTCAACAATT
CGGGTTCGGGTTTGGTTCACTTATAAGCTTTAATCGAGGGTAGTTGTAAACATGTAATCCGCAT
GCACGCTATTAATCCTACGGTCCACTACTACATATAATCGGCCTATAAAATTATAGGTTAAGAT
GACCAGTCGTAGGCGTCGAGATGTCCTTATGGTTGGTCAATTTCTCTATGGTTTTAGGTCGTTT
TTAATGTGAGATAAATTAAATTCGGTATGTTAAGTCTTTATCAAGCAATGGACGTTATATTTAT
TGTTTGATATTGAGAATTAAATTCCATGGGAAAAAAAAAAAAAAAAAAAAAAAAAAA

**FIGURE 5 (continued)**

**SEQ ID NO 21: Helianthus tuberosus cyclin D3 (CycD3;1) AY063461 deduced protein sequence**

MAILSPYSSSFLDTLFCNEQQDHEYHEYEYEDEFTQTTLTDSSDLHLPPLDQLDLSWEHEELVS
LFTKEQEQQKQTPCTLSFGKTSPSVFAARKEAVDWILKVKSCYGFTPLTAILAINYLDRFLSSL
HFQEDKPWMIQLVAVSCLSLAAKVEETQVPLLLDLQVEDTKYLFEAKNIQKMELLVMSTLKWRM
NPVTPISFLDHIVRRLGLTDHVHWDFFKKCEAMILCLVSDSRFVCYKPSVLATATMLHVVDEID
PPNCIDYKSQLLDLLKTTKDDINECYELIVELAYDHHNKRKHDANETTTNPVSPAGVIDFTCDE
SSNESWELNAHHFREPSFKKTRMDSTIRVRVWFTYKL

**SEQ ID NO 22: Lagenaria siceraria cyclin D3.1 AF519810.1 DNA sequence**

AAGAGCACAAGAAAGCTCATCCTTAACTGCTTTCTTCACAGGGCTGGTTGCTTCTGTCGACGGG
GATTCGGGTGTGAGCTTTTATTTTTCTCCGTGTCTCGTACCCCCTCGGAGGGTTAGTCCTATG
AGCTCCCGGTGTCCTCGCTGCTCAATTCAGCTTTGAGGTCCTCTCCTTCTTTTCCACTCTGAAT
AAGATAGAGGAAAAGAGAGAAACCCCAGCTTACAGCCATGGGTGAGGATTGGTCTTCTTCGCCA
TTTTCCTCTGTTTTTCTCCATTTCCTTCTCTCAAGCTCTACAATGAACCAAAATCACAGCCTCT
TTCTTCTCAGAAGAAGATGGTACCACCGTATGCGCTTGATTCTCTTTATTGTTCAGAAGACCAC
TGGGAGAACGACGACGAAGAAGAAGAAAGGGGTTTTCATGAGCAACCTTATTCTAATTTAACAA
CCGAATCAAGTTCTCCGATTCTGGCAGTGGCAGAGCAGGATCTGTTTTGGGAAAACGATGAATT
AATTTCTCTGTTCTCAAGAGAGAAGCCTAATGAACTGTTTAAAACCATTCAGATTGACCCTTCT
CTTGCTGCCGCCCGACGAAGCGCCGTCGGGTGGATGCTGAAGGTTAATGCCCATTATTCTTTCT
CTGCTCTCACTGCGGTTCTCGCCGTCGATTATTTGGATCGGTTTCTGTCCTGTTTTCATTTTCA
AAGAGACAAGCCATGGATGTCTCAGCTTGCTGCTGTTGCTTGTATCTCTCTTGCTGCCAAAGTA
GAGGAGACCCAAGTCCCTCTTTTATTGGACCTACAAGTGGAAGACAGTAGATATCTATTTGAAG
CCAAGACAATTAAGAAAATGGAGCTTCTTGTGCTCTCTACGCTTCAATGGCGGATGAATCCTGT
TACCCCATTTTCTTTTGTGGATTATATCTCAAGGAGGCTTGGATTCAAGGAACATATCTGCTGG
GAAATTCTTTGGCAGTGTGAGCGAACTATTCTCTCTGTTATTTTAGAGTCAGATTTTATGTCCT
TTCTTCCTTCTGTAATGGCCACCGCTACAATGCTGCACGTTTTCAAAGCTATGGAAGAACCCAC
CCTCAGCGTTGAATACGATTCCCAGCTTCTTAACATCCTCGGAATCGACAAGGGGAATGTGGAA
GAATGCTGTAAGCTGATCTCAAATGCATCAAGAAGAAACGGCAACCAATTCAAGAAACGTAAAA
TTGGGTCGATTCCGGGTAGCCCGAACGGCGTGATGGACGTGTCATTCAGCTCCGATAGCTCGAA
CGACTCGTGGTCAGTGGCCTCGTCAGTTTCATCTTCGCCAGAGCCATTAACGAAGAAGAACAGA
GCCAATGGATCAATGTCTGGAGATTGCGAAACATTCAGAACCCTCTCTTAATTAACTTCCCTTT
CTTCTTTTTCCTCGTAATCCTTGTATGTTGAATAAGAATTAGAATCAATCTTTTTATTTATCGA
ATTCTGCGAGTTAAATTGCCTTACCATCTCTGCAGTTAAGAGCCAATGGATGGGCAATCGGAAG
TTTAAAACGCAGAGATGGCTGGCATTTTATCCGGATGCAAAGGACATTGAACGTATAACAATGA
AGAGTAGTTTAAGTTGTTTAAAAAGAAAAA

**SEQ ID NO 23: Lagenaria siceraria cyclin D3.1 AF519810.1 deduced protein sequence**

MVPPYALDSLYCSEDHWENDDEEEERGFHEQPYSNLTTESSSPILAVAEQDLFWENDELISLFS
REKPNELFKTIQIDPSLAAARRSAVGWMLKVNAHYSFSALTAVLAVDYLDRFLSCFHFQRDKPW
MSQLAAVACISLAAKVEETQVPLLLDLQVEDSRYLFEAKTIKKMELLVLSTLQWRMNPVTPFSF
VDYISRRLGFKEHICWEILWQCERTILSVILESDFMSFLPSVMATATMLHVFKAMEEPTLSVEY
DSQLLNILGIDKGNVEECCKLISNASRRNGNQFKKRKIGSIPGSPNGVMDVSFSSDSSNDSWSV
ASSVSSSPEPLTKKNRANGSMSGDCETFRTLS

**FIGURE 5 (continued)**

**SEQ ID NO 24: Lagenaria siceraria cyclin D3.2 AF519811 DNA sequence**

TGGACACGAATTTCTTCGACTAGTAAGCACCAAAAGCTCCATAAAAGAGAAAAAAAAAAGGGGG
GAAATGGGGTTTCTCTTCCTCTCCCTATAATGACTCCGCAATACTCAATCCTCTTCTTCCAAGA
AGAAGAAGATGAAGAAGATGGCTTTGCACTCAAATAAACACAGAACCCAACGCCTCCATAACTC
TCTCTTCTTCTTCGACTTCCTCCACTGCACTGAACAACAACACCTTCAAACAGAGCATCCCATT
TTCCTTAACAATGGGGGCACCAACGACTTCCCTCTTTTCCAACAAACAACCACCCATTTCCTTG
TTTACGAAGACGAGGAGCTCAATCATTTGTTGTCCAAAGAAAAGGACCAAAATCTCCAAACCGG
TGCTGTTTTGAAAACCTTGGTTCAAACAGATAATGCTCTGTCTCTCGCTAGAACAGAGGCCATC
GACTGGTTGCTTAAAGTTAATGCCTTTTATGGTTTCTCCTCTCTCACAGCTCTCTTAGCCATTA
ATTACCTCGATAGAATCCTCTCTGGGCCCTATTTTCAAAGAGATAAGCCATGGATGCTTCAGCT
TGCTGCTGTAACTTGCATCTCTTTAGCTGCTAAAGTCGAAGAAATTCGTGTCCCTCTTCTTCTA
GACCTCCAGGTGGAAGATTCAAAGTACATTTTTGAAGCGAAAACGATACAGAGGATGGAGCTTT
TAGTGCTTACTGCTCTGCAATGGAAGATGCACCCAGTGGCCCCTGTTTCGTTTCTTGGCATTAT
CACAAAAGGACTTGGAATGAAGAATCAGTACATTCAAAGAGAGTTTCTTAGACGCTGTGAGCGT
ATTCTTCTCTCTCTCGTCTCTGATTCGAGATCGGTGGGATTCTTCCTTCTATAATGGCGGTAT
CAGCAATGGTGAGCGTTGTTGAAGAGATGGGAAACTGTAACCCATTGGAGGAGTTTCAGGATCA
GCTTCTTAATGCCCTCAAAATAAATAAGGGGAGAGTGAAGGAGTGTTGTAAAGTGATAATGGAG
GCAAAAATAAAAGGATCAGGGAAGAGGAAGCATGTGGAGGAGGAAGCAGAAGCAGAAGCAGAAT
CAGAATCATCAGAAGCAGAAACAGAGGGAGAAGCAGAAGCAGAAGCAGGGAGCCCAAATGGAGT
AATGGAGGCTAATTTCAGCTGTGAAAGCTCCAACGATTCGTGGGAAATGGGGACGATTGTGTCA
GAATACACACATTTTTCTTCTTCTTCTTCTTCTTCTTCCAAAAGAATCAGACCCACTCGATGAA
TTATTTGAATAATGAATTGAACCAAATTTGCAGAGATTCAATATCCATTACATTAGCCTTCCCC
TGTTCTGAAATGGCACATTGGCAAACACACAAAAAACCAGAGACAAAAAAGTGGGGAAAAACAGA
GAAGTAATGATGACGATGATGATGAGAGAGGTTGTTCTTCTCTTTCTTTATAGCTCATTAATTA
TGTTGTATATGATGAAATTGATGAGTATTTGCTTCTATTAATATTGCTCTCTTCTTCATTTCAA
AAAAAAAAAAAAGTTGACCACGCGTGGCC

**SEQ ID NO 25: Lagenaria siceraria cyclin D3.2 AF519811 deduced protein sequence**

MKKMALHSNKHRTQRLHNSLFFFDFLHCTEQQHLQTEHPIFLNNGGTNDFPLFQQTTTHFLVYE
DEELNHLLSKEKDQNLQTGAVLKTLVQTDNALSLARTEAIDWLLKVNAFYGFSSLTALLAINYL
DRILSGPYFQRDKPWMLQLAAVTCISLAAKVEEIRVPLLLDLQVEDSKYIFEAKTIQRMELLVL
TALQWKMHPVAPVSFLGIITKGLGMKNQYIQREFLRRCERILLSLVSDSRSVGILPSIMAVSAM
VSVVEEMGNCNPLEEFQDQLLNALKINKGRVKECCKVIMEAKIKGSGKRKHVEEEAEAEAESES
SEAETEGEAEAEAGSPNGVMEANFSCESSNDSWEMGTIVSEYTHFSSSSSSSSKRIRPTR

**SEQ ID NO 26: Lycopersicon esculentum mRNA for D-type cyclin AJ002588 DNA sequence**

CTCCTTTTGGCTCTTCCTATTCCCTCTCTCTCTTCCCCTTTTTTCTGTCCTTTAGAGAGAGAAA
AAAAATCCATAAGCCAAGCAGCAGATATGTTACTGGGTCCAAGATTGAGTTTTGGCTTACCTTA
AAGATAATGAGTAGAGCCTTCATTGTCTTCTTCCCTCTAGAAGAAGGAGAAGATGGTTTTCCCT
TTAGATTCCCAGCTCCAAAATCCTATTTCTGCTCTTCTTGATGGCCTTTACTGTGAGGAAGATC
GATTCTTGGATGATGATTTAGGGGAATGGTCTAGTTTAGATGTCGGAAATGAAAATGTTAAAAA

## FIGURE 5 (continued)

GACTCTGCCTTTATTAGAATGTGACATGTTTTGGGAACATGATGAGCTTGCCACACTTTTATCT
AAGGAAAATGAGTTTCATTTGGGTTTTCAATCTTTAATCTCAGATGGGTCTTTAATGGGGGCTA
GAAAAGAGGCTTTGGATTGGATGTTGAGGGTCATTGCTTACTATGGTTTTACTGCTACCACTGC
TGTTTTAGCTGTGAACTATTTTGATAGGTTTGTGTCTGGATGGTGCTTTCAGAAAGATAAGCCT
TGGATGAGTCAGCTTGCTGCTGTTGCCTGTCTTTCCATTGCTGCTAAAGTGGAGGAGACCCAAG
TTCCCCTTTTGTTAGACCTACAAGTTGCTGATTCTAGATTTGTGTTTGAGGCAAAGACTATACA
GAGAATGGAACTCTTGGTGCTTTCTACTCTTAAGTGGAAAATGAATCTGGTGACACCATTATCT
TTCATTGATCATATTATGAGGAGATTTGGATTCATGAGCAACCTGCATATGGATTTTCTTAAGA
AGTGTGAACGCCTCATTCTTGATATCATCACTGATTCTAGGCTCTTGCATTATCCTCCATCTGT
TATTGCAACTGCATCGATGTTTTATGTGATCAATGACATTGAGCCTAGCAATGCTATGGAATAC
CAAAATCAGCTCATGAGTGTTCTTAAAGTCAGAAAGGACATCTTTGAGGAATGCCATGATCTTA
TTCTTGAGCTAATGGACACTGCCTGTTACAAGCTCTGCCAAAGCCTCAAGCGCAAACATCATTC
AGTACCTGGTAGTCCAAGTGGTGTTATTGATGCATATTTTAGTAGTGAGAGCTCGAATGAATCA
TGGTCAGTAGCATCTTCGATTTCATCCTCACCTGAGCCTCAGTATAAGAGAAACAAAACTCAAG
ATCAGCGAATGACACTAGCTCCACTGGGTAGTAATCTTCACTGATCGATATCTTGTTCTCTAGA
TTACCTAGTATTTCGGCAATGGTTTACTCTCTTTTTTGGTATGTTCTCTTAAAAATGCAATTGC
ACAATGCTCTGATGCTCCATTTAAGTTTTACTGGACTTAATTTGTCCGATGATCGTCTAGACTA
TGTGAACATCAACTCCACCCCCCTCCTTCATTGGACATGGGATTGGTGGAGTTTTCCCTTGAGT
TGAATCAATGCTGCTGAATTATGTTGAAAAAAAAAAAAAAAAAAAA

**SEQ ID NO 27: Lycopersicon esculentum mRNA for D-type cyclin AJ002588 deduced protein sequence**

MVFPLDSQLQNPISALLDGLYCEEDRFLDDDLGEWSSLDVGNENVKKTLPLLECDMFWEHDELA
TLLSKENEFHLGFQSLISDGSLMGARKEALDWMLRVIAYYGFTATTAVLAVNYFDRFVSGWCFQ
KDKPWMSQLAAVACLSIAAKVEETQVPLLLDLQVADSRFVFEAKTIQRMELLVLSTLKWKMNLV
TPLSFIDHIMRRFGFMSNLHMDFLKKCERLILDIITDSRLLHYPPSVIATASMFYVINDIEPSN
AMEYQNQLMSVLKVRKDIFEECHDLILELMDTACYKLCQSLKRKHHSVPGSPSGVIDAYFSSES
SNESWSVASSISSSPEPQYKRNKTQDQRMTLAPLGSNLH

**SEQ ID NO 28: Lycopersicon esculentum mRNA for D-type cyclin AJ002589 DNA sequence**

ACAGAGATATTAGAAGGGGAAAAAAATGGCAATAGAGAATAATGATCAATCTTTTTTTTAGAT
GTGCTTTACTGTGAAGAAGAAGAAGAAAAATGGGGTGATTTGTTAGAGGATGAAGAAGGGGTTA
TTATTAACCCATTGTTGTTATCTTCCGAAGGAACAACAAAAACTAATTCTTTATTATTATTACC
TCTGCTTCTGTTGGAACAAGATTTGTTTTGGGAAGATGAAGAGCTTCTTTCACTTTTCGTTAAA
GAAAAAGAAACTCGTTGTTGTTTTGAAAGTTTTGGGAGTGACCCTTTTCTCTGTTCAGCTCGTG
TTGATGTTGTTGAATGGATTCTTAAAGTGAATGCTCATTATGATTTCTCAGCATTGACTGCCAT
TTTAGCCATTAATTATCTTGACAGGTTTCTTTCTAGCCTTCAATTTCAGAAAGATAAGCCATGG
ATGACTCAACTTGCTGCTGTCACTTGTCTTTCTTTAGCGGCTAAAGTTGAAGAAACTCAAGTTC
CCCTTCTTCTTGACTTCCAAGTGGAGGATGCAAAATATGTGTTTGAGGCAAAGACTATACAAAG
AATGGAGCTTCTGGTACTGTCATCACTGAAATGGAGGATGAATCCAGTGACCCCACTTTCATTT
CTTGATCATATTATAAGAAGGCTTGGGCTAAAGAACAATGTTCATTGGGAATTTCTCAGAAGAT
GTGAAAGTCTTCTTCTATCTGTCATGATTGATTGTAGATTTGTACGTTATATGCCTTCTGTATT
GGCTACTGCAATTATGCTTCATGTTATTCATCAAATTGAGCCTTGTAATGCTATTGACTATCAA
AATCAACTTCTTGGGGTTCTCAAAATTAGCAAGGAGAATGTGAATAATTGCTATGAACTCATAT

## FIGURE 5 (continued)

CCGAAGTGTCATCAAAGCCTATTACATCACACAAACGCAAATATGATGAAAATCCCAGTAGTCC
AAGTGGTGTAATAGATCCAATTTACACTTCAGAAAGTTCAAATGATTCATGGGATTTAGATTTG
CCTTCGTTCAAGAAAAGCAAAGTTCAAGAACAGCAAATGAAAATGTCATCATCATTGAGCAGAG
TTTTTGTGGAAGCTGTTGGTAGTCCTCATTAAAATGTCTCTTTTAAATATTTTAATTACATATT
CAAGAAGTATTTTGCTGTTATGTGTTAGCTGTGGTTGTTGGCAGAGAAGAGAAGATGAGTGGCT
TTATTTTTTGCAGGAGTGTAGTCTACTACTACTACTGTGAAGCCAGAGAGAGAAAGAGAAAAGA
CAGAATATGTGCAATCTTTGTTTTTCTCTCTATTTATTTCAATTTTCTCTCTCAAGTCACTTTC
ATGCATGCATACTTTTGATGGACTACTCTATTTATATTGCCTTTACTTATTAGTACTTAATATA
TATATATATATATATATATATATATATAAATCATATAAAAAAAAAAAAAAAAAA

**SEQ ID NO 29: Lycopersicon esculentum mRNA for D-type cyclin AJ002589 deduced protein sequence**

MAIENNDQSFFLDVLYCEEEEEKWGDLLEDEEGVIINPLLLSSEGTTKTNSLLLLPLLLLEQDL
FWEDEELLSLFVKEKETRCCFESFGSDPFLCSARVDVVEWILKVNAHYDFSALTAILAINYLDR
FLSSLQFQKDKPWMTQLAAVTCLSLAAKVEETQVPLLLDFQVEDAKYVFEAKTIQRMELLVLSS
LKWRMNPVTPLSFLDHIIRRLGLKNNVHWEFLRRCESLLLSVMIDCRFVRYMPSVLATAIMLHV
IHQIEPCNAIDYQNQLLGVLKISKENVNNCYELISEVSSKPITSHKRKYDENPSSPSGVIDPIY
TSESSNDSWDLDLPSFKKSKVQEQQMKMSSSLSRVFVEAVGSPH

**SEQ ID NO 30: Lycopersicon esculentum mRNA for D-type cyclin AJ002590 DNA sequence**

ACAAACCTTATTTTTCTTTTTCTTTTTTTAAATTCAGTTTCTTGAATTAGTATGAAGTAAATGA
AAACACAACTAAACTTCTTCTTCTCAGAAGAAGATAATGTCTCACCATTATCAAGAACAAGAAC
AACTAGAAGCACAAAAGATTCCATTTTTATTAGATTCACTTTACTGTGAAGAAAATAATATACT
CACTGAAGTATCAATAGAGACAGAGAGTTTTTCTTCACATGATTTGTTATGGGAAGAAGAAGAA
CTTACCTCTCTGTTTTCTAAAGAAACAGAGTATGAAATAAGCTACAATGTGTTAGAAAAAAACC
AGTCTTTTATTTCATCAAGAAGAGAATCAGTTGAATGGATACTCAAAACAACTGCTTATTACTC
TTTTTCTGCTCAAACTGGATTTCTTGCAGTTAATTACTTTGATAGATTTCTGTTATTTAGTTTT
AATCAGTCTCTGAATCATAAGCCATGGATGAATCAACTTGTTGCTGTTACTTGTCTTTCATTAG
CTGCTAAAGTTGAAGAAACTGATGTTCCTCTGCTTCTTGACCTTCAAGTTGAGGAATCAGGATT
TTTGTTTGAATCTAAAACAATACAGAGAATGGAGATGTTGATTCTGTCTACACTTAAATGGAAG
ATGAATCCAGTAACCCCATTTTCATTTCTTGATTTTATAACTAGAAGACTTGGATTGAAGCACT
GTCTATCTTTGGAATTTCTGAGGAGATGTGAGAAAGTGCTTCTTTACACAATTACTGATGATAG
ATTCATTGGTTACCTTCCTTCTGCAATGGCATCTGCCACAATGTTGCATGTTCTTGATAGGCTT
AAGCCTTGCATTGGAGAAAAGTACCAAGATCAACTTTTGGGCATTCTTGGAATTGTCAAGGAGA
AGGTGGAAGGATGTTACAGGCTAATACAAGAAGTGGCTTGCAACATTGACTTTGGTTCAAATAA
GAGAAAGTTTGGGACATTACCAGGGAGTCCAACAGGGGTTATGGATATGTCATTTAGCTCAGAT
TACTCCAATGACTCATGGTCAGTGGCTACATCAGTTACTTCATCACCTGAGCCATTGTCCAAGA
AGATTAGGGAGTCAAATGAATGACTAATTAAATGTCTTTTAAATATTTCCTTATTAGTAGACTA
GTTATTATTGTTATTATTAGATATGTATAATGTCCAATAACAGTGATCCACTTGTCTCTTAGTT
TAAGTATTAGTAATTAATTAAGTTCTTGTTTATTCACATGTTAATTTTGCTTACATGTAAAAAT
TCAAACCCCCTTTTGACAATTATCACAGCTAGCTAGCTAATGGACCATTTGTTTAATGCTGTAC
TGTCTATAGTGGGGCTATGAGATTTTGTACTAGTTAAAAGATGGTTTGGCACTTTATTCCAAGG
AAATAAAAACTTGCATGAAAAACAAAAAAAAAAAAAAAAAA

## FIGURE 5 (continued)

**SEQ ID NO 31: Lycopersicon esculentum mRNA for D-type cyclin AJ002590 deduced protein sequence**

MSHHYQEQEQLEAQKIPFLLDSLYCEENNILTEVSIETESFSSHDLLWEEEELTSLFSKETEYE
ISYNVLEKNQSFISSRRESVEWILKTTAYYSFSAQTGFLAVNYFDRFLLFSFNQSLNHKPWMNQ
LVAVTCLSLAAKVEETDVPLLLDLQVEESGFLFESKTIQRMEMLILSTLKWKMNPVTPFSFLDF
ITRRLGLKHCLSLEFLRRCEKVLLYTITDDRFIGYLPSAMASATMLHVLDRLKPCIGEKYQDQL
LGILGIVKEKVEGCYRLIQEVACNIDFGSNKRKFGTLPGSPTGVMDMSFSSDYSNDSWSVATSV
TSSPEPLSKKIRESNE

**SEQ ID NO 32: Medicago sativa cyclin X88864.1 DNA sequence**

GAATTCGGCACGAGCTCTTCTGCTACGACTACCTCTCCCTATACTCTCTACTCTTTCTAGTTCT
ACTACTTTTCTTTCTTTCTCTGTTCTCTCTCTCTTCTTCATTTCTTCACATTTTCACACACACA
GAGAAGACAGAACAAAGAGGAAAAGAGAGAGCGATGGATGTGAGACTCTTCAGTACTGTTTCCT
TCTTTTTATAATGAACAAAGGACCACACACCCTCTTCTTCACTGAAGAAGATGGCTATCCATCA
TCATCATCACAATCACCAACAACTTCAACAACACACTTCTTCTCTTTTTGATGCACTTTACTGT
GATGAAGAAGAAAAATGGGAAGATGATGATGAAGGAGAAGTTGTAGATGAAGGAGCACAAAGTG
ATGTCACAACAACAAACTATGATATATTGGACTCTACTTCCCTTTTACCTCTGCTTTTGTTAGA
ACAGAACTTGTTCAATGAAGATGAAGAACTCAACACTCTTTTCTCCAAAGAGATAACTCAACAA
GAAACATATTACGAGGATCTGAAAAATGTGATCAACTTTGACTCACTCTCTCAACCACGTCGTG
AAGCTGTTGAATGGATGCTTAAAGTCAATGCTCATTATGGTTTCTCTGCTCTCACTGCAACACT
TGCTGTTAACTATCTTGATAGGTTTCTTTTAAGCTTCCATTTCCAAAAAGAGAAACCATGGATG
ATTCAGCTTGTTGCTGTTACTTGCATCTCTTTAGCTGCTAAAGTTGAAGAAACTCAAGTTCCTC
TTCTCTTAGACCTTCAAGTGCAAGATACTAAATATGTGTTTGAGGCAAAGACTATTCAGAGAAT
GGAGCTATTGATTCTGTCAACACTGAAATGGAAGATGCATCCAGTGACAACACACTCTTTTCTA
GATCACATTATAAGAAGGCTTGGATTGAAAACTAATCTTCATTGGGAGTTTCTTAGGCGCTGTG
AGAATCTTCTTCTATCTGTACTTTTAGATTCAAGATTTGTTGGTTGTGTTCCTTCTGTGTTGGC
CACTGCTACAATGTTGCATGTTATAGACCAGATTGAACAGAGTGATGATAATGGTGTGGATTAC
AAAAATCAGCTTCTTAATGTTCTCAAAATCAGCAAGGAGAAAGTTGATGAATGTTATAATGCGA
TTCTTCATCTTACAAATGCAAATAATTATGGTCATAAACGAAAATATGAAGAAATCCCTGGTAG
TCCAAGTGGCGTAATTGATGCTGTTTTTAGTTCTGATGGTTCTAACGATTCGTGGACAGTGGGA
GCATCATCATATTCAACCTCAGAGCCTGTGTTTAAGAAGACCAAGAATCAAGGACAAAATATGA
ATTTGTCACCGATTAACAGGGTCATTGTCGGAATTCTTGCCACTGCAACCTCTCCTTAAAACCC
TCTATCCGTTTTCTGTCCTTTTTATTTAAAAAAAAAATAACCATATAAAAAATTACCCCCAAAAA
AAAGATCTATATTTATTTACTATGGTTATGTTCATGTTGCTACTAAACTCTAGTTAGTTTAGTA
GTCTTTCTTTCTATCTCTTCATTTCCAACAATGTCCCAAATTCATTTACATGAATCTCTTGAAG
AGGCAGTGGCAAGATGATGATAGAGGATTAAAGGAATGGTTAATTTCTGATGAGTTAAAAAGGA
AAGGACAAAGTTGGCAATGAAGATTTTTATTACTATGAGCAGAAAAGAACCCTATGATATCTGT
TTCATTTCAAGGCACTGTTTTTTTATTTTATTCAATGGTTCTCTTCTAGACCATACCCAATTTG
GACATATTTATATCATATTTCTATAATAAATTGGGAATAATTTTTGGTCCAAAAAAAAAAAAAA
AAAAA

**FIGURE 5 (continued)**

**SEQ ID NO 33: Medicago sativa cyclin X88864.1 deduced protein sequence**

MAIHHHHHNHQQLQQHTSSLFDALYCDEEEKWEDDDEGEVVDEGAQSDVTTTNYDILDSTSLLP
LLLLEQNLFNEDEELNTLFSKEITQQETYYEDLKNVINFDSLSQPRREAVEWMLKVNAHYGFSA
LTATLAVNYLDRFLLSFHFQKEKPWMIQLVAVTCISLAAKVEETQVPLLLDLQVQDTKYVFEAK
TIQRMELLILSTLKWKMHPVTTHSFLDHIIRRLGLKTNLHWEFLRRCENLLLSVLLDSRFVGCV
PSVLATATMLHVIDQIEQSDDNGVDYKNQLLNVLKISKEKVDECYNAILHLTNANNYGHKRKYE
EIPGSPSGVIDAVFSSDGSNDSWTVGASSYSTSEPVFKKTKNQGQNMNLSPINRVIVGILATAT
SP

**SEQ ID NO 34: Nicotiana tabacum cyclin D3.1 (CycD3.1) AJ011893.1 DNA sequence**

AAACGAGTCTCTGTGTACTCCTCCTCCTATAGCTTTTCTCTCTTCTTCTCTTCACACCTCCCAC
AACACACAATCAGACAAAATAGAGAGGAAAATGAGTATGGTGAAAAAGCTTTGTTTTGTATAAT
GAGAAAAAGAGATTTATATACATCTCTTCTTCTACTTCCTTCTTACTAGAAGATGGCAATAGAA
CACAATGAGCAACAAGAACTATCTCAATCTTTTCTTTTAGATGCTCTTTACTGTGAAGAAGAAG
AAGAAAAAATGGGGAGATTTAGTAGATGATGAGACTATTATTACACCACTCTCTTCAGAAGTAAC
AACAACAACAACAACAACAACAAAGCCTAATTCTTTATTACCTTTGCTTTTGTTGGAACAAGAT
TTATTTTGGGAAGATGAAGAGCTTCTTTCACTTTTCTCTAAAGAAAAAGAAACCCATTGTTGGT
TTAACAGTTTTCAAGATGACTCTTTACTCTGTTCTGCCCGTGTTGATTCTGTGGAATGGATTTT
AAAAGTGAATGGTTATTATGGTTTCTCTGCTTTGACTGCCGTTTTAGCCATAAATTACTTTGAC
AGGTTTCTGACTAGTCTTCATTATCAGAAAGATAAACCTTGGATGATTCAACTTGCTGCTGTTA
CTTGTCTTTCTTTAGCTGCTAAAGTTGAAGAAACTCAAGTTCCTCTTCTTTTAGATTTTCAAGT
GGAGGATGCTAAATATGTGTTTGAGGCAAAAACTATTCAAAGAATGGAGCTTTTAGTGTTGTCT
TCACTAAAATGGAGGATGAATCCAGTGACCCCACTTTCATTTCTTGATCATATTATAAGGAGGC
TTGGGCTAAGAAATAATATTCACTGGGAATTTCTTAGAAGATGTGAAAATCTCCTCCTCTCTAT
TATGGCTGATTGTAGATTCGTACGTTATATGCCGTCTGTATTGGCCACTGCAATTATGCTTCAC
GTTATTCATCAAGTTGAGCCTTGTAATTCTGTTGACTACCAAAATCAACTTCTTGGGGTTCTCA
AAATTAACAAGGAGAAAGTGAATAATTGCTTTGAACTCATATCAGAAGTGTGTTCTAAGCCCAT
TTCACACAAACGCAAATATGAGAATCCTAGTCATAGCCCAAGTGGTGTAATTGATCCAATTTAC
AGTTCAGAAAGTTCAAATGATTCATGGGATTTGGAGTCAACATCTTCATATTTTCCTGTTTTCA
AGAAAAGCAGAGTACAAGAACAGCAAATGAAATTGGCATCTTCAATTAGCAGAGTTTTTGTGGA
AGCTGTTGGTAGTCCTCATTAAAATCAATCACCTGATTTATCTCTTTTCTTTCTTATTACCAAC
TATGGTGGTAATAATATTTATTGATATTCAGAAGTATTTACCTTTAATGTCATTTTCAAAAATT
ACATGAAAATGGAAAAAAAGAAAAGAAGAGCTTAGCTGGTGGTTGCAGTTGGCAGAGAAGAGGA
CTGGCTTTTTTTTGCAGGAGTGTAGTCTACTACTACTGGAAAGCAGAGATAGAGAGAGGAGAAA
AGACAGAAAATCTGCACTATTTGTTTTTTCTCTATTCATATCAATTCTCTCTTAGGTCCTTTTC
ATGCATGCATACTTTTGATGGACATATTTTATATATTTACTATAATCATAAATTCTTGAATAAA
AAAAAAAAAAAAAAA

**FIGURE 5 (continued)**

**SEQ ID NO 35: Nicotiana tabacum cyclin D3.1 (CycD3.1) AJ011893.1 deduced protein sequence**

MAIEHNEQQELSQSFLLDALYCEEEEEKWGDLVDDETIITPLSSEVTTTTTTTTKPNSLLPLLL
LEQDLFWEDEELLSLFSKEKETHCWFNSFQDDSLLCSARVDSVEWILKVNGYYGFSALTAVLAI
NYFDRFLTSLHYQKDKPWMIQLAAVTCLSLAAKVEETQVPLLLDFQVEDAKYVFEAKTIQRMEL
LVLSSLKWRMNPVTPLSFLDHIIRRLGLRNNIHWEFLRRCENLLLSIMADCRFVRYMPSVLATA
IMLHVIHQVEPCNSVDYQNQLLGVLKINKEKVNNCFELISEVCSKPISHKRKYENPSHSPSGVI
DPIYSSESSNDSWDLESTSSYFPVFKKSRVQEQQMKLASSISRVFVEAVGSPH

**SEQ ID NO 36: Nicotiana tabacum cyclin D3.2 (CycD3.2) AJ011894.1 DNA sequence**

CACCTTTACTCTCTTCTCCTTTTTGGCTCTTCCCATTCTCTCCTTCTCTTTCTTTATTTTCTGT
CCTGTAGAGAGAGAGAGAAAGTATAAGCAAAGCAGCAGATATGTTACTGGGTCCAAGATTGAGT
TTTGGCTTACCTTGAAGATAATGAGTAGAGCCTCCATTGTCTTCTTCCGTCAAGAAGAAGAAGA
AGAAGATGGTTTTCCCTTTAGATACTCAGCTCCTAAATCCAATCTTTGATGTCCTTTACTGTGA
GGAAGATCGATTCTTGGACGATGATGATTTAGGAGAATGGTCTAGTACTTTAGAACAAGTAGGA
AATAATGTGAAAAAGACTCTACCTTTATTAGAATGTGACATGTTTTGGGAAGATGACCAGCTTG
TCACTCTTTTAACTAAGGAAAAAGAGTCTCATTTGGGTTTTGATTGTTTAATCTCAGATGGAGA
TGGGTTTTTAGTGGAGGTTAGAAAAGAGGCATTGGATTGGATGTTGAGAGTCATTGCTCACTAT
GGTTTCACTGCTATGACTGCTGTTTTAGCTGTGAATTATTTTGATAGGTTTGTATCTGGACTCT
GCTTTCAGAAAGATAAGCCTTGGATGAGTCAACTTGCTGCTGTGGCTTGTCTTTCTATTGCTGC
TAAAGTGGAAGAGACCCAAGTCCCCCTTCTCTTAGACCTCCAAGTGGCTGATTCAAGATTTGTG
TTTGAGGCAAAGACTATTCAGAGAATGGAACTCTTGGTGCTCTCCACTCTTAAGTGGAAAATGA
ATCCAGTGACACCACTATCTTTCATTGATCATATCATGAGGAGATTTGGATTCATGACCAATCT
ACATTTGGATTTTCTTAGGAGATGTGAACGCCTCATTCTTGGTATTATCACTGATTCTAGGCTC
TTGCATTATCCTCCATCTGTTATTGCAACTGCAGTAGTGTATTTCGTGATCAATGAGATTGAGC
CTTGCAATGCAATGGAATACCAGAATCAGCTCATGACTGTTCTTAAAGTCAAACAGGATAGTTT
TGAAGAATGCCATGATCTTATTCTAGAGCTAATGGGCACTTCTGGCTACAATATCTGCCAAAGC
CTCAAGCGCAAACATCAATCTGTACCTGGCAGTCCAAGTGGAGTTATCGATGCATATTTTAGTT
GCGACAGCTCTAATGATTCGTGGTCGGTAGCATCTTCAATTTCATCGTCACCAGAACCTCAGTA
TAAGAGGATCAAAACTCAGGATCAGACAATGACACTGGCTCCACTGAGTTCTGTTTCTGTCGTT
GTGGGCAGTAGTCCTCGTTGATCAGTATCTCATTCTCTAGATTATCTAGTATTACGGCTATGGT
TACTATATGATCTCTCTTTTTTGGTATGTTCTCTTAAACTGCAGTTGCACAATGCTCTGATGTT
CCATTAAAAAAAAAAAAAAAAA

**SEQ ID NO 37: Nicotiana tabacum cyclin D3.2 (CycD3.2) AJ011894.1 deduced protein sequence**

MVFPLDTQLLNPIFDVLYCEEDRFLDDDDLGEWSSTLEQVGNNVKKTLPLLECDMFWEDDQLVT
LLTKEKESHLGFDCLISDGDGFLVEVRKEALDWMLRVIAHYGFTAMTAVLAVNYFDRFVSGLCF
QKDKPWMSQLAAVACLSIAAKVEETQVPLLLDLQVADSRFVFEAKTIQRMELLVLSTLKWKMNP
VTPLSFIDHIMRRFGFMTNLHLDFLRRCERLILGIITDSRLLHYPPSVIATAVVYFVINEIEPC
NAMEYQNQLMTVLKVKQDSFEECHDLILELMGTSGYNICQSLKRKHQSVPGSPSGVIDAYFSCD
SSNDSWSVASSISSSPEPQYKRIKTQDQTMTLAPLSSVSVVVGSSPR

## FIGURE 5 (continued)

**SEQ ID NO 38: Nicotiana tabacum NtcycD3-3 AB015222 DNA sequence**

GCACGAGCTTCCTTCACACAACCAGATAGAGACAAGAACAGAGAGATTCATGGAAATGCCCAAG
AAGAATCACTCTTTTGGTTTCTTGTTTTATATAATGAGAAAGCAACACATTTTCTTCTTTTCTC
AAAGAAGATGGGAATACAACACAATGAGCATAATCAAGACCAAACCCAATCTTTCCTTTTAGAT
GCTCTTTACTGTGAAGAAGAAAGATGGGAAGAAACAATTGAAGATGAGATTTTAGAAAAAGAAG
CAACACTACCACTTCCTCTGCCTTTACTAGAACAAGACTTGTTTTGGGAAGATGAAGAGCTACT
CTCTCTTTTCACAAAAGAAAAAGAAACAATTTCCAACTTTGAAACTATTAAAACAGACCCTTTA
CTTTGTTTATCTCGTAAAGAAGCTGTGAAATGGATTCTTAAAGTAAATGCTCATTATGGATTCT
CAACATTCACTGCTATTCTTGCTATTAATTACTTTGATAGGTTTCTTTCAAGTCTTCATTTTCA
GAAAGATAAGCCTTGGATGATTCAACTTGTAGCTGTTACTTGTCTTTCTTTGGCTGCTAAAGTT
GAAGAAACTCAAGTTCCTCTTCTTTTGGACTTCCAAGTGGAGGATGCAAAATATGTGTTTGAGG
CCAAAACTATTCAAAGAATGGAGCTTTTGGTATTGTCCTCTTTAAAGTGGAGGATGAATCCTGT
AACCCCACTTTCATTTGTTGATCATATAATAAGAAGACTTGGGCTAAAGAGCCATATACACTGG
GAATTTCTCAAGCAGTGTGAGAGAATTCTTCTTTTGGTCATAGCTGATTGTAGATTCTTAAGTT
ATATGCCTTCTGTATTGGCTACTGCTACTATGCTTCACGTTATTCATCAAGTTGAGCCTTGTAA
TGCTGCTGACTACCAAAATCAACTTCTTGAGGTTCTCAACATTAGCAAGGAGAAGGTGAATGAT
TGCTATGAACTTATAACAGAGGTGTCTTACAACTCTATTTCACACAAGCGCAAGTATGAGAGTC
CAATAAATAGCCCAAGTGCTGTTATTGATACATTTTACAGCTCTGAAAACTCAAATGAATCATG
GGATTTGCAAACTTCTTCCTCTATTCCATCCACTTATTCACCTCGTGATCAATTTTTGCCTTTG
TTTAAGAAAAGCAGAGTTCAAGAACAGCAAATGAGATTGACATCTTTAAGCAGAGTTTTTGTGG
ATTATGCTGTTGGCAGCCCTCGCTAATATTATTGACAATGACAATGTATTACTCCTTATTATGT
CCTTTTTGCAAATTCTACTTTGGAAAGATGGAAAATGAAAAGAGGGGATAGTTGGTGGTAGTGG
CTGCAGAGGGTTGGTGTTTGCAGCACAATGGGGGGGTTAAGAGAAAGAAGGATAGGTAAATATT
CAATGTTCTACTGGGAGTAATCGTTAATTCAAGGAACATTTAGTTAATTTTGGTTTAAGATCTC
TTTGGTTGGTCCCCC

**SEQ ID NO 39: Nicotiana tabacum NtcycD3-3 AB015222 deduced protein sequence**

MGIQHNEHNQDQTQSFLLDALYCEEERWEETIEDEILEKEATLPLPLPLLEQDLFWEDEELLSL
FTKEKETISNFETIKTDPLLCLSRKEAVKWILKVNAHYGFSTFTAILAINYFDRFLSSLHFQKD
KPWMIQLVAVTCLSLAAKVEETQVPLLLDFQVEDAKYVFEAKTIQRMELLVLSSLKWRMNPVTP
LSFVDHIIRRLGLKSHIHWEFLKQCERILLLVIADCRFLSYMPSVLATATMLHVIHQVEPCNAA
DYQNQLLEVLNISKEKVNDCYELITEVSYNSISHKRKYESPINSPSAVIDTFYSSENSNESWDL
QTSSSIPSTYSPRDQFLPLFKKSRVQEQQMRLTSLSRVFVDYAVGSPR

**SEQ ID NO 40: Oryza sativa (japonica cultivar-group) cycD3 like AK103499 DNA sequence**

GTCTCTCTCCCTCCACCTCCGCTCCTACTCTGCTGCTCCACCACGACCAAAAGCCATGCCTATG
CTGCTGCTGCCGCTCTCCTCCTCTTCCGCCATTGCCGCCACCTGAGCTGAGCTCTTGTACTCGC
TACGCTACTCAAGATGGCTTTCGCCACGCTCTTTGACTCCCTCTACTGCCCCGAGGAGCACCTC
GACCTCTTCCATGACACCGCCGCCGACGACGACCTCCACCTCGACCTTCACCTGCACCAACCCC
CACCGCCGCCGCCGCTCCTCGACGACGACCTGCCTGCGCTGTTCCACGCGCTCAGGGGGAAGGA
GGACCCGCTGCGCCCCGCCGCCGACGACGACGGCTACGGCGGGGTGTCTGCCCGGGAGGCGGCG
GTCGGGTGGGCGCTGCGCGCCGTCGCGAGGCTCGGCTTCTCCGCGCTCACGGCCGCGCTCGCCG

**FIGURE 5 (continued)**

TCGCCTACCTCGACCGCTGCTTCCTCGGCGGCGCGCTCCGCCTCGGCGACCGCCCCTGGATGGC
GCGCCTCGCCGCCGTCGCCTGCGTCGCGCTCGCCGCCAAGGTGGAGGAGACGCGCGTGCCCGTG
CTCCTCGACCTCCAGCTCTGCGCCGCCGAACGCGCCGACCCCAACGAGGCCTACGTGTTCGAGG
ACAAGACGGTGCGCCGCATGGAGCTGCTGGTGCTCTCGGCGCTCGGATGGCGGATGCACCCCGT
GACGCCCCTCTCCTACCTCCAACCCCTCCTCGGCACCGCCCACGCCGCGCGCCTTCACCACTGC
GACACCGCATTGCTCGCGCTGATGCCCGATTGGAGGTGGCCTCGCCACCGCCCTTCGGCGTGGG
CCGCCGCGGCGTTGCTCGCGACGGCCGGATGGTGCGGCGGCGGCGGCGGCGACGACGCCGAGCT
CCTAGCCCTCATCGATGCCCCCAAGGATGAGATGGCAGAGTGCGCCAAGATCATCTCCGAGGAG
GCGGCGGCGGCGGCGGCGGGGGGCATTGTGATCGGCGGCGAAAATAAGCGCAAGGGCGCGGCGG
GGCTGTACTCGGCGCCGGCGAGCCCGAGCGGCGTGATCGGCGCGTCGGCCTGCTTCAGCTGCGA
CAGCTCGTCCAGCAGCGTCGACTCGCTGTTCGCCGCCTTGGAGCCACCCGGCCGGCCGATCAAG
CGAGGTGCCGCCGCCGCCACCACCGCGGATCCGCTTCCCGCCGACGAGGAGAGCCGCGACGCCT
GGCCGCCGTACGCCGCATGAGGCTGCTCTGGTCGGTATCGGGAAGGGGAGAAGCTAAAGCAAGC
CACTAATTAAGCTTAGGAGGAGGAGGGTTCAGTCCAAGAAAAAGATGTTCCATTAACGCAATGC
AAGAATGCAGGTGCGAAGGGGTAGGTTCATTCATGTAGCCATTGATGGTCTCTTGGAGCTCAGC
TCAGCCTCAACCTCAACCCACCATAGCCATGGATGGAGGTGAGGAAGAAGCTGTTTTGTCCTGT
CTGCTCACTCTGCTGGTACCACCACTGTTAGAACCATGCTTCACACTCTCTCTCTTGTTTCTCT
CTCTAGAGAGAAGAGAGAGAGAGTATAAAGGAGTAGGAGTATGAGGAGTGGCAACAATGGCAATGG
CATCAAATGCTGCAATTGCCCACTTCCCATGAGCAGCTAGAGCTACAACAGCAGACCTGAGGCT
ATTGTGCTCACCTCTTCTTCTTCCACCATTAATGAAAATGCATTACTACC

**SEQ ID NO 41: Oryza sativa (japonica cultivar-group) cycD3 like AK103499 deduced protein sequence**

MAFATLFDSLYCPEEHLDLFHDTAADDDLHLDLHLHQPPPPPPLLDDDLPALFHALRGKEDPLR
PAADDDGYGGVSAREAAVGWALRAVARLGFSALTAALAVAYLDRCFLGGALRLGDRPWMARLAA
VACVALAAKVEETRVPVLLDLQLCAAERADPNEAYVFEDKTVRRMELLVLSALGWRMHPVTPLS
YLQPLLGTAHAARLHHCDTALLALMPDWRWPRHRPSAWAAAALLATAGWCGGGGDDAELLALI
DAPKDEMAECAKIISEEAAAAAAGGIVIGGENKRKGAAGLYSAPASPSGVIGASACFSCDSSSS
SVDSLFAALEPPGRPIKRGAAAATTADPLPADEESRDAWPPYAA

**SEQ ID NO 42: Pisum sativum cyclin D AB008188.1 DNA sequence**

GTGGCGGCCGCTCTAGAACTAGTGGATCAGAACACAAAAACAAAGAGAAAAAAAAAGATGAATA
TGAAACTCTTGATTACTCTCCCCTTCTTTCTATAATGAACAAAGGACCACACAACCTCTTCTTC
ATTGAAGAAGATGGCAATCCATCATCACCACCACCATCATCAACAACTACACCACAACTCTCTT
CTTGATGCTCTTTACTGCGATGAAGAAAAACTCGAAGAAGAACAAGAAGACGTTTCATCTCAAC
AAAGTGATGTCACAACAAACAATGACAACAACATCCTAGACTCCACTTCCCTGTTCCCTCTTCT
TCTCCTGGAACAAAACCTCTTCTCTCAAGATGAAGAACTCACCACACTTTTCTCCAAAGAAAAA
ACCCAACAAGAAACGTACTACGAGGATCTGAAAAATGTCGTGGATTTTGTTTCTCTCTCTCAAC
CTCGTCGTGAAGCTGTTCAATGGATGCTTAAAGTCAATGCTCATTACGCCTTTTCACCTCTCAC
TGCAACACTCGCTGTTACTTACTTTGATAGGTTCCTTCTAACCTTCCATTTCCAAAAAGATAAG
CCATGGATGATTCAGCTTGTTGCTGTTACTTGCATCTCTTTAGCTGCTAAAGTTGAAGAAACTC
AAGTTCCTCTCCTCTTAGACCTACAAGTGCAAGATACTAAATATGTGTTTGAAGCAAAAACTAT
TCAGAGAATGGAGCTTTTGATTCTGTCAACACTGAAATGGAAGATGCATCCTGTGACACCACAC
TCTTTTCTAGATCATATAATAACAAGGCTTGGTTTGAAAACTAATCTTCATTGGGAGTTTTTAA
GACGCTGTGAGAATCTTCTTCTATCTGTACTTTTAGATTCAAGATTTGTTGGTTGTGTTCCCTC

**FIGURE 5 (continued)**

TGTGTTGGCTACTGCTACAATGCTGCATGTGATAGACCAGATTGAAGAGAGTGATGATAATGGT
GTGGACTACAAAAATCAGCTTCTTAGTATTCTCAAAATCAACAAGGAGAAAGTGGATGAATGTT
ATAATGCTATTGTTGAGGTTACTAATGAAAATAATTATGGTCATAAACGAAAATATGAACAAAT
CCCTGGAAGTCCAAGTGGCGTAATTGATGCTGTTTTAGTTCTGATGGTTCCAATGATTCATGG
AAAGTGGGTTCATCCTCGTATTCAACCTCAGAGCCTGTTTTAAGAAAACAAAAACTCAAGGGC
AAAATAGGAATTTGTCACCTCTTAATAGGGTCATTGTTGGAATTCTTGCCACTGCTAGTGCTAC
TACCTCTCCTTAATATCCTCTCTCTGTCCTTTATAAAAAAAACAAATAATAACCATATGCAAAA
AATCTATATTTATTTAGTATATATGGTTATGTTGATGTTTCTAAGATCTCTTCATTTCCAACAA
TGTCCCTAATGAATCTCTTGAAGAGGCAGAGACAGAGAAAGAGGCAAGATGAAAGAGGAGTGAA
AGAGGAGAGGACAAAGTTGGCAATGAAGATTATGTTTTTTTTTTAATTTGAAGGCACTTTTTA
TTTACTATTGAATGGTTGTTCTCTTCTAGACCATACCAAATTTGAACATATTTATATGATATTT
CTATAATAAATTGGGATAATTTTTGTTC

**SEQ ID NO 43: Pisum sativum cyclin D AB008188.1 deduced protein sequence**

MAIHHHHHHHQQLHHNSLLDALYCDEEKLEEEQEDVSSQQSDVTTNNDNNILDSTSLFPLLLLE
QNLFSQDEELTTLFSKEKTQQETYYEDLKNVVDFVSLSQPRREAVQWMLKVNAHYAFSPLTATL
AVTYFDRFLLTFHFQKDKPWMIQLVAVTCISLAAKVEETQVPLLLDLQVQDTKYVFEAKTIQRM
ELLILSTLKWKMHPVTPHSFLDHIITRLGLKTNLHWEFLRRCENLLLSVLLDSRFVGCVPSVLA
TATMLHVIDQIEESDDNGVDYKNQLLSILKINKEKVDECYNAIVEVTNENNYGHKRKYEQIPGS
PSGVIDAVFSSDGSNDSWKVGSSSYSTSEPVFKKTKTQGQNRNLSPLNRVIVGILATASATTSP

**SEQ ID NO 44: Populus alba cyclin D AY230139.1 DNA sequence**

GAGAAAGATATGCAATATATGCAAAGAGAAGCCTGAGAGATTTTTATCTCCCTTTTTCATTTTT
GATGGTGTTCTATAATGAGAAGAGGATTAAATCTCTTCTTCTCTGAAGAAGATGTCTTTCTTAC
AACAACAAGAGACTCATAATCAAAGCCCAGCATTGGCTCTTGACGGGCTTTACTGTGAAGAGGA
TGGATTTGGAGAGGATTATTCTTGTGGTTTGGATGATGAAACTAGCCAGGTTTATGATCAAAAT
GTGAAAAAGGAGCAAAATTTATCTTCTGTTTTGCTTGAGCAAGACTTGTTTTGGGAAGATAGCG
AGTTACTGTCTTTAATCTCCAAAGAGAAAGAGACCCATGTTGTTTTTGATAGTGTAGGATCTAG
AGATGGATCTTTAATGGTGGTTCGTAGAGAGGCAGTTGAGTGGTTTTTGAGGGTAAAGGCACAT
TATGGGTTCAGTGCTTTGACTGGTGTTCTTGCTGTGAACTACTTTGATAGGTTCATTTCAAGTT
CAAGGTTTCGAAGAGATAAGCCATGGATGGGTCAACTTGCTGCTGTGGCTTGTTTGTCTCTGGC
TGCTAAAGTGGAGGAAACCCAAGTGCCTCTTCTTTTAGACTTGCAAGTGGAGGATGCAAAGTAC
GTTTTTGAAGCCAAGACCATAAAGAGAATGGAGCTGTGGGTGCTGTCAACTCTTCATTGGAGGA
TGAATCCTGTAACCTCAATTTCTTTCTTTGATCACATTATAAGGAGACTTGGATTAAAGACCCA
CATGCATTGGGAGTTTTTATGGAGGTGTGAGCGATTGCTTCTTTCTGTCATTTCTGATTCAAGG
TTCATGAGTTATCTTCCTTCTATATTAGCAACTGCGACAATGTTGCATGTTATCAAGGAGGTTG
AGCCACGTAATCAACTGCAATACCAAACTCAGCTCATGGCTGTGCTAAAAACCAATGAGGATGA
AGTGAATGAGTGTTACAGGCTCATTTTAGAGCAACCAGGCAGCCAAAACCAACGCCACAAGCGC
AAGTACCTGTCCACACCCAGCAGCCCAAATGGTGTCATCGATGCATCTTTCAGCTCTGAGAACT
CAAATGATTCGTGGGCTGTGGCATCATCAATCTCATCATCATCATCAGTGCCTCAATTCAAAAG
AAGCAGGGCCCAGGTTCAGCAGATGCGATTGCCTTCACTAAATCGTATGTGCGTGGATGTGCTT
AGCAGTCCTCATTAGTCTTTCCTTGTCTTAATGCCTCGAGCTATCATAATCTTCCTGTTTTGCG
TATTAATTGTTGTATGTGTAAGAGGTACAGACGTTTTATGAAACTGACCCATTCTTGGATGAGA
AATGAGGTATCTGCTCTGCTCTCTCTTTGTTCGAGAGAGGAATGAAGAGATTGTGGTGTTTTTC
CAATAGGTATGAACATGATGTCTGTATTTCAACCATTGCTCTATTAAATGTTACATGATTCACT
AGGAAAAAAAAAAAAAAAA

**FIGURE 5 (continued)**

**SEQ ID NO 45: Populus alba cyclin D AY230139.1 deduced protein sequence**

MSFLQQQETHNQSPALALDGLYCEEDGFGEDYSCGLDDETSQVYDQNVKKEQNLSSVLLEQDLF
WEDSELLSLISKEKETHVVFDSVGSRDGSLMVVRREAVEWFLRVKAHYGFSALTGVLAVNYFDR
FISSSRFRRDKPWMGQLAAVACLSLAAKVEETQVPLLLDLQVEDAKYVFEAKTIKRMELWVLST
LHWRMNPVTSISFFDHIIRRLGLKTHMHWEFLWRCERLLLSVISDSRFMSYLPSILATATMLHV
IKEVEPRNQLQYQTQLMAVLKTNEDEVNECYRLILEQPGSQNQRHKRKYLSTPSSPNGVIDASF
SSENSNDSWAVASSISSSSSVPQFKRSRAQVQQMRLPSLNRMCVDVLSSPH

**SEQ ID NO 46: Populus tremula x Populus tremuloides cyclin D AF181993.1 DNA sequence**

CTGGAAGAAGATGGCATCAATGTATAACCCAGAAACGAGTGCGGTACAAGACCAACAACAAAAC
CCTACATTACTTTATGATGCTCTCTATTGTTCTGAAGAGAATTGGGTGGAAGAAGTTAGAGAGG
ACTGGTTTCAAGATGAACTAGAAGGAGAGAGCTATTGTAGCAACAATAGCAATAAACTAAACAC
TTTTCCAATATTGCTAGAACAGGACTTAAGCTGGGAAGACGAGGAGCTTTCCTCTTTGTTTGCC
AAGGAGGAGCAAAATCAGCTGTGCAAAGACTTAGAAACCAACCCGTCTTTGGCTAGGGCTCGCT
GTGAGGCTGTAGAGTGGATTCTAAAGGTCAATGAACACTACTCTTTCACCGCTCTAACTGCAGT
TTTGGCAGTGAACTATCTTGATAGGTTTTTATTCAGTGTCCACCTTCAGAAAGAGAAGCCATGG
ATGGCCCAACTTGCAGCTGTGTCTTGCCTCTCACTTGCTGCCAAAGTGGAGGAGACGCAAGTGC
CCCTTCTATTGGATTTTCAGGTGGAGGACAGTAAATACGTGTTCGAGGCCAAAACTATCCAGAG
AATGGAGATCCTGGTGCTTTCTACTCTTAAATGGAAGATGAATCCAGTAACCCCAATATCGTTT
CTTGATTACATCACTAGAAGGCTTGGCCTAGAACACTATCTTTGTTTGGAATTTCTCAAGAGGT
GTGAGCGCATGGTCCTCTCTATCTTGGCAGATTCTAGGTCTATGCCTTATGTTCCTTCTGTAAT
GGCCGCTGCCACGATGCTCTATGTTATTGATAACATAGAACCCAGTCTTGCAGCAGAATACCAA
AGCCAGCTGTTGAGCATTCTTGGAATCGATAAAGACAAGGTAGAGGATTGCAGCAAGTTCTTAA
TGGAATTTGCTCTAAGAGACCATTTTAAGCTTCTCTCAAACAAACGCAAGTTTTGTTCACTTCC
AGGCAGTCCTAGCGGTGTGGTTGATGTGTCTTTTAGCTCAGACAGCTCAAATGATTCATGGTCT
GTGGCATCATCCGTGTCTTCATCACCAAAGCCTCTGTCCAAGAAGAGTAGGGCACTGCAGAGTC
TAAACAACGCAACAACTTCAGATTTTTCTCAGCATTCCTCGCCAGTGCCTTAAAATTACTGTTT
TTCCCTAATGGACCACCTCTTGTACGGTTAATAAGTCTTGTGCTTTTTCAATGTTCAAGTTAAA
TTGCCTGCCATCTCTGCTTTTCCAGCTCAGACCAATGCTTAGAACGCTGAAATATTGATTGGGT
ACTGGGAATTGGAAGCAGAGATGGTTGGCATTTTACCGGAAATGAAGAACAGAACAGAACAAAA
AAAAAGAAAAAAAAAAGAGCCGATGAGGAAGAGTTCTGGTAGTAAAAAAAAAAAAAAAAAAA

**SEQ ID NO 47: Populus tremula x Populus tremuloides cyclin D AF181993.1 deduced protein sequence**

MASMYNPETSAVQDQQQNPTLLYDALYCSEENWVEEVREDWFQDELEGESYCSNNSNKLNTFPI
LLEQDLSWEDEELSSLFAKEEQNQLCKDLETNPSLARARCEAVEWILKVNEHYSFTALTAVLAV
NYLDRFLFSVHLQKEKPWMAQLAAVSCLSLAAKVEETQVPLLLDFQVEDSKYVFEAKTIQRMEI
LVLSTLKWKMNPVTPISFLDYITRRLGLEHYLCLEFLKRCERMVLSILADSRSMPYVPSVMAAA
TMLYVIDNIEPSLAAEYQSQLLSILGIDKDKVEDCSKFLMEFALRDHFKLLSNKRKFCSLPGSP
SGVVDVSFSSDSSNDSWSVASSVSSSPKPLSKKSRALQSLNNATTSDFSQHSSPVP

## FIGURE 5 (continued)

**SEQ ID NO: 48 Arabidopsis thaliana modified Arath_cycD3;3 nucleic acid sequence**

ATGGCTTTAGAAGAGGAGGAAGAGAGTCAAAACGCACCGTTTTGTGTTCTTGATGGTCTTTTCT
GTGAGGAAGAGAGTGAGTTTCACGAACAAGTAGATTTGTGCGACGAGAGTGTTGAAAAGTTTCC
TTTTTTAAATCTGGGTTTGTCTGATCATGATATGTTGTGGGATGATGATGAGTTATCAACTTTG
ATTTCGAAACAAGAACCGTGTCTTTATGACGAAATCTTAGATGATGAGTTTCTGGTTTTGTGTC
GTGAAAAGGCTCTTGATTGGATTTTTAAAGTGAAATCTCATTATGGGTTTAATTCATTGACGGC
TCTTTTAGCTGTTAATTACTTCGATAGGTTTATTACAAGCAGGAAGTTTCAGACAGATAAGCCA
TGGATGTCTCAGCTTACTGCTTTGGCTTGTCTGTCTTTAGCTGCTAAGGTTGAAGAGATCCGTG
TTCCTTTTCTCTTAGATTTTCAAGTGGAAGAAGCAAGATATGTCTTTGAAGCTAAGACTATACA
GAGAATGGAGCTTCTTGTTCTGTCTACTCTTGACTGGAGGATGCATCCTGTGACTCCAATCTCG
TTTTTCGATCACATTATTCGACGATACAGCTTTAAATCTCATCATCAATTGGAGTTCTTGAGTA
GATGTGAATCTTTATTACTCTCCATTATTCCTGATTCGAGATTTCTGAGTTTTAGTCCTTCTGT
GTTAGCCACTGCAATAATGGTCTCTGTTATTAGAGATTTGAAGATGTGTGACGAAGCTGTATAC
CAATCTCAGCTCATGACTCTACTCAAAGTTGATTCGGAGAAGGTAAATAAATGCTATGAGTTAG
TGTTAGACCACAGTCCAAGCAAGAAAAGGATGATGAATTGGATGCAACAACCCGCTAGTCCGAT
CGGTGTGTTTGATGCGTCATTCAGTTCTGATAGCTCGAATGAGTCGTGGGTTGTGTCTGCTTCT
GCTTCAGTGTCGTCTTCACCATCTTCAGAGCCTTTGCTCAAGAGGAGAAGAGTGCAAGAGCAGC
AGATGAGGCTATCTTCAATAAACCGAATGTTTTTCGATGTGCTTATAGTAGTCCTCGCTAAACC
CAGCTTTCTTGTACAAAGTGGTGATATCACAAGCCCGGGCGGTCTTCTAGGGATAACAGGGTAA

**SEQ ID NO: 49 Arabidopsis thaliana modified Arath_cycD3;3 polypepide sequence**

MALEEEEESQNAPFCVLDGLFCEEESEFHEQVDLCDESVEKFPFLNLGLSDHDMLWDDDELSTL
ISKQEPCLYDEILDDEFLVLCREKALDWIFKVKSHYGFNSLTALLAVNYFDRFITSRKFQTDKP
WMSQLTALACLSLAAKVEEIRVPFLLDFQVEEARYVFEAKTIQRMELLVLSTLDWRMHPVTPIS
FFDHIIRRYSFKSHHQLEFLSRCESLLLSIIPDSRFLSFSPSVLATAIMVSVIRDLKMCDEAVY
QSQLMTLLKVDSEKVNKCYELVLDHSPSKKRMMNWMQQPASPIGVFDASFSSDSSNESWVVSAS
ASVSSSPSSEPLLKRRRVQEQQMRLSSINRMFFDVLIVVLAKPSFLVQSGDITSPGGLLGITG

**SEQ ID NO: 50 Aquilegia formosa x Aquilegia pubescens Aqufo_CycD3 nucleic acid sequence compiled from DT755971.1 and DT749271**

ATGGCTCTTCACCACCAATATCAACAAGAACAACAACAAGAATCAACAGATCCCCATTTCCTTG
TAGACTCACTTTTCTGCGAAGAAGAGAAATGGGTAGAAGAAGAAGAGGACTTAAATGAGAGTAG
TATAAGTATCATCAATAATAATGGAACAACAACAACAACAACAACAACAACATCATCAGTAGTT
GAACTTGTTCCACTTTTGTTGTTAGAACAAGACTTGTTTTGGGAAGATGAAGAGCTTATCTCTT
TGTTTAGAAAAGAACAAGATACCCATCTTGTTATATCTTCTCAACTTGATTCTGATCCATCTAT
TGCTATTGCTCGTCGTGGGGTTATTGATTGGATGTTAAGGGTCAATGCTCATTATGCTTTCTCT
GCTCTCACTGCAGTTCTATCTGTTAATTATCTTGATAGATTCCTTTCAAGTTTTAAGTTTCAGA
AAGATAAACCATGGATGATTCAACTTGCTGCTGTTGCTTGTTTATCTATAGCTGCTAAAGTGGA
AGAAACCCAAGTTCCTCTTCTATTAGACTTTCAAGTTGAAGAGACTATGTATGTGTTTGAAGCA
AAAACTATTCAGAGAATGGAGCTTTTGGTGCTTTCTACTCTTCATTGGAAGATGAATCCAGTAA

## FIGURE 5 (continued)

CCCCTCTTTCTTTTCTTGATCACATTATAAGAAGGCTTGGATTGAAAAACCATCTACATTGGGA
ATTTTTTAGAAGGTGTGAAGGTCTTCTTTTGTCTATAATTGCAGATTCAAGGTTTGCTTGTTTT
CTTCCATCTGTGTTGGCTACTTCAACAATGCTGCATGTTATAGACCAAGTTGAGCCTTGTAATG
CAATTGAATATCAAAACCAGCTAATGGGCATTCTTAAAATCAGCAAGGATAAAGTGGATGAATG
TTATAAACTCATACTGGAATCAACATTGAGCTTCAATAGGCATGGTTACGGCAACAAACGCAAG
TTTCAATCTATCCCAAGTAGCCCAAATGGTGTAATTGATGCATCATTCAGTTGCGAGAATTCAA
ATGATTCATGGGCATTGGCTTCATCTGTTACATCATCCCCAGAACCATTTTTCAAGAAGAGCAG
AGCTCAAGACCAACAGATGAGATTACCATCCTTCAGTAGAGTGTTTGTGGATGTTCTAAGCAGT
CCTCCTTAA

**SEQ ID NO: 51 Aquilegia formosa x Aquilegia pubescens Aqufo_CycD3 deduced polypeptide sequence**

MALHHQYQQEQQQESTDPHFLVDSLFCEEEKWVEEEEDLNESSISIINNNGTTTTTTTTTSSVV
ELVPLLLLEQDLFWEDEELISLFRKEQDTHLVISSQLDSDPSIAIARRGVIDWMLRVNAHYAFS
ALTAVLSVNYLDRFLSSFKFQKDKPWMIQLAAVACLSIAAKVEETQVPLLLDFQVEETMYVFEA
KTIQRMELLVLSTLHWKMNPVTPLSFLDHIIRRLGLKNHLHWEFFRRCEGLLLSIIADSRFACF
LPSVLATSTMLHVIDQVEPCNAIEYQNQLMGILKISKDKVDECYKLILESTLSFNRHGYGNKRK
FQSIPSSPNGVIDASFSCENSNDSWALASSVTSSPEPFFKKSRAQDQQMRLPSFSRVFVDVLSS
PP

**SEQ ID NO: 52 Camellia sinensis Camsi_CycD3 AB247282 nucleic acid sequence**

ATGGCTCAACCCCAACCCCAACCCCAACCCCAACCCCAACAACAGCAACAAAATCTTCCATTTG
TTTTAGATGCTCTGTATTGCCAAGAACAACATTTGGAGGGAGGAGAGACAGAGGACTACTTCGA
TTCAGAAGAGGAAGATTGTCATTACAGTGATAATATTGTTAGCCCTAAGCCTCCACAGCTACTG
GAACAAGACCTGTTCTGGGAAGACGAAGAGTTGACCTCTTTGCTGTCAAAAGAACAAGAAAACC
CCTTATTCCATAGTCTCGAAACAGACCCATCTTTGGGTGGGGCTCGGAGAGCCGCCGTGGAGTG
GTTGCTGAAGGTCAACGCCCACTACTCATTCTCTGCTCTCACGGCGGTCCTCGCCGTGAACTAC
CTCGATAGGTTCCTCTTCAGCTTCCATTTCCAGAGAGAGAAACCATGGATGACCCAACTTGCTG
CTGTGGCTTGTCTCTCTCTTGCTGCCAAAGTTGAGGAGACTGAAGTCCCACTCCTATTAGACCT
CCAGGTGGAAGATAGTAGGTATGTTTTTGAGGCTAAAACAATTCAGAGAATGGAGATGCTGATA
CTATCAACTCTTCAATGGAAGATGAATCCTGTGACTCCACTCTCATTTCTTGATCACATTACAA
GGAGGTTAGGTTTGAAGAACAGACTTTGTTGTGAATTTCTCAAGAGATGCGAGTCAATCCTCCT
CTGTATCATTTCTGATTCTAGGTTCATGCTTTATCTTCCCTCCGTATTATCCACTGCCACAATG
CTGCTCGTTTTTAGTAGTCTAGAGCCCTGTCTCGCAGTAGAATACCAAAACCAACTCTTGGGTA
TTCTTCAAATCGACAAGGACAAAGTGGAGGATTGCTATAAGTTAATGCTAGAATCAACATCAGG
AATTCACCAATCCAACAAACGAAAGTTCCGATCAATGCCGGGCAGCCCAAATTGTGTCACAGAT
GTTTGTTTCAGCTCCGACAGCTCGAACGACTCGTGGGCCGTGACATCCTCGGCATCTGCTTCGG
CGTCGGTCTGTTCCTCGCCGGAGCCATTGTCAAAGAAGAGCAGAGCTCAGGACCATAATGCAAC
TGCAGATATTCTGAGCTTCCATTGCTAG

**FIGURE 5 (continued)**

**SEQ ID NO: 53 Camellia sinensis Camsi_CycD3 AB247282 deduced polypeptide sequence**

MAQPQPQPQPQPQQQQQNLPFVLDALYCQEQHLEGGETEDYFDSEEEDCHYSDNIVSPKPPQLL
EQDLFWEDEELTSLLSKEQENPLFHSLETDPSLGGARRAAVEWLLKVNAHYSFSALTAVLAVNY
LDRFLFSFHFQREKPWMTQLAAVACLSLAAKVEETEVPLLLDLQVEDSRYVFEAKTIQRMEMLI
LSTLQWKMNPVTPLSFLDHITRRLGLKNRLCCEFLKRCESILLCIISDSRFMLYLPSVLSTATM
LLVFSSLEPCLAVEYQNQLLGILQIDKDKVEDCYKLMLESTSGIHQSNKRKFRSMPGSPNCVTD
VCFSSDSSNDSWAVTSSASASASVCSSPEPLSKKSRAQDHNATADILSFHC

**SEQ ID NO: 54 Camellia sinensis Camsi_CycD3;2 AB247283 nucleic acid sequence**

ATGAAGAGGATGTCTCCTTACCCAGAGCAAGACTCACATCTACAGAACCCAATGTTTGTCTTTG
ACGGTCTCTACTGTGAAGAAGAGCATTTTGAGGATGATTTGGGAGAGTATGGTTTGGAACAAGG
GAGTGACAACTGCGATGAGAATGTGAAAGGACCTTTAGTTTTCTTGGAACATGACTGGGATTGG
GATGATGATGAGCTTGTTTCTTTAATTTCCAAAGAGAAAGAGACCCATTTGGGTCTGAGTGTTT
TGAACTCAGACGAGTCCTTAATGGTGGCAAGGAGAGAATCTGTTGATTGGATTTTAAGGGTCAT
TGCTCACTATGGTTTCACTGTTTTGACCACTGTTTTAGCAGTTAACTACTTTGATAGATTCATT
TCAAGCCTTTCATTTCAGAGAGAGAAGCCATGGATGAGTCAACTTGTTGCTGTTGCTTGTCTCT
CTTTAGCTGCCAAAGTTGAGGAGACCCAAGTGCCCCTTCTCTTAGACTTCCAAGTGGAGGAATC
AAAGTTTGTGTTTGAAGCCAAGACAATCCAGAGAATGGAGCTTCTGGTGCTATCTACTCTTCAA
TGGAAGATGAATCCTGTGACCCCACTTTCATTTGTTGATCACATTGTGAGGAGGTTTGGATTTA
AGACAAATTTGCATTTGGAGTTTCTGTGGAGGTGTGAGCGCCTTCTTCTCTCTGCCATCACTGA
TTCAAGGTTTGGGTGTTATCTTCCTTCTGTATTGGCTGCTGCAACAATGTTACATGTTATCAAA
GAGGTTGAGCCTTCTAATGTATTGGACTGTCAAAATGAGCTTATGGATGTTCTCAAAATGAGCA
AGGACAAAGTAGATGATTGCTACAAACTCATCCTCGAACTGCCTGGCAACAACAGTCAAATGCA
ATGCCAAACCCACAAGCGCAAGTATCAGTCCATACCCAACAGCCCAAATGGTGTCATCGATGTG
AATTTCAGCTGCGATAGCTCGAACGATTCTTGGGCAGTGACATCCTCAGTTTCATCATCACCAG
AACCCCTGTTCAAGAAGAGCAGAGTTCATGGTCAGCAGATGAGATTGGCTCCATTAAGGCATAT
GTCTGTGGGTGTAGTTGGCAGCCCTCGTTAA

**SEQ ID NO: 55 Camellia sinensis Camsi_CycD3;2 AB247283 deduced polypeptide sequence**

MKRMSPYPEQDSHLQNPMFVFDGLYCEEEHFEDDLGEYGLEQGSDNCDENVKGPLVFLEHDWDW
DDDELVSLISKEKETHLGLSVLNSDESLMVARRESVDWILRVIAHYGFTVLTTVLAVNYFDRFI
SSLSFQREKPWMSQLVAVACLSLAAKVEETQVPLLLDFQVEESKFVFEAKTIQRMELLVLSTLQ
WKMNPVTPLSFVDHIVRRFGFKTNLHLEFLWRCERLLLSAITDSRFGCYLPSVLAAATMLHVIK
EVEPSNVLDCQNELMDVLKMSKDKVDDCYKLILELPGNNSQMQCQTHKRKYQSIPNSPNGVIDV
NFSCDSSNDSWAVTSSVSSSPEPLFKKSRVHGQQMRLAPLRHMSVGVVGSPR

**FIGURE 5 (continued)**

**SEQ ID NO: 56 Citrus sinensis Citsi_CycD3 nucleic acid sequence compiled from CX676162 and CX676163**

ATGGCATTTGGAGATGAACAATACCCTTCTTCATTCTTGCTTGATGCACTCTATTGTGAAGAAG
AAGAGTTAGAAGATGAGGTTATTGACCAAGAAGATGATGAATGTAGCCAAAACAAAAACCCAGC
TTGTTTGTTTTCACTTCTTTTGTTAGAACAAGACTTGTTCTGGGAAGATGAAGAGCTCTTGTCT
CTCTTCTCCAAAGAAGAGCAACAGCTTTTAAAGCAAGAAACACAAACCCATTATAAAGATTCCG
ATGTTCTTGTTGTTGCTAGGAGTGAGGCTGTTGAGTGGGTGCTCAAAGTTAATGCTCATTATGG
GTTCTCTACTCTCACTGCAATACTGGCTATTAACTATCTGGATAGGTTCCTCCGTAGCTTCCAT
TTTCAAATAGATAAGCCTTGGATGATTCAGCTTTTGGCTGTCACTTGTCTCTCCCTGGCTGCTA
AAGTTGAAGAAACCCAAGTGCCCCTTCTCTTAGACCTTCAAGTTGAGGGGGCAAAATATGTTTT
TGAGACCAAAGCCATACAAAGAATGGAGCTTTTGGTGCTCTCAACACTTGAATGGAAGATGCAT
CCAGTGACTCCAATTTCATTTCTTGACCACATCATAAGAAGGCTTGGATTGAAGACATCTCTTC
ACTGGGAGTTTCTCAAGAGATGTGAGCGTCTGCTTCTCACTTTGGTCTCTGATTCAAGATCTGT
AAGTTACCTTCCTTCAGTGTTGGCCACTGCCACAATGATGCACATAATAGACCAAGTTGAGCCT
GTGAATCCCGTTGATTATCAAAACCAGCTTCTAGGTGTGCTTAAAATAAGCAAGGAAAAAGTAA
GTGACTGTTACAAGTTGATTCTTGAGCTGGCTAATGCAAAAACCAATGCTAATAGTAATCCTCA
CAAGCGCAAGTTTGAAGCAATCCCTGGAAGCCCTGGTGGCGTGATTGATGCTACTGTGTTTAGC
TGTGATGAAAGCTCAAACGATTCATGGTCAGTGGCATCATCATCAGTCCTATCATCACCATCGT
CACCAGAGCCTCTCTTCAAAAAGAGCAGAGTCCAAGACCCACAAATGACTTTGCCAATGCCATC
TCTCAATCTCAATAGGGTCATTGTGGGCAGTCCAAGTTGA

**SEQ ID NO: 57 Citrus sinensis Citsi_CycD3 deduced polypeptide sequence**

MAFGDEQYPSSFLLDALYCEEEELEDEVIDQEDDECSQNKNPACLFSLLLLEQDLFWEDEELLS
LFSKEEQQLLKQETQTHYKDSDVLVVARSEAVEWVLKVNAHYGFSTLTAILAINYLDRFLRSFH
FQIDKPWMIQLLAVTCLSLAAKVEETQVPLLLDLQVEGAKYVFETKAIQRMELLVLSTLEWKMH
PVTPISFLDHIIRRLGLKTSLHWEFLKRCERLLLTLVSDSRSVSYLPSVLATATMMHIIDQVEP
VNPVDYQNQLLGVLKISKEKVSDCYKLILELANAKTNANSNPHKRKFEAIPGSPGGVIDATVFS
CDESSNDSWSVASSSVLSSPSSPEPLFKKSRVQDPQMTLPMPSLNLNRVIVGSPS

**SEQ ID NO: 58 Glycine max Glyma_CycD3 nucleic acid sequence AY439098**

ATGGCAATTCAGCACCACAATGAACAACTAGAGCATAATGAAAATGTCTCATCTGTCCTTGATG
CCCTTTACTGCGATGAAGGAAAGTGGGAGGATGAAGAGGAGGAGGAAGAAGANTATGAAGAAAG
TGAAGTAACAACAAACACTGGAACTTCTCTTTTCCCTCTGCTCATGTTGGAGCAAGACTTGTTC
TGGGAAGATGAGGAACTAAACTCTCTCTTTTCCAAAGAGAAGGTTCAACATGAAGAAGCCTATG
ACTATAACAATCTGAACAGTGATGATAATAGCAATGATCACAGTAATAATAACAATAATGTGCT
GTCGGACTCTTGTCTCTCTCAGCCTCGTCGTGAGGCAGTGGAATGGATACTGAAAGTCAATGCT
CACTATGGATTCTCTGCTCTCACTGCAACACTGGCCGTTACTTACCTGGATAGGTTCCTTCTAA
GCTTCCATTTTCAAAGGGAGAAGCCATGGATGATCCAGCTTGTGGCTGTCACTTGCATCTCTTT
GGCTGCAAAAGTTGAAGAAACTCAAGTGCCTCTTCTCTTGGACCTTCAAGTGCAAGACACAAAG
TATGTGTTTGAGGCAAAGACTATTCAGAGAATGGAGCTCCTGGTGCTGTCCACCCTCAAATGGA
AGATGCACCCCGTGACACCCCTCTCCTTTCTAGATCACATTATAAGAAGGCTTGGATTGAAAAC
ACATCTTCACTGGGAGTTTCTCAGGCGCTGTGAGCATCTTCTTTTGTCTGTGCTTTTAGATTCA

**FIGURE 5 (continued)**

AGATTTGTTGGTTGTCTTCCTTCTGTGTTGGCCACTGCAACAATGCTGCATGTTATAGACCAGA
TTAAACACAATGGTGGGATGGAATACAAAACTCAGCTTCTGAGTGTTCTCAAAATTAGCAAGGA
GAAAGTAGATGAGTGTTATAATGCAATTCTCCAACTCTCAAAGGCCAATAAATATGGTCATAAC
AACATCAACAACACTAGCAAGCGCAAGTATGAGCAAATCCCAAGCAGCCCAAGTGGCGTAATTG
ATGCTGCATTTTGCTCTGATGGTTCCAACGATTCGTGGGCAGTGGGGTCATCATTGTTATATTC
ACCACCAGAGCCTCTCTTCAAGAAGAGCAGAACCCAAGGACAACAAATGAATTTGTCACCACTT
AAACGGTTCATTATCGGAATTGTTGGCACCCCTCCTTAA

**SEQ ID NO: 59 Glycine max Glyma_CycD3 deduced polypeptide sequence**

MAIQHHNEQLEHNENVSSVLDALYCDEGKWEDEEEEEEXYEESEVTTNTGTSLFPLLMLEQDLF
WEDEELNSLFSKEKVQHEEAYDYNNLNSDDNSNDHSNNNNNVLSDSCLSQPRREAVEWILKVNA
HYGFSALTATLAVTYLDRFLLSFHFQREKPWMIQLVAVTCISLAAKVEETQVPLLLDLQVQDTK
YVFEAKTIQRMELLVLSTLKWKMHPVTPLSFLDHIIRRLGLKTHLHWEFLRRCEHLLLSVLLDS
RFVGCLPSVLATATMLHVIDQIKHNGGMEYKTQLLSVLKISKEKVDECYNAILQLSKANKYGHN
NINNTSKRKYEQIPSSPSGVIDAAFCSDGSNDSWAVGSSLLYSPPEPLFKKSRTQGQQMNLSPL
KRFIIGIVGTPP

**SEQ ID NO: 60 Gossypium hirsutum Goshi_CycD3 nucleic acid sequence compiled from DT571998 and DT543827.1**

ATGGCAATACAGCAATATGAACAGCAGCAACAACAACCAGAGAATCACCCTTCCTTCTTGCTAG
ATGCTCTCTACTGTGAGGAAGAAGCGGATGCAGGGGAAGTTTTAGAGGAAGAGAGTTCTTGTGT
GGGCTGTAACAATGGCGGAAACCCTTCATTTTTCCCACTGTTGTTGTTAGAGCAGGATTTGTTT
TGGGAAGACGGGGAGCTTCTTTCACTTTTTGCTAAAGAAACAGAGCAGCAGCCGTCTTGTTTCA
ATGTGGGAACCGATGAGTCCCTAGCAATGGCTCGCCGAGAGGCTGCCGAGTGGATGCTTAAAGT
CAATGCTCGATTTGGATTCTCCACTCTCACGGCTGTACTTTCCATTAACTATTTGGACAGGTTC
TTAAGTACCTTTCAGTTTCAAAGAGATAATCCTTGGATGATCCAACTTCTGGGTGTCACTTGTC
TCTCTTTGGCTGCAAAAGTTGAAGAGACACAAGTGCCTCTGCTCCTAGACCTACAAGTGGAGGA
GACAAAGTATGTTTTCGAGGCCAAAACTATCCAAAGAATGGAGCTTTTGGTGCTCTCCACACTG
AAATGGAAGATGCATCCAATTACACCCCTTTCATTTCTAGATCACATCATAAGAAGACTGGGGT
TGAAAACCCACCTCCATTGGGAGTTTCTTAAGCGATGTGAGCGTCTCCTCCTCTGTGTAATCTC
TGATGCAAGATCCATCCATTATCTTCCCTCTGTATTGGCTACTGCAACCATGATGCACGTCATA
GACCAAGTTGAGCTTTTCAATCCCATTGACTACCAAAATCAGCTGCTGAGTGTTCTTAAAATTA
GCAAGGAAAAAGTAAACGATTGTTACAAGCTCATCCTTGATGTATCAACAAGACCCCAGGCCCA
AGGCAATGGTGGTGCATGTAAGAGGAAGGTGGAGGAGAGGGTTCCTAGCAGCCCTAGTGGAGTG
ATTGATGCTGCATTTGGCAGTGATAGCTCGAGCGATTCTTGGGGCACGGTGTCCTTATCGCCTG
AGCAGCAGCCACCTTTTAAGAAGAGCAGAGCCCAAGAGCAAGTAATGCGTTTGCCATCACTCAA
CCGAGTCTTTGTAGACATTGTTGGCAGCCCTTCTTAA

FIGURE 5 (continued)

**SEQ ID NO: 61 Gossypium hirsutum Goshi_CycD3 deduced polypeptide sequence**

MAIQQYEQQQQQPENHPSFLLDALYCEEEADAGEVLEEESSCVGCNNGGNPSFFPLLLLEQDLF
WEDGELLSLFAKETEQQPSCFNVGTDESLAMARREAAEWMLKVNARFGFSTLTAVLSINYLDRF
LSTFQFQRDNPWMIQLLGVTCLSLAAKVEETQVPLLLDLQVEETKYVFEAKTIQRMELLVLSTL
KWKMHPITPLSFLDHIIRRLGLKTHLHWEFLKRCERLLLCVISDARSIHYLPSVLATATMMHVI
DQVELFNPIDYQNQLLSVLKISKEKVNDCYKLILDVSTRPQAQGNGGACKRKVEERVPSSPSGV
IDAAFGSDSSSDSWGTVSLSPEQQPPFKKSRAQEQVMRLPSLNRVFVDIVGSPS

**SEQ ID NO: 62 Lotus corniculatus Lotco_CycD3 nucleic acid sequence AP008090**

ATGGCAATCCATCAACATCATCACAACAATGTCATTGACCAGCTAGAACAAAATGAAAATGTTT
CTTCTGTCTTGGATGCTCTTTACTGTGATGAAGAAAAATGGGAGGAAGAGGAAGTAGAACAAGT
GGTTGGAGAGTTATCTGAAGAAGAAACAAGTGATGTGACAACAAACAATGACCCTAACAACACT
TGTTCTCTGTTTCCCCTGCTTTTGTTGGAGCAAGACTTGTTCTGGGAAGATGAAGAACTCAACT
CTCTCTTCTCCAAAGAGAAGATCCAACACCAAAACTATTATAATGATGTGAACTCGGACCCTTT
TCTCTCTCAGCCTCGTCATGAGGCAGTGAAATGGATGCTTAAAGTCAATGCTCATTATGGATTC
TCTGCTCTCACTGCAACACTTGCTGTTACCTACTTTGATAACTTCCTTTTGAGCTTCCATTTTC
AAAGTGAGAAGCCATGGATGATCCAGCTTGCTGCTGTTACTTGCATCTCTTTGGCAGCTAAAGT
TGAAGAAACCCAAGTGCCACTTCTCTTAGACCTTCAAGTGCAAGATGCTAAGTTTGTGTTTGAG
GCAAAGACCATTCTGAAAATGGAGCTTCTGGTTCTGTCCACACTCAAATGGAAGATGCATCCTG
TGACTCCACTTTCATTTCTGGATCACATTATCAGAAGGCTTGGATTGAAAACACACCTTCATTG
GGAGTTTCTCAGGCGCTGTGAGCATCTTCTTTTGTCTGTGCTTTTAGATTCAAGATTTGTTGGT
GTTCTTCCTTCTGTGTTGGCCACTGCAACAATGCTGCATGTTATAGACCAGATTGAGAAGAGTG
ATGGGGTGGAATACAAAAAGCAGCTTCTGGGTGTTCTCAAAATTAACAAGGGGAAAGTAGATGA
ATGCTATGATGCCATGCTTGAGCTTACAAATGCCAATGATTATGATGATAACAAGAAGCTTAAT
AAGCGCAAGTATGAGGAAATAATCCCTGGTAGCCCAAGTGGCGTCATTGATGCCGCATTTAACT
CTGATGGTTCCAACGATTCGTGGACAGTGGGGTCATCATTGTTTTCATCCTCAGGCCCAGAGTC
TCCTCTGTTCAAGAAAAGCAGAACCCAAATGAAATTGTCACCACTTAACAGGGTCATTGTTGGA
ATTGTTAGCACTTCACCTTGA

**SEQ ID NO: 63 Lotus corniculatus Lotco_CycD3 deduced polypeptide sequence**

MAIHQHHHNNVIDQLEQNENVSSVLDALYCDEEKWEEEEVEQVVGELSEEETSDVTTNNDPNNT
CSLFPLLLLEQDLFWEDEELNSLFSKEKIQHQNYYNDVNSDPFLSQPRHEAVKWMLKVNAHYGF
SALTATLAVTYFDNFLLSFHFQSEKPWMIQLAAVTCISLAAKVEETQVPLLLDLQVQDAKFVFE
AKTILKMELLVLSTLKWKMHPVTPLSFLDHIIRRLGLKTHLHWEFLRRCEHLLLSVLLDSRFVG
VLPSVLATATMLHVIDQIEKSDGVEYKKQLLGVLKINKGKVDECYDAMLELTNANDYDDNKKLN
KRKYEEIIPGSPSGVIDAAFNSDGSNDSWTVGSSLFSSSGPESPLFKKSRTQMKLSPLNRVIVG
IVSTSP

## FIGURE 5 (continued)

**SEQ ID NO: 64 Medicago truncatula Medtr_CycD3 nucleic acid sequence DY615448.1**

ATGGCTATCCATCATCATCATCACAATCACCAACAACTTCAACAACACACTTCTTCTCTTTTTG
ATGCACTTTACTGTGATGAAGAAGAAAAATGGGAAGATGATGATGAAGGAGAAGTTGTAGATGA
AGGAGCACAAAGTGATGTCACAACAACAAACTATGATATATTGGACTCTACTTCCCTTTTACCT
CTGCTTTTGTTAGAACAGAACTTGTTCAATGAAGATGAAGAACTCAACACTCTTTTCTCCAAAG
AGATAACTCAACAAGAAACATATTACGAGGATCTGAAAAATGTGATCAACTTTGACTCACTCTC
TCAACCACGTCGTGAAGCTGTTGAATGGATGCTTAAAGTCAATGCTCATTATGGTTTCTCTGCT
CTCACTGCAACACTTGCTGTTAACTATCTTGATAGGTTTCTTTTAAGCTTCCATTTCCAAAAAG
AGAAACCATGGATGATTCAGCTTGTTGCTGTTACTTGCATCTCTTTAGCTGCTAAAGTTGAAGA
AACTCAAGTTCCTCTTCTCTTAGACCTTCAAGTGCAAGATACTAAATATGTGTTTGAGGCAAAG
ACTATTCAGAGAATGGAGCTATTGATTCTGTCAACACTGAAATGGAAGATGCATCCAGTGACAA
CACACTCTTTTCTAGATCACATTATAAGAAGGCTTGGATTGAAAACTAATCTTCATTGGGAGTT
TCTTAGGCGCTGTGAGAATCTTCTTCTATCTGTACTTTTAGATTCAAGATTTGTTGGTTGTGTT
CCTTCTGTGTTGGCCACTGCTACAATGTTGCATGTTATAGACCAGATTGAACAGAGTGATGATA
ATGGTGTGGATTACAAAAATCAGCTTCTTAATGTTCTCAAAATCAGCAAGGAGAAAGTTGATGA
ATGTTATAATGCGATTCTTCATCTTACAAATGCAAATAATTATGGTCATAAACGAAAATATGAA
GAAATCCCTGGTAGTCCAAGTGGCGTAATTGATGCTGTTTTTAGTTCTGATGGTTCTAACGATT
CGTGGACAGTGGGAGCATCATCATATTCAACCTCAGAGCCTGTGTTTAAGAAGACCAAGAATCA
AGGACAAAATATGAATTTGTCACCGATTAACAGGGTCATTGTCGGAATTCTTGCCACTGCAACC
TCTCCTTAA

**SEQ ID NO: 65 Medicago truncatula Medtr_CycD3 deduced polypeptide sequence**

MAIHHHHHNHQQLQQHTSSLFDALYCDEEEKWEDDDEGEVVDEGAQSDVTTTNYDILDSTSLLP
LLLLEQNLFNEDEELNTLFSKEITQQETYYEDLKNVINFDSLSQPRREAVEWMLKVNAHYGFSA
LTATLAVNYLDRFLLSFHFQKEKPWMIQLVAVTCISLAAKVEETQVPLLLDLQVQDTKYVFEAK
TIQRMELLILSTLKWKMHPVTTHSFLDHIIRRLGLKTNLHWEFLRRCENLLLSVLLDSRFVGCV
PSVLATATMLHVIDQIEQSDDNGVDYKNQLLNVLKISKEKVDECYNAILHLTNANNYGHKRKYE
EIPGSPSGVIDAVFSSDGSNDSWTVGASSYSTSEPVFKKTKNQGQNMNLSPINRVIVGILATAT
SP

**SEQ ID NO: 66 Scutellaria baicalensis Scuba_CycD3 nucleic acid sequence AB205135.1**

ATGGTTTCGGAATTTCAGGAGCACGAATCCCTTCTCCAAAACCCTATCTTTGATGCCCTTTATT
GTGACGAGGAGCGTTTTGATGAATGTGTAGGCGGCGCTGGTTCGGGCTTCAAAGAGCCCGAAAT
CAACGATTTTAATGAGATTCACAATAACCCTTTTGCTTTTCTGTTTGAGCACGACCTTTTCTGG
GAGAGTGAGGAGCTTGACGCCCTGTTAACGAAGGAGAAAACGCAGACCCATTTGACTTTTGATG
AAATAAACTCAGATGCGTCTTTGAAGGCGATGAGAAATGAGGCGATTAACTGGATGCTGAAGGT
GATTGCCCACTACGGCTTCAATGCGCTGACTGCTGTTTTGGCTGTCAACTATTATGATAGATTC
ATCACTAGTGTTTGTTTTCAGAAGGATAAGCCATGGATGAGTCAATTAGCTGCTGTGGCTTGTC
TTTCTGTAGCTGCCAAGGTGGAGGAAACTCAAGTGCCTCTTTTGTTGGATTTACAAGTTGAAGA
ATCTAAGTATTTGTTTGAAGCTAAGACCATCCAAAGAATGGAGCTTTTGGTGCTTTCTACCCTC
CAATGGAGGATGAATCCTGTGACGCCAATCTCATTCTTTGACCACATTGCAAGGAGATTTGAGT

FIGURE 5 (continued)

TTGTAAAGAACCTACATTCTGTATTTTTAAGGAGGTGTGAGAGTTTAATCCTCTCCATTATCAC
TGATTGTAGATTGGTAAAGTATTTTCCTTCAGTTATTGCTTCTGCAGCAATGATATATGCGATT
AGAGAGTTTGAGACTCCTGATGCTCTGGAATATGAGGATCAACTCTTGAGTGTGCTAAGAACTA
GCAAGGACAAAGTTGATGATTGCCGCAAACTCATTGTGGATGCAATGTATGGTGGTTTCAGCCA
CAAGCCTTGCTATAAACGCAAATATGAGTCGATCCCAAGCAGTCCAAGTGGTGTCATTGATGCG
TATTTGAGCTCTGATAGCTCTGTTGATTCGTGGGCTGTTACATTATCAGTGTCATCGTCGCCAG
AGCCTTCGTTTAAGAGAAGCAAAGCTCAAGATCAGCACATGAGATTGGCTCCACTAAGCAGTGT
ATCTCTTGGCCTTGCTCATCGTATTAATTGA

**SEQ ID NO: 67 Scutellaria baicalensis Scuba_CycD3 deduced polypeptide sequence**

MVSEFQEHESLLQNPIFDALYCDEERFDECVGGAGSGFKEPEINDFNEIHNNPFAFLFEHDLFW
ESEELDALLTKEKTQTHLTFDEINSDASLKAMRNEAINWMLKVIAHYGFNALTAVLAVNYYDRF
ITSVCFQKDKPWMSQLAAVACLSVAAKVEETQVPLLLDLQVEESKYLFEAKTIQRMELLVLSTL
QWRMNPVTPISFFDHIARRFEFVKNLHSVFLRRCESLILSIITDCRLVKYFPSVIASAAMIYAI
REFETPDALEYEDQLLSVLRTSKDKVDDCRKLIVDAMYGGFSHKPCYKRKYESIPSSPSGVIDA
YLSSDSSVDSWAVTLSVSSSPEPSFKRSKAQDQHMRLAPLSSVSLGLAHRIN

**SEQ ID NO: 68 Zea mays Zeama_CycD3 like 2 nucleic acid sequence compiled from DV509394.1 and DV028752.1**

ATGGCAGCTTTCGCCGCGTTGTTCGACCCCCTCTACTGCCCGGAGGAGCACCTCGATCTGTACC
ACGAAGGACCCGTCGAGGTTGTGGACGAGCAGTGGCAGGACCAGCGCGGACAGCAGCAACCGGC
GGCTCTTGACGACGAGCTGCCGGCGCTGTTCGAGGCGCTCCGGGACAAGGAGGGGGTGGTGCTG
GCGGGTGATGGGGAGGAGGATGGGTACGGCGGCTCGGCAGGCCGGGAGGCCGCAGTCGGCTGGG
CGTCACGCGCCGCGGCACGGCTGGGCTTCTCTGCGCTCACTTCCGCGCTGTCCGCCGCCTACCT
GGACCGCTGCTTCCTCCCCGGGGGCGCGCTCCGTCTCGGCGACCAGCCCTGGATGTCGCGCCTC
GCCGCCGTCGCCTGTGTCGCGCTCGCCGCCAAGGTCGAGGAAACGCGCGTGCCGCTGCTCCTCG
ACCTCCAGCTCTGCGCCGCCGCCAGCTCCGACGCTGACGCAGCGGACGCGGACGTGTTCGAGGC
CAAGACGGTGCGCCGGATGGAGCTGCTCGTTCTCTCCGCGCTAGGGTGGCGGATGCACCCTGTC
ACGCCCTTCTCCTACCTCCAGCCTGTCCTCGCCGACGCTGCGATGCGCCTACGCAACTGCGAGG
CCGTCCTGCTCGCGGTCATGGCCGATTGGAGGTGGCCTCGGCACCGGCCCTCGGCGTGGGCCGC
CGCCGCATTGCTCACCACAGCCGGCGGCGGCGACGACGACTCGGAGCTGCTCGCGCTCATCAAT
GCCCCCGAGGACGAGACCGCGGAGTGCGCCAAGATCATCTCCGAGGTGACAGGCATGAGCTTCC
TTGTCTGCGACGTCGGCGGCATGATCGCCGGGAATAAGCGTAAGCACGCGGCGGCGCGGATGTA
CTCGCCGCCGCTGAGCCCGAGCGGCGTGATCGGCGCGCTGTCCTGCTTCAGCTGCGAGAGCTCG
TTGTCCGCCACAGCGGACTCGCGCACCCTCGCTACTACGGCTGCGGGGGTCGGCCCGTGGGCAC
CGTCAGCGCCCGTGTCCGTGTCGTCTTCCCCTGAGCCCCCAGGTCGGGCCCCCAAGCGCGCTGC
GGCGGCGGGGGTCCCGCATCCGCTTCCCCCCGACGAGGAGAGCCGCGACGCCTGGCCGTCCACC
TGCGCCGCGTGA

FIGURE 5 (continued)

**SEQ ID NO: 69 Zea mays Zeama_CycD3 like 2 deduced polypeptide sequence**

MAAFAALFDPLYCPEEHLDLYHEGPVEVVDEQWQDQRGQQQPAALDDELPALFEALRDKEGVVL
AGDGEEDGYGGSAGREAAVGWASRAAARLGFSALTSALSAAYLDRCFLPGGALRLGDQPWMSRL
AAVACVALAAKVEETRVPLLLDLQLCAAASSDADAADADVFEAKTVRRMELLVLSALGWRMHPV
TPFSYLQPVLADAAMRLRNCEAVLLAVMADWRWPRHRPSAWAAAALLTTAGGGDDDSELLALIN
APEDETAECAKIISEVTGMSFLVCDVGGMIAGNKRKHAAARMYSPPLSPSGVIGALSCFSCESS
LSATADSRTLATTAAGVGPWAPSAPVSVSSSPEPPGRAPKRAAAAGVPHPLPPDEESRDAWPST
CAA

**SEQ ID NO: 70 Zea mays Zeama_CycD3 like 3 nucleic acid sequence compiled from DT948601.1 and DT642394.1**

ATGGCAGCTTTCGCCGCGCTGTTCGACCCCCTGTACTGCCCGGAGGAGCACCTCGATCTGTACC
GCGACGAACCCGGCGAGGGTGCGGACGAGCAGTGGCCGGGCCAGCACGGACAGCAGGAGCCGGC
TGTCCTCGACGACGAGCTGCCGGCGCTGTTCGAGGCACACCGGGCCAAGGAGGGGGTGGTGCTG
GCGGAGGATGGCGGGTACGGCGGCGCAGCTGGGCGTGAGGCCGCGGTCGGCTGGGTTTCACGCG
CCGCGGCGCGGCTAGGCTTCTCCGCGCTCACCGCCGCGCTCGCCGCCGCCTACCTCGACCGCTG
CTTCCTCCCCGGGGGCGCGCTCCGGCTCGGCGACCAGCCCTGGATGGCGCGCCTAGCCGCCGTC
ACCTGCTTCGCGCTCGCCGCCAAGGTCGAGGAGACGCGCGTGCCGCCGCTCCTCGACCTCCAGC
TCTACGCCGCCGCTGACGCCGCGGATCCGTACGTATTCGAGGCCAAGACGGTGCGCCGGATGGA
GCTGCTCGTGCTCTCCGCGCTTGGGTGGCGGATGCACCCTGTCACGCCCTTCTCCTACCTCCAG
CCCGTCCTCGCCGACGCTGCGACGCGCCTGCGTAGCTGCGAGGGCGTCCTGCTCGCGGTCATGG
CCGACTGGAGGTGGCCTCGGCACCGGCCTTCGGCGTGGGCCGCCGCCGCGTTGCTGATCACAGC
CGCCGCCGGCGACGGCGGCGACGGCGACGGCGACACGGAGCTCCTGGCGCTCCTCATTGCCCCC
GAGGACAAGACCGCCGAGTGTGCCAAGATCATCTCCGAGGTGACGGGCATGAGCTTCCTCGCCT
GCGATGTCGGCGTGAGCGCCGGAAATAAGCGTAAGCACGCGGCGGCGCAGTTGTACTCGCCGCC
GCCGAGCCCGAGCGGCGTGATCGGCGCGCTGTCCTGCTTCAGCTGCGAGAGCTCGACGTCCGCC
ACCGCTATGGCTGCGGCGGTCGGCCCGTGGGCGCCGTCGGCGTCCGTGTCCGTGTCGTCCTCTC
CAGAGCCACCAGGTCGGGCCCCCAAGCGCGCAGCGGCGGCGTCGGCGTCGGCGTCGGCGTCAGC
CGGGGTCGCGCCACCGGTCCAGGTCCCGCATCAGCTACCCCCCGACGAGGAGAGCCGCGACGCC
TGGCCGTCCACCTGCGCCGCGTGA

**SEQ ID NO: 71 Zea mays_CycD3 like 3 deduced polypeptide sequence**

MAAFAALFDPLYCPEEHLDLYRDEPGEGADEQWPGQHGQQEPAVLDDELPALFEAHRAKEGVVL
AEDGGYGGAAGREAAVGWVSRAAARLGFSALTAALAAAYLDRCFLPGGALRLGDQPWMARLAAV
TCFALAAKVEETRVPPLLDLQLYAAADAADPYVFEAKTVRRMELLVLSALGWRMHPVTPFSYLQ
PVLADAATRLRSCEGVLLAVMADWRWPRHRPSAWAAAALLITAAAGDGGDGDGDTELLALLIAP
EDKTAECAKIISEVTGMSFLACDVGVSAGNKRKHAAAQLYSPPPSPSGVIGALSCFSCESSTSA
TAMAAAVGPWAPSASVSVSSSPEPPGRAPKRAAAASASASASAGVAPPVQVPHQLPPDEESRDA
WPSTCAA

FIGURE 5 (continued)

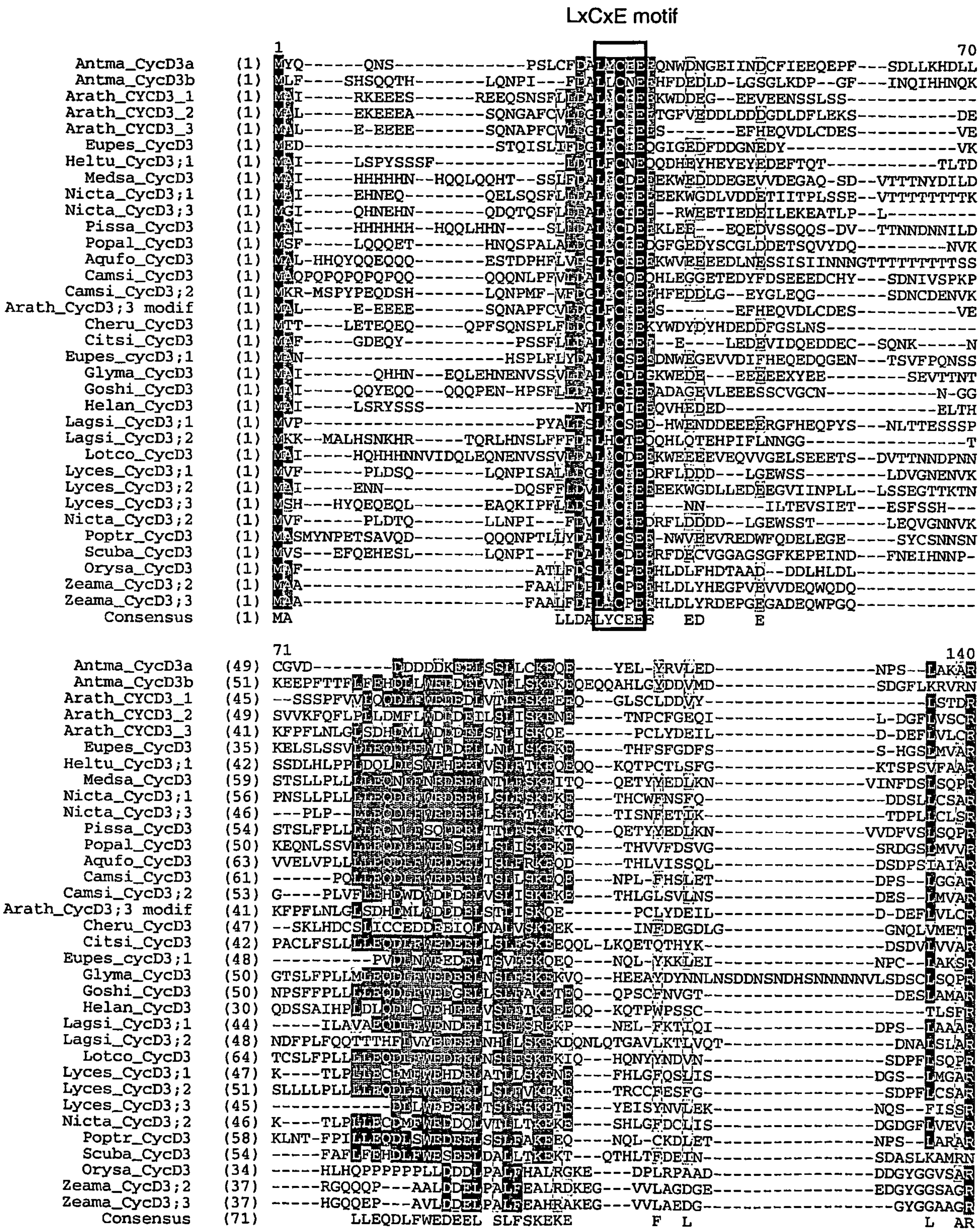
LxCxE motif
1 70
Antma_CycD3a (1) MYQ------QNS-------------PSLCFDALYCEEEQNWDNGEIINDCFIEEQEPF---SDLLKHDLL
Antma_CycD3b (1) MLF----SHSQQTH-------LQNPI---FDALLCNEEHFDEDLD-LGSGLKDP--GF---INQIHHNQK
Arath_CYCD3_1 (1) MAI-----RKEEES------REEQSNSFLLDALYCEEEKWDDEG--EEVEENSSLSS------------
Arath_CYCD3_2 (1) MAL-----EKEEEA-------SQNGAFCVLDGLYCEEETGFVEDDLDDDGDLDFLEKS----------DE
Arath_CYCD3_3 (1) MAL-----E-EEEE-------SQNAPFCVLDGLFCEEES-------EFHEQVDLCDES----------VE
Eupes_CycD3 (1) MED-------------------STQISLIFDGLYCEEQGIGEDFDDGNEDY-----------------VK
Heltu_CycD3;1 (1) MAI-------LSPYSSSF------------LDTLFCNEDQDHEYHEYEYEDEFTQT-----------TLTD
Medsa_CycD3 (1) MAI-----HHHHHN--HQQLQQHT--SSLFDALYCDEEEKWEDDDEGEVVDEGAQ-SD--VTTTNYDILD
Nicta_CycD3;1 (1) MAI-----EHNEQ--------QELSQSFLLDALYCEEEEEKWGDLVDDETIITPLSSE--VTTTTTTTTK
Nicta_CycD3;3 (1) MGI-----QHNEHN-------QDQTQSFLLDALYCEEE--RWEETIEDEILEKEATLP---L---------
Pissa_CycD3 (1) MAI-----HHHHHH--HQQLHHN----SLFDALYCDEEKLEE---EQEDVSSQQS-DV--TTNNDNNILD
Popal_CycD3 (1) MSF-----LQQQET-------HNQSPALALDGLYCEEDGFGEDYSCGLDDETSQVYDQ---------NVK
Aqufo_CycD3 (1) MAL-HHQYQQEQQQ-------ESTDPHFLVLSLFCEEEKWVEEEEDLNESSISIINNNGTTTTTTTTSS
Camsi_CycD3 (1) MAQPQPQPQPQPQQ-------QQQNLPFVLDALYCQEQHLEGGETEDYFDSEEEDCHY---SDNIVSPKP
Camsi_CycD3;2 (1) MKR-MSPYPEQDSH-------LQNPMF-VFDGLYCEEHFEDDLG--EYGLEQG-------SDNCDENVK
Arath_CycD3;3 modif (1) MAL-----E-EEEE-------SQNAPFCVLDGLFCEEES-------EFHEQVDLCDES----------VE
Cheru_CycD3 (1) MTT----LETEQEQ-----QPFSQNSPLFLDGLYCEEKYWDYDYHDEDDFGSLNS-------------
Citsi_CycD3 (1) MAF-----GDEQY-----------PSSFLLDALYCEEE--E----LEDEVIDQEDDEC--SQNK-----N
Eupes_cycD3;1 (1) MAN--------------------HSPLFLLYDALYCSEEDNWEGEVVDIFHEQEDQGEN---TSVFPQNSS
Glyma_CycD3 (1) MAI-------QHHN---EQLEHNENVSSVLDALYCDEEGKWEDE---EEEEEXYEE--------SEVTTNT
Goshi_CycD3 (1) MAI-----QQYEQQ---QQQPEN-HPSFLLDALYCEEEADAGEVLEEESSCVGCN-----------N-GG
Helan_CycD3 (1) MAI-----LSRYSSS---------------NTLFCIEEQVHEDED---------------------ELTH
Lagsi_CycD3;1 (1) MVP--------------------PYALDSLYCSEED-HWENDDEEEERGFHEQPYS---NLTTESSSP
Lagsi_CycD3;2 (1) MKK--MALHSNKHR-----TQRLHNSLFFFDHLHCTEQQHLQTEHPIFLNNGG----------------T
Lotco_CycD3 (1) MAI-----HQHHHNNVIDQLEQNENVSSVLDALYCDEEKWEEEEVEQVVGELSEEETS--DVTTNNDPNN
Lyces_CycD3;1 (1) MVF------PLDSQ-------LQNPISALLDGLYCEEDRFLDDD---LGEWSS--------LDVGNENVK
Lyces_CycD3;2 (1) MAI-----ENN-------------DQSFFLDVLYCEEEEEKWGDLLEDEEGVIINPLL--LSSEGTTKTN
Lyces_CycD3;3 (1) MSH---HYQEQEQL-------EAQKIPFLLDSLYCEE---NN-----ILTEVSIET---ESFSSH---
Nicta_CycD3;2 (1) MVF------PLDTQ-------LLNPI---FDVLYCEEDRFLDDDD--LGEWSST-------LEQVGNNVK
Poptr_CycD3 (1) MASMYNPETSAVQD-------QQQNPTLLYDALYCSEE-NWVEEVREDWFQDELEGE-----SYCSNNSN
Scuba_CycD3 (1) MVS---EFQEHESL-------LQNPI---FDALYCDEERFDECVGGAGSGFKEPEIND---FNEIHNNP-
Orysa_CycD3 (1) MAF----------------------ATLLFDSLYCPEEHLDLFHDTAAD--DDLHLDL------------
Zeama_CycD3;2 (1) MAA----------------------FAALFDHLLCPEEHLDLYHEGPVEVVDEQWQDQ------------
Zeama_CycD3;3 (1) MAA----------------------FAALFDHLLCPEEHLDLYRDEPGEGADEQWPGQ------------
Consensus (1) MA LLDALYCEEE ED E
71 140
Antma_CycD3a (49) CGVD--------PDDDDKEEESSLLCKEQE----YEL-YRVLED----------------NPS--LAKAR
Antma_CycD3b (51) KEEPFTTFLFEHDLLWEDDELVNLLSKKEQEQQAHLGYDDVMD----------------SDGFLKRVRN
Arath_CYCD3_1 (45) --SSSPFVVLEQDLFWEDEDLVTLFSKEEEQ---GLSCLDDVY----------------------LSTDR
Arath_CYCD3_2 (49) SVVKFQFLPLLDMFLWDDDEILSLISKENE----TNPCFGEQI-----------------L-DGFLVSCR
Arath_CYCD3_3 (41) KFPFLNLGLSDHDMLWDDDELSTLISKQE-------PCLYDEIL----------------D-DEFLVLCR
Eupes_CycD3 (35) KELSLSSVLLEQDLFWEDDELNSLISKEKE-----THFSFGDFS----------------S-HGSLMVAR
Heltu_CycD3;1 (42) SSDLHLPPLDQLDLSWEHEELVSLFTKEQEQQ--KQTPCTLSFG----------------KTSPSVFAAR
Medsa_CycD3 (59) STSLLPLLLLEQNLFWEDEELNTLFSKEITQ---QETYYEDLKN---------------VINFDSLSQPR
Nicta_CycD3;1 (56) PNSLLPLLLLEQDLFWEDEELLSLFSKEKE----THCWFNSFQ-----------------DDSLLCSAR
Nicta_CycD3;3 (46) ---PLP--LLEQDLFWEDEELLSLFTKEKE----TISNFETLK----------------TDPLLCLSR
Pissa_CycD3 (54) STSLFPLLLLEQNLFSQDEELTTLFSKDKTQ---QETYYEDLKN---------------VVDFVSLSQPR
Popal_CycD3 (50) KEQNLSSVLLEQDLFWEDSELLSLISKEKE----THVVFDSVG----------------SRDGSLMVVR
Aqufo_CycD3 (63) VVELVPLLLLEQDLFWEDEELISLFRKEQD-----THLVISSQL----------------DSDPSIAIAR
Camsi_CycD3 (61) -------PQLLEQDLFWEDEELTSLLSKEQE----NPL-FHSLET---------------DPS--LGGAR
Camsi_CycD3;2 (53) G----PLVFLEHDWDWDDDELVSLISKEKE----THLGLSVLNS----------------DES--LMVAR
Arath_CycD3;3 modif (41) KFPFLNLGLSDHDMLWDDDELSTLISKQE-------PCLYDEIL----------------D-DEFLVLCR
Cheru_CycD3 (47) --SKLHDCSLICCEDDEEIQLNALVSKEEK------INFDEGDLG-----------------GNQLVMETR
Citsi_CycD3 (42) PACLFSLLLLEQDLFWEDEELLSLFSKEEQQL-LKQETQTHYK-----------------DSDVLVVAR
Eupes_cycD3;1 (48) ----------PVLDENWEDEELTSVLSKEQEQ----NQL-YKKLEI----------------NPC--LAKSR
Glyma_CycD3 (50) GTSLFPLLMLEQDLFWEDEELNSLFSKEKVQ---HEEAYYDYNNLNSDDNSNDHSNNNNNVLSDSCLSQPR
Goshi_CycD3 (50) NPSFFPLLLLEQDLFWEDGELLSLFAKETEQ---QPSCFNVGT-----------------DESLAMAR
Helan_CycD3 (30) QDSSAIHPLDLQDLCWEHEELVSLFTKEEQQ--KQTPWPSSC------------------TLSFR
Lagsi_CycD3;1 (44) -----ILAVAEQDLFWVNDELISLFSREKP----NEL-FKTLQI----------------DPS--LAAAR
Lagsi_CycD3;2 (48) NDFPLFQQTTTHFLVYEDEELNHLLSKEKDQNLQTGAVLKTLVQT---------------DNALSLAR
Lotco_CycD3 (64) TCSLFPLLLLEQDLFWEDEELNSLFSKEKIQ---HQNYYNDVN----------------SDPFLSQPR
Lyces_CycD3;1 (47) K----TLPLLECDMFWEHDELATLLSKENE----FHLGFQSLIS----------------DGS--LMGAR
Lyces_CycD3;2 (51) SLLLLPLLLLEQDLFWEDEELLSLFVKEKE----TRCCFESFG----------------SDPFLLCSAR
Lyces_CycD3;3 (45) -----------DELLWEDEELTSLFSKETE----YEISYNVLEK----------------NQS--FISSR
Nicta_CycD3;2 (46) K----TLPLLECDMFWEDDQLVTLLTKEKE----SHLGFDCLIS----------------DGDGFLVEVR
Poptr_CycD3 (58) KLNT-FPILLEQDLSWEDEELSSLFAKEEQ----NQL-CKDLET----------------NPS--LARAR
Scuba_CycD3 (54) -----FAFLFEHDLFWESEELDALLTKEKT---QTHLTFDEIN-----------------SDASLKAMRN
Orysa_CycD3 (34) -----HLHQPPPPPPLLDDDLPALFHALRGKE---DPLRPAAD----------------DDGYGGVSAR
Zeama_CycD3;2 (37) -----RGQQQP---AALLDDELPALFEALRDKEG---VVLAGDGE----------------EDGYGGSAGR
Zeama_CycD3;3 (37) -----HGQQEP---AVLLDDELPALFEAHRAKEG---VVLAEDG------------------GYGGAAGR
Consensus (71) LLEQDLFWEDEEL SLFSKEKE F L L AR

# FIGURE 6

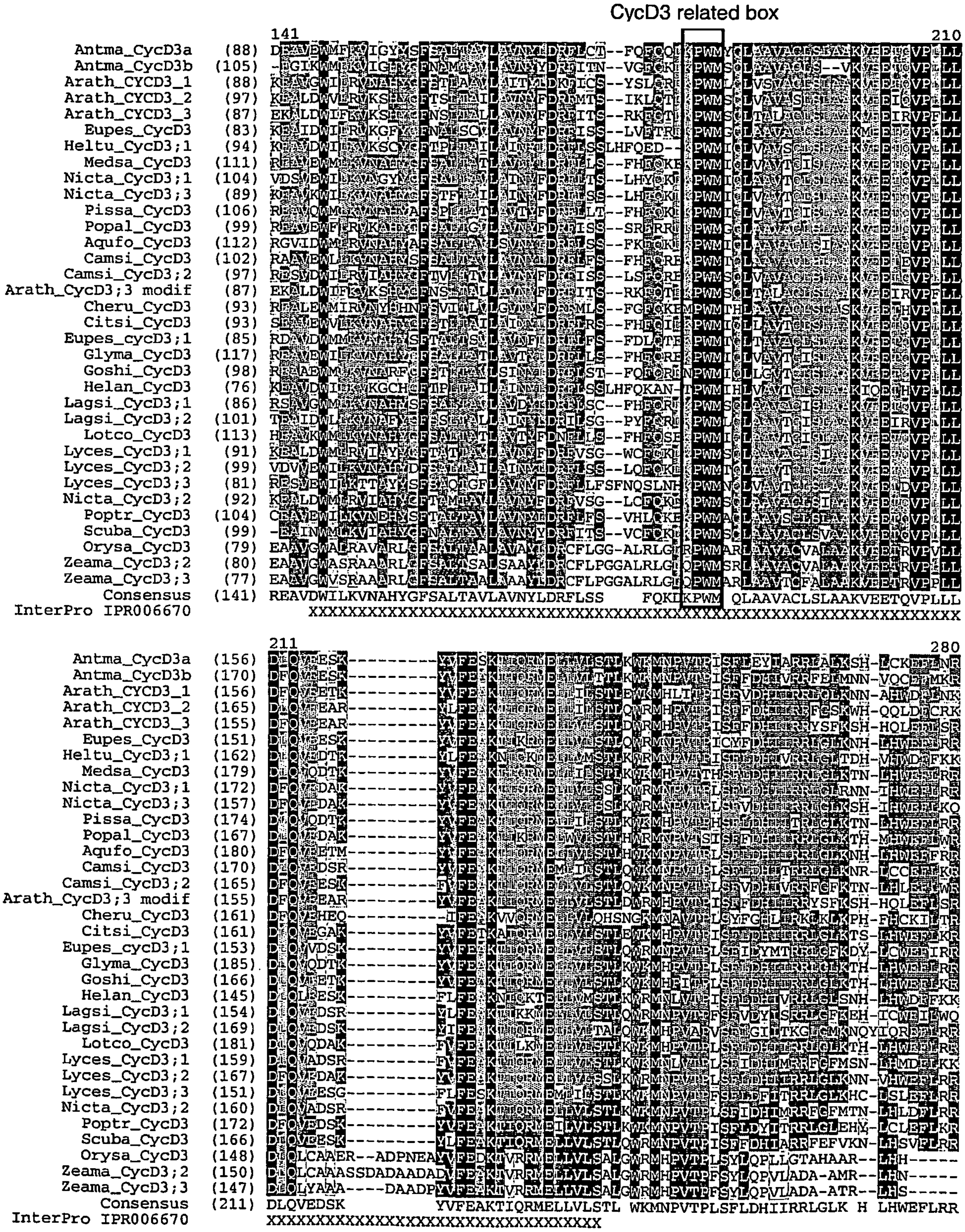


# FIGURE 6 (continued)

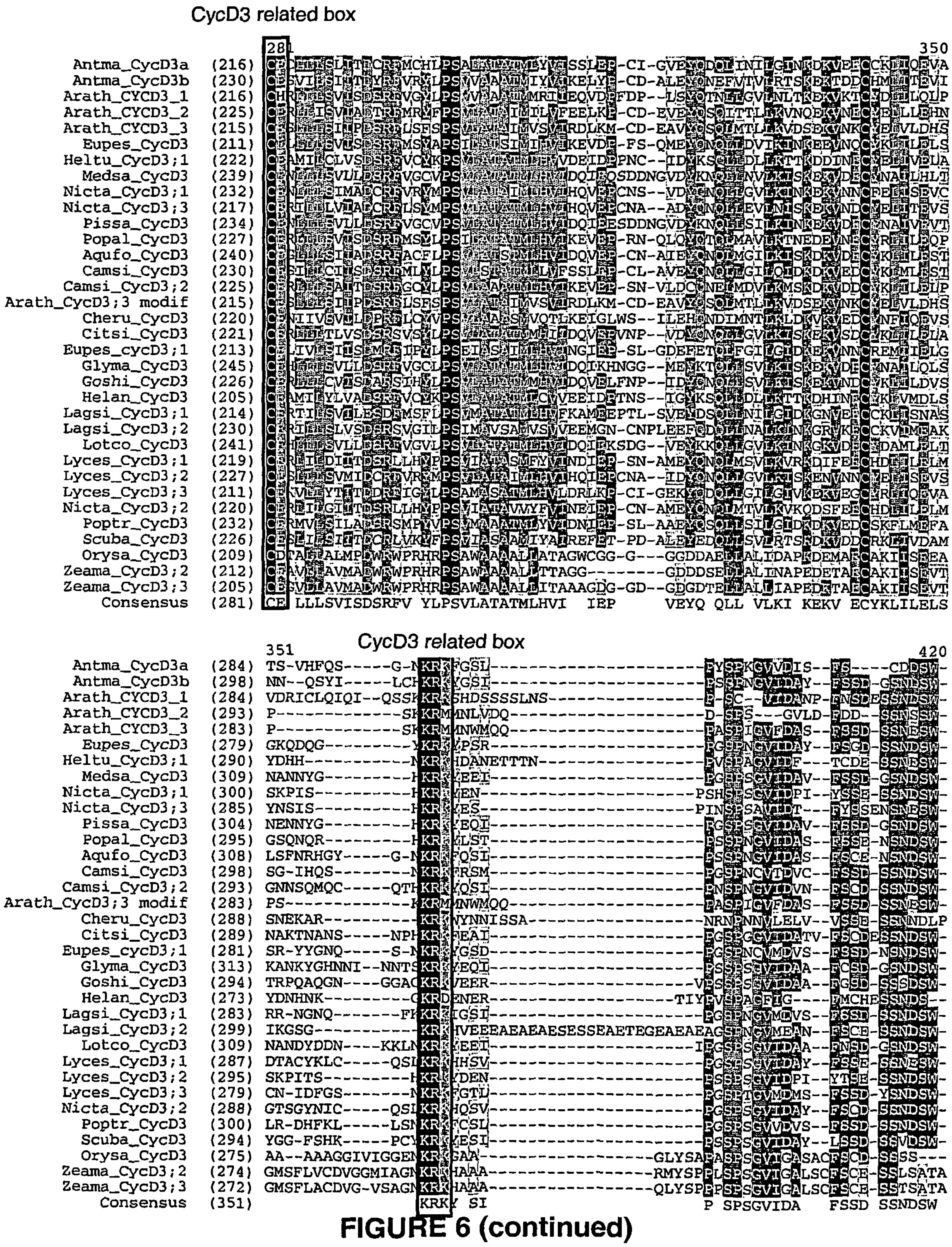


**FIGURE 6 (continued)**

```
                            421                                                                  490
        Antma_CycD3a  (318) ----------------PLDSTASVSSS---PEHL---SKKIKTQNPDH----------------------
        Antma_CycD3b  (335) ------------------SAVSSVSSS---PEPV---FKRIRAIGGANPPH-------------------
        Arath_CYCD3_1 (331) --------------SASSCNPPTSSSS-PQQQPP---LKK-------MRGAEENEK---KKPILHLPWAI
        Arath_CYCD3_2 (322) -------------NVSTTASVSSSSSS---PEPL---LKRRRVQEQQMR----------LPSINRMFLDV
        Arath_CYCD3_3 (316) -------------VVSASASVSSSPSS----EEL---LKRRRVQEQQMR----------LSSINRMFFDV
         Eupes_CycD3  (314) ---------------GVSSSISSSPS-----IPR---FKRIKSQDQQMR----------LPSINRMFVDV
       Heltu_CycD3;1  (327) ----------ELNAHH-------------FREPS---FKKTR-----MD-----------STIRVRVWFT
         Medsa_CycD3  (344) -----------------TVGASSYSTS----EPV---FKKTKNQGQNMN----------LSPINRVIVGI
       Nicta_CycD3;1  (334) ----------------DLESTSSY-------FPV---FKKSRVQEQQMK----------LASSISRVFVE
       Nicta_CycD3;3  (319) ------------DLQTSSSIPSTYSPRDQF-LPL---FKKSRVQEQQMR----------LTSLSRVFVDY
         Pissa_CycD3  (339) -----------------KVGSSSYSTS----EPV---FKKTKTQGQNRN----------LSPLNRVIVGI
         Popal_CycD3  (330) ------------------AVASSISSSSS--VPQ---FKRSRAQVQQMR----------LPSLNRMCVDV
         Aqufo_CycD3  (346) ------------------ALASSVTSS---PEPF---FKKSRAQDQQMR----------LPSFSRVFVDV
         Camsi_CycD3  (333) ------------AVTSSASASASVCSS---PEPL---SKKSRAQDHN----------------ATADTLS
       Camsi_CycD3;2  (332) ------------------AVTSSVSSS---PEPL---FKKSRVHGQQMR----------LAPLRHMSVGV
Arath_CycD3;3 modif  (316) ----------------VVSASASVSSSPS-SEPL---LKRRRVQEQQMR----------LSSINRMFFDV
         Cheru_CycD3  (327) ------------------------------SETL---PKKCRT----MGPPCFG----------------
         Citsi_CycD3  (330) -----------------SVASSSVLSSPSSPEPL---FKKSRVQDPQMT----------LPMPSLNLNRV
       Eupes_cycD3;1  (318) ---------------AVGSKSSSVSSS-----PA---AKKLRAVSG-MNH-------------ENAITLS
         Glyma_CycD3  (355) ------------------AVGSSLLYSP--PEPL---FKKSRTQGQQMN----------LSPLKRFITGI
         Goshi_CycD3  (335) ---------------------GTVSLS---PEQQPP-FKKSRAQEQVMR----------LPSLNRVFVDI
         Helan_CycD3  (309) ----------------------------------------------------------------------
       Lagsi_CycD3;1  (319) ------------------SVASSVSSS---PEPL---TKKNRANGS-MSG----------DCETFRTLS-
       Lagsi_CycD3;2  (354) -------EMGTIVSEYTHFSSSSSSSSS----------KRIRPTR-------------------------
         Lotco_CycD3  (350) -----------------TVGSSLFSSSG--PESPL--FKKSRTQ---MK----------LSPLNRVIVGI
       Lyces_CycD3;1  (326) ------------------SVASSISSS---PEPQ---YKRNKTQDQRMT----------LAPLGSNLH--
       Lyces_CycD3;2  (330) ---------------------DL-------DLPS---FKKSKVQEQQMK---------MSSSLSRVFVEA
       Lyces_CycD3;3  (315) ------------------SVATSVTSS---PEPL---SKKIRESNE------------------------
       Nicta_CycD3;2  (327) ------------------SVASSISSS---PEPQ---YKRIKTQDQTMT----------LAPLSSVSVVV
         Poptr_CycD3  (338) ------------------SVASSVSSS---PKPL---SKKSRALQSLNN-----------ATTSDFSQHS
         Scuba_CycD3  (332) ------------------AVTLSVSSS---PEPS---FKRSKAQDQHMR----------LAPLSSVSLGL
         Orysa_CycD3  (321) ------------------SVDS-LFAA---LEPPGRPIKRGAAAATTAD------------PLPADEESR
       Zeama_CycD3;2  (326) DSRTLATTAAGVGPWAPSAPVSVSSS---PEPPGRAPKRAAAAG-------------VPHPLPPDEESR
       Zeama_CycD3;3  (323) -------MAAAVGPWAPSASVSVSSS---PEPPGRAPKRAAAASASASASAGVAPPVQVPHQLPPDEESR
           Consensus  (421)                   S  SSVSSS   PEPL   FKK R Q Q M           L  L    V

                            491                        518
        Antma_CycD3a  (344) ----------------------------
        Antma_CycD3b  (362) ----------------------------
        Arath_CYCD3_1 (373) VATP------------------------
        Arath_CYCD3_2 (363) LSSPR-----------------------
        Arath_CYCD3_3 (356) LSSSPR----------------------
         Eupes_CycD3  (351) LSSPH-----------------------
       Heltu_CycD3;1  (355) YKL-------------------------
         Medsa_CycD3  (380) LATATSP---------------------
       Nicta_CycD3;1  (368) AVGSPH----------------------
       Nicta_CycD3;3  (363) AVGSPR----------------------
         Pissa_CycD3  (375) LATASATTSP------------------
         Popal_CycD3  (367) LSSPH-----------------------
         Aqufo_CycD3  (382) LSSPP-----------------------
         Camsi_CycD3  (369) FHC-------------------------
       Camsi_CycD3;2  (368) VGSPR-----------------------
Arath_CycD3;3 modif  (356) LIVVLAKPSFLVQSGDITSPGGLLGITG
         Cheru_CycD3  (344) ----------------------------
         Citsi_CycD3  (370) IVGSPS----------------------
       Eupes_cycD3;1  (351) ----------------------------
         Glyma_CycD3  (392) VGTPP-----------------------
         Goshi_CycD3  (370) VGSPS-----------------------
         Helan_CycD3  (309) ----------------------------
       Lagsi_CycD3;1  (353) ----------------------------
       Lagsi_CycD3;2  (381) ----------------------------
         Lotco_CycD3  (386) VSTSP-----------------------
       Lyces_CycD3;1  (360) ----------------------------
       Lyces_CycD3;2  (360) VGSPH-----------------------
       Lyces_CycD3;3  (337) ----------------------------
       Nicta_CycD3;2  (363) GSSPR-----------------------
         Poptr_CycD3  (373) SPVP------------------------
         Scuba_CycD3  (368) AHRIN-----------------------
         Orysa_CycD3  (357) DAWPPYAA--------------------
       Zeama_CycD3;2  (379) DAWPSTCAA-------------------
       Zeama_CycD3;3  (383) DAWPSTCAA-------------------
           Consensus  (491)
```

# FIGURE 6 (continued)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5811238 A
- US 6395547 B
- US 5565350 A
- WO 9322443 A
- EP 1198985 A1
- EP 05102444 A
- US 60668076 B


### Non-patent literature cited in the description


- **Meinkoth ; Wahl.** *Anal. Biochem.,* 1984, vol. 138, 267-284
- **Sambrook et al.** *Molecular Cloning: a laboratory manual,* 2001
- *Current Protocols in Molecular Biology,* 1989
- **Castle et al.** *Science,* 2004, vol. 304 (5674), 1151-4
- **Buchman ; Berg.** *Mol. Cell biol.,* 1988, vol. 8, 4395-4405
- **Callis et al.** *Genes Dev.,* 1987, vol. 1, 1183-1200
- The Maize Handbook. 1994
- **Wen et al.** *Plant Physiol,* 1993, vol. 101 (3), 1115-6
- **Takaiwa et al.** *Mol Gen Genet,* 1986, vol. 208, 15-22
- **Takaiwa et al.** *FEBS Letts.,* 1987, vol. 221, 43-47
- **Matzke et al.** *Plant Mol Biol,* 1990, vol. 14 (3), 323-32
- **Colot et al.** *Mol Gen Genet,* 1989, vol. 216, 81-90
- **Anderson et al.** *NAR,* 1989, vol. 17, 461-2
- **Albani.** *Plant Cell,* 1997, vol. 9, 171-184
- **Rafalski et al.** *EMBO,* 1984, vol. 3, 1409-15
- **Diaz et al.** *Mol Gen Genet,* 1995, vol. 248 (5), 592-8
- **Cho et al.** *Theor Appl Genet,* 1999, vol. 98, 1253-62
- **Muller et al.** *Plant J,* 1993, vol. 4, 343-55
- **Sorenson et al.** *Mol Gen Genet,* 1996, vol. 250, 750-60
- **Mena et al.** *Plant J,* 1998, vol. 116 (1), 53-62
- **Onate et al.** *J Biol Chem,* 1999, vol. 274 (14), 9175-82
- **Vicente-Carbajosa et al.** *Plant J,* 1998, vol. 13, 629-640
- **Wu et al.** *Plant Cell Physiol,* 1998, vol. 39 (8), 885-889
- **Nakase et al.** *Plant Molec Biol,* 1997, vol. 33, 513-522
- **Russell et al.** *Trans Res,* 1997, vol. 6, 157-68
- **Opsahl-Ferstad et al.** *Plant J,* 1997, vol. 12, 235-46
- **DeRose et al.** *Plant Molec Biol,* 1996, vol. 32, 1029-35
- **Krens, F.A. et al.** *Nature,* 1982, vol. 296, 72-74
- **Negrutiu I et al.** *Plant Mol Biol,* 1987, vol. 8, 363-373
- **Shillito R.D. et al.** *Bio/Technol,* 1985, vol. 3, 1099-1102
- **Crossway A et al.** *Mol. Gen Genet,* 1986, vol. 202, 179-185
- **Klein TM et al.** *Nature,* 1987, vol. 327, 70
- **Aldemita ; Hodges.** *Planta,* 1996, vol. 199, 612-617
- **Chan et al.** *Plant Mol Biol,* 1993, vol. 22 (3), 491-506
- **Hiei et al.** *Plant J,* 1994, vol. 6 (2), 271-282
- **Ishida.** *Nat. Biotechnol,* 1996, vol. 14 (6), 745-50
- **Frame et al.** *Plant Physiol,* 2002, vol. 129 (1), 13-22
- **Sambrook J ; Fritsch EF ; Maniatis T.** *Molecular Cloning, A Laboratory Manual,* 1989
- **Lander et al.** *Genomics,* 1987, vol. 1, 174-181
- **Botstein et al.** *Am. J. Hum. Genet.,* 1980, vol. 32, 314-331
- **Bematzky ; Tanksley.** *Plant Mol. Biol. Reporter,* 1986, vol. 4, 37-41
- **Hoheisel et al.** Non-mammalian Genomic Analysis. *A Practical Guide,* 1996, 319-346
- **Trask.** *Trends Genet.,* 1991, vol. 7, 149-154
- **Laan et al.** *Genome Res.,* 1995, vol. 5, 13-20
- **Kazazian.** *J. Lab. Clin. Med,* 1989, vol. 11, 95-96
- **Sheffield et al.** *Genomics,* 1993, vol. 16, 325-332
- **Landegren et al.** *Science,* 1988, vol. 241, 1077-1080

- **Sokolov.** *Nucleic Acid Res.,* 1990, vol. 18, 3671
- **Walter et al.** *Nat. Genet.,* 1997, vol. 7, 22-28
- **Dear ; Cook.** *Nucleic Acid Res.,* 1989, vol. 17, 6795-6807
- **Hayashi et al.** *Science,* 1992, 1350-1353
- **Redei GP ; Koncz C.** In Methods in Arabidopsis Research. World Scientific Publishing, 1992, 16-82
- **Feldmann et al.** *Arabidopsis.,* 1994, 137-172
- **Lightner J ; Caspar T.** *Methods on Molecular Biology,* 1998, vol. 82, 91-104
- **McCallum et al.** *Nat Biotechnol,* 2000, vol. 18 (2), 455-457
- **Stemple.** *Nat Rev Genet,* 2004, vol. 5 (2), 145-50
- **Offringa et al.** *EMBO J,* 1990, vol. 9 (10), 3077-84
- **Terada et al.** *Nat Biotech,* 2002, vol. 20 (10), 1030-4
- **Iida ; Terada.** *Curr Opin Biotech,* 2004, vol. 15 (2), 132-8
- **Sambrook.** *Molecular Cloning: a laboratory manual,* 2001, vol. 1 and 2
- *Plant Molecular Biology Labfase,* 1993

SEQUENCE LISTING

<110> CropDesign N.V.

<120> Plants having increased yield and a method for making the same

<130> CD-130-PCT

<150> EP 05102444.6
<151> 2005-03-25

<150> US 60/668,076
<151> 2005-04-05

<160> 71

<170> PatentIn version 3.3

<210> 1
<211> 1086
<212> DNA
<213> Arabidopsis thaliana

<400> 1

```
atggctttag aagaggagga agagagtcaa aacgcaccgt tttgtgttct tgatggtctt     60
ttctgtgagg aagagagtga gtttcacgaa caagtagatt tgtgcgacga gagtgttgaa    120
aagtttcctt ttttaaatct gggtttgtct gatcatgata tgttgtggga tgatgatgag    180
ttatcaactt tgatttcgaa acaagaaccg tgtctttatg acgaaatctt agatgatgag    240
tttctggttt tgtgtcgtga aaaggctctt gattggattt ttaaagtgaa atctcattat    300
gggtttaatt cattgacggc tcttttagct gttaattact tcgataggtt tattacaagc    360
aggaagtttc agacagataa gccatggatg tctcagctta ctgctttggc ttgtctgtct    420
ttagctgcta aggttgaaga gatccgtgtt ccttttctct tagattttca agtggaagaa    480
gcaagatatg tctttgaagc taagactata cagagaatgg agcttcttgt tctgtctact    540
cttgactgga ggatgcatcc tgtgactcca atctcgtttt tcgatcacat tattcgacga    600
tacagcttta aatctcatca tcaattggag ttcttgagta gatgtgaatc tttattactc    660
tccattattc ctgattcgag atttctgagt tttagtcctt ctgtgttagc cactgcaata    720
atggtctctg ttattagaga tttgaagatg tgtgacgaag ctgtatacca atctcagctc    780
atgactctac tcaaagttga ttcggagaag gtaaataaat gctatgagtt agtgttagac    840
cacagtccaa gcaagaaaag gatgatgaat tggatgcaac aacccgctag tccgatcggt    900
gtgtttgatg cgtcattcag ttctgatagc tcgaatgagt cgtgggttgt gtctgcttct    960
gcttcagtgt cgtcttcacc atcttcagag cctttgctca agaggagaag agtgcaagag   1020
cagcagatga ggctatcttc aataaaccga atgtttttcg atgtgcttag tagtagtcct   1080
cgctaa                                                              1086
```

<210> 2
<211> 361
<212> PRT
<213> Arabidopsis thaliana

<400> 2

```
Met Ala Leu Glu Glu Glu Glu Glu Ser Gln Asn Ala Pro Phe Cys Val
1               5                   10                  15
Leu Asp Gly Leu Phe Cys Glu Glu Glu Ser Glu Phe His Glu Gln Val
            20                  25                  30

Asp Leu Cys Asp Glu Ser Val Glu Lys Phe Pro Phe Leu Asn Leu Gly
        35                  40                  45
Leu Ser Asp His Asp Met Leu Trp Asp Asp Asp Glu Leu Ser Thr Leu
    50                  55                  60
Ile Ser Lys Gln Glu Pro Cys Leu Tyr Asp Glu Ile Leu Asp Asp Glu
65                  70                  75                  80
Phe Leu Val Leu Cys Arg Glu Lys Ala Leu Asp Trp Ile Phe Lys Val
                85                  90                  95
Lys Ser His Tyr Gly Phe Asn Ser Leu Thr Ala Leu Leu Ala Val Asn
            100                 105                 110
Tyr Phe Asp Arg Phe Ile Thr Ser Arg Lys Phe Gln Thr Asp Lys Pro
        115                 120                 125
Trp Met Ser Gln Leu Thr Ala Leu Ala Cys Leu Ser Leu Ala Ala Lys
    130                 135                 140
Val Glu Glu Ile Arg Val Pro Phe Leu Leu Asp Phe Gln Val Glu Glu
145                 150                 155                 160
Ala Arg Tyr Val Phe Glu Ala Lys Thr Ile Gln Arg Met Glu Leu Leu
                165                 170                 175
Val Leu Ser Thr Leu Asp Trp Arg Met His Pro Val Thr Pro Ile Ser
            180                 185                 190
Phe Phe Asp His Ile Ile Arg Arg Tyr Ser Phe Lys Ser His His Gln
        195                 200                 205
Leu Glu Phe Leu Ser Arg Cys Glu Ser Leu Leu Leu Ser Ile Ile Pro
    210                 215                 220
Asp Ser Arg Phe Leu Ser Phe Ser Pro Ser Val Leu Ala Thr Ala Ile
225                 230                 235                 240
Met Val Ser Val Ile Arg Asp Leu Lys Met Cys Asp Glu Ala Val Tyr
                245                 250                 255
Gln Ser Gln Leu Met Thr Leu Leu Lys Val Asp Ser Glu Lys Val Asn
            260                 265                 270
Lys Cys Tyr Glu Leu Val Leu Asp His Ser Pro Ser Lys Lys Arg Met
        275                 280                 285
Met Asn Trp Met Gln Gln Pro Ala Ser Pro Ile Gly Val Phe Asp Ala
    290                 295                 300
Ser Phe Ser Ser Asp Ser Ser Asn Glu Ser Trp Val Val Ser Ala Ser
305                 310                 315                 320
Ala Ser Val Ser Ser Ser Pro Ser Ser Glu Pro Leu Leu Lys Arg Arg
                325                 330                 335
Arg Val Gln Glu Gln Gln Met Arg Leu Ser Ser Ile Asn Arg Met Phe
            340                 345                 350
Phe Asp Val Leu Ser Ser Ser Pro Arg
        355                 360
```

<210> 3
<211> 654
<212> DNA
<213> Oryza sativa

<400> 3

```
cttctacatc ggcttaggtg tagcaacacg actttattat tattattatt attattatta     60
ttattttaca aaaatataaa atagatcagt ccctcaccac aagtagagca agttggtgag    120
ttattgtaaa gttctacaaa gctaatttaa aagttattgc attaacttat ttcatattac    180
aaacaagagt gtcaatggaa caatgaaaac catatgacat actataattt tgtttttatt    240
attgaaatta tataattcaa agagaataaa tccacatagc cgtaaagttc tacatgtggt    300

gcattaccaa aatatatata gcttacaaaa catgacaagc ttagtttgaa aaattgcaat    360
ccttatcaca ttgacacata aagtgagtga tgagtcataa tattattttc tttgctaccc    420
atcatgtata tatgatagcc acaaagttac tttgatgatg atatcaaaga acatttttag    480
gtgcacctaa cagaatatcc aaataatatg actcacttag atcataatag agcatcaagt    540
aaaactaaca ctctaaagca accgatggga aagcatctat aaatagacaa gcacaatgaa    600
aatcctcatc atccttcacc acaattcaaa tattatagtt gaagcatagt agta          654
```

<210> 4
<211> 54
<212> DNA
<213> Artificial sequence

<220>
<223> primer: prm0360

<400> 4
ggggacaagt ttgtacaaaa aagcaggctt cacaatggct ttagaagagg agga 54

<210> 5
<211> 51
<212> DNA
<213> Artificial sequence

<220>
<223> primer: prm0361

<400> 5
ggggaccact ttgtacaaga aagctgggtt tagcgaggac tactataagc a 51

<210> 6
<211> 1140
<212> DNA
<213> Antirrhinum majus

<400> 6

```
gagagaagag atggctcaaa gctcaactct aggcctctct ttcttcacac ctttgtttga     60
cttagaatga aatgaatgca gcttcacaca actctcttct cctcagaaga tgtatcaaca    120
aaactctcca tcactctgtt ttgatgctct gtactgtgag gaagaacaaa actgggacaa    180
tggtgaaatc atcaatgact gtttcattga agaacaagaa cccttttctg atttattgaa    240
acatgatttg ttatgtggtg tagatgatga tgatgatgat aaagaagagc ttagctcttt    300
attgtgtaaa gagcaggaat atgaactgta cagagtcctt gaggacaatc catctctagc    360
aaaagctaga gatgaggctg ttgaatggat gtttaaggtc attgggtact attcttttc     420
tgctctcact gcggttcttg cagttaacta tttggataga tttctatgca catttcagtt    480
tcaacaagat aagccatgga tgtatcagtt ggctgctgtg gcttgtctct ctttggctgc    540
taaagttgaa gaaactcaag tccctcttct gttagacctt caagttgagg aatctaagta    600
tgtgtttgag tcaaaaacca ttcaaagaat ggagcttttg gtgctttcaa cacttaaatg    660
gaagatgaat ccagtcaccc caatttcatt ccttgagtac attgctagga ggctagcatt    720
gaagagccat ctttgtaaag agttcctcaa cagatgtgaa tgcctccttt tgtcccttat    780
taccgattgt agattcatgt gccatcttcc atctgcattg gccactgcaa cgatgctgta    840
tgttataagc agcttagagc cctgcattgg tgtggagtac caagatcaac tcatcaacat    900
tcttggaatc aacaaggaca aagtggagga atgttgtaag ttaatacaag aagtggccac    960
aagtgttcat tttcaatcag gcaacaaaag aaagtttgga tctttgcctt atagccccaa   1020
aggggtagtg gacatctcat tcagttgtga tgattcatgg ccgttggatt caactgcatc   1080
agtttcttcc tcaccagagc atttgtccaa gaaaatcaag acccaaaatc cagaccatga   1140
```

<210> 7
<211> 343
<212> PRT
<213> Antirrhinum majus

<400> 7

```
Met Tyr Gln Gln Asn Ser Pro Ser Leu Cys Phe Asp Ala Leu Tyr Cys
1               5                   10                  15
Glu Glu Glu Gln Asn Trp Asp Asn Gly Glu Ile Ile Asn Asp Cys Phe
            20                  25                  30
Ile Glu Glu Gln Glu Pro Phe Ser Asp Leu Leu Lys His Asp Leu Leu
        35                  40                  45
Cys Gly Val Asp Asp Asp Asp Asp Asp Lys Glu Glu Leu Ser Ser Leu
    50                  55                  60
Leu Cys Lys Glu Gln Glu Tyr Glu Leu Tyr Arg Val Leu Glu Asp Asn
65                  70                  75                  80
Pro Ser Leu Ala Lys Ala Arg Asp Glu Ala Val Glu Trp Met Phe Lys
                85                  90                  95
Val Ile Gly Tyr Tyr Ser Phe Ser Ala Leu Thr Ala Val Leu Ala Val
            100                 105                 110
Asn Tyr Leu Asp Arg Phe Leu Cys Thr Phe Gln Phe Gln Gln Asp Lys
        115                 120                 125
Pro Trp Met Tyr Gln Leu Ala Ala Val Ala Cys Leu Ser Leu Ala Ala
    130                 135                 140
Lys Val Glu Glu Thr Gln Val Pro Leu Leu Leu Asp Leu Gln Val Glu
145                 150                 155                 160
Glu Ser Lys Tyr Val Phe Glu Ser Lys Thr Ile Gln Arg Met Glu Leu
                165                 170                 175
Leu Val Leu Ser Thr Leu Lys Trp Lys Met Asn Pro Val Thr Pro Ile
            180                 185                 190
Ser Phe Leu Glu Tyr Ile Ala Arg Arg Leu Ala Leu Lys Ser His Leu
        195                 200                 205
Cys Lys Glu Phe Leu Asn Arg Cys Glu Cys Leu Leu Leu Ser Leu Ile
    210                 215                 220
Thr Asp Cys Arg Phe Met Cys His Leu Pro Ser Ala Leu Ala Thr Ala
225                 230                 235                 240
Thr Met Leu Tyr Val Ile Ser Ser Leu Glu Pro Cys Ile Gly Val Glu
                245                 250                 255
Tyr Gln Asp Gln Leu Ile Asn Ile Leu Gly Ile Asn Lys Asp Lys Val
            260                 265                 270
Glu Glu Cys Cys Lys Leu Ile Gln Glu Val Ala Thr Ser Val His Phe
        275                 280                 285
Gln Ser Gly Asn Lys Arg Lys Phe Gly Ser Leu Pro Tyr Ser Pro Lys
    290                 295                 300
Gly Val Val Asp Ile Ser Phe Ser Cys Asp Asp Ser Trp Pro Leu Asp
305                 310                 315                 320
Ser Thr Ala Ser Val Ser Ser Ser Pro Glu His Leu Ser Lys Lys Ile
                325                 330                 335
Lys Thr Gln Asn Pro Asp His
            340
```

<210> 8
<211> 1451
<212> DNA
<213> Antirrhinum majus

<400> 8

```
taaaaaaaag cgtaaaatta aacagaaaaa aatatgatat taagcccaag attcaatctt      60
ttgagctttt cttaaagata atgagcagag ccccctgtat cttcttctcc gaagaagatg     120
ttgttttcac attctcaaca aacccatctc caaaatccaa tctttgatgc tcttctctgt     180
aacgaggagc atttcgatga agatttggat cttgggtccg ggttaaaaga cccgggtttt     240
ataaatcaga ttcatcataa tcaaaaaaaa gaagaaccat ttactacttt tctgtttgag     300
cacgaccttt tgtgggaaga tgacgagctt gttaatctct tgtctaagga gaaagaacaa     360
gaacaacaag cccatttggg gtacgatgat gtaatggact ctgatgggtt tttgaaaagg     420
gtgagaaatg aagggattaa gtggatgttg aaggtgattg gacactatgg gttcaatgcg     480
atgactgctg ttttagctgt gaattattat gatagattta ttacaaacgt tgggtttcaa     540
aaggataagc cttggatgag tcaattggct gctgttgctt gtctttctgt aaaagtggag     600
gagactcaag tgcctctgtt gctggatttt caagtagagg aatcaaagta tgtgtttgag     660
gcaaagacta tacagaggat ggagcttttg gtgctcacta ctttgaaatg gaagatgaac     720
cctgtgacgc ctatctcgtt ctttgaccac attgtgagga ggtttgagtt gatgaacaat     780
gtgcaatgcg agtttatgaa gaggtgtgag agtgtcattc tctccatcat caccgattat     840
cgatttgtgc gctatcttcc ttctgttgtt gctgctgcaa ccatgatata tgtaatcaaa     900
gagctttatc cttgtgatgc attggaatac cagaatgagt ttgtgactgt gctgagaact     960
agcaaggaaa agactgatga ttgccatatg ctaatcactg aagtaatcaa caatcaaagc    1020
tacatccttt gtcacaagcg caagtacggt tccataccaa gcagtccaaa tggtgtgatc    1080
gatgcctatt tcagctctga tggctctaac gattcgtggt cagcagtgtc atccgtttca    1140
tcatcaccag agcccgtgtt taagagaatc agagccattg ggggggctaa tcctcctcat    1200
tgaactcatt tcttatttta tctgatattt agcaacggta cttcataatc gctcttttgc    1260
tatggttttt tccgtcataa gacgatgcag ttatgttaca tttctgttat atcttgctgt    1320
gatggatcag acatgtttta acagacaatt gactcttatc acctctttga ttgagggatg    1380
gagagccaaa gggattatgt gagtttttgt tcctggagta aaatcgatga acttttagta    1440
ttaatgaaaa a                                                         1451
```

<210> 9
<211> 361
<212> PRT
<213> Antirrhinum majus

<400> 9

```
Met Leu Phe Ser His Ser Gln Gln Thr His Leu Gln Asn Pro Ile Phe
1               5                   10                  15
Asp Ala Leu Leu Cys Asn Glu Glu His Phe Asp Glu Asp Leu Asp Leu
            20                  25                  30
Gly Ser Gly Leu Lys Asp Pro Gly Phe Ile Asn Gln Ile His His Asn
        35                  40                  45
Gln Lys Lys Glu Glu Pro Phe Thr Thr Phe Leu Phe Glu His Asp Leu
    50                  55                  60
Leu Trp Glu Asp Asp Glu Leu Val Asn Leu Leu Ser Lys Glu Lys Glu
65                  70                  75                  80
Gln Glu Gln Gln Ala His Leu Gly Tyr Asp Asp Val Met Asp Ser Asp
                85                  90                  95
Gly Phe Leu Lys Arg Val Arg Asn Glu Gly Ile Lys Trp Met Leu Lys
            100                 105                 110
Val Ile Gly His Tyr Gly Phe Asn Ala Met Thr Ala Val Leu Ala Val
        115                 120                 125
Asn Tyr Tyr Asp Arg Phe Ile Thr Asn Val Gly Phe Gln Lys Asp Lys
    130                 135                 140
```

```
Pro Trp Met Ser Gln Leu Ala Ala Val Ala Cys Leu Ser Val Lys Val
145                 150                 155                 160
Glu Glu Thr Gln Val Pro Leu Leu Leu Asp Phe Gln Val Glu Glu Ser
                165                 170                 175
Lys Tyr Val Phe Glu Ala Lys Thr Ile Gln Arg Met Glu Leu Leu Val
            180                 185                 190
Leu Thr Thr Leu Lys Trp Lys Met Asn Pro Val Thr Pro Ile Ser Phe
        195                 200                 205
Phe Asp His Ile Val Arg Arg Phe Glu Leu Met Asn Asn Val Gln Cys
    210                 215                 220
Glu Phe Met Lys Arg Cys Glu Ser Val Ile Leu Ser Ile Ile Thr Asp
225                 230                 235                 240
Tyr Arg Phe Val Arg Tyr Leu Pro Ser Val Val Ala Ala Ala Thr Met
                245                 250                 255
Ile Tyr Val Ile Lys Glu Leu Tyr Pro Cys Asp Ala Leu Glu Tyr Gln
            260                 265                 270
Asn Glu Phe Val Thr Val Leu Arg Thr Ser Lys Glu Lys Thr Asp Asp
        275                 280                 285
Cys His Met Leu Ile Thr Glu Val Ile Asn Asn Gln Ser Tyr Ile Leu
    290                 295                 300
Cys His Lys Arg Lys Tyr Gly Ser Ile Pro Ser Ser Pro Asn Gly Val
305                 310                 315                 320
Ile Asp Ala Tyr Phe Ser Ser Asp Gly Ser Asn Asp Ser Trp Ser Ala
                325                 330                 335
Val Ser Ser Val Ser Ser Ser Pro Glu Pro Val Phe Lys Arg Ile Arg
            340                 345                 350
Ala Ile Gly Gly Ala Asn Pro Pro His
        355                 360
```

<210> 10
<211> 1646
<212> DNA
<213> Arabidopsis thaliana

<400> 10

```
atcactctcc gaaacccact tccagctttt tcctctctct ttctctctct agtctctctt     60
ttgtagctct cccctgctaa gctaaccact gcacgtttcc atagagagga aagatgagtc    120
tctctccgag agatttctc tctatcatct tatcttcttc cgtgtaatgc tctgagccaa     180
aacccaataa ctaaatcaac aacaatatag aagagaagag aaagatctta tctttcttct    240
cattcttgag tttagtcccc cacaatggcg attcggaagg aggaagaaag tagagaagaa    300
cagagcaatt cgtttcttct tgatgctctc tactgcgaag aagagaaatg ggacgatgaa    360
ggagaagaag ttgaagaaaa ctcttccttg tcttcttctt cttctccatt cgttgttttg    420
caacaagatt tgttctggga agatgaagat ctggttacac tcttctccaa agaagaagaa    480
caaggactca gctgtctcga tgatgtttat ctttccacgg atcgaaaaga agctgttggt    540
tggattctga gagtcaacgc tcattatggc ttctctactt tagcagctgt tttagccata    600
acttatctcg ataagttcat ctgtagctac agcttacaga gagacaaacc atggatgctt    660
cagctcgttt ctgtcgcgtg tctctcatta gctgctaaag tcgaagaaac ccaagtccct    720
cttcttctag actttcaagt ggaggagaca aagtatgtgt ttgaagcaaa aaccatacag    780
agaatggagc tactgattct gtctactctc gagtggaaga tgcatctcat tactccaatt    840
tcgttcgtag accacattat caggagattg ggacttaaga acaatgctca ctgggatttc    900
ctcaacaaat gccaccgtct cctcctctct gtaatctccg attcaagatt tgtcgggtac    960
ctcccatcag tagttgccgc agctaccatg atgcgaatta tagagcaagt tgatcccttt   1020
gaccctcttt cataccaaac taatctcctc ggtgtcctta acttaaccaa ggaaaaggtg   1080
aaaacttgct acgatctaat cctccaacta ccagtggacc gcatcggttt acagatccaa   1140
```

```
atccaatctt ccaagaaacg caagagtcac gattcatcat catcgttgaa cagtccaagc    1200
tgcgtgattg atgcaaaccc tttcaatagc gacgaaagct caaacgattc gtggtcagcg    1260
agttcgtgca acccaccaac gtcgtcgtcg tccccgcagc aacaacctcc attgaagaag    1320
atgagaggag ctgaagagaa tgagaagaag aagccgattt tgcatctgcc atgggcaatc    1380
gtagccactc cataatcgaa agctcgattt cgtttatatg atatttactg tttttttaaa    1440
ctttgagaac aatctttgtt gtattaagct ttacccgttt gcatatacga aatgtcgcga    1500
atgcccttac gtgccatggc ttgatagagt taatgggtaa agggtattca tgacatttga    1560
ctgcatggga tgtgacgaag gagagaatta gaaataataa taataatatt gcgtaaattt    1620
tgaggcttgc ccaatctttg ggccgt                                          1646
```

<210> 11
<211> 376
<212> PRT
<213> Arabidopsis thaliana

<400> 11

```
Met Ala Ile Arg Lys Glu Glu Glu Ser Arg Glu Glu Gln Ser Asn Ser
1               5                   10                  15
Phe Leu Leu Asp Ala Leu Tyr Cys Glu Glu Glu Lys Trp Asp Asp Glu
            20                  25                  30
Gly Glu Glu Val Glu Glu Asn Ser Ser Leu Ser Ser Ser Ser Ser Pro
        35                  40                  45
Phe Val Val Leu Gln Gln Asp Leu Phe Trp Glu Asp Glu Asp Leu Val
    50                  55                  60
Thr Leu Phe Ser Lys Glu Glu Glu Gln Gly Leu Ser Cys Leu Asp Asp
65                  70                  75                  80
Val Tyr Leu Ser Thr Asp Arg Lys Glu Ala Val Gly Trp Ile Leu Arg
                85                  90                  95
Val Asn Ala His Tyr Gly Phe Ser Thr Leu Ala Ala Val Leu Ala Ile
            100                 105                 110
Thr Tyr Leu Asp Lys Phe Ile Cys Ser Tyr Ser Leu Gln Arg Asp Lys
        115                 120                 125
Pro Trp Met Leu Gln Leu Val Ser Val Ala Cys Leu Ser Leu Ala Ala
    130                 135                 140
Lys Val Glu Glu Thr Gln Val Pro Leu Leu Leu Asp Phe Gln Val Glu
145                 150                 155                 160
Glu Thr Lys Tyr Val Phe Glu Ala Lys Thr Ile Gln Arg Met Glu Leu
                165                 170                 175
Leu Ile Leu Ser Thr Leu Glu Trp Lys Met His Leu Ile Thr Pro Ile
            180                 185                 190
Ser Phe Val Asp His Ile Ile Arg Arg Leu Gly Leu Lys Asn Asn Ala
        195                 200                 205
His Trp Asp Phe Leu Asn Lys Cys His Arg Leu Leu Leu Ser Val Ile
    210                 215                 220
Ser Asp Ser Arg Phe Val Gly Tyr Leu Pro Ser Val Val Ala Ala Ala
225                 230                 235                 240
Thr Met Met Arg Ile Ile Glu Gln Val Asp Pro Phe Asp Pro Leu Ser
                245                 250                 255
Tyr Gln Thr Asn Leu Leu Gly Val Leu Asn Leu Thr Lys Glu Lys Val
            260                 265                 270
Lys Thr Cys Tyr Asp Leu Ile Leu Gln Leu Pro Val Asp Arg Ile Gly
        275                 280                 285
Leu Gln Ile Gln Ile Gln Ser Ser Lys Lys Arg Lys Ser His Asp Ser
    290                 295                 300
```

Ser Ser Ser Leu Asn Ser Pro Ser Cys Val Ile Asp Ala Asn Pro Phe
305 310 315 320
Asn Ser Asp Glu Ser Ser Asn Asp Ser Trp Ser Ala Ser Ser Cys Asn
325 330 335
Pro Pro Thr Ser Ser Ser Ser Pro Gln Gln Gln Pro Pro Leu Lys Lys
340 345 350
Met Arg Gly Ala Glu Glu Asn Glu Lys Lys Lys Pro Ile Leu His Leu
355 360 365
Pro Trp Ala Ile Val Ala Thr Pro
370 375

<210> 12
<211> 1415
<212> DNA
<213> Arabidopsis thaliana

<400> 12

```
aaggcgtgag ataataaaac cctttggctt tctcatagag atttgtccgg tctcttgttc     60
ctctttctcc tttcttcact gtagaatccg tcgaccaaac aactagctcc aatggcataa    120
tgagcattgt agtttgcaat ttcttcttcc gtgaagaaga agaagatggc tttggagaaa    180
gaggaagaag cgtcacaaaa cggtgcgttt tgtgtccttg atgggctcta ttgcgaggaa    240
gaaaccgggt ttgtggagga cgatcttgat gacgatggag atttagattt tctcgagaaa    300
tctgatgaga gtgttgtaaa gtttcagttt ttacctcttt tggatatgtt cttatgggat    360
gacgatgaga ttctgagttt gatttcaaag gaaaacgaaa cgaatccatg ttttggggaa    420
caaatcttag atggcttttt ggtttcttgt aggaaagagg ctttagattg ggttcttagg    480
gttaaatctc attatgggtt tacttcattg acggctatac ttgctgtgaa ctacttcgat    540
aggtttatga caagtataaa gcttcagact gataagccat ggatgtctca gcttgttgct    600
gtggcttctt tgtctttagc tgctaaagtt gaagagattc aagttccatt gctcttagac    660
ctccaagtgg aagaagcaag atatctcttt gaagctaaga cgattcaaag aatggagctt    720
ttgattcttt ctactcttca atggagaatg caccctgtga ctccaatctc tttctttgat    780
cacattatcc ggcgatttgg ctctaaatgg caccagcaat tagacttctg taggaagtgt    840
gagcgtcttc tgatctctgt tattgctgat acgaggttta tgaggtactt cccttctgtc    900
ttagctactg caataatgat ccttgtcttc gaggaattga agccatgtga tgaagttgaa    960
taccaatctc aaataacgac tctactcaaa gtcaatcagg agaaagtaaa tgaatgctat   1020
gaactgttgt tggagcacaa tccaagcaag aagaggatga tgaatttggt tgatcaggac   1080
agtccaagtg gtgtattaga ctttgatgac agctcaaata gctcctggaa tgtctccact   1140
actgcttcag tgtcctcatc atcttcgtct ccagagcctc tgctcaagag aagaagagtt   1200
caggagcagc aaatgagatt gccctcaata aaccgtatgt ttctcgatgt gcttagtagt   1260
cctcgctagt acctttcttt gatcaaatgt gtcaaaacat aaattcaatc tctcttttgc   1320
ttattattat cggccatcgg ctacaatttg aaggcagaac attttgtgat aactctaagt   1380
taattctgcc tcttaaatca taatattcat tgatc                              1415
```

<210> 13
<211> 367
<212> PRT
<213> Arabidopsis thaliana

<400> 13

Met Ala Leu Glu Lys Glu Glu Glu Ala Ser Gln Asn Gly Ala Phe Cys
1 5 10 15
Val Leu Asp Gly Leu Tyr Cys Glu Glu Glu Thr Gly Phe Val Glu Asp
20 25 30
Asp Leu Asp Asp Asp Gly Asp Leu Asp Phe Leu Glu Lys Ser Asp Glu
35 40 45

```
Ser Val Val Lys Phe Gln Phe Leu Pro Leu Leu Asp Met Phe Leu Trp
    50                  55                  60
Asp Asp Asp Glu Ile Leu Ser Leu Ile Ser Lys Glu Asn Glu Thr Asn
65                  70                  75                  80
Pro Cys Phe Gly Glu Gln Ile Leu Asp Gly Phe Leu Val Ser Cys Arg
                85                  90                  95
Lys Glu Ala Leu Asp Trp Val Leu Arg Val Lys Ser His Tyr Gly Phe
            100                 105                 110
Thr Ser Leu Thr Ala Ile Leu Ala Val Asn Tyr Phe Asp Arg Phe Met
        115                 120                 125
Thr Ser Ile Lys Leu Gln Thr Asp Lys Pro Trp Met Ser Gln Leu Val
    130                 135                 140
Ala Val Ala Ser Leu Ser Leu Ala Ala Lys Val Glu Glu Ile Gln Val
145                 150                 155                 160
Pro Leu Leu Leu Asp Leu Gln Val Glu Glu Ala Arg Tyr Leu Phe Glu
                165                 170                 175
Ala Lys Thr Ile Gln Arg Met Glu Leu Leu Ile Leu Ser Thr Leu Gln
            180                 185                 190
Trp Arg Met His Pro Val Thr Pro Ile Ser Phe Phe Asp His Ile Ile
        195                 200                 205
Arg Arg Phe Gly Ser Lys Trp His Gln Gln Leu Asp Phe Cys Arg Lys
    210                 215                 220
Cys Glu Arg Leu Leu Ile Ser Val Ile Ala Asp Thr Arg Phe Met Arg
225                 230                 235                 240
Tyr Phe Pro Ser Val Leu Ala Thr Ala Ile Met Ile Leu Val Phe Glu
                245                 250                 255
Glu Leu Lys Pro Cys Asp Glu Val Glu Tyr Gln Ser Gln Ile Thr Thr
            260                 265                 270
Leu Leu Lys Val Asn Gln Glu Lys Val Asn Glu Cys Tyr Glu Leu Leu
        275                 280                 285
Leu Glu His Asn Pro Ser Lys Lys Arg Met Met Asn Leu Val Asp Gln
    290                 295                 300
Asp Ser Pro Ser Gly Val Leu Asp Phe Asp Asp Ser Ser Asn Ser Ser
305                 310                 315                 320
Trp Asn Val Ser Thr Thr Ala Ser Val Ser Ser Ser Ser Ser Ser Pro
                325                 330                 335
Glu Pro Leu Leu Lys Arg Arg Arg Val Gln Glu Gln Gln Met Arg Leu
            340                 345                 350
Pro Ser Ile Asn Arg Met Phe Leu Asp Val Leu Ser Ser Pro Arg
        355                 360                 365
```

<210> 14
<211> 1425
<212> DNA
<213> Euphorbia esula

<220>
<221> misc_feature
<222> (1347)..(1347)
<223> n is a, c, g, or t

<400> 14

```
tttttttttc ttctctgcct ctctatccat tccttctctt ctctctgtct ctgctatcaa     60
tagaccccta gtgagagaaa cagagataaa gcaaattgta tggtgatatg atttgcaatg    120
```

```
agattgagat tgggatttta ttttctttga tggtataatg agcaaaggat tgaatctctt    180
cttctctgaa gaagaacaaa aatggaagac agcactcaga tttcattgat ttttgatggg    240
ttgtactgcg aagaacaagg cattggtgaa gattttgacg atgggaatga agattatgtg    300
aaaaaggagt tatctttatc ttctgttttg cttgagcagg acttgttttg gaccgatgat    360
gagttgttaa atctgatttc aaaagaaaaa gagactcatt ttagttttgg ggattttttcc   420
tctcatgggt ctttaatggt ggctcgtaaa gaggcaatag attggatttt gagggtaaaa    480
gggttttatg gattcaatgc tttgagctgt gttcttgctg ttaattattt tgatagattc    540
atttcgagtt tagtttttac aagagataaa ccatggatgg gtcaacttgc tgctgttgct    600
tgtttatctt tggctgctaa aatggaggag actcaagttc ctcttcttct agatttacaa    660
gtggaagaat caaagtatgt gtttgaggca aagactataa agagaatgga gcttcttgtg    720
ctctctactc ttcaatggag gatgaatcct gtgaccccaa tttgctactt tgatcacatt    780
ataaggaggc taggacttaa aaaccatctg cattgggaat ttttgaggag atgtgagctt    840
ttacttctct ctgtcatttc tgattcaaga ttcatgagtt atgcaccttc tatattagca    900
acttcaatta tgatccatgt gattaaggag gttgacccat ttagtcaaat ggaataccag    960
aaccagcttt tggatgtgat caaaatcaac aaggaggaag tgaaccagtg ttacaagctc   1020
atcttggagc tatcgggtaa gcaagatcaa gggtacaaac gcaagtatcc ctcaagaccc   1080
gggagcccaa atggtgtgat tgatgcctat tttagtggag atagctcgaa tgattcgtgg   1140
ggagtttctt cctcaatctc atcatcacca tcgattcctc gatttaaaag gatcaaatcc   1200
caggatcaac agatgaggtt gccttcaata aaccgtatgt ttgtggatgt gcttagtagt   1260
cctcattgat cttttacttt tgttatctat tgcctacttc gaaacaatgc cattataaat   1320
ttctctattt tgcatattat ttgtatnccc gtggttactt gggtacattt ccggcctaat   1380
atttgaactc actcaagcta gacaaattga aaaaaaaaaa aaaaa                   1425
```

<210> 15
<211> 355
<212> PRT
<213> Euphorbia esula

<400> 15

```
Met Glu Asp Ser Thr Gln Ile Ser Leu Ile Phe Asp Gly Leu Tyr Cys
1               5                   10                  15
Glu Glu Gln Gly Ile Gly Glu Asp Phe Asp Asp Gly Asn Glu Asp Tyr
            20                  25                  30
Val Lys Lys Glu Leu Ser Leu Ser Ser Val Leu Leu Glu Gln Asp Leu
        35                  40                  45
Phe Trp Thr Asp Asp Glu Leu Leu Asn Leu Ile Ser Lys Glu Lys Glu
    50                  55                  60
Thr His Phe Ser Phe Gly Asp Phe Ser Ser His Gly Ser Leu Met Val
65                  70                  75                  80
Ala Arg Lys Glu Ala Ile Asp Trp Ile Leu Arg Val Lys Gly Phe Tyr
                85                  90                  95
Gly Phe Asn Ala Leu Ser Cys Val Leu Ala Val Asn Tyr Phe Asp Arg
            100                 105                 110
Phe Ile Ser Ser Leu Val Phe Thr Arg Asp Lys Pro Trp Met Gly Gln
        115                 120                 125
Leu Ala Ala Val Ala Cys Leu Ser Leu Ala Ala Lys Met Glu Glu Thr
    130                 135                 140
Gln Val Pro Leu Leu Leu Asp Leu Gln Val Glu Glu Ser Lys Tyr Val
145                 150                 155                 160
Phe Glu Ala Lys Thr Ile Lys Arg Met Glu Leu Leu Val Leu Ser Thr
                165                 170                 175
Leu Gln Trp Arg Met Asn Pro Val Thr Pro Ile Cys Tyr Phe Asp His
            180                 185                 190
Ile Ile Arg Arg Leu Gly Leu Lys Asn His Leu His Trp Glu Phe Leu
```

195 200 205
Arg Arg Cys Glu Leu Leu Leu Leu Ser Val Ile Ser Asp Ser Arg Phe
210 215 220
Met Ser Tyr Ala Pro Ser Ile Leu Ala Thr Ser Ile Met Ile His Val
225 230 235 240
Ile Lys Glu Val Asp Pro Phe Ser Gln Met Glu Tyr Gln Asn Gln Leu
245 250 255
Leu Asp Val Ile Lys Ile Asn Lys Glu Glu Val Asn Gln Cys Tyr Lys
260 265 270
Leu Ile Leu Glu Leu Ser Gly Lys Gln Asp Gln Gly Tyr Lys Arg Lys
275 280 285
Tyr Pro Ser Arg Pro Gly Ser Pro Asn Gly Val Ile Asp Ala Tyr Phe
290 295 300
Ser Gly Asp Ser Ser Asn Asp Ser Trp Gly Val Ser Ser Ser Ile Ser
305 310 315 320
Ser Ser Pro Ser Ile Pro Arg Phe Lys Arg Ile Lys Ser Gln Asp Gln
325 330 335
Gln Met Arg Leu Pro Ser Ile Asn Arg Met Phe Val Asp Val Leu Ser
340 345 350
Ser Pro His
355

<210> 16
<211> 1501
<212> DNA
<213> Euphorbia esula

<400> 16

aaagaattcc tacaacctat gccttcttcc ccactcagat acccaaataa aaacacatac 60
ccttagataa ctggtcacta cccctcttcc tgctcttttt gtttctttga caaagagaga 120
gaaatggctc aaactgagtc tttaactctc tctttaatgt tcatcttcta ttcctttgcc 180
ctataatgaa ccccaatctc aattktatct tcttagaaag aagatggcaa atcattctcc 240
attatttctc tatgatgctc tttactgctc agaagaagat aactgggaag gagaagttgt 300
tgatattttt catgaacaag aagatcaagg agaaaacacc tctgtctttc cccaaaattc 360
ttccccagta gacttaaatt gggaagaaga tgagcttacg tctgtatttt ctaaacaaga 420
gcaaaaccaa ctatataaaa aactagaaat caacccatgt ctagctaaat ctcgccgtga 480
tgctgtggat tggatgatga aagtcaatgc ccattactct ttcactgctt tgacttcagt 540
tttggccgtt aattttcttg atagattcct ttttagcttc gatcttcaaa cggagaagcc 600
atggatgacc cagctcacag ctgtagcttg tctctcctta gcagcaaaag tagaagagac 660
acaagtccca cttttattgg accttcaggt ggtggacagt aagtatgtgt ttgaggctaa 720
aactatacaa agaatggagc ttttggtgct ttctactctt caatggagaa tgaaccctgt 780
aactccatta tcatttattg attacatgac aagaaggctt ggttttaagg attatctttg 840
ctgggaattt atccggagat gcgagcttat tgttctctct ataatctcag atatgagatt 900
tataccttat ctccctagtg aaattgcttc cgcaataatg ctacatgtga ttaatggtat 960
agaacccagt cttggagatg aattcgaaac ccagctattc gggattcttg gaattgataa 1020
ggagaaggtg aataattgca gagaaatgat aatcgagtta ggatcaagat attacggcaa 1080
ccaatcaaac aaaagaaaat acgggtcgga tccgggtagt ccaaattgcg taatggatgt 1140
ctcatttagt tcagataatt caaacgattc ttgggcagtc ggatctaaat catcatcagt 1200
gtcttcctca cccgcggcga agaaactcag ggcagtttca gggatgaacc atgaaaatgc 1260
gataatactt tcctaagcat gaacattttt taatccttaa aatcttttta atttatttca 1320
tttgaatccc cttttgagga cttggttgta ttaaactgtt aataattgat gttgttagtt 1380
aaattgccgg gcatctctgc ttctccaatc tcaattaaaa tcttaattag aattttggaa 1440
gcagagatgg ttggcatttt atccggaaat tagtagtaaa agagaaattg cactaaaaaa 1500
a 1501

<210> 17
<211> 350
<212> PRT

<213> Euphorbia esula

<400> 17

```
Met Ala Asn His Ser Pro Leu Phe Leu Tyr Asp Ala Leu Tyr Cys Ser
1               5                   10                  15
Glu Glu Asp Asn Trp Glu Gly Glu Val Val Asp Ile Phe His Glu Gln
            20                  25                  30
Glu Asp Gln Gly Glu Asn Thr Ser Val Phe Pro Gln Asn Ser Ser Pro
        35                  40                  45
Val Asp Leu Asn Trp Glu Glu Asp Glu Leu Thr Ser Val Phe Ser Lys
    50                  55                  60
Gln Glu Gln Asn Gln Leu Tyr Lys Lys Leu Glu Ile Asn Pro Cys Leu
65                  70                  75                  80
Ala Lys Ser Arg Arg Asp Ala Val Asp Trp Met Met Lys Val Asn Ala
                85                  90                  95
His Tyr Ser Phe Thr Ala Leu Thr Ser Val Leu Ala Val Asn Phe Leu
            100                 105                 110
Asp Arg Phe Leu Phe Ser Phe Asp Leu Gln Thr Glu Lys Pro Trp Met
        115                 120                 125
Thr Gln Leu Thr Ala Val Ala Cys Leu Ser Leu Ala Ala Lys Val Glu
    130                 135                 140
Glu Thr Gln Val Pro Leu Leu Leu Asp Leu Gln Val Val Asp Ser Lys
145                 150                 155                 160
Tyr Val Phe Glu Ala Lys Thr Ile Gln Arg Met Glu Leu Leu Val Leu
                165                 170                 175
Ser Thr Leu Gln Trp Arg Met Asn Pro Val Thr Pro Leu Ser Phe Ile
            180                 185                 190
Asp Tyr Met Thr Arg Arg Leu Gly Phe Lys Asp Tyr Leu Cys Trp Glu
        195                 200                 205
Phe Ile Arg Arg Cys Glu Leu Ile Val Leu Ser Ile Ile Ser Asp Met
    210                 215                 220
Arg Phe Ile Pro Tyr Leu Pro Ser Glu Ile Ala Ser Ala Ile Met Leu
225                 230                 235                 240
His Val Ile Asn Gly Ile Glu Pro Ser Leu Gly Asp Glu Phe Glu Thr
                245                 250                 255
Gln Leu Phe Gly Ile Leu Gly Ile Asp Lys Glu Lys Val Asn Asn Cys
            260                 265                 270
Arg Glu Met Ile Ile Glu Leu Gly Ser Arg Tyr Tyr Gly Asn Gln Ser
        275                 280                 285
Asn Lys Arg Lys Tyr Gly Ser Asp Pro Gly Ser Pro Asn Cys Val Met
    290                 295                 300
Asp Val Ser Phe Ser Ser Asp Asn Ser Asn Asp Ser Trp Ala Val Gly
305                 310                 315                 320
Ser Lys Ser Ser Ser Val Ser Ser Ser Pro Ala Ala Lys Lys Leu Arg
                325                 330                 335
Ala Val Ser Gly Met Asn His Glu Asn Ala Ile Ile Leu Ser
            340                 345                 350
```

<210> 18
<211> 1193
<212> DNA
<213> Helianthus annuus

<400> 18

```
aatacccaat ggccatttta tcacgatatt catcttcgaa cacactcttc tgcattgaag     60
aacaagttca tgaagatgaa gatgagttaa cacatcaaga ttcctctgca atccatccat    120
tagacctaca agatttgtgt tgggaacatg aagaacttgt ctctcttttc acaaaagaag    180
aagaacaaca aaagcaaacc ccttggcctt cttcttgtac tttgtctttt cgtaaagagg    240
ctgtggattg gatccttaaa gtcaaaggtt gtcatggatt cacacctcta acagccattt    300
tagccatcaa ttatcttgat cggtttctgt ccagtctcca ttttcagaaa gctaacacac    360
cttggatgat tcaccttgtt gctgttactt gtctttcttt ggctgctaaa attcaagaaa    420
ctcatgtgcc tttgctctta gatcttcagc tagaggagag taagttcttg tttgaggcca    480
agaacataca aaagacggag cttttggtga tgtcaacact gaaatggagg atgaacctag    540
tgacaccaat ctcatttctt gatcacattg taagaaggct tggattatca aatcatcttc    600
attgggattt cttcaagaaa tgtgaagcta tgattcttta cctagtggct gattcaagat    660
ttgtgtgcta taaaccatct gtgttggcaa ccgctacaat gctttgtgtt gtagaggaaa    720
tcgacccgac caattccatt ggctacaaaa gtcaacttct ggatcttctc aaaaccacta    780
aggaccacat aaatgagtgt tacaagcttg ttatggatct atcctatgat aatcacaaca    840
aaggaaagcg tgatgaaaac gagagaacaa tttatccggt tagtccagct ggttttattg    900
gttttatgtg ccacgaaagt tcaaatgatt catgatcctc gctcaagaat tgatcaataa    960
ttagggtttg gctcacttgt aagctttaaa ccactcgcaa gcactcgtta tccataacct   1020
atacaatcag cagaagttgc gctaataata gacccgtcgg tccaccacta gttatgttgt   1080
atggtatggt ctttaatttc tctgttgttt taggtcgttt ttaatgtgag ataagttaaa   1140
ctcggtgatg ttatcatgtc ttattcaagc aatgaattta tatattttac atc          1193
```

<210> 19
<211> 308
<212> PRT
<213> Helianthus annuus

<400> 19

```
Met Ala Ile Leu Ser Arg Tyr Ser Ser Ser Asn Thr Leu Phe Cys Ile
1               5                   10                  15
Glu Glu Gln Val His Glu Asp Glu Asp Glu Leu Thr His Gln Asp Ser
            20                  25                  30
Ser Ala Ile His Pro Leu Asp Leu Gln Asp Leu Cys Trp Glu His Glu
        35                  40                  45
Glu Leu Val Ser Leu Phe Thr Lys Glu Glu Glu Gln Gln Lys Gln Thr
    50                  55                  60
Pro Trp Pro Ser Ser Cys Thr Leu Ser Phe Arg Lys Glu Ala Val Asp
65                  70                  75                  80
Trp Ile Leu Lys Val Lys Gly Cys His Gly Phe Thr Pro Leu Thr Ala
                85                  90                  95
Ile Leu Ala Ile Asn Tyr Leu Asp Arg Phe Leu Ser Ser Leu His Phe
            100                 105                 110
Gln Lys Ala Asn Thr Pro Trp Met Ile His Leu Val Ala Val Thr Cys
        115                 120                 125
Leu Ser Leu Ala Ala Lys Ile Gln Glu Thr His Val Pro Leu Leu Leu
    130                 135                 140
Asp Leu Gln Leu Glu Glu Ser Lys Phe Leu Phe Glu Ala Lys Asn Ile
145                 150                 155                 160
Gln Lys Thr Glu Leu Leu Val Met Ser Thr Leu Lys Trp Arg Met Asn
                165                 170                 175
Leu Val Thr Pro Ile Ser Phe Leu Asp His Ile Val Arg Arg Leu Gly
```

```
                180                     185                     190
Leu Ser Asn His Leu His Trp Asp Phe Phe Lys Lys Cys Glu Ala Met
            195                     200                     205
Ile Leu Tyr Leu Val Ala Asp Ser Arg Phe Val Cys Tyr Lys Pro Ser
    210                     215                     220
Val Leu Ala Thr Ala Thr Met Leu Cys Val Val Glu Glu Ile Asp Pro
225                     230                     235                     240
Thr Asn Ser Ile Gly Tyr Lys Ser Gln Leu Leu Asp Leu Leu Lys Thr
                245                     250                     255
Thr Lys Asp His Ile Asn Glu Cys Tyr Lys Leu Val Met Asp Leu Ser
            260                     265                     270
Tyr Asp Asn His Asn Lys Gly Lys Arg Asp Glu Asn Glu Arg Thr Ile
        275                     280                     285
Tyr Pro Val Ser Pro Ala Gly Phe Ile Gly Phe Met Cys His Glu Ser
    290                     295                     300
Ser Asn Asp Ser
305
```

<210> 20
<211> 1400
<212> DNA
<213> Helianthus tuberosus

<400> 20

```
ttgaaccttc atttcttttc ttttcttctt tctaatcacc aaccccaatg gccattttat      60
caccatattc atcttctttc ttagacacac tcttttgcaa tgaacaacaa gatcatgaat     120
atcatgaata tgagtatgaa gatgaattta cacaaaccac cctcacagat tcatctgatc     180
tccatcttcc ccccctggac caactagatt tgtcatggga acatgaagag cttgtgtcct     240
tgttcacaaa agaacaagag cagcaaaaac aaaccccttg tactctctct tttggcaaaa     300
ctagtccctc agtttttgct gctcgtaaag aggctgtaga ttggatcctt aaggtcaaaa     360
gttgttatgg attcacacct cttacagcca ttttagccat caattatctt gataggtttc     420
tttctagcct ccattttcaa gaagataaac cttggatgat tcaacttgtt gctgttagtt     480
gtctctcttt agctgctaaa gttgaagaaa ctcaagtgcc actcttacta gatcttcaag     540
tagaggacac taagtacttg tttgaggcta aaaacataca aaaaatggag cttttggtga     600
tgtcaacttt gaaatggagg atgaacccag tgacaccaat ctcatttctt gatcacattg     660
taagaaggct tggattaact gatcatgttc attgggattt tttcaagaaa tgtgaagcta     720
tgatcctttg tttagtttca gattcaagat tcgtgtgtta taaaccatcc gtgttggcca     780
cagctacaat gcttcacgtt gtagatgaaa ttgatcctcc caattgtatt gactacaaaa     840
gtcaacttct ggatcttctc aaaaccacta aggacgacat aaacgagtgt tacgagctca     900
ttgtcgagct agcttacgat catcacaaca aacgaaaaca tgatgcaaac gagacaacaa     960
ccaatccggt tagtccagct ggcgtgatcg atttcacttg tgatgaaagt tcaaatgagt    1020
catgggaact taatgctcat catttccgcg agccttcatt caagaaaaca agaatggatt    1080
caacaattcg ggttcgggtt tggttcactt ataagcttta atcgagggta gttgtaaaca    1140
tgtaatccgc atgcacgcta ttaatcctac ggtccactac tacatataat cggcctataa    1200
aattataggt taagatgacc agtcgtaggc gtcgagatgt ccttatggtt ggtcaatttc    1260
tctatggttt taggtcgttt ttaatgtgag ataaattaaa ttcggtatgt taagtcttta    1320
tcaagcaatg gacgttatat ttattgtttg atattgagaa ttaaattcca tgggaaaaaa    1380
aaaaaaaaaa aaaaaaaaaa                                                1400
```

<210> 21
<211> 357
<212> PRT
<213> Helianthus tuberosus

<400> 21

```
Met Ala Ile Leu Ser Pro Tyr Ser Ser Ser Phe Leu Asp Thr Leu Phe
1               5                   10                  15
Cys Asn Glu Gln Gln Asp His Glu Tyr His Glu Tyr Glu Tyr Glu Asp
            20                  25                  30
Glu Phe Thr Gln Thr Thr Leu Thr Asp Ser Ser Asp Leu His Leu Pro
        35                  40                  45
Pro Leu Asp Gln Leu Asp Leu Ser Trp Glu His Glu Glu Leu Val Ser
    50                  55                  60
Leu Phe Thr Lys Glu Gln Glu Gln Gln Lys Gln Thr Pro Cys Thr Leu
65                  70                  75                  80
Ser Phe Gly Lys Thr Ser Pro Ser Val Phe Ala Ala Arg Lys Glu Ala
                85                  90                  95
Val Asp Trp Ile Leu Lys Val Lys Ser Cys Tyr Gly Phe Thr Pro Leu
            100                 105                 110
Thr Ala Ile Leu Ala Ile Asn Tyr Leu Asp Arg Phe Leu Ser Ser Leu
        115                 120                 125
His Phe Gln Glu Asp Lys Pro Trp Met Ile Gln Leu Val Ala Val Ser
    130                 135                 140
Cys Leu Ser Leu Ala Ala Lys Val Glu Glu Thr Gln Val Pro Leu Leu
145                 150                 155                 160
Leu Asp Leu Gln Val Glu Asp Thr Lys Tyr Leu Phe Glu Ala Lys Asn
                165                 170                 175
Ile Gln Lys Met Glu Leu Leu Val Met Ser Thr Leu Lys Trp Arg Met
            180                 185                 190
Asn Pro Val Thr Pro Ile Ser Phe Leu Asp His Ile Val Arg Arg Leu
        195                 200                 205
Gly Leu Thr Asp His Val His Trp Asp Phe Phe Lys Lys Cys Glu Ala
    210                 215                 220
Met Ile Leu Cys Leu Val Ser Asp Ser Arg Phe Val Cys Tyr Lys Pro
225                 230                 235                 240
Ser Val Leu Ala Thr Ala Thr Met Leu His Val Val Asp Glu Ile Asp
                245                 250                 255
Pro Pro Asn Cys Ile Asp Tyr Lys Ser Gln Leu Leu Asp Leu Leu Lys
            260                 265                 270
Thr Thr Lys Asp Asp Ile Asn Glu Cys Tyr Glu Leu Ile Val Glu Leu
        275                 280                 285
Ala Tyr Asp His His Asn Lys Arg Lys His Asp Ala Asn Glu Thr Thr
    290                 295                 300
Thr Asn Pro Val Ser Pro Ala Gly Val Ile Asp Phe Thr Cys Asp Glu
305                 310                 315                 320
Ser Ser Asn Glu Ser Trp Glu Leu Asn Ala His His Phe Arg Glu Pro
                325                 330                 335
Ser Phe Lys Lys Thr Arg Met Asp Ser Thr Ile Arg Val Arg Val Trp
            340                 345                 350
Phe Thr Tyr Lys Leu
        355
```

<210> 22
<211> 1630
<212> DNA
<213> Lagenaria siceraria

<400> 22

```
aagagcacaa gaaagctcat ccttaactgc tttcttcaca gggctggttg cttctgtcga     60
cggggattcg ggtgtgagct ttttattttt ctccgtgtct cgtacccccct cggagggtta   120
gtcctatgag ctcccggtgt cctcgctgct caattcagct ttgaggtcct ctccttcttt    180
tccactctga ataagataga ggaaaagaga gaaaccccag cttacagcca tgggtgagga    240
ttggtcttct tcgccatttt cctctgtttt tctccatttc cttctctcaa gctctacaat    300
gaaccaaaat cacagcctct ttcttctcag aagaagatgg taccaccgta tgcgcttgat    360
tctctttatt gttcagaaga ccactgggag aacgacgacg aagaagaaga aaggggtttt    420
catgagcaac cttattctaa tttaacaacc gaatcaagtt ctccgattct ggcagtggca    480
gagcaggatc tgttttggga aaacgatgaa ttaatttctc tgttctcaag agagaagcct    540
aatgaactgt ttaaaaccat tcagattgac ccttctcttg ctgccgcccg acgaagcgcc    600
gtcgggtgga tgctgaaggt taatgcccat tattctttct ctgctctcac tgcggttctc    660
gccgtcgatt atttggatcg gtttctgtcc tgttttcatt ttcaaagaga caagccatgg    720
atgtctcagc ttgctgctgt tgcttgtatc tctcttgctg ccaaagtaga ggagacccaa    780
gtccctcttt tattggacct acaagtggaa gacagtagat atctatttga agccaagaca    840
attaagaaaa tggagcttct tgtgctctct acgcttcaat ggcggatgaa tcctgttacc    900
ccattttctt ttgtggatta tatctcaagg aggcttggat tcaaggaaca tatctgctgg    960
gaaattcttt ggcagtgtga gcgaactatt ctctctgtta ttttagagtc agattttatg   1020
tcctttcttc cttctgtaat ggccaccgct acaatgctgc acgttttcaa agctatggaa   1080
gaacccaccc tcagcgttga atacgattcc cagcttctta acatcctcgg aatcgacaag   1140
gggaatgtgg aagaatgctg taagctgatc tcaaatgcat caagaagaaa cggcaaccaa   1200
ttcaagaaac gtaaaattgg gtcgattccg ggtagcccga acggcgtgat ggacgtgtca   1260
ttcagctccg atagctcgaa cgactcgtgg tcagtggcct cgtcagtttc atcttcgcca   1320
gagccattaa cgaagaagaa cagagccaat ggatcaatgt ctggagattg cgaaacattc   1380
agaaccctct cttaattaac ttccctttct tctttttcct cgtaatcctt gtatgttgaa   1440
taagaattag aatcaatctt tttatttatc gaattctgcg agttaaattg ccttaccatc   1500
tctgcagtta agagccaatg gatgggcaat cggaagttta aaacgcagag atggctggca   1560
ttttatccgg atgcaaagga cattgaacgt ataacaatga agagtagttt aagttgttta   1620
aaaagaaaaa                                                          1630
```

<210> 23
<211> 352
<212> PRT
<213> Lagenaria siceraria

<400> 23

```
Met Val Pro Pro Tyr Ala Leu Asp Ser Leu Tyr Cys Ser Glu Asp His
1               5                   10                  15
Trp Glu Asn Asp Asp Glu Glu Glu Glu Arg Gly Phe His Glu Gln Pro
            20                  25                  30
Tyr Ser Asn Leu Thr Thr Glu Ser Ser Ser Pro Ile Leu Ala Val Ala
        35                  40                  45
Glu Gln Asp Leu Phe Trp Glu Asn Asp Glu Leu Ile Ser Leu Phe Ser
    50                  55                  60
Arg Glu Lys Pro Asn Glu Leu Phe Lys Thr Ile Gln Ile Asp Pro Ser
65                  70                  75                  80
Leu Ala Ala Ala Arg Arg Ser Ala Val Gly Trp Met Leu Lys Val Asn
                85                  90                  95
Ala His Tyr Ser Phe Ser Ala Leu Thr Ala Val Leu Ala Val Asp Tyr
            100                 105                 110
Leu Asp Arg Phe Leu Ser Cys Phe His Phe Gln Arg Asp Lys Pro Trp
        115                 120                 125
Met Ser Gln Leu Ala Ala Val Ala Cys Ile Ser Leu Ala Ala Lys Val
    130                 135                 140
Glu Glu Thr Gln Val Pro Leu Leu Leu Asp Leu Gln Val Glu Asp Ser
```

```
145                 150                 155                 160
Arg Tyr Leu Phe Glu Ala Lys Thr Ile Lys Lys Met Glu Leu Leu Val
                165                 170                 175
Leu Ser Thr Leu Gln Trp Arg Met Asn Pro Val Thr Pro Phe Ser Phe
            180                 185                 190
Val Asp Tyr Ile Ser Arg Arg Leu Gly Phe Lys Glu His Ile Cys Trp
        195                 200                 205
Glu Ile Leu Trp Gln Cys Glu Arg Thr Ile Leu Ser Val Ile Leu Glu
    210                 215                 220
Ser Asp Phe Met Ser Phe Leu Pro Ser Val Met Ala Thr Ala Thr Met
225                 230                 235                 240
Leu His Val Phe Lys Ala Met Glu Glu Pro Thr Leu Ser Val Glu Tyr
                245                 250                 255
Asp Ser Gln Leu Leu Asn Ile Leu Gly Ile Asp Lys Gly Asn Val Glu
            260                 265                 270
Glu Cys Cys Lys Leu Ile Ser Asn Ala Ser Arg Arg Asn Gly Asn Gln
        275                 280                 285
Phe Lys Lys Arg Lys Ile Gly Ser Ile Pro Gly Ser Pro Asn Gly Val
    290                 295                 300
Met Asp Val Ser Phe Ser Ser Asp Ser Ser Asn Asp Ser Trp Ser Val
305                 310                 315                 320
Ala Ser Ser Val Ser Ser Ser Pro Glu Pro Leu Thr Lys Lys Asn Arg
                325                 330                 335
Ala Asn Gly Ser Met Ser Gly Asp Cys Glu Thr Phe Arg Thr Leu Ser
            340                 345                 350
```

<210> 24
<211> 1565
<212> DNA
<213> Lagenaria siceraria

<400> 24

```
tggacacgaa tttcttcgac tagtaagcac caaaagctcc ataaaagaga aaaaaaaaag      60
gggggaaatg gggtttctct tcctctccct ataatgactc cgcaatactc aatcctcttc     120
ttccaagaag aagaagatga agaagatggc tttgcactca aataaacaca gaacccaacg     180
cctccataac tctctcttct tcttcgactt cctccactgc actgaacaac aacaccttca     240
aacagagcat cccattttcc ttaacaatgg gggcaccaac gacttccctc ttttccaaca     300
aacaaccacc catttccttg tttacgaaga cgaggagctc aatcatttgt tgtccaaaga     360
aaaggaccaa aatctccaaa ccggtgctgt tttgaaaacc ttggttcaaa cagataatgc     420
tctgtctctc gctagaacag aggccatcga ctggttgctt aaagttaatg ccttttatgg     480
tttctcctct ctcacagctc tcttagccat taattacctc gatagaatcc tctctgggcc     540
ctattttcaa agagataagc catggatgct tcagcttgct gctgtaactt gcatctcttt     600
agctgctaaa gtcgaagaaa ttcgtgtccc tcttcttcta gacctccagg tggaagattc     660
aaagtacatt tttgaagcga aaacgataca gaggatggag cttttagtgc ttactgctct     720
gcaatggaag atgcacccag tggcccctgt ttcgtttctt ggcattatca caaaaggact     780
tggaatgaag aatcagtaca ttcaaagaga gtttcttaga cgctgtgagc gtattcttct     840
ctctctcgtc tctgattcga gatcggtggg gattcttcct tctataatgg cggtatcagc     900
aatggtgagc gttgttgaag agatgggaaa ctgtaaccca ttggaggagt ttcaggatca     960
gcttcttaat gccctcaaaa taaataaggg gagagtgaag gagtgttgta aagtgataat    1020
ggaggcaaaa ataaaaggat cagggaagag gaagcatgtg gaggaggaag cagaagcaga    1080
agcagaatca gaatcatcag aagcagaaac agagggagaa gcagaagcag aagcagggag    1140
cccaaatgga gtaatggagg ctaatttcag ctgtgaaagc tccaacgatt cgtgggaaat    1200
ggggacgatt gtgtcagaat acacacattt ttcttcttct tcttcttctt cttccaaaag    1260
aatcagaccc actcgatgaa ttatttgaat aatgaattga accaaatttg cagagattca    1320
```

```
atatccatta cattagcctt cccctgttct gaaatggcac attggcaaac acacaaaaac   1380
cagagacaaa aaagtgggga aaaacagaga agtaatgatg acgatgatga tgagagaggt   1440
tgttcttctc tttctttata gctcattaat tatgttgtat atgatgaaat tgatgagtat   1500
ttgcttctat taatattgct ctcttcttca tttcaaaaaa aaaaaaaagt tgaccacgcg   1560
tggcc                                                                1565
```

<210> 25
<211> 380
<212> PRT
<213> Lagenaria siceraria

<400> 25

```
Met Lys Lys Met Ala Leu His Ser Asn Lys His Arg Thr Gln Arg Leu
1               5                   10                  15
His Asn Ser Leu Phe Phe Phe Asp Phe Leu His Cys Thr Glu Gln Gln
            20                  25                  30
His Leu Gln Thr Glu His Pro Ile Phe Leu Asn Asn Gly Gly Thr Asn
        35                  40                  45
Asp Phe Pro Leu Phe Gln Gln Thr Thr Thr His Phe Leu Val Tyr Glu
    50                  55                  60
Asp Glu Glu Leu Asn His Leu Leu Ser Lys Glu Lys Asp Gln Asn Leu
65                  70                  75                  80
Gln Thr Gly Ala Val Leu Lys Thr Leu Val Gln Thr Asp Asn Ala Leu
                85                  90                  95
Ser Leu Ala Arg Thr Glu Ala Ile Asp Trp Leu Leu Lys Val Asn Ala
            100                 105                 110
Phe Tyr Gly Phe Ser Ser Leu Thr Ala Leu Leu Ala Ile Asn Tyr Leu
        115                 120                 125
Asp Arg Ile Leu Ser Gly Pro Tyr Phe Gln Arg Asp Lys Pro Trp Met
    130                 135                 140
Leu Gln Leu Ala Ala Val Thr Cys Ile Ser Leu Ala Ala Lys Val Glu
145                 150                 155                 160
Glu Ile Arg Val Pro Leu Leu Leu Asp Leu Gln Val Glu Asp Ser Lys
                165                 170                 175
Tyr Ile Phe Glu Ala Lys Thr Ile Gln Arg Met Glu Leu Leu Val Leu
            180                 185                 190
Thr Ala Leu Gln Trp Lys Met His Pro Val Ala Pro Val Ser Phe Leu
        195                 200                 205
Gly Ile Ile Thr Lys Gly Leu Gly Met Lys Asn Gln Tyr Ile Gln Arg
    210                 215                 220
Glu Phe Leu Arg Arg Cys Glu Arg Ile Leu Leu Ser Leu Val Ser Asp
225                 230                 235                 240
Ser Arg Ser Val Gly Ile Leu Pro Ser Ile Met Ala Val Ser Ala Met
                245                 250                 255
Val Ser Val Val Glu Glu Met Gly Asn Cys Asn Pro Leu Glu Glu Phe
            260                 265                 270
Gln Asp Gln Leu Leu Asn Ala Leu Lys Ile Asn Lys Gly Arg Val Lys
        275                 280                 285
Glu Cys Cys Lys Val Ile Met Glu Ala Lys Ile Lys Gly Ser Gly Lys
    290                 295                 300
Arg Lys His Val Glu Glu Glu Ala Glu Ala Glu Ala Glu Ser Glu Ser
305                 310                 315                 320
Ser Glu Ala Glu Thr Glu Gly Glu Ala Glu Ala Glu Ala Gly Ser Pro
                325                 330                 335
```

```
Asn Gly Val Met Glu Ala Asn Phe Ser Cys Glu Ser Ser Asn Asp Ser
            340                 345                 350
Trp Glu Met Gly Thr Ile Val Ser Glu Tyr Thr His Phe Ser Ser Ser
        355                 360                 365
Ser Ser Ser Ser Ser Lys Arg Ile Arg Pro Thr Arg
    370                 375                 380
```

<210> 26
<211> 1518
<212> DNA
<213> Lycopersicon esculentum

<400> 26

```
ctcctttgg ctcttcctat tccctctctc tcttcccctt ttttctgtcc tttagagaga      60
gaaaaaaaat ccataagcca agcagcagat atgttactgg gtccaagatt gagttttggc   120
ttaccttaaa gataatgagt agagccttca ttgtcttctt ccctctagaa gaaggagaag   180
atggttttcc ctttagattc ccagctccaa aatcctattt ctgctcttct tgatggcctt   240
tactgtgagg aagatcgatt cttggatgat gatttagggg aatggtctag tttagatgtc   300
ggaaatgaaa atgttaaaaa gactctgcct ttattagaat gtgacatgtt ttgggaacat   360
gatgagcttg ccacactttt atctaaggaa aatgagtttc atttgggttt tcaatcttta   420
atctcagatg ggtctttaat gggggctaga aaagaggctt tggattggat gttgagggtc   480
attgcttact atggttttac tgctaccact gctgttttag ctgtgaacta ttttgatagg   540
tttgtgtctg gatggtgctt tcagaaagat aagccttgga tgagtcagct tgctgctgtt   600
gcctgtcttt ccattgctgc taaagtggag gagacccaag ttccccttttt gttagaccta   660
caagttgctg attctagatt tgtgtttgag gcaaagacta tacagagaat ggaactcttg   720
gtgctttcta ctcttaagtg gaaaatgaat ctggtgacac cattatcttt cattgatcat   780
attatgagga gatttggatt catgagcaac ctgcatatgg attttcttaa gaagtgtgaa   840
cgcctcattc ttgatatcat cactgattct aggctcttgc attatcctcc atctgttatt   900
gcaactgcat cgatgtttta tgtgatcaat gacattgagc ctagcaatgc tatggaatac   960
caaaatcagc tcatgagtgt tcttaaagtc agaaaggaca tctttgagga atgccatgat  1020
cttattcttg agctaatgga cactgcctgt tacaagctct gccaaagcct caagcgcaaa  1080
catcattcag tacctggtag tccaagtggt gttattgatg catattttag tagtgagagc  1140
tcgaatgaat catggtcagt agcatcttcg atttcatcct cacctgagcc tcagtataag  1200
agaaacaaaa ctcaagatca gcgaatgaca ctagctccac tgggtagtaa tcttcactga  1260
tcgatatctt gttctctaga ttacctagta tttcggcaat ggtttactct ctttttttggt  1320
atgttctctt aaaaatgcaa ttgcacaatg ctctgatgct ccatttaagt tttactggac  1380
ttaatttgtc cgatgatcgt ctagactatg tgaacatcaa ctccaccccc ctccttcatt  1440
ggacatggga ttggtggagt tttcccttga gttgaatcaa tgctgctgaa ttatgttgaa  1500
aaaaaaaaaa aaaaaaaa                                                 1518
```

<210> 27
<211> 359
<212> PRT
<213> Lycopersicon esculentum

<400> 27

```
Met Val Phe Pro Leu Asp Ser Gln Leu Gln Asn Pro Ile Ser Ala Leu
1               5                   10                  15
Leu Asp Gly Leu Tyr Cys Glu Glu Asp Arg Phe Leu Asp Asp Asp Leu
            20                  25                  30
Gly Glu Trp Ser Ser Leu Asp Val Gly Asn Glu Asn Val Lys Lys Thr
        35                  40                  45
Leu Pro Leu Leu Glu Cys Asp Met Phe Trp Glu His Asp Glu Leu Ala
    50                  55                  60
```

```
Thr Leu Leu Ser Lys Glu Asn Glu Phe His Leu Gly Phe Gln Ser Leu
65                  70                  75                  80
Ile Ser Asp Gly Ser Leu Met Gly Ala Arg Lys Glu Ala Leu Asp Trp
                85                  90                  95
Met Leu Arg Val Ile Ala Tyr Tyr Gly Phe Thr Ala Thr Thr Ala Val
            100                 105                 110
Leu Ala Val Asn Tyr Phe Asp Arg Phe Val Ser Gly Trp Cys Phe Gln
        115                 120                 125
Lys Asp Lys Pro Trp Met Ser Gln Leu Ala Ala Val Ala Cys Leu Ser
    130                 135                 140
Ile Ala Ala Lys Val Glu Glu Thr Gln Val Pro Leu Leu Leu Asp Leu
145                 150                 155                 160
Gln Val Ala Asp Ser Arg Phe Val Phe Glu Ala Lys Thr Ile Gln Arg
                165                 170                 175
Met Glu Leu Leu Val Leu Ser Thr Leu Lys Trp Lys Met Asn Leu Val
            180                 185                 190
Thr Pro Leu Ser Phe Ile Asp His Ile Met Arg Arg Phe Gly Phe Met
        195                 200                 205
Ser Asn Leu His Met Asp Phe Leu Lys Lys Cys Glu Arg Leu Ile Leu
    210                 215                 220
Asp Ile Ile Thr Asp Ser Arg Leu Leu His Tyr Pro Pro Ser Val Ile
225                 230                 235                 240
Ala Thr Ala Ser Met Phe Tyr Val Ile Asn Asp Ile Glu Pro Ser Asn
                245                 250                 255
Ala Met Glu Tyr Gln Asn Gln Leu Met Ser Val Leu Lys Val Arg Lys
            260                 265                 270
Asp Ile Phe Glu Glu Cys His Asp Leu Ile Leu Glu Leu Met Asp Thr
        275                 280                 285
Ala Cys Tyr Lys Leu Cys Gln Ser Leu Lys Arg Lys His His Ser Val
    290                 295                 300
Pro Gly Ser Pro Ser Gly Val Ile Asp Ala Tyr Phe Ser Ser Glu Ser
305                 310                 315                 320
Ser Asn Glu Ser Trp Ser Val Ala Ser Ser Ile Ser Ser Ser Pro Glu
                325                 330                 335
Pro Gln Tyr Lys Arg Asn Lys Thr Gln Asp Gln Arg Met Thr Leu Ala
            340                 345                 350
Pro Leu Gly Ser Asn Leu His
        355
```

<210> 28
<211> 1459
<212> DNA
<213> Lycopersicon esculentum

<400> 28

```
acagagatat tagaagggga aaaaaatggc aatagagaat aatgatcaat cttttttttt     60
agatgtgctt tactgtgaag aagaagaaga aaaatggggt gatttgttag aggatgaaga    120
aggggttatt attaacccat tgttgttatc ttccgaagga acaacaaaaa ctaattcttt    180
attattatta cctctgcttc tgttggaaca agatttgttt tgggaagatg aagagcttct    240
ttcacttttc gttaaagaaa aagaaactcg ttgttgtttt gaaagttttg ggagtgaccc    300
ttttctctgt tcagctcgtg ttgatgttgt tgaatggatt cttaaagtga atgctcatta    360
tgatttctca gcattgactg ccattttagc cattaattat cttgacaggt ttctttctag    420
ccttcaattt cagaaagata agccatggat gactcaactt gctgctgtca cttgtctttc    480
tttagcggct aaagttgaag aaactcaagt tccccttctt cttgacttcc aagtggagga    540
```

```
tgcaaaatat gtgtttgagg caaagactat acaaagaatg gagcttctgg tactgtcatc    600
actgaaatgg aggatgaatc cagtgacccc actttcattt cttgatcata ttataagaag    660
gcttgggcta aagaacaatg ttcattggga atttctcaga agatgtgaaa gtcttcttct    720
atctgtcatg attgattgta gatttgtacg ttatatgcct tctgtattgg ctactgcaat    780
tatgcttcat gttattcatc aaattgagcc ttgtaatgct attgactatc aaaatcaact    840
tcttggggtt ctcaaaatta gcaaggagaa tgtgaataat tgctatgaac tcatatccga    900
agtgtcatca aagcctatta catcacacaa acgcaaatat gatgaaaatc ccagtagtcc    960
aagtggtgta atagatccaa tttacacttc agaaagttca aatgattcat gggatttaga   1020
tttgccttcg ttcaagaaaa gcaaagttca agaacagcaa atgaaaatgt catcatcatt   1080
gagcagagtt tttgtggaag ctgttggtag tcctcattaa aatgtctctt ttaaatattt   1140
taattacata ttcaagaagt attttgctgt tatgtgttag ctgtggttgt tggcagagaa   1200
gagaagatga gtggctttat tttttgcagg agtgtagtct actactacta ctgtgaagcc   1260
agagagagaa agagaaaaga cagaatatgt gcaatctttg tttttctctc tatttatttc   1320
aattttctct ctcaagtcac tttcatgcat gcatactttt gatggactac tctatttata   1380
ttgcctttac ttattagtac ttaatatata tatatatata tatatatata tataaatcat   1440
ataaaaaaaa aaaaaaaaa                                                1459
```

<210> 29
<211> 364
<212> PRT
<213> Lycopersicon esculentum

<400> 29

```
Met Ala Ile Glu Asn Asn Asp Gln Ser Phe Phe Leu Asp Val Leu Tyr
1               5                   10                  15
Cys Glu Glu Glu Glu Glu Lys Trp Gly Asp Leu Leu Glu Asp Glu Glu
            20                  25                  30
Gly Val Ile Ile Asn Pro Leu Leu Leu Ser Ser Glu Gly Thr Thr Lys
        35                  40                  45
Thr Asn Ser Leu Leu Leu Leu Pro Leu Leu Leu Leu Glu Gln Asp Leu
    50                  55                  60
Phe Trp Glu Asp Glu Glu Leu Leu Ser Leu Phe Val Lys Glu Lys Glu
65                  70                  75                  80
Thr Arg Cys Cys Phe Glu Ser Phe Gly Ser Asp Pro Phe Leu Cys Ser
                85                  90                  95
Ala Arg Val Asp Val Val Glu Trp Ile Leu Lys Val Asn Ala His Tyr
            100                 105                 110
Asp Phe Ser Ala Leu Thr Ala Ile Leu Ala Ile Asn Tyr Leu Asp Arg
        115                 120                 125
Phe Leu Ser Ser Leu Gln Phe Gln Lys Asp Lys Pro Trp Met Thr Gln
    130                 135                 140
Leu Ala Ala Val Thr Cys Leu Ser Leu Ala Ala Lys Val Glu Glu Thr
145                 150                 155                 160
Gln Val Pro Leu Leu Leu Asp Phe Gln Val Glu Asp Ala Lys Tyr Val
                165                 170                 175
Phe Glu Ala Lys Thr Ile Gln Arg Met Glu Leu Leu Val Leu Ser Ser
            180                 185                 190
Leu Lys Trp Arg Met Asn Pro Val Thr Pro Leu Ser Phe Leu Asp His
        195                 200                 205
Ile Ile Arg Arg Leu Gly Leu Lys Asn Asn Val His Trp Glu Phe Leu
    210                 215                 220
Arg Arg Cys Glu Ser Leu Leu Leu Ser Val Met Ile Asp Cys Arg Phe
225                 230                 235                 240
Val Arg Tyr Met Pro Ser Val Leu Ala Thr Ala Ile Met Leu His Val
```

```
                245                 250                 255
Ile His Gln Ile Glu Pro Cys Asn Ala Ile Asp Tyr Gln Asn Gln Leu
            260                 265                 270
Leu Gly Val Leu Lys Ile Ser Lys Glu Asn Val Asn Asn Cys Tyr Glu
        275                 280                 285
Leu Ile Ser Glu Val Ser Ser Lys Pro Ile Thr Ser His Lys Arg Lys
    290                 295                 300
Tyr Asp Glu Asn Pro Ser Ser Pro Ser Gly Val Ile Asp Pro Ile Tyr
305                 310                 315                 320
Thr Ser Glu Ser Ser Asn Asp Ser Trp Asp Leu Asp Leu Pro Ser Phe
                325                 330                 335
Lys Lys Ser Lys Val Gln Glu Gln Gln Met Lys Met Ser Ser Ser Leu
            340                 345                 350
Ser Arg Val Phe Val Glu Ala Val Gly Ser Pro His
        355                 360
```

<210> 30
<211> 1449
<212> DNA
<213> Lycopersicon esculentum

<400> 30

```
acaaacctta ttttctttt tcttttttta aattcagttt cttgaattag tatgaagtaa     60
atgaaaacac aactaaactt cttcttctca gaagaagata atgtctcacc attatcaaga    120
acaagaacaa ctagaagcac aaaagattcc atttttatta gattcacttt actgtgaaga    180
aaataatata ctcactgaag tatcaataga gacagagagt ttttcttcac atgatttgtt    240
atgggaagaa gaagaactta cctctctgtt ttctaaagaa acagagtatg aaataagcta    300
caatgtgtta gaaaaaaacc agtcttttat ttcatcaaga agagaatcag ttgaatggat    360
actcaaaaca actgcttatt actcttttc tgctcaaact ggatttcttg cagttaatta    420
ctttgataga tttctgttat ttagttttaa tcagtctctg aatcataagc catggatgaa    480
tcaacttgtt gctgttactt gtctttcatt agctgctaaa gttgaagaaa ctgatgttcc    540
tctgcttctt gaccttcaag ttgaggaatc aggatttttg tttgaatcta aaacaataca    600
gagaatggag atgttgattc tgtctacact taaatggaag atgaatccag taacccatt    660
ttcatttctt gattttataa ctagaagact tggattgaag cactgtctat ctttggaatt    720
tctgaggaga tgtgagaaag tgcttcttta cacaattact gatgatagat tcattggtta    780
ccttccttct gcaatggcat ctgccacaat gttgcatgtt cttgataggc ttaagccttg    840
cattggagaa aagtaccaag atcaactttt gggcattctt ggaattgtca aggagaaggt    900
ggaaggatgt tacaggctaa tacaagaagt ggcttgcaac attgactttg gttcaaataa    960
gagaaagttt gggacattac cagggagtcc aacaggggtt atggatatgt catttagctc   1020
agattactcc aatgactcat ggtcagtggc tacatcagtt acttcatcac ctgagccatt   1080
gtccaagaag attagggagt caaatgaatg actaattaaa tgtcttttaa atatttcctt   1140
attagtagac tagttattat tgttattatt agatatgtat aatgtccaat aacagtgatc   1200
cacttgtctc ttagtttaag tattagtaat taattaagtt cttgtttatt cacatgttaa   1260
ttttgcttac atgtaaaaat tcaaaccccc ttttgacaat tatcacagct agctagctaa   1320
tggaccattt gtttaatgct gtactgtcta tagtggggct atgagatttt gtactagtta   1380
aaagatggtt tggcacttta ttccaaggaa ataaaaactt gcatgaaaaa caaaaaaaaa   1440
aaaaaaaaa                                                           1449
```

<210> 31
<211> 336
<212> PRT
<213> Lycopersicon esculentum

<400> 31

```
Met Ser His His Tyr Gln Glu Gln Glu Gln Leu Glu Ala Gln Lys Ile
1               5                   10                  15
Pro Phe Leu Leu Asp Ser Leu Tyr Cys Glu Glu Asn Asn Ile Leu Thr
            20                  25                  30
Glu Val Ser Ile Glu Thr Glu Ser Phe Ser Ser His Asp Leu Leu Trp
        35                  40                  45
Glu Glu Glu Glu Leu Thr Ser Leu Phe Ser Lys Glu Thr Glu Tyr Glu
    50                  55                  60
Ile Ser Tyr Asn Val Leu Glu Lys Asn Gln Ser Phe Ile Ser Ser Arg
65                  70                  75                  80
Arg Glu Ser Val Glu Trp Ile Leu Lys Thr Thr Ala Tyr Tyr Ser Phe
                85                  90                  95
Ser Ala Gln Thr Gly Phe Leu Ala Val Asn Tyr Phe Asp Arg Phe Leu
            100                 105                 110
Leu Phe Ser Phe Asn Gln Ser Leu Asn His Lys Pro Trp Met Asn Gln
        115                 120                 125
Leu Val Ala Val Thr Cys Leu Ser Leu Ala Ala Lys Val Glu Glu Thr
    130                 135                 140
Asp Val Pro Leu Leu Leu Asp Leu Gln Val Glu Glu Ser Gly Phe Leu
145                 150                 155                 160
Phe Glu Ser Lys Thr Ile Gln Arg Met Glu Met Leu Ile Leu Ser Thr
                165                 170                 175
Leu Lys Trp Lys Met Asn Pro Val Thr Pro Phe Ser Phe Leu Asp Phe
            180                 185                 190
Ile Thr Arg Arg Leu Gly Leu Lys His Cys Leu Ser Leu Glu Phe Leu
        195                 200                 205
Arg Arg Cys Glu Lys Val Leu Leu Tyr Thr Ile Thr Asp Asp Arg Phe
    210                 215                 220
Ile Gly Tyr Leu Pro Ser Ala Met Ala Ser Ala Thr Met Leu His Val
225                 230                 235                 240
Leu Asp Arg Leu Lys Pro Cys Ile Gly Glu Lys Tyr Gln Asp Gln Leu
                245                 250                 255
Leu Gly Ile Leu Gly Ile Val Lys Glu Lys Val Glu Gly Cys Tyr Arg
            260                 265                 270
Leu Ile Gln Glu Val Ala Cys Asn Ile Asp Phe Gly Ser Asn Lys Arg
        275                 280                 285
Lys Phe Gly Thr Leu Pro Gly Ser Pro Thr Gly Val Met Asp Met Ser
    290                 295                 300
Phe Ser Ser Asp Tyr Ser Asn Asp Ser Trp Ser Val Ala Thr Ser Val
305                 310                 315                 320
Thr Ser Ser Pro Glu Pro Leu Ser Lys Lys Ile Arg Glu Ser Asn Glu
                325                 330                 335
```

<210> 32
<211> 1861
<212> DNA
<213> Medicago sativa

<400> 32

```
gaattcggca cgagctcttc tgctacgact acctctccct atactctcta ctctttctag      60
ttctactact tttctttctt tctctgttct ctctctcttc ttcatttctt cacattttca     120
cacacacaga gaagacagaa caaagaggaa aagagagagc gatggatgtg agactcttca     180
gtactgtttc cttcttttta taatgaacaa aggaccacac accctcttct tcactgaaga     240
agatggctat ccatcatcat catcacaatc accaacaact tcaacaacac acttcttctc     300
```

```
tttttgatgc actttactgt gatgaagaag aaaaatggga agatgatgat gaaggagaag    360
ttgtagatga aggagcacaa agtgatgtca caacaacaaa ctatgatata ttggactcta    420
cttccctttt acctctgctt ttgttagaac agaacttgtt caatgaagat gaagaactca    480
acactctttt ctccaaagag ataactcaac aagaaacata ttacgaggat ctgaaaaatg    540
tgatcaactt tgactcactc tctcaaccac gtcgtgaagc tgttgaatgg atgcttaaag    600
tcaatgctca ttatggtttc tctgctctca ctgcaacact tgctgttaac tatcttgata    660
ggtttctttt aagcttccat ttccaaaaag agaaaccatg gatgattcag cttgttgctg    720
ttacttgcat ctctttagct gctaaagttg aagaaactca agttcctctt ctcttagacc    780
ttcaagtgca agatactaaa tatgtgtttg aggcaaagac tattcagaga atggagctat    840
tgattctgtc aacactgaaa tggaagatgc atccagtgac aacacactct tttctagatc    900
acattataag aaggcttgga ttgaaaacta atcttcattg ggagtttctt aggcgctgtg    960
agaatcttct tctatctgta cttttagatt caagatttgt tggttgtgtt ccttctgtgt   1020
tggccactgc tacaatgttg catgttatag accagattga acagagtgat gataatggtg   1080
tggattacaa aaatcagctt cttaatgttc tcaaaatcag caaggagaaa gttgatgaat   1140
gttataatgc gattcttcat cttacaaatg caaataatta tggtcataaa cgaaaatatg   1200
aagaaatccc tggtagtcca agtggcgtaa ttgatgctgt ttttagttct gatggttcta   1260
acgattcgtg gacagtggga gcatcatcat attcaacctc agagcctgtg tttaagaaga   1320
ccaagaatca aggacaaaat atgaatttgt caccgattaa cagggtcatt gtcggaattc   1380
ttgccactgc aacctctcct taaaaccctc tatccgtttt ctgtcctttt tatttaaaaa   1440
aaaataacca tataaaaaat tacccccaaa aaaaagatct atatttattt actatggtta   1500
tgttcatgtt gctactaaac tctagttagt ttagtagtct ttctttctat ctcttcattt   1560
ccaacaatgt cccaaattca tttacatgaa tctcttgaag aggcagtggc aagatgatga   1620
tagaggatta aaggaatggt taatttctga tgagttaaaa aggaaaggac aaagttggca   1680
atgaagattt ttattactat gagcagaaaa gaaccctatg atatctgttt catttcaagg   1740
cactgttttt ttattttatt caatggttct cttctagacc atacccaatt tggacatatt   1800
tatatcatat ttctataata aattgggaat aatttttggt ccaaaaaaaa aaaaaaaaaa   1860
a                                                                   1861
```

<210> 33
<211> 386
<212> PRT
<213> Medicago sativa

<400> 33

```
Met Ala Ile His His His His His Asn His Gln Gln Leu Gln Gln His
1               5                   10                  15
Thr Ser Ser Leu Phe Asp Ala Leu Tyr Cys Asp Glu Glu Glu Lys Trp
            20                  25                  30
Glu Asp Asp Asp Glu Gly Glu Val Val Asp Glu Gly Ala Gln Ser Asp
        35                  40                  45
Val Thr Thr Thr Asn Tyr Asp Ile Leu Asp Ser Thr Ser Leu Leu Pro
    50                  55                  60
Leu Leu Leu Leu Glu Gln Asn Leu Phe Asn Glu Asp Glu Glu Leu Asn
65                  70                  75                  80
Thr Leu Phe Ser Lys Glu Ile Thr Gln Gln Glu Thr Tyr Tyr Glu Asp
                85                  90                  95
Leu Lys Asn Val Ile Asn Phe Asp Ser Leu Ser Gln Pro Arg Arg Glu
            100                 105                 110
Ala Val Glu Trp Met Leu Lys Val Asn Ala His Tyr Gly Phe Ser Ala
        115                 120                 125
Leu Thr Ala Thr Leu Ala Val Asn Tyr Leu Asp Arg Phe Leu Leu Ser
    130                 135                 140
Phe His Phe Gln Lys Glu Lys Pro Trp Met Ile Gln Leu Val Ala Val
145                 150                 155                 160
```

```
Thr Cys Ile Ser Leu Ala Ala Lys Val Glu Glu Thr Gln Val Pro Leu
                165                 170                 175
Leu Leu Asp Leu Gln Val Gln Asp Thr Lys Tyr Val Phe Glu Ala Lys
            180                 185                 190
Thr Ile Gln Arg Met Glu Leu Leu Ile Leu Ser Thr Leu Lys Trp Lys
        195                 200                 205
Met His Pro Val Thr Thr His Ser Phe Leu Asp His Ile Ile Arg Arg
    210                 215                 220
Leu Gly Leu Lys Thr Asn Leu His Trp Glu Phe Leu Arg Arg Cys Glu
225                 230                 235                 240
Asn Leu Leu Leu Ser Val Leu Leu Asp Ser Arg Phe Val Gly Cys Val
                245                 250                 255
Pro Ser Val Leu Ala Thr Ala Thr Met Leu His Val Ile Asp Gln Ile
            260                 265                 270
Glu Gln Ser Asp Asp Asn Gly Val Asp Tyr Lys Asn Gln Leu Leu Asn
        275                 280                 285
Val Leu Lys Ile Ser Lys Glu Lys Val Asp Glu Cys Tyr Asn Ala Ile
    290                 295                 300
Leu His Leu Thr Asn Ala Asn Asn Tyr Gly His Lys Arg Lys Tyr Glu
305                 310                 315                 320
Glu Ile Pro Gly Ser Pro Ser Gly Val Ile Asp Ala Val Phe Ser Ser
                325                 330                 335
Asp Gly Ser Asn Asp Ser Trp Thr Val Gly Ala Ser Ser Tyr Ser Thr
            340                 345                 350
Ser Glu Pro Val Phe Lys Lys Thr Lys Asn Gln Gly Gln Asn Met Asn
        355                 360                 365
Leu Ser Pro Ile Asn Arg Val Ile Val Gly Ile Leu Ala Thr Ala Thr
    370                 375                 380
Ser Pro
385
```

<210> 34
<211> 1679
<212> DNA
<213> Nicotiana tabacum

<400> 34

```
aaacgagtct ctgtgtactc ctcctcctat agcttttctc tcttcttctc ttcacacctc     60
ccacaacaca caatcagaca aaatagagag gaaaatgagt atggtgaaaa agctttgttt    120
tgtataatga gaaaaagaga tttatataca tctcttcttc tacttccttc ttactagaag    180
atggcaatag aacacaatga gcaacaagaa ctatctcaat cttttctttt agatgctctt    240
tactgtgaag aagaagaaga aaaatgggga gatttagtag atgatgagac tattattaca    300
ccactctctt cagaagtaac aacaacaaca acaacaacaa caaagcctaa ttctttatta    360
cctttgcttt tgttggaaca agatttattt tgggaagatg aagagcttct ttcacttttc    420
tctaaagaaa aagaaaccca ttgttggttt aacagttttc aagatgactc tttactctgt    480
tctgcccgtg ttgattctgt ggaatggatt ttaaaagtga atggttatta tggtttctct    540
gctttgactg ccgttttagc cataaattac tttgacaggt ttctgactag tcttcattat    600
cagaaagata aaccttggat gattcaactt gctgctgtta cttgtctttc tttagctgct    660
aaagttgaag aaactcaagt tcctcttctt ttagattttc aagtggagga tgctaaatat    720
gtgtttgagg caaaaactat tcaaagaatg gagcttttag tgttgtcttc actaaaatgg    780
aggatgaatc cagtgacccc actttcattt cttgatcata ttataaggag gcttgggcta    840
agaaataata ttcactggga atttcttaga agatgtgaaa atctcctcct ctctattatg    900
gctgattgta gattcgtacg ttatatgccg tctgtattgg ccactgcaat tatgcttcac    960
gttattcatc aagttgagcc ttgtaattct gttgactacc aaaatcaact tcttggggtt   1020
```

```
ctcaaaatta acaaggagaa agtgaataat tgctttgaac tcatatcaga agtgtgttct   1080
aagcccattt cacacaaacg caaatatgag aatcctagtc atagcccaag tggtgtaatt   1140
gatccaattt acagttcaga aagttcaaat gattcatggg atttggagtc aacatcttca   1200
tattttcctg ttttcaagaa aagcagagta caagaacagc aaatgaaatt ggcatcttca   1260
attagcagag tttttgtgga agctgttggt agtcctcatt aaaatcaatc acctgattta   1320
tctcttttct ttcttattac caactatggt ggtaataata tttattgata ttcagaagta   1380
tttaccttta atgtcatttt caaaaattac atgaaaatgg aaaaaaagaa aagaagagct   1440
tagctggtgg ttgcagttgg cagagaagag gactggcttt tttttgcagg agtgtagtct   1500
actactactg gaaagcagag atagagagag gagaaaagac agaaaatctg cactatttgt   1560
tttttctcta ttcatatcaa ttctctctta ggtccttttc atgcatgcat acttttgatg   1620
gacatatttt atatatttac tataatcata aattcttgaa taaaaaaaaa aaaaaaaaa    1679
```

<210> 35
<211> 373
<212> PRT
<213> Nicotiana tabacum

<400> 35

```
Met Ala Ile Glu His Asn Glu Gln Gln Glu Leu Ser Gln Ser Phe Leu
1               5                   10                  15
Leu Asp Ala Leu Tyr Cys Glu Glu Glu Glu Glu Lys Trp Gly Asp Leu
            20                  25                  30
Val Asp Asp Glu Thr Ile Ile Thr Pro Leu Ser Ser Glu Val Thr Thr
        35                  40                  45
Thr Thr Thr Thr Thr Thr Lys Pro Asn Ser Leu Leu Pro Leu Leu Leu
    50                  55                  60
Leu Glu Gln Asp Leu Phe Trp Glu Asp Glu Glu Leu Leu Ser Leu Phe
65                  70                  75                  80
Ser Lys Glu Lys Glu Thr His Cys Trp Phe Asn Ser Phe Gln Asp Asp
                85                  90                  95
Ser Leu Leu Cys Ser Ala Arg Val Asp Ser Val Glu Trp Ile Leu Lys
            100                 105                 110
Val Asn Gly Tyr Tyr Gly Phe Ser Ala Leu Thr Ala Val Leu Ala Ile
        115                 120                 125
Asn Tyr Phe Asp Arg Phe Leu Thr Ser Leu His Tyr Gln Lys Asp Lys
    130                 135                 140
Pro Trp Met Ile Gln Leu Ala Ala Val Thr Cys Leu Ser Leu Ala Ala
145                 150                 155                 160
Lys Val Glu Glu Thr Gln Val Pro Leu Leu Leu Asp Phe Gln Val Glu
                165                 170                 175
Asp Ala Lys Tyr Val Phe Glu Ala Lys Thr Ile Gln Arg Met Glu Leu
            180                 185                 190
Leu Val Leu Ser Ser Leu Lys Trp Arg Met Asn Pro Val Thr Pro Leu
        195                 200                 205
Ser Phe Leu Asp His Ile Ile Arg Arg Leu Gly Leu Arg Asn Asn Ile
    210                 215                 220
His Trp Glu Phe Leu Arg Arg Cys Glu Asn Leu Leu Leu Ser Ile Met
225                 230                 235                 240
Ala Asp Cys Arg Phe Val Arg Tyr Met Pro Ser Val Leu Ala Thr Ala
                245                 250                 255
Ile Met Leu His Val Ile His Gln Val Glu Pro Cys Asn Ser Val Asp
            260                 265                 270
Tyr Gln Asn Gln Leu Leu Gly Val Leu Lys Ile Asn Lys Glu Lys Val
        275                 280                 285
```

```
Asn Asn Cys Phe Glu Leu Ile Ser Glu Val Cys Ser Lys Pro Ile Ser
    290                 295                 300
His Lys Arg Lys Tyr Glu Asn Pro Ser His Ser Pro Ser Gly Val Ile
305                 310                 315                 320
Asp Pro Ile Tyr Ser Ser Glu Ser Ser Asn Asp Ser Trp Asp Leu Glu
                325                 330                 335
Ser Thr Ser Ser Tyr Phe Pro Val Phe Lys Lys Ser Arg Val Gln Glu
            340                 345                 350
Gln Gln Met Lys Leu Ala Ser Ser Ile Ser Arg Val Phe Val Glu Ala
        355                 360                 365
Val Gly Ser Pro His
    370
```

<210> 36
<211> 1430
<212> DNA
<213> Nicotiana tabacum

<400> 36

```
cacctttact ctcttctcct ttttggctct tcccattctc tccttctctt tctttatttt      60
ctgtcctgta gagagagaga gaaagtataa gcaaagcagc agatatgtta ctgggtccaa     120
gattgagttt tggcttacct tgaagataat gagtagagcc tccattgtct tcttccgtca     180
agaagaagaa gaagaagatg gttttccctt tagatactca gctcctaaat ccaatctttg     240
atgtccttta ctgtgaggaa gatcgattct tggacgatga tgatttagga gaatggtcta     300
gtactttaga acaagtagga aataatgtga aaaagactct acctttatta gaatgtgaca     360
tgttttggga agatgaccag cttgtcactc ttttaactaa ggaaaaagag tctcatttgg     420
gttttgattg tttaatctca gatggagatg ggtttttagt ggaggttaga aaagaggcat     480
tggattggat gttgagagtc attgctcact atggtttcac tgctatgact gctgttttag     540
ctgtgaatta ttttgatagg tttgtatctg gactctgctt tcagaaagat aagccttgga     600
tgagtcaact tgctgctgtg gcttgtcttt ctattgctgc taaagtggaa gagacccaag     660
tcccccttct cttagacctc caagtggctg attcaagatt tgtgtttgag gcaaagacta     720
ttcagagaat ggaactcttg gtgctctcca ctcttaagtg gaaaatgaat ccagtgacac     780
cactatcttt cattgatcat atcatgagga gatttggatt catgaccaat ctacatttgg     840
attttcttag gagatgtgaa cgcctcattc ttggtattat cactgattct aggctcttgc     900
attatcctcc atctgttatt gcaactgcag tagtgtattt cgtgatcaat gagattgagc     960
cttgcaatgc aatggaatac cagaatcagc tcatgactgt tcttaaagtc aaacaggata    1020
gttttgaaga atgccatgat cttattctag agctaatggg cacttctggc tacaatatct    1080
gccaaagcct caagcgcaaa catcaatctg tacctggcag tccaagtgga gttatcgatg    1140
catattttag ttgcgacagc tctaatgatt cgtggtcggt agcatcttca atttcatcgt    1200
caccagaacc tcagtataag aggatcaaaa ctcaggatca gacaatgaca ctggctccac    1260
tgagttctgt ttctgtcgtt gtgggcagta gtcctcgttg atcagtatct cattctctag    1320
attatctagt attacggcta tggttactat atgatctctc ttttttggta tgttctctta    1380
aactgcagtt gcacaatgct ctgatgttcc attaaaaaaa aaaaaaaaaa               1430
```

<210> 37
<211> 367
<212> PRT
<213> Nicotiana tabacum

<400> 37

```
Met Val Phe Pro Leu Asp Thr Gln Leu Leu Asn Pro Ile Phe Asp Val
1               5                   10                  15
Leu Tyr Cys Glu Glu Asp Arg Phe Leu Asp Asp Asp Asp Leu Gly Glu
            20                  25                  30
```

Trp Ser Ser Thr Leu Glu Gln Val Gly Asn Asn Val Lys Lys Thr Leu
35 40 45
Pro Leu Leu Glu Cys Asp Met Phe Trp Glu Asp Asp Gln Leu Val Thr
50 55 60
Leu Leu Thr Lys Glu Lys Glu Ser His Leu Gly Phe Asp Cys Leu Ile
65 70 75 80
Ser Asp Gly Asp Gly Phe Leu Val Glu Val Arg Lys Glu Ala Leu Asp
85 90 95
Trp Met Leu Arg Val Ile Ala His Tyr Gly Phe Thr Ala Met Thr Ala
100 105 110
Val Leu Ala Val Asn Tyr Phe Asp Arg Phe Val Ser Gly Leu Cys Phe
115 120 125
Gln Lys Asp Lys Pro Trp Met Ser Gln Leu Ala Ala Val Ala Cys Leu
130 135 140
Ser Ile Ala Ala Lys Val Glu Glu Thr Gln Val Pro Leu Leu Leu Asp
145 150 155 160
Leu Gln Val Ala Asp Ser Arg Phe Val Phe Glu Ala Lys Thr Ile Gln
165 170 175
Arg Met Glu Leu Leu Val Leu Ser Thr Leu Lys Trp Lys Met Asn Pro
180 185 190
Val Thr Pro Leu Ser Phe Ile Asp His Ile Met Arg Arg Phe Gly Phe
195 200 205
Met Thr Asn Leu His Leu Asp Phe Leu Arg Arg Cys Glu Arg Leu Ile
210 215 220
Leu Gly Ile Ile Thr Asp Ser Arg Leu Leu His Tyr Pro Pro Ser Val
225 230 235 240
Ile Ala Thr Ala Val Val Tyr Phe Val Ile Asn Glu Ile Glu Pro Cys
245 250 255
Asn Ala Met Glu Tyr Gln Asn Gln Leu Met Thr Val Leu Lys Val Lys
260 265 270
Gln Asp Ser Phe Glu Glu Cys His Asp Leu Ile Leu Glu Leu Met Gly
275 280 285
Thr Ser Gly Tyr Asn Ile Cys Gln Ser Leu Lys Arg Lys His Gln Ser
290 295 300
Val Pro Gly Ser Pro Ser Gly Val Ile Asp Ala Tyr Phe Ser Cys Asp
305 310 315 320
Ser Ser Asn Asp Ser Trp Ser Val Ala Ser Ser Ile Ser Ser Ser Pro
325 330 335
Glu Pro Gln Tyr Lys Arg Ile Lys Thr Gln Asp Gln Thr Met Thr Leu
340 345 350
Ala Pro Leu Ser Ser Val Ser Val Val Val Gly Ser Ser Pro Arg
355 360 365

<210> 38
<211> 1487
<212> DNA
<213> Nicotiana tabacum

<400> 38

gcacgagctt ccttcacaca accagataga gacaagaaca gagagattca tggaaatgcc 60
caagaagaat cactcttttg gtttcttgtt ttatataatg agaaagcaac acattttctt 120
cttttctcaa agaagatggg aatacaacac aatgagcata atcaagacca aacccaatct 180
ttcctttag atgctcttta ctgtgaagaa gaaagatggg aagaaacaat tgaagatgag 240
attttagaaa aagaagcaac actaccactt cctctgcctt tactagaaca agacttgttt 300

```
tgggaagatg aagagctact ctctcttttc acaaaagaaa aagaaacaat ttccaacttt    360
gaaactatta aaacagaccc tttactttgt ttatctcgta aagaagctgt gaaatggatt    420
cttaaagtaa atgctcatta tggattctca acattcactg ctattcttgc tattaattac    480
tttgataggt ttctttcaag tcttcatttt cagaaagata agccttggat gattcaactt    540
gtagctgtta cttgtctttc tttggctgct aaagttgaag aaactcaagt tcctcttctt    600
ttggacttcc aagtggagga tgcaaaatat gtgtttgagg ccaaaactat tcaaagaatg    660
gagcttttgg tattgtcctc tttaaagtgg aggatgaatc ctgtaacccc actttcattt    720
gttgatcata taataagaag acttgggcta aagagccata tacactggga atttctcaag    780
cagtgtgaga gaattcttct tttggtcata gctgattgta gattcttaag ttatatgcct    840
tctgtattgg ctactgctac tatgcttcac gttattcatc aagttgagcc ttgtaatgct    900
gctgactacc aaaatcaact tcttgaggtt ctcaacatta gcaaggagaa ggtgaatgat    960
tgctatgaac ttataacaga ggtgtcttac aactctattt cacacaagcg caagtatgag   1020
agtccaataa atagcccaag tgctgttatt gatacatttt acagctctga aaactcaaat   1080
gaatcatggg atttgcaaac ttcttcctct attccatcca cttattcacc tcgtgatcaa   1140
tttttgcctt tgtttaagaa aagcagagtt caagaacagc aaatgagatt gacatcttta   1200
agcagagttt ttgtggatta tgctgttggc agccctcgct aatattattg acaatgacaa   1260
tgtattactc cttattatgt cctttttgca aattctactt tggaaagatg gaaaatgaaa   1320
agaggggata gttggtggta gtggctgcag agggttggtg tttgcagcac aatggggggg   1380
ttaagagaaa gaaggatagg taaatattca atgttctact gggagtaatc gttaattcaa   1440
ggaacattta gttaattttg gtttaagatc tctttggttg gtccccc                1487
```

<210> 39
<211> 368
<212> PRT
<213> Nicotiana tabacum

<400> 39

```
Met Gly Ile Gln His Asn Glu His Asn Gln Asp Gln Thr Gln Ser Phe
1               5                   10                  15
Leu Leu Asp Ala Leu Tyr Cys Glu Glu Glu Arg Trp Glu Glu Thr Ile
            20                  25                  30
Glu Asp Glu Ile Leu Glu Lys Glu Ala Thr Leu Pro Leu Pro Leu Pro
        35                  40                  45
Leu Leu Glu Gln Asp Leu Phe Trp Glu Asp Glu Glu Leu Leu Ser Leu
    50                  55                  60
Phe Thr Lys Glu Lys Glu Thr Ile Ser Asn Phe Glu Thr Ile Lys Thr
65                  70                  75                  80
Asp Pro Leu Leu Cys Leu Ser Arg Lys Glu Ala Val Lys Trp Ile Leu
                85                  90                  95
Lys Val Asn Ala His Tyr Gly Phe Ser Thr Phe Thr Ala Ile Leu Ala
            100                 105                 110
Ile Asn Tyr Phe Asp Arg Phe Leu Ser Ser Leu His Phe Gln Lys Asp
        115                 120                 125
Lys Pro Trp Met Ile Gln Leu Val Ala Val Thr Cys Leu Ser Leu Ala
    130                 135                 140
Ala Lys Val Glu Glu Thr Gln Val Pro Leu Leu Leu Asp Phe Gln Val
145                 150                 155                 160
Glu Asp Ala Lys Tyr Val Phe Glu Ala Lys Thr Ile Gln Arg Met Glu
                165                 170                 175
Leu Leu Val Leu Ser Ser Leu Lys Trp Arg Met Asn Pro Val Thr Pro
            180                 185                 190
Leu Ser Phe Val Asp His Ile Ile Arg Arg Leu Gly Leu Lys Ser His
        195                 200                 205
Ile His Trp Glu Phe Leu Lys Gln Cys Glu Arg Ile Leu Leu Leu Val
```

```
    210                 215                 220
Ile Ala Asp Cys Arg Phe Leu Ser Tyr Met Pro Ser Val Leu Ala Thr
225                 230                 235                 240
Ala Thr Met Leu His Val Ile His Gln Val Glu Pro Cys Asn Ala Ala
                245                 250                 255
Asp Tyr Gln Asn Gln Leu Leu Glu Val Leu Asn Ile Ser Lys Glu Lys
            260                 265                 270
Val Asn Asp Cys Tyr Glu Leu Ile Thr Glu Val Ser Tyr Asn Ser Ile
        275                 280                 285
Ser His Lys Arg Lys Tyr Glu Ser Pro Ile Asn Ser Pro Ser Ala Val
    290                 295                 300
Ile Asp Thr Phe Tyr Ser Ser Glu Asn Ser Asn Glu Ser Trp Asp Leu
305                 310                 315                 320
Gln Thr Ser Ser Ser Ile Pro Ser Thr Tyr Ser Pro Arg Asp Gln Phe
                325                 330                 335
Leu Pro Leu Phe Lys Lys Ser Arg Val Gln Glu Gln Gln Met Arg Leu
            340                 345                 350
Thr Ser Leu Ser Arg Val Phe Val Asp Tyr Ala Val Gly Ser Pro Arg
        355                 360                 365
```

<210> 40
<211> 1714
<212> DNA
<213> Oryza sativa

<400> 40

```
gtctctctcc ctccacctcc gctcctactc tgctgctcca ccacgaccaa aagccatgcc      60
tatgctgctg ctgccgctct cctcctcttc cgccattgcc gccacctgag ctgagctctt     120
gtactcgcta cgctactcaa gatggctttc gccacgctct ttgactccct ctactgcccc     180
gaggagcacc tcgacctctt ccatgacacc gccgccgacg acgacctcca cctcgacctt     240
cacctgcacc aacccccacc gccgccgccg ctcctcgacg acgacctgcc tgcgctgttc     300
cacgcgctca gggggaagga ggacccgctg cgcccccgccg ccgacgacga cggctacggc    360
ggggtgtctg cccgggaggc ggcggtcggg tgggcgctgc gcgccgtcgc gaggctcggc     420
ttctccgcgc tcacggccgc gctcgccgtc gcctacctcg accgctgctt cctcggcggc     480
gcgctccgcc tcggcgaccg cccctggatg gcgcgcctcg ccgccgtcgc ctgcgtcgcg     540
ctcgccgcca aggtggagga gacgcgcgtg cccgtgctcc tcgacctcca gctctgcgcc     600
gccgaacgcg ccgaccccaa cgaggcctac gtgttcgagg acaagacggt gcgccgcatg     660
gagctgctgg tgctctcggc gctcggatgg cggatgcacc ccgtgacgcc cctctcctac     720
ctccaacccc tcctcggcac cgcccacgcc gcgcgccttc accactgcga caccgcattg     780
ctcgcgctga tgcccgattg gaggtggcct cgccaccgcc cttcggcgtg ggccgccgcg     840
gcgttgctcg cgacggccgg atggtgcggc ggcggcggcg gcgacgacgc cgagctccta     900
gccctcatcg atgcccccaa ggatgagatg gcagagtgcg ccaagatcat ctccgaggag     960
gcggcggcgg cggcggcggg gggcattgtg atcggcggcg aaaataagcg caagggcgcg    1020
gcggggctgt actcggcgcc ggcgagcccg agcggcgtga tcggcgcgtc ggcctgcttc    1080
agctgcgaca gctcgtccag cagcgtcgac tcgctgttcg ccgccttgga gccacccggc    1140
cggccgatca agcgaggtgc cgccgccgcc accaccgcgg atccgcttcc cgccgacgag    1200
gagagccgcg acgcctggcc gccgtacgcc gcatgaggct gctctggtcg gtatcgggaa    1260
ggggagaagc taaagcaagc cactaattaa gcttaggagg aggagggttc agtccaagaa    1320
aaagatgttc cattaacgca atgcaagaat gcaggtgcga aggggtaggt tcattcatgt    1380
agccattgat ggtctcttgg agctcagctc agcctcaacc tcaacccacc atagccatgg    1440
atggaggtga ggaagaagct gttttgtcct gtctgctcac tctgctggta ccaccactgt    1500
tagaaccatg cttcacactc tctctcttgt ttctctctct agagagaaga gagagagtat    1560
aaaggagtag gagtatgagg agtggcaaca atggcaatgg catcaaatgc tgcaattgcc    1620
cacttcccat gagcagctag agctacaaca gcagacctga ggctattgtg ctcacctctt    1680

cttcttccac cattaatgaa aatgcattac tacc                                 1714
```

<210> 41
<211> 364
<212> PRT
<213> Oryza sativa

<400> 41

```
Met Ala Phe Ala Thr Leu Phe Asp Ser Leu Tyr Cys Pro Glu Glu His
1               5                   10                  15
Leu Asp Leu Phe His Asp Thr Ala Ala Asp Asp Asp Leu His Leu Asp
            20                  25                  30
Leu His Leu His Gln Pro Pro Pro Pro Pro Pro Leu Leu Asp Asp Asp
        35                  40                  45
Leu Pro Ala Leu Phe His Ala Leu Arg Gly Lys Glu Asp Pro Leu Arg
    50                  55                  60
Pro Ala Ala Asp Asp Asp Gly Tyr Gly Gly Val Ser Ala Arg Glu Ala
65                  70                  75                  80
Ala Val Gly Trp Ala Leu Arg Ala Val Ala Arg Leu Gly Phe Ser Ala
                85                  90                  95
Leu Thr Ala Ala Leu Ala Val Ala Tyr Leu Asp Arg Cys Phe Leu Gly
            100                 105                 110
Gly Ala Leu Arg Leu Gly Asp Arg Pro Trp Met Ala Arg Leu Ala Ala
        115                 120                 125
Val Ala Cys Val Ala Leu Ala Ala Lys Val Glu Glu Thr Arg Val Pro
    130                 135                 140
Val Leu Leu Asp Leu Gln Leu Cys Ala Ala Glu Arg Ala Asp Pro Asn
145                 150                 155                 160
Glu Ala Tyr Val Phe Glu Asp Lys Thr Val Arg Arg Met Glu Leu Leu
                165                 170                 175
Val Leu Ser Ala Leu Gly Trp Arg Met His Pro Val Thr Pro Leu Ser
            180                 185                 190
Tyr Leu Gln Pro Leu Leu Gly Thr Ala His Ala Ala Arg Leu His His
        195                 200                 205
Cys Asp Thr Ala Leu Leu Ala Leu Met Pro Asp Trp Arg Trp Pro Arg
    210                 215                 220
His Arg Pro Ser Ala Trp Ala Ala Ala Ala Leu Leu Ala Thr Ala Gly
225                 230                 235                 240
Trp Cys Gly Gly Gly Gly Gly Asp Asp Ala Glu Leu Leu Ala Leu Ile
                245                 250                 255
Asp Ala Pro Lys Asp Glu Met Ala Glu Cys Ala Lys Ile Ile Ser Glu
            260                 265                 270
Glu Ala Ala Ala Ala Ala Ala Gly Gly Ile Val Ile Gly Gly Glu Asn
        275                 280                 285
Lys Arg Lys Gly Ala Ala Gly Leu Tyr Ser Ala Pro Ala Ser Pro Ser
    290                 295                 300
Gly Val Ile Gly Ala Ser Ala Cys Phe Ser Cys Asp Ser Ser Ser Ser
305                 310                 315                 320
Ser Val Asp Ser Leu Phe Ala Ala Leu Glu Pro Pro Gly Arg Pro Ile
                325                 330                 335
Lys Arg Gly Ala Ala Ala Ala Thr Thr Ala Asp Pro Leu Pro Ala Asp
            340                 345                 350
Glu Glu Ser Arg Asp Ala Trp Pro Pro Tyr Ala Ala
        355                 360
```

<210> 42
<211> 1628
<212> DNA
<213> Pisum sativum

<400> 42

```
gtggcggccg ctctagaact agtggatcag aacacaaaaa caaagagaaa aaaaaagatg      60
aatatgaaac tcttgattac tctccccttc tttctataat gaacaaagga ccacacaacc     120
tcttcttcat tgaagaagat ggcaatccat catcaccacc accatcatca acaactacac     180
cacaactctc ttcttgatgc tctttactgc gatgaagaaa aactcgaaga agaacaagaa     240
gacgtttcat ctcaacaaag tgatgtcaca acaaacaatg acaacaacat cctagactcc     300
acttccctgt tccctcttct tctcctggaa caaaacctct tctctcaaga tgaagaactc     360
accacacttt tctccaaaga aaaaacccaa caagaaacgt actacgagga tctgaaaaat     420
gtcgtggatt ttgtttctct ctctcaacct cgtcgtgaag ctgttcaatg gatgcttaaa     480
gtcaatgctc attacgcctt ttcacctctc actgcaacac tcgctgttac ttactttgat     540
aggttccttc taaccttcca tttccaaaaa gataagccat ggatgattca gcttgttgct     600
gttacttgca tctctttagc tgctaaagtt gaagaaactc aagttcctct cctcttagac     660
ctacaagtgc aagatactaa atatgtgttt gaagcaaaaa ctattcagag aatggagctt     720
ttgattctgt caacactgaa atggaagatg catcctgtga caccacactc ttttctagat     780
catataataa caaggcttgg tttgaaaact aatcttcatt gggagttttt aagacgctgt     840
gagaatcttc ttctatctgt acttttagat tcaagatttg ttggttgtgt tccctctgtg     900
ttggctactg ctacaatgct gcatgtgata gaccagattg aagagagtga tgataatggt     960
gtggactaca aaaatcagct tcttagtatt ctcaaaatca acaaggagaa agtggatgaa    1020
tgttataatg ctattgttga ggttactaat gaaaataatt atggtcataa acgaaaatat    1080
gaacaaatcc ctggaagtcc aagtggcgta attgatgctg tttttagttc tgatggttcc    1140
aatgattcat ggaaagtggg ttcatcctcg tattcaacct cagagcctgt ttttaagaaa    1200
acaaaaactc aagggcaaaa taggaatttg tcacctctta atagggtcat tgttggaatt    1260
cttgccactg ctagtgctac tacctctcct taatatcctc tctctgtcct ttataaaaaa    1320
aacaaataat aaccatatgc aaaaaatcta tatttattta gtatatatgg ttatgttgat    1380
gtttctaaga tctcttcatt tccaacaatg tccctaatga atctcttgaa gaggcagaga    1440
cagagaaaga ggcaagatga aagaggagtg aaagaggaga ggacaaagtt ggcaatgaag    1500
attatgtttt ttttttaat ttgaaggcac ttttattta ctattgaatg gttgttctct      1560
tctagaccat accaaatttg aacatattta tatgatattt ctataataaa ttgggataat    1620
ttttgttc                                                             1628
```

<210> 43
<211> 384
<212> PRT
<213> Pisum sativum

<400> 43

```
Met Ala Ile His His His His His His His Gln Gln Leu His His Asn
1               5                   10                  15
Ser Leu Leu Asp Ala Leu Tyr Cys Asp Glu Glu Lys Leu Glu Glu Glu
            20                  25                  30
Gln Glu Asp Val Ser Ser Gln Gln Ser Asp Val Thr Thr Asn Asn Asp
        35                  40                  45
Asn Asn Ile Leu Asp Ser Thr Ser Leu Phe Pro Leu Leu Leu Leu Glu
    50                  55                  60
Gln Asn Leu Phe Ser Gln Asp Glu Glu Leu Thr Thr Leu Phe Ser Lys
65                  70                  75                  80
Glu Lys Thr Gln Gln Glu Thr Tyr Tyr Glu Asp Leu Lys Asn Val Val
                85                  90                  95
```

```
Asp Phe Val Ser Leu Ser Gln Pro Arg Arg Glu Ala Val Gln Trp Met
            100                 105                 110
Leu Lys Val Asn Ala His Tyr Ala Phe Ser Pro Leu Thr Ala Thr Leu
        115                 120                 125
Ala Val Thr Tyr Phe Asp Arg Phe Leu Leu Thr Phe His Phe Gln Lys
    130                 135                 140
Asp Lys Pro Trp Met Ile Gln Leu Val Ala Val Thr Cys Ile Ser Leu
145                 150                 155                 160
Ala Ala Lys Val Glu Glu Thr Gln Val Pro Leu Leu Leu Asp Leu Gln
                165                 170                 175
Val Gln Asp Thr Lys Tyr Val Phe Glu Ala Lys Thr Ile Gln Arg Met
            180                 185                 190
Glu Leu Leu Ile Leu Ser Thr Leu Lys Trp Lys Met His Pro Val Thr
        195                 200                 205
Pro His Ser Phe Leu Asp His Ile Ile Thr Arg Leu Gly Leu Lys Thr
    210                 215                 220
Asn Leu His Trp Glu Phe Leu Arg Arg Cys Glu Asn Leu Leu Leu Ser
225                 230                 235                 240
Val Leu Leu Asp Ser Arg Phe Val Gly Cys Val Pro Ser Val Leu Ala
                245                 250                 255
Thr Ala Thr Met Leu His Val Ile Asp Gln Ile Glu Glu Ser Asp Asp
            260                 265                 270
Asn Gly Val Asp Tyr Lys Asn Gln Leu Leu Ser Ile Leu Lys Ile Asn
        275                 280                 285
Lys Glu Lys Val Asp Glu Cys Tyr Asn Ala Ile Val Glu Val Thr Asn
    290                 295                 300
Glu Asn Asn Tyr Gly His Lys Arg Lys Tyr Glu Gln Ile Pro Gly Ser
305                 310                 315                 320
Pro Ser Gly Val Ile Asp Ala Val Phe Ser Ser Asp Gly Ser Asn Asp
                325                 330                 335
Ser Trp Lys Val Gly Ser Ser Ser Tyr Ser Thr Ser Glu Pro Val Phe
            340                 345                 350
Lys Lys Thr Lys Thr Gln Gly Gln Asn Arg Asn Leu Ser Pro Leu Asn
        355                 360                 365
Arg Val Ile Val Gly Ile Leu Ala Thr Ala Ser Ala Thr Thr Ser Pro
    370                 375                 380
```

<210> 44
<211> 1491
<212> DNA
<213> Populus alba

<400> 44

```
gagaaagata tgcaatatat gcaaagagaa gcctgagaga tttttatctc ccttttttcat      60
ttttgatggt gttctataat gagaagagga ttaaatctct tcttctctga agaagatgtc     120
tttcttacaa caacaagaga ctcataatca aagcccagca ttggctcttg acgggcttta     180
ctgtgaagag gatggatttg gagaggatta ttcttgtggt ttggatgatg aaactagcca     240
ggtttatgat caaaatgtga aaaaggagca aaatttatct tctgttttgc ttgagcaaga     300
cttgttttgg gaagatagcg agttactgtc tttaatctcc aaagagaaag agacccatgt     360
tgtttttgat agtgtaggat ctagagatgg atctttaatg gtggttcgta gagaggcagt     420
tgagtggttt ttgagggtaa aggcacatta tgggttcagt gctttgactg gtgttcttgc     480
tgtgaactac tttgataggt tcatttcaag ttcaaggttt cgaagagata agccatggat     540
gggtcaactt gctgctgtgg cttgtttgtc tctggctgct aaagtggagg aaacccaagt     600
gcctcttctt ttagacttgc aagtggagga tgcaaagtac gtttttgaag ccaagaccat     660
```

```
aaagagaatg gagctgtggg tgctgtcaac tcttcattgg aggatgaatc ctgtaacctc    720
aatttctttc tttgatcaca ttataaggag acttggatta aagacccaca tgcattggga    780
gtttttatgg aggtgtgagc gattgcttct ttctgtcatt tctgattcaa ggttcatgag    840
ttatcttcct tctatattag caactgcgac aatgttgcat gttatcaagg aggttgagcc    900
acgtaatcaa ctgcaatacc aaactcagct catggctgtg ctaaaaacca atgaggatga    960
agtgaatgag tgttacaggc tcattttaga gcaaccaggc agccaaaacc aacgccacaa   1020
gcgcaagtac ctgtccacac ccagcagccc aaatggtgtc atcgatgcat ctttcagctc   1080
tgagaactca aatgattcgt gggctgtggc atcatcaatc tcatcatcat catcagtgcc   1140
tcaattcaaa agaagcaggg cccaggttca gcagatgcga ttgccttcac taaatcgtat   1200
gtgcgtggat gtgcttagca gtcctcatta gtctttcctt gtcttaatgc ctcgagctat   1260
cataatcttc ctgttttgcg tattaattgt tgtatgtgta agaggtacag acgttttatg   1320
aaactgaccc attcttggat gagaaatgag gtatctgctc tgctctctct ttgttcgaga   1380
gaggaatgaa gagattgtgg tgtttttcca ataggtatga acatgatgtc tgtatttcaa   1440
ccattgctct attaaatgtt acatgattca ctaggaaaaa aaaaaaaaaa a            1491
```

<210> 45
<211> 371
<212> PRT
<213> Populus alba

<400> 45

```
Met Ser Phe Leu Gln Gln Gln Glu Thr His Asn Gln Ser Pro Ala Leu
1               5                   10                  15
Ala Leu Asp Gly Leu Tyr Cys Glu Glu Asp Gly Phe Gly Glu Asp Tyr
            20                  25                  30
Ser Cys Gly Leu Asp Asp Glu Thr Ser Gln Val Tyr Asp Gln Asn Val
        35                  40                  45
Lys Lys Glu Gln Asn Leu Ser Ser Val Leu Leu Glu Gln Asp Leu Phe
    50                  55                  60
Trp Glu Asp Ser Glu Leu Leu Ser Leu Ile Ser Lys Glu Lys Glu Thr
65                  70                  75                  80
His Val Val Phe Asp Ser Val Gly Ser Arg Asp Gly Ser Leu Met Val
                85                  90                  95
Val Arg Arg Glu Ala Val Glu Trp Phe Leu Arg Val Lys Ala His Tyr
            100                 105                 110
Gly Phe Ser Ala Leu Thr Gly Val Leu Ala Val Asn Tyr Phe Asp Arg
        115                 120                 125
Phe Ile Ser Ser Ser Arg Phe Arg Arg Asp Lys Pro Trp Met Gly Gln
    130                 135                 140
Leu Ala Ala Val Ala Cys Leu Ser Leu Ala Ala Lys Val Glu Glu Thr
145                 150                 155                 160
Gln Val Pro Leu Leu Leu Asp Leu Gln Val Glu Asp Ala Lys Tyr Val
                165                 170                 175
Phe Glu Ala Lys Thr Ile Lys Arg Met Glu Leu Trp Val Leu Ser Thr
            180                 185                 190
Leu His Trp Arg Met Asn Pro Val Thr Ser Ile Ser Phe Phe Asp His
        195                 200                 205
Ile Ile Arg Arg Leu Gly Leu Lys Thr His Met His Trp Glu Phe Leu
    210                 215                 220
Trp Arg Cys Glu Arg Leu Leu Leu Ser Val Ile Ser Asp Ser Arg Phe
225                 230                 235                 240
Met Ser Tyr Leu Pro Ser Ile Leu Ala Thr Ala Thr Met Leu His Val
                245                 250                 255
Ile Lys Glu Val Glu Pro Arg Asn Gln Leu Gln Tyr Gln Thr Gln Leu
```

```
            260                 265                 270
Met Ala Val Leu Lys Thr Asn Glu Asp Glu Val Asn Glu Cys Tyr Arg
        275                 280                 285
Leu Ile Leu Glu Gln Pro Gly Ser Gln Asn Gln Arg His Lys Arg Lys
    290                 295                 300
Tyr Leu Ser Thr Pro Ser Ser Pro Asn Gly Val Ile Asp Ala Ser Phe
305                 310                 315                 320
Ser Ser Glu Asn Ser Asn Asp Ser Trp Ala Val Ala Ser Ser Ile Ser
                325                 330                 335
Ser Ser Ser Ser Val Pro Gln Phe Lys Arg Ser Arg Ala Gln Val Gln
            340                 345                 350
Gln Met Arg Leu Pro Ser Leu Asn Arg Met Cys Val Asp Val Leu Ser
        355                 360                 365
Ser Pro His
    370
```

<210> 46
<211> 1405
<212> DNA
<213> Populus tremula x Populus tremuloides

<400> 46

```
ctggaagaag atggcatcaa tgtataaccc agaaacgagt gcggtacaag accaacaaca      60
aaaccctaca ttactttatg atgctctcta ttgttctgaa gagaattggg tggaagaagt     120
tagagaggac tggtttcaag atgaactaga aggagagagc tattgtagca acaatagcaa     180
taaactaaac acttttccaa tattgctaga acaggactta agctgggaag acgaggagct     240
ttcctctttg tttgccaagg aggagcaaaa tcagctgtgc aaagacttag aaaccaaccc     300
gtctttggct agggctcgct gtgaggctgt agagtggatt ctaaaggtca atgaacacta     360
ctctttcacc gctctaactg cagttttggc agtgaactat cttgataggt tttattcag      420
tgtccacctt cagaaagaga agccatggat ggcccaactt gcagctgtgt cttgcctctc     480
acttgctgcc aaagtggagg agacgcaagt gccccttcta ttggattttc aggtggagga     540
cagtaaatac gtgttcgagg ccaaaactat ccagagaatg gagatcctgg tgctttctac     600
tcttaaatgg aagatgaatc cagtaacccc aatatcgttt cttgattaca tcactagaag     660
gcttggccta gaacactatc tttgtttgga atttctcaag aggtgtgagc gcatggtcct     720
ctctatcttg gcagattcta ggtctatgcc ttatgttcct tctgtaatgg ccgctgccac     780
gatgctctat gttattgata acatagaacc cagtcttgca gcagaatacc aaagccagct     840
gttgagcatt cttggaatcg ataaagacaa ggtagaggat tgcagcaagt tcttaatgga     900
atttgctcta agagaccatt ttaagcttct ctcaaacaaa cgcaagtttt gttcacttcc     960
aggcagtcct agcggtgtgg ttgatgtgtc ttttagctca gacagctcaa atgattcatg    1020
gtctgtggca tcatccgtgt cttcatcacc aaagcctctg tccaagaaga gtagggcact    1080
gcagagtcta aacaacgcaa caacttcaga tttttctcag cattcctcgc cagtgcctta    1140
aaattactgt ttttccctaa tggaccacct cttgtacggt taataagtct tgtgcttttt    1200
caatgttcaa gttaaattgc ctgccatctc tgcttttcca gctcagacca atgcttagaa    1260
cgctgaaata ttgattgggt actgggaatt ggaagcagag atggttggca ttttaccgga    1320
aatgaagaac agaacagaac aaaaaaaaag aaaaaaaaag agccgatgag gaagagttct    1380
ggtagtaaaa aaaaaaaaaa aaaaa                                          1405
```

<210> 47
<211> 376
<212> PRT
<213> Populus tremula x Populus tremuloides

<400> 47

```
Met Ala Ser Met Tyr Asn Pro Glu Thr Ser Ala Val Gln Asp Gln Gln
```

```
1               5                   10                  15
Gln Asn Pro Thr Leu Leu Tyr Asp Ala Leu Tyr Cys Ser Glu Glu Asn
            20                  25                  30
Trp Val Glu Glu Val Arg Glu Asp Trp Phe Gln Asp Glu Leu Glu Gly
        35                  40                  45
Glu Ser Tyr Cys Ser Asn Asn Ser Asn Lys Leu Asn Thr Phe Pro Ile
    50                  55                  60
Leu Leu Glu Gln Asp Leu Ser Trp Glu Asp Glu Glu Leu Ser Ser Leu
65                  70                  75                  80
Phe Ala Lys Glu Glu Gln Asn Gln Leu Cys Lys Asp Leu Glu Thr Asn
                85                  90                  95
Pro Ser Leu Ala Arg Ala Arg Cys Glu Ala Val Glu Trp Ile Leu Lys
            100                 105                 110
Val Asn Glu His Tyr Ser Phe Thr Ala Leu Thr Ala Val Leu Ala Val
        115                 120                 125
Asn Tyr Leu Asp Arg Phe Leu Phe Ser Val His Leu Gln Lys Glu Lys
    130                 135                 140
Pro Trp Met Ala Gln Leu Ala Ala Val Ser Cys Leu Ser Leu Ala Ala
145                 150                 155                 160
Lys Val Glu Glu Thr Gln Val Pro Leu Leu Leu Asp Phe Gln Val Glu
                165                 170                 175
Asp Ser Lys Tyr Val Phe Glu Ala Lys Thr Ile Gln Arg Met Glu Ile
            180                 185                 190
Leu Val Leu Ser Thr Leu Lys Trp Lys Met Asn Pro Val Thr Pro Ile
        195                 200                 205
Ser Phe Leu Asp Tyr Ile Thr Arg Arg Leu Gly Leu Glu His Tyr Leu
    210                 215                 220
Cys Leu Glu Phe Leu Lys Arg Cys Glu Arg Met Val Leu Ser Ile Leu
225                 230                 235                 240
Ala Asp Ser Arg Ser Met Pro Tyr Val Pro Ser Val Met Ala Ala Ala
                245                 250                 255
Thr Met Leu Tyr Val Ile Asp Asn Ile Glu Pro Ser Leu Ala Ala Glu
            260                 265                 270
Tyr Gln Ser Gln Leu Leu Ser Ile Leu Gly Ile Asp Lys Asp Lys Val
        275                 280                 285
Glu Asp Cys Ser Lys Phe Leu Met Glu Phe Ala Leu Arg Asp His Phe
    290                 295                 300
Lys Leu Leu Ser Asn Lys Arg Lys Phe Cys Ser Leu Pro Gly Ser Pro
305                 310                 315                 320
Ser Gly Val Val Asp Val Ser Phe Ser Ser Asp Ser Ser Asn Asp Ser
                325                 330                 335
Trp Ser Val Ala Ser Ser Val Ser Ser Ser Pro Lys Pro Leu Ser Lys
            340                 345                 350
Lys Ser Arg Ala Leu Gln Ser Leu Asn Asn Ala Thr Thr Ser Asp Phe
        355                 360                 365
Ser Gln His Ser Ser Pro Val Pro
    370                 375
```

<210> 48
<211> 1152
<212> DNA
<213> Arabidopsis thaliana

<400> 48

```
atggctttag aagaggagga agagagtcaa aacgcaccgt tttgtgttct tgatggtctt      60
ttctgtgagg aagagagtga gtttcacgaa caagtagatt tgtgcgacga gagtgttgaa     120
aagtttcctt ttttaaatct gggtttgtct gatcatgata tgttgtggga tgatgatgag     180
ttatcaactt tgatttcgaa acaagaaccg tgtctttatg acgaaatctt agatgatgag     240
tttctggttt tgtgtcgtga aaaggctctt gattggattt ttaaagtgaa atctcattat     300
gggtttaatt cattgacggc tcttttagct gttaattact tcgataggtt tattacaagc     360
aggaagtttc agacagataa gccatggatg tctcagctta ctgctttggc ttgtctgtct     420
ttagctgcta aggttgaaga gatccgtgtt ccttttctct tagattttca agtggaagaa     480
gcaagatatg tctttgaagc taagactata cagagaatgg agcttcttgt tctgtctact     540
cttgactgga ggatgcatcc tgtgactcca atctcgtttt tcgatcacat tattcgacga     600
tacagcttta aatctcatca tcaattggag ttcttgagta gatgtgaatc tttattactc     660
tccattattc ctgattcgag atttctgagt tttagtcctt ctgtgttagc cactgcaata     720
atggtctctg ttattagaga tttgaagatg tgtgacgaag ctgtatacca atctcagctc     780
atgactctac tcaaagttga ttcggagaag gtaaataaat gctatgagtt agtgttagac     840
cacagtccaa gcaagaaaag gatgatgaat tggatgcaac aacccgctag tccgatcggt     900
gtgtttgatg cgtcattcag ttctgatagc tcgaatgagt cgtgggttgt gtctgcttct     960
gcttcagtgt cgtcttcacc atcttcagag cctttgctca agaggagaag agtgcaagag    1020
cagcagatga ggctatcttc aataaaccga atgtttttcg atgtgcttat agtagtcctc    1080
gctaaaccca gctttcttgt acaaagtggt gatatcacaa gcccgggcgg tcttctaggg    1140
ataacagggt aa                                                        1152
```

<210> 49
<211> 383
<212> PRT
<213> Arabidopsis thaliana

<400> 49

```
Met Ala Leu Glu Glu Glu Glu Glu Ser Gln Asn Ala Pro Phe Cys Val
1               5                   10                  15
Leu Asp Gly Leu Phe Cys Glu Glu Glu Ser Glu Phe His Glu Gln Val
            20                  25                  30
Asp Leu Cys Asp Glu Ser Val Glu Lys Phe Pro Phe Leu Asn Leu Gly
        35                  40                  45
Leu Ser Asp His Asp Met Leu Trp Asp Asp Asp Glu Leu Ser Thr Leu
    50                  55                  60
Ile Ser Lys Gln Glu Pro Cys Leu Tyr Asp Glu Ile Leu Asp Asp Glu
65                  70                  75                  80
Phe Leu Val Leu Cys Arg Glu Lys Ala Leu Asp Trp Ile Phe Lys Val
                85                  90                  95
Lys Ser His Tyr Gly Phe Asn Ser Leu Thr Ala Leu Leu Ala Val Asn
            100                 105                 110
Tyr Phe Asp Arg Phe Ile Thr Ser Arg Lys Phe Gln Thr Asp Lys Pro
        115                 120                 125
Trp Met Ser Gln Leu Thr Ala Leu Ala Cys Leu Ser Leu Ala Ala Lys
    130                 135                 140
Val Glu Glu Ile Arg Val Pro Phe Leu Leu Asp Phe Gln Val Glu Glu
145                 150                 155                 160
Ala Arg Tyr Val Phe Glu Ala Lys Thr Ile Gln Arg Met Glu Leu Leu
                165                 170                 175
Val Leu Ser Thr Leu Asp Trp Arg Met His Pro Val Thr Pro Ile Ser
            180                 185                 190
Phe Phe Asp His Ile Ile Arg Arg Tyr Ser Phe Lys Ser His His Gln
        195                 200                 205
Leu Glu Phe Leu Ser Arg Cys Glu Ser Leu Leu Leu Ser Ile Ile Pro
```

```
    210                 215                 220
Asp Ser Arg Phe Leu Ser Phe Ser Pro Ser Val Leu Ala Thr Ala Ile
225                 230                 235                 240
Met Val Ser Val Ile Arg Asp Leu Lys Met Cys Asp Glu Ala Val Tyr
                245                 250                 255
Gln Ser Gln Leu Met Thr Leu Leu Lys Val Asp Ser Glu Lys Val Asn
            260                 265                 270
Lys Cys Tyr Glu Leu Val Leu Asp His Ser Pro Ser Lys Lys Arg Met
        275                 280                 285
Met Asn Trp Met Gln Gln Pro Ala Ser Pro Ile Gly Val Phe Asp Ala
    290                 295                 300
Ser Phe Ser Ser Asp Ser Ser Asn Glu Ser Trp Val Val Ser Ala Ser
305                 310                 315                 320
Ala Ser Val Ser Ser Ser Pro Ser Ser Glu Pro Leu Leu Lys Arg Arg
                325                 330                 335
Arg Val Gln Glu Gln Gln Met Arg Leu Ser Ser Ile Asn Arg Met Phe
            340                 345                 350
Phe Asp Val Leu Ile Val Val Leu Ala Lys Pro Ser Phe Leu Val Gln
        355                 360                 365
Ser Gly Asp Ile Thr Ser Pro Gly Gly Leu Leu Gly Ile Thr Gly
    370                 375                 380
```

<210> 50
<211> 1161
<212> DNA
<213> Aquilegia formosa x Aquilegia pubescens

<400> 50

```
atggctcttc accaccaata tcaacaagaa caacaacaag aatcaacaga tccccatttc      60
cttgtagact cacttttctg cgaagaagag aaatgggtag aagaagaaga ggacttaaat     120
gagagtagta taagtatcat caataataat ggaacaacaa caacaacaac aacaacaaca     180
tcatcagtag ttgaacttgt tccacttttg ttgttagaac aagacttgtt ttgggaagat     240
gaagagctta tctctttgtt tagaaaagaa caagataccc atcttgttat atcttctcaa     300
cttgattctg atccatctat tgctattgct cgtcgtgggg ttattgattg gatgttaagg     360
gtcaatgctc attatgcttt ctctgctctc actgcagttc tatctgttaa ttatcttgat     420
agattccttt caagttttaa gtttcagaaa gataaaccat ggatgattca acttgctgct     480
gttgcttgtt tatctatagc tgctaaagtg gaagaaaccc aagttcctct tctattagac     540
tttcaagttg aagagactat gtatgtgttt gaagcaaaaa ctattcagag aatggagctt     600
ttggtgcttt ctactcttca ttggaagatg aatccagtaa cccctctttc ttttcttgat     660
cacattataa gaaggcttgg attgaaaaac catctacatt gggaattttt tagaaggtgt     720
gaaggtcttc ttttgtctat aattgcagat tcaaggtttg cttgttttct tccatctgtg     780
ttggctactt caacaatgct gcatgttata gaccaagttg agccttgtaa tgcaattgaa     840
tatcaaaacc agctaatggg cattcttaaa atcagcaagg ataaagtgga tgaatgttat     900
aaactcatac tggaatcaac attgagcttc aataggcatg gttacggcaa caaacgcaag     960
tttcaatcta tcccaagtag cccaaatggt gtaattgatg catcattcag ttgcgagaat    1020
tcaaatgatt catgggcatt ggcttcatct gttacatcat ccccagaacc atttttcaag    1080
aagagcagag ctcaagacca acagatgaga ttaccatcct tcagtagagt gtttgtggat    1140
gttctaagca gtcctcctta a                                              1161
```

<210> 51
<211> 386
<212> PRT
<213> Aquilegia formosa x Aquilegia pubescens

<400> 51

```
Met Ala Leu His His Gln Tyr Gln Gln Glu Gln Gln Gln Glu Ser Thr
1               5                   10                  15
Asp Pro His Phe Leu Val Asp Ser Leu Phe Cys Glu Glu Glu Lys Trp
            20                  25                  30
Val Glu Glu Glu Glu Asp Leu Asn Glu Ser Ser Ile Ser Ile Ile Asn
        35                  40                  45
Asn Asn Gly Thr Thr Thr Thr Thr Thr Thr Thr Thr Ser Ser Val Val
    50                  55                  60
Glu Leu Val Pro Leu Leu Leu Leu Glu Gln Asp Leu Phe Trp Glu Asp
65                  70                  75                  80
Glu Glu Leu Ile Ser Leu Phe Arg Lys Glu Gln Asp Thr His Leu Val
                85                  90                  95
Ile Ser Ser Gln Leu Asp Ser Asp Pro Ser Ile Ala Ile Ala Arg Arg
            100                 105                 110
Gly Val Ile Asp Trp Met Leu Arg Val Asn Ala His Tyr Ala Phe Ser
        115                 120                 125
Ala Leu Thr Ala Val Leu Ser Val Asn Tyr Leu Asp Arg Phe Leu Ser
    130                 135                 140
Ser Phe Lys Phe Gln Lys Asp Lys Pro Trp Met Ile Gln Leu Ala Ala
145                 150                 155                 160
Val Ala Cys Leu Ser Ile Ala Ala Lys Val Glu Glu Thr Gln Val Pro
                165                 170                 175
Leu Leu Leu Asp Phe Gln Val Glu Glu Thr Met Tyr Val Phe Glu Ala
            180                 185                 190
Lys Thr Ile Gln Arg Met Glu Leu Leu Val Leu Ser Thr Leu His Trp
        195                 200                 205
Lys Met Asn Pro Val Thr Pro Leu Ser Phe Leu Asp His Ile Ile Arg
    210                 215                 220
Arg Leu Gly Leu Lys Asn His Leu His Trp Glu Phe Phe Arg Arg Cys
225                 230                 235                 240
Glu Gly Leu Leu Leu Ser Ile Ile Ala Asp Ser Arg Phe Ala Cys Phe
                245                 250                 255
Leu Pro Ser Val Leu Ala Thr Ser Thr Met Leu His Val Ile Asp Gln
            260                 265                 270
Val Glu Pro Cys Asn Ala Ile Glu Tyr Gln Asn Gln Leu Met Gly Ile
        275                 280                 285
Leu Lys Ile Ser Lys Asp Lys Val Asp Glu Cys Tyr Lys Leu Ile Leu
    290                 295                 300
Glu Ser Thr Leu Ser Phe Asn Arg His Gly Tyr Gly Asn Lys Arg Lys
305                 310                 315                 320
Phe Gln Ser Ile Pro Ser Ser Pro Asn Gly Val Ile Asp Ala Ser Phe
                325                 330                 335
Ser Cys Glu Asn Ser Asn Asp Ser Trp Ala Leu Ala Ser Ser Val Thr
            340                 345                 350
Ser Ser Pro Glu Pro Phe Phe Lys Lys Ser Arg Ala Gln Asp Gln Gln
        355                 360                 365
Met Arg Leu Pro Ser Phe Ser Arg Val Phe Val Asp Val Leu Ser Ser
    370                 375                 380
Pro Pro
385
```

<210> 52
<211> 1116

<212> DNA
<213> Camellia sinensis

<400> 52

```
atggctcaac cccaacccca accccaaccc caaccccaac aacagcaaca aaatcttcca     60
tttgttttag atgctctgta ttgccaagaa caacatttgg agggaggaga gacagaggac    120
tacttcgatt cagaagagga agattgtcat tacagtgata atattgttag ccctaagcct    180
ccacagctac tggaacaaga cctgttctgg gaagacgaag agttgacctc tttgctgtca    240
aaagaacaag aaaacccctt attccatagt ctcgaaacag acccatcttt gggtggggct    300
cggagagccg ccgtggagtg gttgctgaag gtcaacgccc actactcatt ctctgctctc    360
acggcggtcc tcgccgtgaa ctacctcgat aggttcctct tcagcttcca tttccagaga    420
gagaaaccat ggatgaccca acttgctgct gtggcttgtc tctctcttgc tgccaaagtt    480
gaggagactg aagtcccact cctattagac ctccaggtgg aagatagtag gtatgttttt    540
gaggctaaaa caattcagag aatggagatg ctgatactat caactcttca atggaagatg    600
aatcctgtga ctccactctc atttcttgat cacattacaa ggaggttagg tttgaagaac    660
agactttgtt gtgaatttct caagagatgc gagtcaatcc tcctctgtat catttctgat    720
tctaggttca tgctttatct tccctccgta ttatccactg ccacaatgct gctcgttttt    780
agtagtctag agccctgtct cgcagtagaa taccaaaacc aactcttggg tattcttcaa    840
atcgacaagg acaaagtgga ggattgctat aagttaatgc tagaatcaac atcaggaatt    900
caccaatcca acaaacgaaa gttccgatca atgccgggca gcccaaattg tgtcacagat    960
gtttgtttca gctccgacag ctcgaacgac tcgtgggccg tgacatcctc ggcatctgct   1020
tcggcgtcgg tctgttcctc gccggagcca ttgtcaaaga agagcagagc tcaggaccat   1080
aatgcaactg cagatattct gagcttccat tgctag                             1116
```

<210> 53
<211> 371
<212> PRT
<213> Camellia sinensis

<400> 53

```
Met Ala Gln Pro Gln Pro Gln Pro Gln Pro Gln Pro Gln Gln Gln Gln
1               5                   10                  15
Gln Asn Leu Pro Phe Val Leu Asp Ala Leu Tyr Cys Gln Glu Gln His
            20                  25                  30
Leu Glu Gly Gly Glu Thr Glu Asp Tyr Phe Asp Ser Glu Glu Glu Asp
        35                  40                  45
Cys His Tyr Ser Asp Asn Ile Val Ser Pro Lys Pro Pro Gln Leu Leu
    50                  55                  60
Glu Gln Asp Leu Phe Trp Glu Asp Glu Glu Leu Thr Ser Leu Leu Ser
65                  70                  75                  80
Lys Glu Gln Glu Asn Pro Leu Phe His Ser Leu Glu Thr Asp Pro Ser
                85                  90                  95
Leu Gly Gly Ala Arg Arg Ala Ala Val Glu Trp Leu Leu Lys Val Asn
            100                 105                 110
Ala His Tyr Ser Phe Ser Ala Leu Thr Ala Val Leu Ala Val Asn Tyr
        115                 120                 125
Leu Asp Arg Phe Leu Phe Ser Phe His Phe Gln Arg Glu Lys Pro Trp
    130                 135                 140
Met Thr Gln Leu Ala Ala Val Ala Cys Leu Ser Leu Ala Ala Lys Val
145                 150                 155                 160
Glu Glu Thr Glu Val Pro Leu Leu Leu Asp Leu Gln Val Glu Asp Ser
                165                 170                 175
Arg Tyr Val Phe Glu Ala Lys Thr Ile Gln Arg Met Glu Met Leu Ile
            180                 185                 190
```

```
Leu Ser Thr Leu Gln Trp Lys Met Asn Pro Val Thr Pro Leu Ser Phe
        195                 200                 205
Leu Asp His Ile Thr Arg Arg Leu Gly Leu Lys Asn Arg Leu Cys Cys
    210                 215                 220
Glu Phe Leu Lys Arg Cys Glu Ser Ile Leu Leu Cys Ile Ile Ser Asp
225                 230                 235                 240
Ser Arg Phe Met Leu Tyr Leu Pro Ser Val Leu Ser Thr Ala Thr Met
                245                 250                 255
Leu Leu Val Phe Ser Ser Leu Glu Pro Cys Leu Ala Val Glu Tyr Gln
            260                 265                 270
Asn Gln Leu Leu Gly Ile Leu Gln Ile Asp Lys Asp Lys Val Glu Asp
        275                 280                 285
Cys Tyr Lys Leu Met Leu Glu Ser Thr Ser Gly Ile His Gln Ser Asn
    290                 295                 300
Lys Arg Lys Phe Arg Ser Met Pro Gly Ser Pro Asn Cys Val Thr Asp
305                 310                 315                 320
Val Cys Phe Ser Ser Asp Ser Ser Asn Asp Ser Trp Ala Val Thr Ser
                325                 330                 335
Ser Ala Ser Ala Ser Ala Ser Val Cys Ser Ser Pro Glu Pro Leu Ser
            340                 345                 350
Lys Lys Ser Arg Ala Gln Asp His Asn Ala Thr Ala Asp Ile Leu Ser
        355                 360                 365
Phe His Cys
    370
```

<210> 54
<211> 1119
<212> DNA
<213> Camellia sinensis

<400> 54

```
atgaagagga tgtctcctta cccagagcaa gactcacatc tacagaaccc aatgtttgtc      60
tttgacggtc tctactgtga agaagagcat tttgaggatg atttgggaga gtatggtttg     120
gaacaaggga gtgacaactg cgatgagaat gtgaaaggac ctttagtttt cttggaacat     180
gactgggatt gggatgatga tgagcttgtt tctttaattt ccaaagagaa agagacccat     240
ttgggtctga gtgttttgaa ctcagacgag tccttaatgg tggcaaggag agaatctgtt     300
gattggattt taagggtcat tgctcactat ggtttcactg ttttgaccac tgttttagca     360
gttaactact ttgatagatt catttcaagc ctttcatttc agagagagaa gccatggatg     420
agtcaacttg ttgctgttgc ttgtctctct ttagctgcca aagttgagga gacccaagtg     480
ccccttctct tagacttcca agtggaggaa tcaaagtttg tgtttgaagc caagacaatc     540
cagagaatgg agcttctggt gctatctact cttcaatgga agatgaatcc tgtgacccca     600
ctttcatttg ttgatcacat tgtgaggagg tttggattta agacaaattt gcatttggag     660
tttctgtgga ggtgtgagcg ccttcttctc tctgccatca ctgattcaag gtttgggtgt     720
tatcttcctt ctgtattggc tgctgcaaca atgttacatg ttatcaaaga ggttgagcct     780
tctaatgtat tggactgtca aaatgagctt atggatgttc tcaaaatgag caaggacaaa     840
gtagatgatt gctacaaact catcctcgaa ctgcctggca acaacagtca aatgcaatgc     900
caaacccaca agcgcaagta tcagtccata cccaacagcc caaatggtgt catcgatgtg     960
aatttcagct gcgatagctc gaacgattct tgggcagtga catcctcagt ttcatcatca    1020
ccagaacccc tgttcaagaa gagcagagtt catggtcagc agatgagatt ggctccatta    1080
aggcatatgt ctgtgggtgt agttggcagc cctcgttaa                           1119
```

<210> 55
<211> 372
<212> PRT
<213> Camellia sinensis

<400> 55

```
Met Lys Arg Met Ser Pro Tyr Pro Glu Gln Asp Ser His Leu Gln Asn
1               5                   10                  15
Pro Met Phe Val Phe Asp Gly Leu Tyr Cys Glu Glu Glu His Phe Glu
            20                  25                  30
Asp Asp Leu Gly Glu Tyr Gly Leu Glu Gln Gly Ser Asp Asn Cys Asp
        35                  40                  45
Glu Asn Val Lys Gly Pro Leu Val Phe Leu Glu His Asp Trp Asp Trp
    50                  55                  60
Asp Asp Asp Glu Leu Val Ser Leu Ile Ser Lys Glu Lys Glu Thr His
65                  70                  75                  80
Leu Gly Leu Ser Val Leu Asn Ser Asp Glu Ser Leu Met Val Ala Arg
                85                  90                  95
Arg Glu Ser Val Asp Trp Ile Leu Arg Val Ile Ala His Tyr Gly Phe
            100                 105                 110
Thr Val Leu Thr Thr Val Leu Ala Val Asn Tyr Phe Asp Arg Phe Ile
        115                 120                 125
Ser Ser Leu Ser Phe Gln Arg Glu Lys Pro Trp Met Ser Gln Leu Val
    130                 135                 140
Ala Val Ala Cys Leu Ser Leu Ala Ala Lys Val Glu Glu Thr Gln Val
145                 150                 155                 160
Pro Leu Leu Leu Asp Phe Gln Val Glu Glu Ser Lys Phe Val Phe Glu
                165                 170                 175
Ala Lys Thr Ile Gln Arg Met Glu Leu Leu Val Leu Ser Thr Leu Gln
            180                 185                 190
Trp Lys Met Asn Pro Val Thr Pro Leu Ser Phe Val Asp His Ile Val
        195                 200                 205
Arg Arg Phe Gly Phe Lys Thr Asn Leu His Leu Glu Phe Leu Trp Arg
    210                 215                 220
Cys Glu Arg Leu Leu Leu Ser Ala Ile Thr Asp Ser Arg Phe Gly Cys
225                 230                 235                 240
Tyr Leu Pro Ser Val Leu Ala Ala Ala Thr Met Leu His Val Ile Lys
                245                 250                 255
Glu Val Glu Pro Ser Asn Val Leu Asp Cys Gln Asn Glu Leu Met Asp
            260                 265                 270
Val Leu Lys Met Ser Lys Asp Lys Val Asp Asp Cys Tyr Lys Leu Ile
        275                 280                 285
Leu Glu Leu Pro Gly Asn Asn Ser Gln Met Gln Cys Gln Thr His Lys
    290                 295                 300
Arg Lys Tyr Gln Ser Ile Pro Asn Ser Pro Asn Gly Val Ile Asp Val
305                 310                 315                 320
Asn Phe Ser Cys Asp Ser Ser Asn Asp Ser Trp Ala Val Thr Ser Ser
                325                 330                 335
Val Ser Ser Ser Pro Glu Pro Leu Phe Lys Lys Ser Arg Val His Gly
            340                 345                 350
Gln Gln Met Arg Leu Ala Pro Leu Arg His Met Ser Val Gly Val Val
        355                 360                 365
Gly Ser Pro Arg
    370
```

<210> 56
<211> 1128
<212> DNA
<213> Citrus sinensis

<400> 56

```
atggcatttg gagatgaaca atacccttct tcattcttgc ttgatgcact ctattgtgaa      60
gaagaagagt tagaagatga ggttattgac caagaagatg atgaatgtag ccaaaacaaa     120
aacccagctt gtttgttttc acttcttttg ttagaacaag acttgttctg ggaagatgaa     180
gagctcttgt ctctcttctc caaagaagag caacagcttt taaagcaaga aacacaaacc     240
cattataaag attccgatgt tcttgttgtt gctaggagtg aggctgttga gtgggtgctc     300
aaagttaatg ctcattatgg gttctctact ctcactgcaa tactggctat taactatctg     360
gataggttcc tccgtagctt ccattttcaa atagataagc cttggatgat tcagcttttg     420
gctgtcactt gtctctccct ggctgctaaa gttgaagaaa cccaagtgcc ccttctctta     480
gaccttcaag ttgagggggc aaaatatgtt tttgagacca aagccataca aagaatggag     540
cttttggtgc tctcaacact tgaatggaag atgcatccag tgactccaat ttcatttctt     600
gaccacatca taagaaggct tggattgaag acatctcttc actgggagtt tctcaagaga     660
tgtgagcgtc tgcttctcac tttggtctct gattcaagat ctgtaagtta ccttccttca     720
gtgttggcca ctgccacaat gatgcacata atagaccaag ttgagcctgt gaatcccgtt     780
gattatcaaa accagcttct aggtgtgctt aaaataagca aggaaaaagt aagtgactgt     840
tacaagttga ttcttgagct ggctaatgca aaaaccaatg ctaatagtaa tcctcacaag     900
cgcaagtttg aagcaatccc tggaagccct ggtggcgtga ttgatgctac tgtgtttagc     960
tgtgatgaaa gctcaaacga ttcatggtca gtggcatcat catcagtcct atcatcacca    1020
tcgtcaccag agcctctctt caaaaagagc agagtccaag acccacaaat gactttgcca    1080
atgccatctc tcaatctcaa tagggtcatt gtgggcagtc caagttga                 1128
```

<210> 57
<211> 375
<212> PRT
<213> Citrus sinensis

<400> 57

```
Met Ala Phe Gly Asp Glu Gln Tyr Pro Ser Ser Phe Leu Leu Asp Ala
1               5                   10                  15
Leu Tyr Cys Glu Glu Glu Glu Leu Glu Asp Glu Val Ile Asp Gln Glu
            20                  25                  30
Asp Asp Glu Cys Ser Gln Asn Lys Asn Pro Ala Cys Leu Phe Ser Leu
        35                  40                  45
Leu Leu Leu Glu Gln Asp Leu Phe Trp Glu Asp Glu Glu Leu Leu Ser
    50                  55                  60
Leu Phe Ser Lys Glu Glu Gln Gln Leu Leu Lys Gln Glu Thr Gln Thr
65                  70                  75                  80
His Tyr Lys Asp Ser Asp Val Leu Val Val Ala Arg Ser Glu Ala Val
                85                  90                  95
Glu Trp Val Leu Lys Val Asn Ala His Tyr Gly Phe Ser Thr Leu Thr
            100                 105                 110
Ala Ile Leu Ala Ile Asn Tyr Leu Asp Arg Phe Leu Arg Ser Phe His
        115                 120                 125
Phe Gln Ile Asp Lys Pro Trp Met Ile Gln Leu Leu Ala Val Thr Cys
    130                 135                 140
Leu Ser Leu Ala Ala Lys Val Glu Glu Thr Gln Val Pro Leu Leu Leu
145                 150                 155                 160
Asp Leu Gln Val Glu Gly Ala Lys Tyr Val Phe Glu Thr Lys Ala Ile
                165                 170                 175
Gln Arg Met Glu Leu Leu Val Leu Ser Thr Leu Glu Trp Lys Met His
            180                 185                 190
```

```
Pro Val Thr Pro Ile Ser Phe Leu Asp His Ile Ile Arg Arg Leu Gly
        195                 200                 205
Leu Lys Thr Ser Leu His Trp Glu Phe Leu Lys Arg Cys Glu Arg Leu
    210                 215                 220
Leu Leu Thr Leu Val Ser Asp Ser Arg Ser Val Ser Tyr Leu Pro Ser
225                 230                 235                 240
Val Leu Ala Thr Ala Thr Met Met His Ile Ile Asp Gln Val Glu Pro
                245                 250                 255
Val Asn Pro Val Asp Tyr Gln Asn Gln Leu Leu Gly Val Leu Lys Ile
            260                 265                 270
Ser Lys Glu Lys Val Ser Asp Cys Tyr Lys Leu Ile Leu Glu Leu Ala
        275                 280                 285
Asn Ala Lys Thr Asn Ala Asn Ser Asn Pro His Lys Arg Lys Phe Glu
    290                 295                 300
Ala Ile Pro Gly Ser Pro Gly Gly Val Ile Asp Ala Thr Val Phe Ser
305                 310                 315                 320
Cys Asp Glu Ser Ser Asn Asp Ser Trp Ser Val Ala Ser Ser Ser Val
                325                 330                 335
Leu Ser Ser Pro Ser Ser Pro Glu Pro Leu Phe Lys Lys Ser Arg Val
            340                 345                 350
Gln Asp Pro Gln Met Thr Leu Pro Met Pro Ser Leu Asn Leu Asn Arg
        355                 360                 365
Val Ile Val Gly Ser Pro Ser
    370                 375
```

<210> 58
<211> 1191
<212> DNA
<213> Glycine max

<220>
<221> misc_feature
<222> (117)..(117)
<223> n is a, c, g, or t

<400> 58

```
atggcaattc agcaccacaa tgaacaacta gagcataatg aaaatgtctc atctgtcctt      60
gatgcccttt actgcgatga aggaaagtgg gaggatgaag aggaggagga agaagantat     120
gaagaaagtg aagtaacaac aaacactgga acttctcttt tccctctgct catgttggag     180
caagacttgt tctgggaaga tgaggaacta aactctctct tttccaaaga gaaggttcaa     240
catgaagaag cctatgacta taacaatctg aacagtgatg ataatagcaa tgatcacagt     300
aataataaca ataatgtgct gtcggactct tgtctctctc agcctcgtcg tgaggcagtg     360
gaatggatac tgaaagtcaa tgctcactat ggattctctg ctctcactgc aacactggcc     420
gttacttacc tggataggtt ccttctaagc ttccattttc aaagggagaa gccatggatg     480
atccagcttg tggctgtcac ttgcatctct ttggctgcaa aagttgaaga aactcaagtg     540
cctcttctct tggaccttca agtgcaagac acaaagtatg tgtttgaggc aaagactatt     600
cagagaatgg agctcctggt gctgtccacc ctcaaatgga agatgcaccc cgtgacaccc     660
ctctcctttc tagatcacat tataagaagg cttggattga aaacacatct tcactgggag     720
tttctcaggc gctgtgagca tcttcttttg tctgtgcttt tagattcaag atttgttggt     780
tgtcttcctt ctgtgttggc cactgcaaca atgctgcatg ttatagacca gattaaacac     840
aatggtggga tggaatacaa aactcagctt ctgagtgttc tcaaaattag caaggagaaa     900
gtagatgagt gttataatgc aattctccaa ctctcaaagg ccaataaata tggtcataac     960
aacatcaaca acactagcaa gcgcaagtat gagcaaatcc caagcagccc aagtggcgta    1020
attgatgctg cattttgctc tgatggttcc aacgattcgt gggcagtggg gtcatcattg    1080

ttatattcac caccagagcc tctcttcaag aagagcagaa cccaaggaca acaaatgaat    1140
ttgtcaccac ttaaacggtt cattatcgga attgttggca cccctcctta a             1191
```

<210> 59
<211> 396
<212> PRT
<213> Glycine max

<220>
<221> misc_feature
<222> (39)..(39)
<223> Xaa can be any naturally occurring amino acid

<400> 59

```
Met Ala Ile Gln His His Asn Glu Gln Leu Glu His Asn Glu Asn Val
1               5                   10                  15
Ser Ser Val Leu Asp Ala Leu Tyr Cys Asp Glu Gly Lys Trp Glu Asp
            20                  25                  30
Glu Glu Glu Glu Glu Glu Xaa Tyr Glu Glu Ser Glu Val Thr Thr Asn
        35                  40                  45
Thr Gly Thr Ser Leu Phe Pro Leu Leu Met Leu Glu Gln Asp Leu Phe
    50                  55                  60
Trp Glu Asp Glu Glu Leu Asn Ser Leu Phe Ser Lys Glu Lys Val Gln
65                  70                  75                  80
His Glu Glu Ala Tyr Asp Tyr Asn Asn Leu Asn Ser Asp Asp Asn Ser
                85                  90                  95
Asn Asp His Ser Asn Asn Asn Asn Asn Val Leu Ser Asp Ser Cys Leu
            100                 105                 110
Ser Gln Pro Arg Arg Glu Ala Val Glu Trp Ile Leu Lys Val Asn Ala
        115                 120                 125
His Tyr Gly Phe Ser Ala Leu Thr Ala Thr Leu Ala Val Thr Tyr Leu
    130                 135                 140
Asp Arg Phe Leu Leu Ser Phe His Phe Gln Arg Glu Lys Pro Trp Met
145                 150                 155                 160
Ile Gln Leu Val Ala Val Thr Cys Ile Ser Leu Ala Ala Lys Val Glu
                165                 170                 175
Glu Thr Gln Val Pro Leu Leu Leu Asp Leu Gln Val Gln Asp Thr Lys
            180                 185                 190
Tyr Val Phe Glu Ala Lys Thr Ile Gln Arg Met Glu Leu Leu Val Leu
        195                 200                 205
Ser Thr Leu Lys Trp Lys Met His Pro Val Thr Pro Leu Ser Phe Leu
    210                 215                 220
Asp His Ile Ile Arg Arg Leu Gly Leu Lys Thr His Leu His Trp Glu
225                 230                 235                 240
Phe Leu Arg Arg Cys Glu His Leu Leu Leu Ser Val Leu Leu Asp Ser
                245                 250                 255
Arg Phe Val Gly Cys Leu Pro Ser Val Leu Ala Thr Ala Thr Met Leu
            260                 265                 270
His Val Ile Asp Gln Ile Lys His Asn Gly Gly Met Glu Tyr Lys Thr
        275                 280                 285
Gln Leu Leu Ser Val Leu Lys Ile Ser Lys Glu Lys Val Asp Glu Cys
    290                 295                 300
Tyr Asn Ala Ile Leu Gln Leu Ser Lys Ala Asn Lys Tyr Gly His Asn
305                 310                 315                 320
```

```
Asn Ile Asn Asn Thr Ser Lys Arg Lys Tyr Glu Gln Ile Pro Ser Ser
                325                 330                 335
Pro Ser Gly Val Ile Asp Ala Ala Phe Cys Ser Asp Gly Ser Asn Asp
            340                 345                 350
Ser Trp Ala Val Gly Ser Ser Leu Leu Tyr Ser Pro Pro Glu Pro Leu
        355                 360                 365
Phe Lys Lys Ser Arg Thr Gln Gly Gln Gln Met Asn Leu Ser Pro Leu
    370                 375                 380
Lys Arg Phe Ile Ile Gly Ile Val Gly Thr Pro Pro
385                 390                 395
```

<210> 60
<211> 1125
<212> DNA
<213> Gossypium hirsutum

<400> 60

```
atggcaatac agcaatatga acagcagcaa caacaaccag agaatcaccc ttccttcttg      60
ctagatgctc tctactgtga ggaagaagcg gatgcagggg aagttttaga ggaagagagt     120
tcttgtgtgg gctgtaacaa tggcggaaac ccttcatttt tcccactgtt gttgttagag     180
caggatttgt tttgggaaga cggggagctt ctttcacttt ttgctaaaga aacagagcag     240
cagccgtctt gtttcaatgt gggaaccgat gagtccctag caatggctcg ccgagaggct     300
gccgagtgga tgcttaaagt caatgctcga tttggattct ccactctcac ggctgtactt     360
tccattaact atttggacag gttcttaagt acctttcagt ttcaaagaga taatccttgg     420
atgatccaac ttctgggtgt cacttgtctc tctttggctg caaaagttga agagacacaa     480
gtgcctctgc tcctagacct acaagtggag gagacaaagt atgttttcga ggccaaaact     540
atccaaagaa tggagctttt ggtgctctcc acactgaaat ggaagatgca tccaattaca     600
ccccttcat ttctagatca catcataaga agactggggt tgaaaaccca cctccattgg      660
gagtttctta agcgatgtga gcgtctcctc ctctgtgtaa tctctgatgc aagatccatc     720
cattatcttc cctctgtatt ggctactgca accatgatgc acgtcataga ccaagttgag     780
cttttcaatc ccattgacta ccaaaatcag ctgctgagtg ttcttaaaat tagcaaggaa     840
aaagtaaacg attgttacaa gctcatcctt gatgtatcaa caagacccca ggcccaaggc     900
aatggtggtg catgtaagag gaaggtggag gagagggttc ctagcagccc tagtggagtg     960
attgatgctg catttggcag tgatagctcg agcgattctt ggggcacggt gtccttatcg    1020
cctgagcagc agccaccttt taagaagagc agagcccaag agcaagtaat gcgtttgcca    1080
tcactcaacc gagtctttgt agacattgtt ggcagccctt cttaa                   1125
```

<210> 61
<211> 374
<212> PRT
<213> Gossypium hirsutum

<400> 61

```
Met Ala Ile Gln Gln Tyr Glu Gln Gln Gln Gln Gln Pro Glu Asn His
1               5                   10                  15
Pro Ser Phe Leu Leu Asp Ala Leu Tyr Cys Glu Glu Glu Ala Asp Ala
            20                  25                  30
Gly Glu Val Leu Glu Glu Glu Ser Ser Cys Val Gly Cys Asn Asn Gly
        35                  40                  45
Gly Asn Pro Ser Phe Phe Pro Leu Leu Leu Leu Glu Gln Asp Leu Phe
    50                  55                  60
Trp Glu Asp Gly Glu Leu Leu Ser Leu Phe Ala Lys Glu Thr Glu Gln
65                  70                  75                  80
Gln Pro Ser Cys Phe Asn Val Gly Thr Asp Glu Ser Leu Ala Met Ala
```

```
                85                  90                  95
Arg Arg Glu Ala Ala Glu Trp Met Leu Lys Val Asn Ala Arg Phe Gly
            100                 105                 110
Phe Ser Thr Leu Thr Ala Val Leu Ser Ile Asn Tyr Leu Asp Arg Phe
        115                 120                 125
Leu Ser Thr Phe Gln Phe Gln Arg Asp Asn Pro Trp Met Ile Gln Leu
    130                 135                 140
Leu Gly Val Thr Cys Leu Ser Leu Ala Ala Lys Val Glu Glu Thr Gln
145                 150                 155                 160
Val Pro Leu Leu Leu Asp Leu Gln Val Glu Glu Thr Lys Tyr Val Phe
                165                 170                 175
Glu Ala Lys Thr Ile Gln Arg Met Glu Leu Leu Val Leu Ser Thr Leu
            180                 185                 190
Lys Trp Lys Met His Pro Ile Thr Pro Leu Ser Phe Leu Asp His Ile
        195                 200                 205
Ile Arg Arg Leu Gly Leu Lys Thr His Leu His Trp Glu Phe Leu Lys
    210                 215                 220
Arg Cys Glu Arg Leu Leu Leu Cys Val Ile Ser Asp Ala Arg Ser Ile
225                 230                 235                 240
His Tyr Leu Pro Ser Val Leu Ala Thr Ala Thr Met Met His Val Ile
                245                 250                 255
Asp Gln Val Glu Leu Phe Asn Pro Ile Asp Tyr Gln Asn Gln Leu Leu
            260                 265                 270
Ser Val Leu Lys Ile Ser Lys Glu Lys Val Asn Asp Cys Tyr Lys Leu
        275                 280                 285
Ile Leu Asp Val Ser Thr Arg Pro Gln Ala Gln Gly Asn Gly Gly Ala
    290                 295                 300
Cys Lys Arg Lys Val Glu Glu Arg Val Pro Ser Ser Pro Ser Gly Val
305                 310                 315                 320
Ile Asp Ala Ala Phe Gly Ser Asp Ser Ser Ser Asp Ser Trp Gly Thr
                325                 330                 335
Val Ser Leu Ser Pro Glu Gln Gln Pro Pro Phe Lys Lys Ser Arg Ala
            340                 345                 350
Gln Glu Gln Val Met Arg Leu Pro Ser Leu Asn Arg Val Phe Val Asp
        355                 360                 365
Ile Val Gly Ser Pro Ser
    370
```

<210> 62
<211> 1173
<212> DNA
<213> Lotus corniculatus

<400> 62

```
atggcaatcc atcaacatca tcacaacaat gtcattgacc agctagaaca aaatgaaaat     60
gtttcttctg tcttggatgc tctttactgt gatgaagaaa aatgggagga agaggaagta    120
gaacaagtgg ttggagagtt atctgaagaa gaaacaagtg atgtgacaac aaacaatgac    180
cctaacaaca cttgttctct gtttcccctg cttttgttgg agcaagactt gttctgggaa    240
gatgaagaac tcaactctct cttctccaaa gagaagatcc aacaccaaaa ctattataat    300
gatgtgaact cggacccttt tctctctcag cctcgtcatg aggcagtgaa atggatgctt    360
aaagtcaatg ctcattatgg attctctgct ctcactgcaa cacttgctgt tacctacttt    420
gataacttcc ttttgagctt ccattttcaa agtgagaagc catggatgat ccagcttgct    480
gctgttactt gcatctcttt ggcagctaaa gttgaagaaa cccaagtgcc acttctctta    540
gaccttcaag tgcaagatgc taagtttgtg tttgaggcaa agaccattct gaaaatggag    600
```

```
cttctggttc tgtccacact caaatggaag atgcatcctg tgactccact ttcatttctg    660
gatcacatta tcagaaggct tggattgaaa acacaccttc attgggagtt tctcaggcgc    720
tgtgagcatc ttcttttgtc tgtgctttta gattcaagat ttgttggtgt tcttccttct    780
gtgttggcca ctgcaacaat gctgcatgtt atagaccaga ttgagaagag tgatggggtg    840
gaatacaaaa agcagcttct gggtgttctc aaaattaaca aggggaaagt agatgaatgc    900
tatgatgcca tgcttgagct tacaaatgcc aatgattatg atgataacaa gaagcttaat    960
aagcgcaagt atgaggaaat aatccctggt agcccaagtg gcgtcattga tgccgcattt   1020
aactctgatg gttccaacga ttcgtggaca gtggggtcat cattgttttc atcctcaggc   1080
ccagagtctc ctctgttcaa gaaaagcaga acccaaatga aattgtcacc acttaacagg   1140
gtcattgttg gaattgttag cacttcacct tga                                1173
```

<210> 63
<211> 390
<212> PRT
<213> Lotus corniculatus

<400> 63

```
Met Ala Ile His Gln His His His Asn Asn Val Ile Asp Gln Leu Glu
1               5                   10                  15
Gln Asn Glu Asn Val Ser Ser Val Leu Asp Ala Leu Tyr Cys Asp Glu
            20                  25                  30
Glu Lys Trp Glu Glu Glu Glu Val Glu Gln Val Val Gly Glu Leu Ser
        35                  40                  45
Glu Glu Glu Thr Ser Asp Val Thr Thr Asn Asn Asp Pro Asn Asn Thr
    50                  55                  60
Cys Ser Leu Phe Pro Leu Leu Leu Leu Glu Gln Asp Leu Phe Trp Glu
65                  70                  75                  80
Asp Glu Glu Leu Asn Ser Leu Phe Ser Lys Glu Lys Ile Gln His Gln
                85                  90                  95
Asn Tyr Tyr Asn Asp Val Asn Ser Asp Pro Phe Leu Ser Gln Pro Arg
            100                 105                 110
His Glu Ala Val Lys Trp Met Leu Lys Val Asn Ala His Tyr Gly Phe
        115                 120                 125
Ser Ala Leu Thr Ala Thr Leu Ala Val Thr Tyr Phe Asp Asn Phe Leu
    130                 135                 140
Leu Ser Phe His Phe Gln Ser Glu Lys Pro Trp Met Ile Gln Leu Ala
145                 150                 155                 160
Ala Val Thr Cys Ile Ser Leu Ala Ala Lys Val Glu Glu Thr Gln Val
                165                 170                 175
Pro Leu Leu Leu Asp Leu Gln Val Gln Asp Ala Lys Phe Val Phe Glu
            180                 185                 190
Ala Lys Thr Ile Leu Lys Met Glu Leu Leu Val Leu Ser Thr Leu Lys
        195                 200                 205
Trp Lys Met His Pro Val Thr Pro Leu Ser Phe Leu Asp His Ile Ile
    210                 215                 220
Arg Arg Leu Gly Leu Lys Thr His Leu His Trp Glu Phe Leu Arg Arg
225                 230                 235                 240
Cys Glu His Leu Leu Leu Ser Val Leu Leu Asp Ser Arg Phe Val Gly
                245                 250                 255
Val Leu Pro Ser Val Leu Ala Thr Ala Thr Met Leu His Val Ile Asp
            260                 265                 270
Gln Ile Glu Lys Ser Asp Gly Val Glu Tyr Lys Lys Gln Leu Leu Gly
        275                 280                 285
Val Leu Lys Ile Asn Lys Gly Lys Val Asp Glu Cys Tyr Asp Ala Met
```

```
    290                 295                 300
Leu Glu Leu Thr Asn Ala Asn Asp Tyr Asp Asp Asn Lys Lys Leu Asn
305                 310                 315                 320
Lys Arg Lys Tyr Glu Glu Ile Ile Pro Gly Ser Pro Ser Gly Val Ile
                325                 330                 335
Asp Ala Ala Phe Asn Ser Asp Gly Ser Asn Asp Ser Trp Thr Val Gly
            340                 345                 350
Ser Ser Leu Phe Ser Ser Ser Gly Pro Glu Ser Pro Leu Phe Lys Lys
        355                 360                 365
Ser Arg Thr Gln Met Lys Leu Ser Pro Leu Asn Arg Val Ile Val Gly
    370                 375                 380
Ile Val Ser Thr Ser Pro
385                 390
```

<210> 64
<211> 1161
<212> DNA
<213> Medicago trunculata

<400> 64

```
atggctatcc atcatcatca tcacaatcac caacaacttc aacaacacac ttcttctctt      60
tttgatgcac tttactgtga tgaagaagaa aaatgggaag atgatgatga aggagaagtt     120
gtagatgaag gagcacaaag tgatgtcaca acaacaaact atgatatatt ggactctact     180
tccctttac ctctgctttt gttagaacag aacttgttca atgaagatga agaactcaac      240
actcttttct ccaaagagat aactcaacaa gaaacatatt acgaggatct gaaaaatgtg     300
atcaactttg actcactctc tcaaccacgt cgtgaagctg ttgaatggat gcttaaagtc     360
aatgctcatt atggtttctc tgctctcact gcaacacttg ctgttaacta tcttgatagg     420
tttcttttaa gcttccattt ccaaaaagag aaaccatgga tgattcagct tgttgctgtt     480
acttgcatct ctttagctgc taaagttgaa gaaactcaag ttcctcttct cttagacctt     540
caagtgcaag atactaaata tgtgtttgag gcaaagacta ttcagagaat ggagctattg     600
attctgtcaa cactgaaatg gaagatgcat ccagtgacaa cacactcttt tctagatcac     660
attataagaa ggcttggatt gaaaactaat cttcattggg agtttcttag gcgctgtgag     720
aatcttcttc tatctgtact tttagattca agatttgttg gttgtgttcc ttctgtgttg     780
gccactgcta caatgttgca tgttatagac cagattgaac agagtgatga taatggtgtg     840
gattacaaaa atcagcttct taatgttctc aaaatcagca aggagaaagt tgatgaatgt     900
tataatgcga ttcttcatct tacaaatgca aataattatg gtcataaacg aaaatatgaa     960
gaaatccctg gtagtccaag tggcgtaatt gatgctgttt ttagttctga tggttctaac    1020
gattcgtgga cagtgggagc atcatcatat tcaacctcag agcctgtgtt taagaagacc    1080
aagaatcaag gacaaaatat gaatttgtca ccgattaaca gggtcattgt cggaattctt    1140
gccactgcaa cctctcctta a                                              1161
```

<210> 65
<211> 386
<212> PRT
<213> Medicago trunculata

<400> 65

```
Met Ala Ile His His His His His Asn His Gln Gln Leu Gln Gln His
1               5                   10                  15
Thr Ser Ser Leu Phe Asp Ala Leu Tyr Cys Asp Glu Glu Glu Lys Trp
            20                  25                  30
Glu Asp Asp Asp Glu Gly Glu Val Val Asp Glu Gly Ala Gln Ser Asp
        35                  40                  45
Val Thr Thr Thr Asn Tyr Asp Ile Leu Asp Ser Thr Ser Leu Leu Pro
```

```
    50                  55                  60
Leu Leu Leu Leu Glu Gln Asn Leu Phe Asn Glu Asp Glu Glu Leu Asn
65                  70                  75                  80
Thr Leu Phe Ser Lys Glu Ile Thr Gln Gln Glu Thr Tyr Tyr Glu Asp
                85                  90                  95
Leu Lys Asn Val Ile Asn Phe Asp Ser Leu Ser Gln Pro Arg Arg Glu
            100                 105                 110
Ala Val Glu Trp Met Leu Lys Val Asn Ala His Tyr Gly Phe Ser Ala
        115                 120                 125
Leu Thr Ala Thr Leu Ala Val Asn Tyr Leu Asp Arg Phe Leu Leu Ser
    130                 135                 140
Phe His Phe Gln Lys Glu Lys Pro Trp Met Ile Gln Leu Val Ala Val
145                 150                 155                 160
Thr Cys Ile Ser Leu Ala Ala Lys Val Glu Glu Thr Gln Val Pro Leu
                165                 170                 175
Leu Leu Asp Leu Gln Val Gln Asp Thr Lys Tyr Val Phe Glu Ala Lys
            180                 185                 190
Thr Ile Gln Arg Met Glu Leu Leu Ile Leu Ser Thr Leu Lys Trp Lys
        195                 200                 205
Met His Pro Val Thr Thr His Ser Phe Leu Asp His Ile Ile Arg Arg
    210                 215                 220
Leu Gly Leu Lys Thr Asn Leu His Trp Glu Phe Leu Arg Arg Cys Glu
225                 230                 235                 240
Asn Leu Leu Leu Ser Val Leu Leu Asp Ser Arg Phe Val Gly Cys Val
                245                 250                 255
Pro Ser Val Leu Ala Thr Ala Thr Met Leu His Val Ile Asp Gln Ile
            260                 265                 270
Glu Gln Ser Asp Asp Asn Gly Val Asp Tyr Lys Asn Gln Leu Leu Asn
        275                 280                 285
Val Leu Lys Ile Ser Lys Glu Lys Val Asp Glu Cys Tyr Asn Ala Ile
    290                 295                 300
Leu His Leu Thr Asn Ala Asn Asn Tyr Gly His Lys Arg Lys Tyr Glu
305                 310                 315                 320
Glu Ile Pro Gly Ser Pro Ser Gly Val Ile Asp Ala Val Phe Ser Ser
                325                 330                 335
Asp Gly Ser Asn Asp Ser Trp Thr Val Gly Ala Ser Ser Tyr Ser Thr
            340                 345                 350
Ser Glu Pro Val Phe Lys Lys Thr Lys Asn Gln Gly Gln Asn Met Asn
        355                 360                 365
Leu Ser Pro Ile Asn Arg Val Ile Val Gly Ile Leu Ala Thr Ala Thr
    370                 375                 380
Ser Pro
385
```

<210> 66
<211> 1119
<212> DNA
<213> Scutellaria baicalensis

<400> 66

```
atggtttcgg aatttcagga gcacgaatcc cttctccaaa accctatctt tgatgccctt    60
tattgtgacg aggagcgttt tgatgaatgt gtaggcggcg ctggttcggg cttcaaagag   120
cccgaaatca acgattttaa tgagattcac aataaccctt ttgcttttct gtttgagcac   180
gaccttttct gggagagtga ggagcttgac gccctgttaa cgaaggagaa aacgcagacc   240
```

```
catttgactt ttgatgaaat aaactcagat gcgtctttga aggcgatgag aaatgaggcg     300
attaactgga tgctgaaggt gattgcccac tacggcttca atgcgctgac tgctgttttg     360
gctgtcaact attatgatag attcatcact agtgtttgtt ttcagaagga taagccatgg     420
atgagtcaat tagctgctgt ggcttgtctt tctgtagctg ccaaggtgga ggaaactcaa     480
gtgcctcttt tgttggattt acaagttgaa gaatctaagt atttgtttga agctaagacc     540
atccaaagaa tggagctttt ggtgctttct accctccaat ggaggatgaa tcctgtgacg     600
ccaatctcat tctttgacca cattgcaagg agatttgagt ttgtaaagaa cctacattct     660
gtatttttaa ggaggtgtga gagtttaatc ctctccatta tcactgattg tagattggta     720
aagtattttc cttcagttat tgcttctgca gcaatgatat atgcgattag agagtttgag     780
actcctgatg ctctggaata tgaggatcaa ctcttgagtg tgctaagaac tagcaaggac     840
aaagttgatg attgccgcaa actcattgtg gatgcaatgt atggtggttt cagccacaag     900
ccttgctata aacgcaaata tgagtcgatc ccaagcagtc caagtggtgt cattgatgcg     960
tatttgagct ctgatagctc tgttgattcg tgggctgtta cattatcagt gtcatcgtcg    1020
ccagagcctt cgtttaagag aagcaaagct caagatcagc acatgagatt ggctccacta    1080
agcagtgtat ctcttggcct tgctcatcgt attaattga                           1119
```

<210> 67

<211> 372

<212> PRT

<213> Scutellaria baicalensis

<400> 67

```
Met Val Ser Glu Phe Gln Glu His Glu Ser Leu Leu Gln Asn Pro Ile
1               5                   10                  15
Phe Asp Ala Leu Tyr Cys Asp Glu Glu Arg Phe Asp Glu Cys Val Gly
            20                  25                  30
Gly Ala Gly Ser Gly Phe Lys Glu Pro Glu Ile Asn Asp Phe Asn Glu
        35                  40                  45
Ile His Asn Asn Pro Phe Ala Phe Leu Phe Glu His Asp Leu Phe Trp
    50                  55                  60
Glu Ser Glu Glu Leu Asp Ala Leu Leu Thr Lys Glu Lys Thr Gln Thr
65                  70                  75                  80
His Leu Thr Phe Asp Glu Ile Asn Ser Asp Ala Ser Leu Lys Ala Met
                85                  90                  95
Arg Asn Glu Ala Ile Asn Trp Met Leu Lys Val Ile Ala His Tyr Gly
            100                 105                 110
Phe Asn Ala Leu Thr Ala Val Leu Ala Val Asn Tyr Tyr Asp Arg Phe
        115                 120                 125
Ile Thr Ser Val Cys Phe Gln Lys Asp Lys Pro Trp Met Ser Gln Leu
    130                 135                 140
Ala Ala Val Ala Cys Leu Ser Val Ala Ala Lys Val Glu Glu Thr Gln
145                 150                 155                 160
Val Pro Leu Leu Leu Asp Leu Gln Val Glu Glu Ser Lys Tyr Leu Phe
                165                 170                 175
Glu Ala Lys Thr Ile Gln Arg Met Glu Leu Leu Val Leu Ser Thr Leu
            180                 185                 190
Gln Trp Arg Met Asn Pro Val Thr Pro Ile Ser Phe Phe Asp His Ile
        195                 200                 205
Ala Arg Arg Phe Glu Phe Val Lys Asn Leu His Ser Val Phe Leu Arg
    210                 215                 220
Arg Cys Glu Ser Leu Ile Leu Ser Ile Ile Thr Asp Cys Arg Leu Val
225                 230                 235                 240
Lys Tyr Phe Pro Ser Val Ile Ala Ser Ala Ala Met Ile Tyr Ala Ile
                245                 250                 255
```

Arg Glu Phe Glu Thr Pro Asp Ala Leu Glu Tyr Glu Asp Gln Leu Leu
260 265 270
Ser Val Leu Arg Thr Ser Lys Asp Lys Val Asp Asp Cys Arg Lys Leu
275 280 285
Ile Val Asp Ala Met Tyr Gly Gly Phe Ser His Lys Pro Cys Tyr Lys
290 295 300
Arg Lys Tyr Glu Ser Ile Pro Ser Ser Pro Ser Gly Val Ile Asp Ala
305 310 315 320
Tyr Leu Ser Ser Asp Ser Ser Val Asp Ser Trp Ala Val Thr Leu Ser
325 330 335
Val Ser Ser Ser Pro Glu Pro Ser Phe Lys Arg Ser Lys Ala Gln Asp
340 345 350
Gln His Met Arg Leu Ala Pro Leu Ser Ser Val Ser Leu Gly Leu Ala
355 360 365
His Arg Ile Asn
370

<210> 68
<211> 1164
<212> DNA
<213> Zea mays

<400> 68

atggcagctt tcgccgcgtt gttcgacccc ctctactgcc cggaggagca cctcgatctg 60
taccacgaag gacccgtcga ggttgtggac gagcagtggc aggaccagcg cggacagcag 120
caaccggcgg ctcttgacga cgagctgccg gcgctgttcg aggcgctccg ggacaaggag 180
ggggtggtgc tggcgggtga tggggaggag gatgggtacg gcggctcggc aggccgggag 240
gccgcagtcg gctgggcgtc acgcgccgcg gcacggctgg gcttctctgc gctcacttcc 300
gcgctgtccg ccgcctacct ggaccgctgc ttcctccccg ggggcgcgct ccgtctcggc 360
gaccagccct ggatgtcgcg cctcgccgcc gtcgcctgtg tcgcgctcgc cgccaaggtc 420
gaggaaacgc gcgtgccgct gctcctcgac ctccagctct gcgccgccgc cagctccgac 480
gctgacgcag cggacgcgga cgtgttcgag gccaagacgg tgcgccggat ggagctgctc 540
gttctctccg cgctagggtg gcggatgcac cctgtcacgc ccttctccta cctccagcct 600
gtcctcgccg acgctgcgat gcgcctacgc aactgcgagg ccgtcctgct cgcggtcatg 660
gccgattgga ggtggcctcg gcaccggccc tcggcgtggg ccgccgccgc attgctcacc 720
acagccggcg gcggcgacga cgactcggag ctgctcgcgc tcatcaatgc ccccgaggac 780
gagaccgcgg agtgcgccaa gatcatctcc gaggtgacag gcatgagctt ccttgtctgc 840
gacgtcggcg gcatgatcgc cgggaataag cgtaagcacg cggcggcgcg gatgtactcg 900
ccgccgctga gcccgagcgg cgtgatcggc gcgctgtcct gcttcagctg cgagagctcg 960
ttgtccgcca cagcggactc gcgcaccctc gctactacgg ctgcgggggt cggcccgtgg 1020
gcaccgtcag cgcccgtgtc cgtgtcgtct tcccctgagc ccccaggtcg ggcccccaag 1080
cgcgctgcgg cggcgggggt cccgcatccg cttcccccg acgaggagag ccgcgacgcc 1140
tggccgtcca cctgcgccgc gtga 1164

<210> 69
<211> 387
<212> PRT
<213> Zea mays

<400> 69

Met Ala Ala Phe Ala Ala Leu Phe Asp Pro Leu Tyr Cys Pro Glu Glu
1 5 10 15
His Leu Asp Leu Tyr His Glu Gly Pro Val Glu Val Val Asp Glu Gln
20 25 30

```
Trp Gln Asp Gln Arg Gly Gln Gln Gln Pro Ala Ala Leu Asp Asp Glu
        35                  40                  45
Leu Pro Ala Leu Phe Glu Ala Leu Arg Asp Lys Glu Gly Val Val Leu
    50                  55                  60
Ala Gly Asp Gly Glu Glu Asp Gly Tyr Gly Gly Ser Ala Gly Arg Glu
65                  70                  75                  80
Ala Ala Val Gly Trp Ala Ser Arg Ala Ala Ala Arg Leu Gly Phe Ser
                85                  90                  95
Ala Leu Thr Ser Ala Leu Ser Ala Ala Tyr Leu Asp Arg Cys Phe Leu
            100                 105                 110
Pro Gly Gly Ala Leu Arg Leu Gly Asp Gln Pro Trp Met Ser Arg Leu
        115                 120                 125
Ala Ala Val Ala Cys Val Ala Leu Ala Ala Lys Val Glu Glu Thr Arg
    130                 135                 140
Val Pro Leu Leu Leu Asp Leu Gln Leu Cys Ala Ala Ala Ser Ser Asp
145                 150                 155                 160
Ala Asp Ala Ala Asp Ala Asp Val Phe Glu Ala Lys Thr Val Arg Arg
                165                 170                 175
Met Glu Leu Leu Val Leu Ser Ala Leu Gly Trp Arg Met His Pro Val
            180                 185                 190
Thr Pro Phe Ser Tyr Leu Gln Pro Val Leu Ala Asp Ala Ala Met Arg
        195                 200                 205
Leu Arg Asn Cys Glu Ala Val Leu Leu Ala Val Met Ala Asp Trp Arg
    210                 215                 220
Trp Pro Arg His Arg Pro Ser Ala Trp Ala Ala Ala Ala Leu Leu Thr
225                 230                 235                 240
Thr Ala Gly Gly Gly Asp Asp Asp Ser Glu Leu Leu Ala Leu Ile Asn
                245                 250                 255
Ala Pro Glu Asp Glu Thr Ala Glu Cys Ala Lys Ile Ile Ser Glu Val
            260                 265                 270
Thr Gly Met Ser Phe Leu Val Cys Asp Val Gly Gly Met Ile Ala Gly
        275                 280                 285
Asn Lys Arg Lys His Ala Ala Ala Arg Met Tyr Ser Pro Pro Leu Ser
    290                 295                 300
Pro Ser Gly Val Ile Gly Ala Leu Ser Cys Phe Ser Cys Glu Ser Ser
305                 310                 315                 320
Leu Ser Ala Thr Ala Asp Ser Arg Thr Leu Ala Thr Thr Ala Ala Gly
                325                 330                 335
Val Gly Pro Trp Ala Pro Ser Ala Pro Val Ser Val Ser Ser Ser Pro
            340                 345                 350
Glu Pro Pro Gly Arg Ala Pro Lys Arg Ala Ala Ala Ala Gly Val Pro
        355                 360                 365
His Pro Leu Pro Pro Asp Glu Glu Ser Arg Asp Ala Trp Pro Ser Thr
    370                 375                 380
Cys Ala Ala
385
```

<210> 70
<211> 1176
<212> DNA
<213> Zea mays

<400> 70

```
atggcagctt tcgccgcgct gttcgacccc ctgtactgcc cggaggagca cctcgatctg      60
```

```
taccgcgacg aacccggcga gggtgcggac gagcagtggc cgggccagca cggacagcag    120
gagccggctg tcctcgacga cgagctgccg gcgctgttcg aggcacaccg ggccaaggag    180
ggggtggtgc tggcggagga tggcgggtac ggcggcgcag ctgggcgtga ggccgcggtc    240
ggctgggttt cacgcgccgc ggcgcggcta ggcttctccg cgctcaccgc cgcgctcgcc    300
gccgcctacc tcgaccgctg cttcctcccc gggggcgcgc tccggctcgg cgaccagccc    360
tggatggcgc gcctagccgc cgtcacctgc ttcgcgctcg ccgccaaggt cgaggagacg    420
cgcgtgccgc cgctcctcga cctccagctc tacgccgccg ctgacgccgc ggatccgtac    480
gtattcgagg ccaagacggt gcgccggatg gagctgctcg tgctctccgc gcttgggtgg    540
cggatgcacc ctgtcacgcc cttctcctac ctccagcccg tcctcgccga cgctgcgacg    600
cgcctgcgta gctgcgaggg cgtcctgctc gcggtcatgg ccgactggag gtggcctcgg    660
caccggcctt cggcgtgggc cgccgccgcg ttgctgatca cagccgccgc cggcgacggc    720
ggcgacggcg acggcgacac ggagctcctg gcgctcctca ttgcccccga ggacaagacc    780
gccgagtgtg ccaagatcat ctccgaggtg acgggcatga gcttcctcgc ctgcgatgtc    840
ggcgtgagcg ccggaaataa gcgtaagcac gcggcggcgc agttgtactc gccgccgccg    900
agcccgagcg gcgtgatcgg cgcgctgtcc tgcttcagct gcgagagctc gacgtccgcc    960
accgctatgg ctgcggcggt cggcccgtgg gcgccgtcgg cgtccgtgtc cgtgtcgtcc   1020
tctccagagc caccaggtcg ggcccccaag cgcgcagcgg cggcgtcggc gtcggcgtcg   1080
gcgtcagccg gggtcgcgcc accggtccag gtcccgcatc agctaccccc cgacgaggag   1140
agccgcgacg cctggccgtc cacctgcgcc gcgtga                              1176
```

<210> 71
<211> 391
<212> PRT
<213> Zea mays

<400> 71

```
Met Ala Ala Phe Ala Ala Leu Phe Asp Pro Leu Tyr Cys Pro Glu Glu
1               5                   10                  15
His Leu Asp Leu Tyr Arg Asp Glu Pro Gly Glu Gly Ala Asp Glu Gln
            20                  25                  30
Trp Pro Gly Gln His Gly Gln Gln Glu Pro Ala Val Leu Asp Asp Glu
        35                  40                  45
Leu Pro Ala Leu Phe Glu Ala His Arg Ala Lys Glu Gly Val Val Leu
    50                  55                  60
Ala Glu Asp Gly Gly Tyr Gly Gly Ala Ala Gly Arg Glu Ala Ala Val
65                  70                  75                  80
Gly Trp Val Ser Arg Ala Ala Ala Arg Leu Gly Phe Ser Ala Leu Thr
                85                  90                  95
Ala Ala Leu Ala Ala Ala Tyr Leu Asp Arg Cys Phe Leu Pro Gly Gly
            100                 105                 110
Ala Leu Arg Leu Gly Asp Gln Pro Trp Met Ala Arg Leu Ala Ala Val
        115                 120                 125
Thr Cys Phe Ala Leu Ala Ala Lys Val Glu Glu Thr Arg Val Pro Pro
    130                 135                 140
Leu Leu Asp Leu Gln Leu Tyr Ala Ala Ala Asp Ala Ala Asp Pro Tyr
145                 150                 155                 160
Val Phe Glu Ala Lys Thr Val Arg Arg Met Glu Leu Leu Val Leu Ser
                165                 170                 175
Ala Leu Gly Trp Arg Met His Pro Val Thr Pro Phe Ser Tyr Leu Gln
            180                 185                 190
Pro Val Leu Ala Asp Ala Ala Thr Arg Leu Arg Ser Cys Glu Gly Val
        195                 200                 205
Leu Leu Ala Val Met Ala Asp Trp Arg Trp Pro Arg His Arg Pro Ser
    210                 215                 220
```

```
Ala Trp Ala Ala Ala Ala Leu Leu Ile Thr Ala Ala Ala Gly Asp Gly
225                 230                 235                 240
Gly Asp Gly Asp Gly Asp Thr Glu Leu Leu Ala Leu Leu Ile Ala Pro
                245                 250                 255
Glu Asp Lys Thr Ala Glu Cys Ala Lys Ile Ile Ser Glu Val Thr Gly
            260                 265                 270
Met Ser Phe Leu Ala Cys Asp Val Gly Val Ser Ala Gly Asn Lys Arg
        275                 280                 285
Lys His Ala Ala Ala Gln Leu Tyr Ser Pro Pro Pro Ser Pro Ser Gly
    290                 295                 300
Val Ile Gly Ala Leu Ser Cys Phe Ser Cys Glu Ser Ser Thr Ser Ala
305                 310                 315                 320
Thr Ala Met Ala Ala Ala Val Gly Pro Trp Ala Pro Ser Ala Ser Val
                325                 330                 335
Ser Val Ser Ser Ser Pro Glu Pro Pro Gly Arg Ala Pro Lys Arg Ala
            340                 345                 350
Ala Ala Ala Ser Ala Ser Ala Ser Ala Ser Ala Gly Val Ala Pro Pro
        355                 360                 365
Val Gln Val Pro His Gln Leu Pro Pro Asp Glu Glu Ser Arg Asp Ala
    370                 375                 380
Trp Pro Ser Thr Cys Ala Ala
385                 390
```